# EUROPEAN PATENT SPECIFICATION

(11) **EP 1 756 156 B1**
(45) Date of publication and mention of the grant of the patent: **22.10.2008**
(21) Application number: 05755072.5
(22) Date of filing: 19.05.2005
(51) Int. Cl.: C07K 14/575

(54) **SELECTIVE VPAC2 RECEPTOR PEPTIDE AGONISTS**
SELEKTIVE PEPTIDISCHE AGONISTEN DES VPAC2-REZEPTORS
AGONISTES PEPTIDIQUES SELECTIFS DU RECEPTEUR VPAC2

(30) Priority: 21.05.2004 US 573739 P; 12.11.2004 US 627880 P; 25.02.2005 US 656601 P
(43) Date of publication of application: 28.02.2007
(73) Proprietor: ELI LILLY AND COMPANY, Indianapolis IN 46285 (US)
(72) Inventor: BOKVIST, Bengt, Krister, Lilly Forschung GmbH, 22419 Hamburg (DE); CUMMINS, Robert, Chadwick, Brownsburg, Indiana 46112 (US); GLAESNER, Wolfgang, Indianapolis, Indiana 46254 (US); GROMADA, Jesper, Lindgren, Lilly Forschung GmbH, 22419 Hamburg (DE); MAYER, John, Philip, Indianapolis, Indiana 46220 (US); ZHANG, Lianshan, Carmel, Indiana 46033 (US); ALSINA-FERNANDEZ, Jorge, Indianapolis, Indiana 46208 (US)
(74) Representative: Kent, Lindsey Ruth
(86) International application number: PCT/US2005/017435
(87) International publication number: WO 2005/113594

(56) References cited:
- WO-A-01/23420
- YUNG S L ET AL: "Generation of highly selective VPAC2 receptor agonists by high throughput mutagenesis of vasoactive intestinal peptide and pituitary adenylate cyclase-activating peptide" JOURNAL OF BIOLOGICAL CHEMISTRY, AMERICAN SOCIETY OF BIOLOCHEMICAL BIOLOGISTS, BIRMINGHAM,, US, vol. 278, no. 12, 21 March 2003 (2003-03-21), pages 10273-10281, XP002256208 ISSN: 0021-9258
- TSUTSUMI MANAMI ET AL: "A potent and highly selective VPAC2 agonist enhances glucose-induced insulin release and glucose disposal: A potential therapy for type 2 diabetes" DIABETES, vol. 51, no. 5, May 2002 (2002-05), pages 1453-1460, XP002348792 ISSN: 0012-1797 cited in the application
- DOYLE MÁIRE E ET AL: "The importance of the nine-amino acid C-terminal sequence of exendin-4 for binding to the GLP-1 receptor and for biological activity." REGULATORY PEPTIDES. 15 JUL 2003, vol. 114, no. 2-3, 15 July 2003 (2003-07-15), pages 153-158, XP002349264 ISSN: 0167-0115

## Description

The present invention is in the field of medicine. More particularly, this invention relates to selective VPAC2 receptor peptide agonists.

Type 2 diabetes, or non -insulin dependent diabetes mellitus (NIDDM), is the most common form of diabetes, affecting 90% of people with diabetes. With NIDDM, patients have impaired β-cell function resulting in insufficient insulin production and/or decreased insulin sensitivity. If NIDDM is not controlled, excess glucose accumulates in the blood, resulting in hyperglycemia. Over time, more serious complications may arise including renal dysfunction, cardiovascular problems, visual loss, lower limb ulceration, neuropathy, and ischemia. Treatments for NIDDM include improving diet, exercise, and weight control as well as using a variety of oral medications. Individuals with NIDDM can initially control their blood glucose levels by taking such oral medications. These medications, however, do not slow the progressive loss of β-cell function that occurs in type 2 diabetes patients and, thus, are not sufficient to control blood glucose levels in the later stages of the disease. Also, treatment with currently available medications exposes NID DM patients to potential side effects such as hypoglycemia, gastrointestinal problems, fluid retention, oedema, and/or weight gain.

Compounds, such as peptides that are selective for a particular G -protein coupled receptor known as the VPAC2 receptor, were initially identified by modifying vasoactive intestinal peptide (VIP) and/or pituitary adenylate cyclase -activating polypeptide (PACAP). (See, for example, Xia et al., J Pharmacol Exp Ther. , 281:629 -633 (1997); Tsutsumi et al., Diabetes, 51:1453-1460 (2002), WO 01/23420, WO 2004/006839.) Many of these peptides are not, however, suitable for commercial candidates as a result of stability issues associated with the polypeptides in formulation, as well as issues with the short half -life of these polypeptides.

Yung et al., J. Biol. Chem., 278(12): 10273 -10281 (2003) describes the generation of a series of recombinant selective VPAC2 receptor agonists by high throughput mutagenesis of VIP and PACAP.

PACAP belongs to the secretin / glucagon / vasoactive intesti nal peptide (VIP) family of peptides and works through three G -protein-coupled receptors that exert their action through the cAMP-mediated and other Ca ²⁺-mediated signal transduction pathways. These receptors are known as the PACAP-preferring type 1 (PAC1) receptor (Isobe, et al., Regul. Pept., 110:213-217 (2003); Ogi, et al., Biochem. Biophys. Res. Commun., 196:1511-1521 (1993)) and the two VIP-shared type 2 receptors (VPAC1 and VPAC2) (Sherwood et al., Endocr. Rev., 21:619-670 (2000); Hammar et al., Pharmacol Rev, 50:265-270 (1998); Couvineau, et al., J. Biol. Chem., 278:24759-24766 (2003); Sreedharan, et al., Biochem. Biophys. Res. Commun., 193:546-553 (1993); Lutz, et al., FEBS Lett., 458: 197-203 (1999); Adamou, et al., Biochem. Biophys. Res. Commun., 209: 385-392 (1995)).

PACAP has comparable activities towards all three receptors, while VIP selectively activates the two VPAC receptors (Tsutsumi 2002). Both VIP (Eriksson et al., Peptides, 10: 481-484 (1989)) and PACAP (Filipsson et al., JCEM, 82:3093-3098 (1997)) have been shown to not only stimulate insulin secretion in man when given intravenously but also increase glucagon secretion and hepatic glucose output. As a consequence, PACAP or VIP stimulation generally does not result in a net improvement of glycemia. Activation of multiple receptors by PACAP or VIP also has broad physiological effects on nervous, endocrine, cardiovascular, reproductive, muscular, and immune systems (Gozes et al., Curr. Med. Chem., 6:1019-1034 (1999)). Furthermore, it appears that VIP-induced watery diarrhoea in rats is mediated by only one of the VPAC receptors, VPAC1 (Ito et al., Peptides, 22:1139-1151 (2001); Tsutsumi 2002). In addition, the VPAC1 and PAC1 receptors are expressed on α-cells and hepatocytes and, thus, are most likely involved in the effects on hepatic glucose output.

Recent studies have shown that peptides selective for the VPAC2 receptor are able to stimulate insulin secretion from the pancreas without gastrointestinal (GI) side effects and without enhancing glucagon release and hepatic glucose output (Tsutsumi 2002). Many of the VPAC2 receptor peptide agonists reported to date have, however, less than desirable potency, selectivity, and stability profiles, which could impede their clinical viability.

There is, therefore, a need for new therapies, which overcome the problems associated with current medications for NIDDM. The present invention seeks to provide improved compounds that are selective for the VPAC2 receptor and which induce insulin secretion from the pancreas only in the presence of high blood glucose levels. The compounds of the present invention are peptides, which are believed to also improve beta cell function. These peptides can, therefore, have the physiological effect of inducing insulin secretion without GI side effects or a corresponding increase in hepatic glucose output and also generally have enhanced sele ctivity, potency, and/or *in vivo* stability of the peptide compared to known VPAC2 receptor peptide agonists. The compounds of the present invention include selective VPAC2 receptor peptide agonists.

According to a first aspect of the present invention, there is provided a VPAC2 receptor peptide agonist comprising a sequence of the formula: wherein:
Xaa₁ is: His, dH, or is absent;
Xaa₂ is: dA, Ser, Val, Gly, Thr, Leu, dS, Pro, or Aib;
Xaa₃ is: Asp or Glu;
Xaa₄ is: Ala, Ile, Tyr, Phe, Val, Thr, Leu, Trp, Gly, dA, Aib, or NMeA;
Xaa₅ is: Val, Leu, Phe, Ile, Thr, Trp, Tyr, dV, Aib, or NMeV;
Xaa₆ is: Phe, Ile, Leu, Thr, Val, Trp, or Tyr;
Xaa₈ is: Asp, Glu, Ala, Lys, Leu, Arg, or Tyr;
Xaa₉ is: Asn, Gln, or Glu;
Xaa₁₀ is: Tyr, Trp, or Tyr(OMe);
Xaa₁₂ is: Arg, Lys, hR, Orn, Aib, Cit, or Ala;
Xaa₁₃ is: Leu, Phe, Glu, Ala, or Aib;
Xaa₁₄ is: Arg, Leu, Lys, Ala, hR, Orns, Phe, Gln, Aib, or Cit;
Xaa₁₅ is: Lys, Ala, Arg, Glu, Leu, hR, Orn, Phe, Gln, Aib, K(Ac), or Cit;
Xaa₁₆ is: Gln, Lys, Ala, hR, Orn, or Cit;
Xaa₁₇ is: Val, Ala, Leu, Ile, Met, Nle, Lys, or A ib;
Xaa₁₉ is: Ala, Gly, or Leu;
Xaa₂₀ is: Lys, Gln, hR, Arg, Ser, Orn, Ala, Aib, Trp, Thr, Leu, Ile, Phe, Tyr, Val, K(Ac), or Cit;
Xaa₂₁ is: Lys, Arg, Ala, Phe, Aib, Leu, Gln, Orn, hR, K(Ac) or Cit;
Xaa₂₂ is: Tyr, Trp, Phe, Thr, Leu, Ile, Val, Tyr(OMe), Al a, or Aib;
Xaa₂₃ is: Leu, Phe, Ile, Ala, Trp, Thr, Val, or Aib;
Xaa₂₄ is: Gln, or Asn;
Xaa₂₅ is: Ser, Asp, Phe, Ile, Leu, Thr, Val, Trp, Gln, Asn, Tyr, Aib, or Glu;
Xaa₂₆ is: Ile, Leu, Thr, Val, Trp, Tyr, Phe or Aib;
Xaa₂₇ is: Lys, hR, Arg, Gln, Orn, or d K;
Xaa₂₈ is: Asn, Gln, Lys, Arg, Aib, Orn, hR, Cit, Pro, or dK;
Xaa₂₉ is: Lys, Ser, Arg, Asn, hR, Orn, Cit, Aib or is absent;
Xaa₃₀ is: Arg, Lys, Ile, hR, Cit, Aib, Orn, or is absent;
Xaa₃₁ is: Tyr, His, Phe, or is absent; and
Xaa₃₂ is: Cys, or is absen t;
provided that if Xaa₂₉, Xaa₃₀, or Xaa₃₁ is absent, the next amino acid present downstream is the next amino acid in the peptide agonist sequence,
and a C-terminal extension wherein the N -terminus of the C -terminal extension is linked to the C-terminus of the peptide of Formula 12 and wherein the C -terminal extension comprises an amino acid sequence of the formula:
Xaa₁-Xaa₂-Xaa₃-Xaa₄-Xaa₅-Xaa₆-Xaa₇-Xaa₈-Xaa₉-Xaa₁₀-Xaa₁₁ Formula 7 (SEQ ID NO:15)
wherein:
Xaa₁ is: Gly, Cys, or absent;
Xaa₂ is: Gly, Arg, or absent;
Xaa₃ is: Pro, Thr, or absent;
Xaa₄ is: Ser, or absent;
Xaa₅ is: Ser, or absent;
Xaa₆ is: Gly, or absent;
Xaa₇ is: Ala, or absent;
Xaa₈ is: Pro, or absent;
Xaa₉ is: Pro, or absent;
Xaa₁₀ is: Pro, or absent; and
Xaa₁₁ is: Ser, Cys, or abs ent;
provided that at least five of Xaa₁ to Xaa₁₁ of the C-terminal extension are present and provided that if Xaa₁, Xaa₂, Xaa₃, Xaa₄, Xaas, Xaa₆, Xaa₇, Xaa₈, Xaa₉, or Xaa₁₀ is absent, the next amino acid present downstream is the next amino acid in the C -terminal extension and wherein the C-terminal amino acid may be amidated.

The VPAC2 receptor peptide agonist preferably comprises a sequence of the formula: wherein:
Xaa₂ is: dA, Ser, Val, dS, or Aib;
Xaa₃ is: Asp or Glu;
Xaa₄ is: Ala, dA, or Aib;
Xaa₅ is: Val, Leu, dV, or Aib;
Xaa₈ is: Asp, Glu, or Ala;
Xaa₉ is: Asn, Gln, or Glu;
Xaa₁₂ is: Ala, Arg, Lys, hR, or Orn;
Xaa₁₄ is: Arg, Leu, Lys, Ala, hR, Orn, Phe, Gln, Aib, or Cit;
Xaa₁₅ is: Lys, Ala, Arg, Leu, Orn, Phe, Gln, Aib, or K(Ac);
Xaa₁₆ is: Gln, or Lys;
Xaa₁₇ is: Val, Ala, Leu, Ile, Met, Nle, or Lys;
Xaa₁₉ is: Ala, or Leu;
Xaa₂₀ is: Lys, Gln, hR, Arg, Ser, Ala, Aib, Trp, Thr, Leu, Ile, Phe, Tyr, Val, or K(Ac);
Xaa₂₁ is: Lys, Arg, Ala, Phe, Aib, Leu, Gln, K(Ac), or Orn;
Xaa₂₂ is: Tyr, Trp, Phe, Leu, Ile, or Val;
Xaa₂₄ is: Gln, or Asn;
Xaa₂₅ is: Ser, Phe, Ile, Leu, Thr, Val, Trp, Gln, Asn, Tyr, or Aib;
Xaa₂₆ is: Ile, Leu, Thr, Val, Trp, Tyr, Phe or Aib;
Xaa₂₇ is: Lys, hR, Arg, dK, or Orn;
Xaa₂₈ is: Asn, Gln, Lys, hR, Aib, Orn, dK, or Pro;
Xaa₂₉ is: Lys, Ser, Arg, hR, Orn, or is absent;
Xaa₃₀ is: Arg, Lys, or is absent; and
Xaa₃₁ is: Tyr, Phe, or is absent;
provided that if Xaa₂₉, or Xaa₃₀ is absent, the next amino acid present downstream is the next amino acid in the peptide agonist sequence,
and a C-terminal extension wherein the N -terminus of the C -terminal extension is linked to the C-terminus of the peptide of Formula 13 and wherein the C -terminal extension comprises an amino acid sequence of the formula:
Xaa₁-Xaa₂-Xaa₃-Xaa₄-Xaa₅-Xaa₆-Xaa₇-Xaa₈-Xaa₉-Xaa₁₀-Xaa₁₁ Formula 7 (SEQ ID NO:15)
wherein:
Xaa₁ is: Gly, Cys, or absent;
Xaa₂ is: Gly, Arg, or absent;
Xaa₃ is: Pro, Thr, or absent;
Xaa₄ is: Ser, or absent;
Xaa₅ is: Ser, or absent;
Xaa₆ is: Gly, or absent;
Xaa₇ is: Ala, or absent;
Xaa₈ is: Pro, or absent;
Xaa₉ is: Pro, or absent;
Xaa₁₀ is: Pro, or absent; and
Xaa₁₁ is: Ser, Cys, or absent;
provided that at least five of Xaa₁ to Xaa₁₁ of the C-terminal extension are present and provided that if Xaa₁, Xaa₂, Xaa₃, Xaa₄, Xaa₅, Xaa₆, Xaa₇, Xaa₈, Xaa₉, or Xaa₁₀ is absent, the next amino acid present d ownstream is the next amino acid in the C -terminal extension and wherein the C -terminal amino acid may be amidated.

Preferably, at least six of Xaa₁ to Xaa₁₁ of the C-terminal extension are present. More preferably at least seven, eight, nine , ten or all of Xaa₁ to Xaa₁₁ of the C-terminal extension are present.

Preferably, the VPAC2 receptor peptide agonist sequence further comprises a histidine residue at the N-terminal extension region of the peptide sequence before Xaa₁.

More preferably, the C -terminal extension of the VPAC2 receptor peptide agonist is selected from:

| | |
|---|---|
| SEQ ID NO: 10 | GGPSSGAPPPS |
| SEQ ID NO: 11 | GGPSSGAPPPS-NH₂ |
| SEQ ID NO: 22 | GGPSSGAPPPC |
| SEQ ID NO: 23 | GGPSSGAPPPC-NN₂ |
| SEQ ID NO: 24 | GRPSSGAPPPS |
| SEQ ID NO: 25 | GRPSSGAPPPS-NH₂ |

It is especially preferred that the C -terminal extension is GGPSSGAPPPS (SEQ ID NO: 10) or GGPSSGAPPPS -NH₂ (SEQ ID NO: 11).

Preferably, the VPAC2 receptor peptide agonist of the present invention comprises a sequence of Formula 12 (SEQ ID NO 20) or Formula 13 (SEQ ID N O 21) wherein Xaa₃ is Asp or Glu, Xaa₈ is Asp or Glu, Xaa₁₂ is Arg, hR, Lys, or Orn, Xaa₁₄ is Arg, Gln, Aib, hR, Orn, Cit, Lys, Ala, or Leu, Xaa₁₅ is Lys, Aib, or Orn, Xaa₁₆ is Gln or Lys, Xaa₁₇ is Val, Leu, Ala, Ile, Lys, or Nle, Xaa₂₀ is Lys, Val, Leu, A ib, Ala, or Gln, Xaa₂₁ is Lys, Aib, Orn, Ala, or Gln, Xaa₂₇ is Lys, Orn, or hR, Xaa₂₈ is Asn, Gln, Lys, hR, Aib, Orn, or Pro and Xaa₂₉ is Lys, Orn, hR, or is absent.

More preferably, the VPAC2 receptor peptide agonist of the present invention comprises a sequence of Formula 12 (SEQ ID NO 20) or Formula 13 (SEQ ID NO 21) wherein Xaa₁₂ is Arg, hR, or Orn, Xaa₁₄ is Arg, Aib, Gln, Ala, Leu, Lys, or Orn, Xaa₁₅ is Lys or Aib, Xaa₁₇ is Val or Leu, Xaa₂₁ is Lys, Aib, or Gln and Xaa₂₈ is Asn or Gln.

Preferably, the VPAC2 receptor peptide agonist of the present invention comprises a sequence of Formula 12 (SEQ ID NO 20) or Formula 13 (SEQ ID NO 21) wherein Xaa ₃₀ and/or Xaa₃₁ are absent. Alternatively, Xaa ₂₉, Xaa₃₀ and Xaa₃₁ are all absent.

Preferably, the VPAC2 re ceptor peptide agonist of the present invention comprises a sequence of Formula 12 (SEQ ID NO 20) or Formula 13 (SEQ ID NO 21) wherein either Xaa₁₄ or Xaa₁₅ is Aib.

Also preferably, the VPAC2 receptor peptide agonist of the present invention comprises a sequence of Formula 12 (SEQ ID NO 20) or Formula 13 (SEQ ID NO 21) wherein either Xaa₂₀ or Xaa₂₁ is Aib.

More preferably, either Xaa₁₄ or Xaa₁₅ is Aib and either Xaa₂₀ or Xaa₂₁ is Aib.

Preferably, the VPAC2 receptor peptide agonist of the present invent ion comprises a sequence of Formula 12 (SEQ ID NO 20) or Formula 13 (SEQ ID NO 21) wherein Xaa ₂₈ is Gln and Xaa ₂₉ is Lys or is absent.

More preferably, Xaa₂₈ is Gln and Xaa ₂₉ is Lys or is absent, and either Xaa ₁₄ or Xaa₁₅ is Aib and either Xaa ₂₀ or Xaa₂₁ is Aib.

Preferably, the VPAC2 receptor peptide agonist of the present invention comprises a sequence of Formula 12 (SEQ ID NO 20) or Formula 13 (SEQ ID NO 21) wherein Xaa ₁₂ is hR or Orn, Xaa₂₇ is hR or Orn and Xaa₂₉ is hR or Orn.

More preferably, Xaa₁₂ is hR or Orn, Xaa₂₇ is hR or Orn and Xaa₂₉ is hR or Orn, and either Xaa₁₄ or Xaa₁₅ is Aib and either Xaa₂₀ or Xaa₂₁ is Aib.

Preferably, the VPAC2 receptor peptide agonist of the present invention further comprises a N-terminal modification at the N -terminus of the peptide agonist wherein the N - terminal modification is selected from:
(a) addition of D-histidine, isoleucine, methionine, or norleucine;
(b) addition of a peptide comprising the sequence Ser-Trp-Cys-Glu-Pro-Gly-Trp-Cys-Arg wherein the Arg is linked to the N-terminus of the peptide agonist;
(c) addition of C₁-C₁₆ alkyl optionally substituted with one or more substituents independently selected from aryl, C ₁-C₆ alkoxy, -NH₂, -OH, halogen and -CF₃;
(d) addition of -C(O)R¹ wherein R¹ is a C₁-C₁₆ alkyl optionally substituted with one or more substituents independently selected from aryl, C ₁-C₆ alkoxy, -NH₂, -OH, halogen, -SH and -CF₃; an aryl or aryl C ₁-C₄ alkyl optionally substituted with one or more substituents independently selected from C ₁-C₆ alkyl, C₂-C₆ alkenyl, C₂-C₆ alkynyl, C ₁-C₆alkoxy, -NH₂, -OH, halogen and -CF₃; -NR²R³ wherein R² and R³ are independently hydrogen, C₁-C₆ alkyl, aryl or aryl C₁-C₄ alkyl; or -OR⁴ wherein R⁴ is C₁-C₁₆ alkyl optionally substituted with one or more substituents independently selected from aryl, C ₁-C₆alkoxy, -NH₂, -OH, halogen and -CF₃, aryl or aryl C ₁-C₄ alkyl optionally substituted with one or more substituents independently selected from C₁-C₆ alkyl, C₂-C₆ alkenyl, C₂-C₆ alkynyl, C ₁-C₆ alkoxy, -NH₂, -OH, halogen and -CF₃;
(e) addition of -SO₂R⁵ wherein R⁵ is aryl, aryl C ₁-C₄ alkyl or C ₁-C₁₆ alkyl;
(f) formation of a succinimide group optionally substituted with C ₁-C₆ alkyl or -SR⁶, wherein R⁶ is hydrogen or C ₁-C₆ alkyl; and
(g) addition of methionine sulfoxide.

Preferably, the N-terminal modification is the addition of a group selected from: acetyl, propionyl, butyryl, pentanoyl, hexanoyl, methionine, methionine sulfoxide, 3 - phenylpropionyl, phenylacetyl, benzoyl, norleucine, D -histidine, isoleucine and -3-mercaptopropionyl and more preferably is the addition of acetyl or hexanoyl. It is especially preferred that the N-terminal modification is the addition of hexanoyl.

It will be appreciated by the skilled person that various combinations of the VPAC2 receptor peptide agonist s equence, C-terminal extension sequence and N -terminal modifications described above may be made based on the above disclosure.

Preferred VPAC2 receptor peptide agonists comprise an amino acid sequence selected from:

| **Agonist #** | **Sequence** |
|---|---|
| P6 SEQ ID NO:28 | HSDAVFTDNYTRLRKQVAAKKYLQSIKNKRYGGPSSGAPPPS |
| P9 SEQ ID NO:31 | HSDAVFTDNYTRLRKQVAAKKYLQSIKNKKGGPSSGAPPPS |
| P18 SEQ ID NO:32 | HSDAVFTDNYTRLRKQVAAhRKYLQSIKNKRYGGPSSGAPPPS |
| P19 SEQ ID NO:33 | HSDAVFTDNYTRLRKQVAAIKYLQSIKNKRYGGPSSGAPPPS |
| P20 SEQ ID NO:34 | HSDAVFTDNYTRLRKQVAARKYLQSIKNKRYGGPSSGAPPPS |
| P21 SEQ ID NO:35 | HSDAVFTDNYTRLRKQVAASKYLQSIKNKRYGGPSSGAPPPS |
| P22 SEQ ID NO:36 | HSDAVFTDNYTRLRKQVAAKKYLQSIhRNKRYGGPSSGAPPPS |
| P23 SEQ ID NO:37 | HSDAVFTDNYTRLRKQVAAKKYLQSIRNKRYGGPSSGAPPPS |
| P24 SEQ ID NO:38 | HSDAVFTDNYTRLRKQVAAKKYLQSIKNHRRYGGPSSGAPPPS |
| P25 SEQ ID NO:39 | HSDAVFTDNYTRLRKQVAAKKYLQSIKNRRYGGPSSGAPPPS |
| P26 SEQ ID NO:40 | HSDAVFTDNYTRFRKQVAAKKYLQSIKNKRYGGPSSGAPPPS |
| P27 SEQ ID NO:41 | HSDAVFTDNWTRLRKQVAAKKYLQSIKNKRYGGPSSGAPPPS |
| P28 SEQ ID NO:42 | HSDAVFTDNYTRLRKQVAAKKYL QSIKNKRHGGPSSGAPPPS |
| P31 SEQ ID NO:43 | C6-HSDAVFTDNYTRLRKQVAAKKYLQSIKNKRYGGPSSGAPPPS |
| P33 SEQ ID NO:44 | Ac-HSDAVFTDNYTRLRKQVAAKKYLQSIKNKRYGGPSSGAPPPS |
| P43 SEQ ID NO:47 | HGDAVFTDNYTRLRKQVAAKKYLQSIKNKRYGGPSSGAPPPS |
| P44 SEQ ID NO:48 | HVDAVFTDNYTRLRKQVAAKKYLQSIKN KRYGGPSSGAPPPS |
| P45 SEQ ID NO:49 | HTDAVFTDNYTRLRKQVAAKKYLQSIKNKRYGGPSSGAPPPS |
| P46 SEQ ID NO:50 | HLDAVFTDNYTRLRKQVAAKKYLQSIKNKRYGGPSSGAPPPS |
| P47 SEQ ID NO:51 | HdADAVFTDNYTRLRKQVAAKKYLQSIKNKRYGGPSSGAPPPS |
| P48 SEQ ID NO:52 | HdSDAVFTDNYTRLRKQVAAKKYLQSIKNKRYGGP SSGAPPPS |
| P49 SEQ ID NO:53 | HPDAVFTDNYTRLRKQVAAKKYLQSIKNKRYGGPSSGAPPPS |
| P50 SEQ ID NO:54 | HSDIVFTDNYTRLRKQVAAKKYLQSIKNKRYGGPSSGAPPPS |
| P51 SEQ ID NO:55 | HSDYVFTDNYTRLRKQVAAKKYLQSIKNKRYGGPSSGAPPPS |
| P52 SEQ ID NO:56 | HSDFVFTDNYTRLRKQVAAKKYLQSIKNKRYGGPSSGAPPPS |
| P53 SEQ ID NO:57 | HSDVVFTDNYTRLRKQVAAKKYLQSIKNKRYGGPSSGAPPPS |
| P54 SEQ NO:58 | HSDTVFTDNYTRLRKQVAAKKYLQSIKNKRYGGPSSGAPPPS |
| P55 SEQ ID NO:59 | HSDLVFTDNYTRLRKQVAAKKYLQSIKNKRYGGPSSGAPPPS |
| P56 SEQ ID NO:60 | HSDWVFTDNYTRLRKQVAAKKYLQSIKNKRYGGPSSGAPPPS |
| P58 SEQ ID NO:61 | HSDAFFTDNYTRLRKQVAAKKYLQSIKNKRYGGPSSGAPPPS |
| P60 SEQ ID NO:62 | HSDAIFTDNYTRLRKQVAAKKYLQSIKNKRYGGPSSGAPPPS |
| P61 SEQ ID NO:63 | HSDALFTDNYTRLRKQVAAKKYLQSIKNKRYGGPSSGAPPPS |
| P62 SEQ ID NO:64 | HSDATFTDNYTRLRKQVAAKLYLQSIKNKRYGGPSSGAPPPS |
| P63 SEQ ID NO:65 | HSDAVITDNYTRILRKQVAAKKYLQSIKNKRYGGPSSGAPPPS |
| P64 SEQ ID NO:66 | HSDAVLTDNYTRLRKQVAAKKYLQSIKNKRYGGPSSGAPPPS |
| P65 SEQ ID NO:67 | HSDAVTTDNYTRLRKQVAAKKYLQSIKNKRYGGPSSGAPPPS |
| P66 SEQ ID NO:68 | HSDAVVTDNYTRLRKQVAAKKYLQSIKNKRYGGPSSGAPPPS |
| P67 SEQ ID NO:69 | HSDAVWTDNYTRLRKQVAAKKYLQSIKNKRYGGPSSGAPPPS |
| P68 SEQ ID NO:70 | HSDAVYTDNYTRLRKQVAAKKYLQSIKNKRYGGPSSGAPPPS |
| P69 SEQ ID NO:71 | HSDAWFTDNYTRLRKQVAAKKYLQSIKNKRYGGPSSGAPPPS |
| P70 SEQ ID NO:72 | HSDAYFTDNYTRLRKQVAAKKYLQSIKNKRYGGPSSGAPPPS |
| P71 SEQ ID NO:73 | HSDAVFTDNYTRLRRQVAARRYLQSIRNRRYGGPSSGAPPPS |
| P72 SEQ ID NO:74 | HSDAVFTDNYTRLRRQVAAKKYLQSIKNKRYGGPSSGAPPPS |
| P73 SEQ ID NO:75 | HSDAVFTDNYTRLRKQVAARKYLQSIKNKRYGGPSSGAPPPS |
| P74 SEQ ID NO:76 | HSDAVFTDNYTRLRKQVAAKKYLQSIQNKRYGGPSSGAPPPS |
| P75 SEQ ID NO:77 | HSDAVFTDNYTRLRKQVAAKKYLQSIKNNRYGGPSSGAPPPS |
| P76 SEQ ID NO:78 | HSDAVFTDNYTRLRKQVAAKKYLQSIKNKIYGGPSSGAPPPS |
| P82 SEQ ID NO:79 | HSDAVFTDNYTRLRKQVAAKKYLQSIKRGGPSSGAPPPS |
| P83 SEQ ID NO:80 | HSDAVFTDNYTRLRKQVAAKKYLQSIKNGGPSSGAPPPS |
| P84 SEQ ID NO:81 | dHSDAVFTDNYTRLRKQVAAKKYLQSIKNKRYGGPSSGAPPPS |
| P85 SEQ ID NO:82 | HSDAVFTDNYTRLRKQVAAKKYLQSIKKGGPSSGAPPPS |
| P87 SEQ ID NO:83 | HSDAVFTDNYTREKEKVAAKKYLQSIKNKRYGGPSSGAPPPS |
| P88 SEQ ID NO:84 | HSDAVFTDNYTRAAAKVAAKKYLQSIKNKRYGGPSSGAPPPS |
| P89 SEQ ID NO:85 | HSDAVFTDNYTRLLAKVAAKKYLQSIKNKRYGGPSSGAPPPS |
| P92 SEQ ID NO:86 | HSDAVFTDNYTRLRKQVAAKKYLQSIKNGRPSSGAPPPS |
| P93 SEQ ID NO:87 | HSDAVFTDNYTRLLLKVAAKKYLQSIKNKRYGGPSSGAPPPS |
| P94 SEQ ID NO:88 | HSDAVFTDNYTRAKAKVAAKKYLQSIKNKRYGGPSSGAPPPS |
| P98 SEQ ID NO:89 | C6-HSDAVFTDNYTRLRRQVAARRYLQSIRNRRYGGPSSGAPPPS |
| P99 SEQ ID NO:90 | C6-HVDAVFTDNYTRLRKQVAAKKYLQSIKNKRYGGPSSGAPPPS |
| P100 SEQ ID NO:91 | M-HSDAVFTDQYTRLRKQVAAKKYLQSIKQKRYGGPSSGAPPPS |
| P101 SEQ ID NO:92 | C6-HdADAVFTDNYTRLRKQVAAKKYLQSIKNKRYGGPSSGAPPPS |
| P102 SEQ ID NO:93 | HSDGVFTDNYTRLRKQVAAKKYLQSIKNKRYGGPSSGAPPPS |
| P103 SEQ ID NO:94 | C6-HSDAVFTDNYTKLKKQVAAKKYLQSIKNKKYGGPSSGAPPPS |
| P104 SEQ ID NO:95 | M-HSDAVFTDNYTRLRKQVAAKKYLQSIKNKRYGGPSSGAPPPS |
| P105 SEQ ID NO:96 | I-HSDAVFTDNYTRLRKQVAAKKYLQSIKNKRYGGPSSGAPPPS |
| P106 SEQ ID NO:97 | C6-HSDAVFTDNYTRLRKQVAAKKYLQSFKNKRYGGPSSGAPPPS |
| P107 SEQ ID NO:98 | C6-HSDAVFTDNYTRLRKQVAAKKYLQSLKNKRYGGPSSGAPPPS |
| P108 SEQ ID NO:99 | C6-HSDAVFTDNYTRLRKQVAAKKYLQSTKNKRYGGPSSGAPPPS |
| P109 SEQ ID NO:100 | C6-HSDAVFTDNYTRLRKQVAAKKYLQSVKNKRYGGPSSGAPPPS |
| P110 SEQ ID NO: 101 | C6-HSDAVFTDNYTRLRKQVAAKKYLQSWKNKRYGGPSSGAPPPS |
| P111 SEQ ID NO: 102 | C6-HSDAVFTDNYTRLRKQVAAKKYLQSYKNKRYGGPSSGAPPPS |
| P112 SEQ ID NO:103 | C6-HSDAVFTDNYTRLRKQVAAKKYLQFIKNKRYGGPSSGAPPPS |
| P113 SEQ ID NO:104 | C6-HSDAVFTDNYTRLRKQVAAKKYLQIIKNKRYGGPSSGAPPPS |
| P114 SEQ ID NO:105 | C6-HSDAVFTDNYTRLRKQVAAKKYLQLIKNKRYGGPSSGAPPPS |
| P115 SEQ ID NO:106 | C6-HSDAVFTDNYTRLRKQVAAKKYLQTIKNKRYGGPSSGAPPPS |
| P116 SEQ ID NO:107 | C6-HSDAVFTDNYTRLRKQVAAKKYLQVIKNKRYGGPSSGAPPPS |
| P117 SEQ ID NO:108 | C6-HSDAVFTDNYTRLRKQVAAKKYLQWIKNKRYGGPSSGAPPPS |
| P119 SEQ ID NO:109 | C6-HSDAVFTDNYTRLLAKLALQKYLQSIKNKRYGGPSSGAPPPS |
| P120 SEQ ID NO:110 | C6-HSDAVFTDNYTRLRKQVAAKKYLQSIKNKRYGGPSSGAPPPC |
| P121 SEQ ID NO:111 | C6-HSDAVFTDNYTRLRKQVAAKKYLQSIKNKKGGPSSGAPPPS |
| P122 SEQ ID NO:112 | C6-HSDAVFTDNYTRLRKQVAAKKYLQSIKNSRGGPSSGAPPPS |
| P123 SEQ ID NO:113 | C6-HSDAVFTDNYTRLRKQVAAKKYLQSIKNKRGGPSSGAPPPS |
| P124 SEQ ID NO:114 | C6-HSDAVFTDNYTRLRKQVAAKKYLQQIKNKRYGGPSSGAPPPS |
| P125 SEQ ID NO:115 | C6-HSDAVFTDNYTRLRKQVAAKKYLQNIKNKRYGGPSSGAPPPS |
| P126 SEQ ID NO:116 | HSDAVFTDNYTRLRKQVAAKKYLQSIKRGRPSSGAPPPS |
| P127 SEQ ID NO:117 | C6-HSDAVFTDNYTRLRKQVAAKKYLQYIKNKRYGGPSSGAPPPS |
| P129 SEQ ID NO:118 | C6-HSDAVFTDNYTRLRKQVAAKKWLQSIKNKRYGGPSSGAPPPS |
| P130 SEQ ID NO:119 | C6-HSDAVFTDNYTRLRKQVAAKKFLQSIKNKRYGGPSSGAPPPS |
| P131 SEQ ID NO:120 | C6-HSDAVFTDNYTRLRKQVAAKKTLQSIKNKRYGGPSSGAPPPS |
| P132 SEQ ID NO:121 | C6-HSDAVFTDNYTRLRKQVAAKKLLQSIKNKRYGGPSSGAPPPS |
| P133 SEQ ID NO:122 | C6-HSDAVFTDNYTRLRKQVAAKKILQSIKNKRYGGPSSGAPPPS |
| P134 SEQ ID NO:123 | C6-HSDAVFTDNYTRLRKQVAAKKVLQSIKNKRYGGPSSGAPPPS |
| P135 SEQ ID NO:124 | C6-HSDAVFTDNYTRLLAKVAAKKYLQSIKNKRYGGPSSGAPPPS |
| P138 SEQ ID NO:125 | C6-HSDAVFTDNYTRLRAQVAAQKYLQSIKNKRYGGPSSGAPPPS |
| P139 SEQ ID NO:126 | C6-HSDAVFTDNYTRLRAQVAAKKYLQSIKNKRYGGPSSGAPPPS |
| P140 SEQ ID NO:127 | M-HSDAVFTDNYTRLLAKLALQKYLQSIKNKRYGGPSSGAPPPS |
| P141 SEQ ID NO:128 | C6-HSDAVFTDNYTRLKAQVAAKKYLQSIKNKRYGGPSSGAPPPS |
| P142 SEQ ID NO:129 | C6-HSDAVFTDNYTRLRAQLAAQKYLQSIKNKRYGGPSSGAPPPS |
| | |
| P143 SEQ ID NO:130 | C6-HSDAVFTDNYTRLRKQMAAQKYLNQLKKGGPSSGAPPPS |
| P144 SEQ ID NO:131 | C6-HSDAVFTDNYTRLRKQVAAQKYLNQLKKGGPSSGAPPPS |
| P146 SEQ ID NO:132 | C6-HSDAVFTDNYTRLRKQVAAVKYLQSIKNKRYGGPSSGAPPPS |
| P147 SEQ ID NO:133 | C6-HSDAVFTDNYTRLRKQVAAYKYLQSIKNKRYGGPSSGAPPPS |
| P148 SEQ ID NO:134 | C6-HSDAVFTDNYTRLRKQVAAFKYLQSIKNKRYGGPSSGAPPPS |
| P149 SEQ ID NO:135 | C6-HSDAVFTDNYTRLRKQVAAIKYLQSIKNKRYGGPSSGAPPPS |
| P150 SEQ ID NO:136 | C6-HSDAVFTDNYTRLRKQVAAQKYLQSIKNKRYGGPSSGAPPPS |
| P151SEQ ID NO: 137 | C6-HSDAVFTDNYTRLRKQVAALKYLQSIKNKRYGGPSSGAPPPS |
| P152 SEQ ID NO:138 | C6-HSDAVFTDNYTRLRKQVAATKYLQSIKNKRYGGPSSGAPPPS |
| P153 SEQ ID NO:139 | C6-HSDAVFTDNYTRLRKQVAAWKYLQSIKNKRYGGPSSGAPPPS |
| P154 SEQ ID NO:140 | C6-HSDAVFTDNYTRLRKQVAAKKYLQSIKNGGPSSGAPPPS |
| P155 SEQ ID NO:141 | C6-HSDAVFTDNYTRLRKQVALKKYLQSIKNKRYGGPSSGAPPPS |
| P158 SEQ ID NO:142 | C6-HSDAVFTANYTRLRKQVAAKKYLQSIKNKRYGGPSSGAPPPS |
| P159 SEQ ID NO:13 | C6-HSDAVFTENYTRLRKQVAAKKYLQSIKNKRYGGPSSGAPPPS |
| P160 SEQ ID NO:144 | C6-HSDAVFTKNYTRLRKQVAAKKYLQSIKNKRYGGPSSGAPPPS |
| | |
| P161 SEQ ID NO:145 | C6-HSDAVFTLNYTRLRKQVAAKKYLQSIKNKRYGGPSSGAPPPS |
| P162 SEQ ID NO:146 | C6-HSDAVFTRNYTRLRKQVAAKKYLQSIKNKRYGGPSSGAPPPS |
| P163 SEQ ID NO:147 | C6-HSDAVFTYNYTRLRKQVAAKKYLQSIKNKRYGGPSSGAPPPS |
| P164 SEQ ID NO:148 | C6-HSDAVFTDNYTRLLAKLALQKYLQSIKNKRYGGPSSGAPPPC |
| P165 SEQ ID NO:149 | C6-HSDAVFTEEYTRLQKQVAAKQYLQSIKNKRYGGPSSGAPPPS |
| P166 SEQ ID NO:150 | C6-HAibDAVFTDNYTRLRKQVAAKKYLQSIKNKRYGGPSSGAPPPS |
| P167 SEQ ID NO:151 | C6-HSDAVFTDNYTRLAibKQVAAKKYLQSIKNKRYGGPSSGAPPPS |
| P168 SEQ ID NO:152 | C6-HSDAVFTDNYTRLRAibQVAAKKYLQSIKNKRYGGPSSGAPPPS |
| P169 SEQ ID NO:153 | C6-HSDAVFTDNYTRLRKQVAAAibKYLQSIKNKRYGGPSSGAPPPS |
| P170 SEQ ID NO:154 | C6-HSDAVFTDNYTRLRKQVAAKAibYLQSIKNKRYGGPSSGAPPPS |
| P171 SEQ ID NO:155 | C6-HSDAVFTDNYTRLKKQVAAKKYLQSIKNKRYGGPSSGAPPPS |
| P172 SEQ ID NO:156 | C6-HSDAVFTDNYTRLQKQVAAKKYLQSIKNKRYGGPSSGAPPPS |
| P173 SEQ ID NO:157 | C6-HSDAVFTDNYTRLAKQVAAKKYLQSIKNKRYGGPSSGAPPPS |
| P174 SEQ ID NO:158 | C6-HSDAVFTDNYTRLLKQVAAKKYLQSIKNKRYGGPSSGAPPPS |
| | |
| P175 SEQ ID NO: 159 | C6-HSDAVFTDNYTRLFKQVAAKKYLQSIKNKRYGGPSSGAPPPS |
| P176 SEQ ID NO:160 | C6-HSDAVFTDNYTRLRRQVAAKKYLQSIKNKRYGGPSSGAPPPS |
| P177 SEQ ID NO:161 | C6-HSDAVFTDNYTRLRQQVAAKKYLQSIKNKRYGGPSSGAPPPS |
| P179 SEQ ID NO:162 | C6-HSDAVFTDNYTRLRLQVAAKKYLQSIKNKRYGGPSSGAPPPS |
| P180 SEQ ID NO:163 | C6-HSDAVFTDNYTRLRFQVAAKKYLQSIKNKRYGGPSSGAPPPS |
| P181 SEQ ID NO:164 | C6-HSDAVFTDNYTRLRKQVAAAKYLQSIKNKRYGGPSSGAPPPS |
| P182 SEQ ID NO:165 | C6-HSDAVFTDNYTRLRKQVAAKRYLQSIKNKRYGGPSSGAPPPS |
| P183 SEQ ID NO:166 | C6-HSDAVFTDNYTRLRKQVAAKQYLQSIKNKRYGGPSSGAPPPS |
| P184 SEQ ID NO:167 | C6-HSDAVFTDNYTRLRKQVAAKAYLQSIKNKRYGGPSSGAPPPS |
| P185 SEQ ID NO:168 | C6-HSDAVFTDNYTRLRKQVAAKLYLQSIKNKRYGGPSSGAPPPS |
| P186 SEQ ID NO:169 | C6-HSDAVFTDNYTRLRKQVAAKFYLQSIKNKRYGGPSSGAPPPS |
| P187 SEQ ID NO:170 | C6-HSDAVFIDNYTRLRKQVAAKKYLQAibIKNKRYGGPSSGAPPPS |
| P188 SEQ ID NO:171 | C6-HSDAVFTDNYTRLRKQVAAKKYLQSAibKNKRYGGPSSGAPPPS |
| P189 SEQ ID NO:172 | C6-HSDAVFTDNYTRLRKQAibAAKKYLQSIKNKRYGGPSSGAPPPS |
| | |
| P191 SEQ ID NO:173 | C6-HHSDAVFTDNYTRLLAKLALQKYLQSIKNKRYGGPSSGAPPPS |
| P192 SEQ ID NO:174 | C6-HSDAVFTDQYTRLLAKLALQKYLQSIKQKRYGGPSSGAPPPS |
| P193 SEQ ID NO:175 | |
| P194 SEQ ID NO: 176 | |
| P195 SEQ ID NO:177 | C6-HSDAVFTDNYTRLLAQLALQKYLQSIKNKRYGGPSSGAPPPS |
| P196 SEQ ID NO:178 | C6-HSDAVFTDNYTRLLAKVALQKYLQSIKNKRYGGPSSGAPPPS |
| P197 SEQ ID NO:179 | C6-HSDAVFTDNYTRLLAKLAAQKYLQSIKNKRYGGPSSGAPPPS |
| P198 SEQ ID NO:180 | C6-HSDAVFTDNYTRLLAKLALQKYLQSIKNKGGPSSGAPPPC |
| P199 SEQ ID NO: 181 | Met(O)-HSDAVFTDNYTRLRKQVAAKKYLQSIKNKRYGGPSSGAPPPS |
| P203 SEQ ID NO:182 | C6-HSDAVFTDNYTRLRKQVAAKKYLQSIKNKRFGGPSSGAPPPS |
| P205 SEQ ID NO:183 | |
| P206 SEQ ID NO:184 | |
| P207 SEQ ID NO:185 | C6-HSDdAVFTDNYTRLRKQVAAKKYLQSIKNKRYGGPSSGAPPPS |
| P208 SEQ ID NO:186 | C6-HSDNMeAVFTDNYTRLRKQVAAKKYLQSIKNKRYGGPSSGAPPPS |
| P209 SEQ ID NO:187 | C6-HSDAibVFTDNYTRLRKQVAAKKYLQSIKNKRYGGPSSGAPPPS |
| P210 SEQ ID NO:188 | C6-HSDAdVFFDNYTRLRKQVAAKKYLQSIKNKRYGGPSSGAPPPS |
| P211 SEQ ID NO:189 | C6-HSDANMeVFTDNYTRLRKQVAAKKYLQSIKNKRYGGPSSGAPPPS |
| P212 SEQ ID NO:190 | C6-HSDAAibFTDNYTRLRKQVAAKKYLQSIKNKRYGGPSSGAPPPS |
| P213 SEQ ID NO:191 | C6-HSDAVFTDNYTRLRKQVAAKRYLQSIRNGGPSSGAPPPS |
| P214 SEQ ID NO:192 | C6-HSDAVFTDNYTRLRKQVAARRYLQSIRNGGPSSGAPPPS |
| P215 SEQ ID NO:193 | C6-HSDAVFTDNYTRLRRQVAAKRYLQSIRNGGPSSGAPPPS |
| P216 SEQ ID NO:194 | C6-HSDAVFTDNYTRLRRQVAARKYLQSIRNGGPSSGAPPPS |
| P220 SEQ ID NO:195 | C6-HSDAVFTDQYTRLRRQVAARKYLQSIRQGGPSSGAPPPS |
| P221 SEQ ID NO:196 | C6-HSDIVFTDNYTRLRKQVAAKKYLQSIKNKRYGGPSSGAPPPS |
| P222 SEQ ID NO:197 | C6-HGEGTFTDNYTRLRKQVAAKKYLQSIKNKRYGGPSSGAPPPS |
| P223 SEQ ID NO:198 | C6-HSDLVFTDNYTRLRKQVAAKKYLQSIKNKRYGGPSSGAPPPS |
| P224 SEQ ID NO:199 | C6-HSEAVFTDNYTRLRKQVAAKKYLQSIKNKRYGGPSSGAPPPS |
| P228 SEQ ID NO:200 | C6-HSDAVFTDNYTRLRKQVAAKKAibLQSIKNKRYGGPSSGAPPPS |
| P229 SEQ ID NO:201 | C6-HSDAVFTDNYTRLRKQVAAKKALQSIKNKRYGGPSSGAPPPS |
| | |
| P230 SEQ ID NO:202 | C6-HSDAVFTDNYTRLRKQVAAKKYWQSIKNKRYGGPSSGAPPPS |
| P231 SEQ ID NO: 203 | C6-HSDAVFTDNYTRLRKQVAAKKYFQSIKNKRYGGPSSGAPPPS |
| P232 SEQ ID NO:204 | C6-HSDAVFTDNYTRLRKQVAAKKYTQSIKNKRYGGPSSGAPPPS |
| P233 SEQ ID NO:205 | C6-HSDAVFTDNYTRLRKQVAAKKYIQSIKNKRYGGPSSGAPPPS |
| P234 SEQ ID NO:206 | C6-HSDAVFTDNYTRLRKQVAAKKYVQSIKNKRYGGPSSGAPPPS |
| P235 SEQ ID NO:207 | C6-HSDAVFTDNYTRLRKQVAAKKYAQSIKNKRYGGPSSGAPPPS |
| P236 SEQ ID NO:208 | C6-HSDAVFTDNYTRLRKQVAAKKYAibQSIKNKRYGGPSSGAPPPS |
| P240 SEQ ID NO:209 | C6-HSDAVFTDNYTRLAibKQLAAAibKYLQSIKNKRYGGPSSGAPPPS |
| P241 SEQ ID NO:210 | C6-HSDAVFTDNYTRLAibKQLAAKAibYLQSIKNKRYGGPSSGAPPPS |
| P242 SEQ ID NO:211 | C6-HSDAVFTDNYTRLAibKQVAAAibKYLQSIKNKRYGGPSSGAPPPS |
| P243 SEQ ID NO:212 | C6-HSDAVFTDNYTRLAibKQVAAQKYLQSIKNKRYGGPSSGAPPPS |
| P244 SEQ ID NO:213 | C6-HSDAVFTDNYTRLAibKQVAAKAibYLQSIKNKRYGGPSSGAPPPS |
| P249 SEQ ID NO:214 | C6-HSDAVFTDNYTRLAibKQVAAKQYLQSIKNKRYGGPSSGAPPPS |
| P250 SEQ ID NO:215 | C6-HSDAVFTDNYTRLQKQVAAAibKYLQSIKNKRYGGPSSGAPPPS |
| P251 SEQ ID NO:216 | C6-HSDAVFTDNYTRLQKQVAAKAibYLQSIKNKRYGGPSSGAPPPS |
| P252 SEQ ID NO:217 | C6-HSDAVFTDNYTRLQKQVAAQKYLQSIKNKRYGGPSSGAPPPS |
| P253 SEQ ID NO:218 | C6-HSDAVFTDNYTRLQKQVAAKQYLQSIKNKRYGGPSSGAPPPS |
| P258 SEQ ID NO:219 | C6-HSDAVFTDNYTRLQAibQVAAKKYLQSIKNKRYGGPSSGAPPPS |
| P259 SEQ ID NO:220 | C6-HSDAVFTDNYTRLAibKQVAALKYLQSIKNKRYGGPSSGAPPPS |
| P260 SEQ ID NO:221 | C6-HSDAVFTDNYTRLAibKQVAAAKYLQSIKNKRYGGPSSGAPPPS |
| P261 SEQ ID NO:222 | C6-HSDAVFTDNYTRLRAibQVAAAibKYLQSIKNKRYGGPSSGAPPPS |
| P262 SEQ ID NO:223 | C6-HSDAVFTDNYTRLRAibQVAAVKYLQSIKNKRYGGPSSGAPPPS |
| P263 SEQ ID NO:224 | C6-HSDAVFTDNYTRLRAibQVAAAKYLQSIKNKRYGGPSSGAPPPS |
| P264 SEQ ID NO: 225 | C6-HSDAVFTDNYTRLRAibQVAAKAibYLQSIKNKRYGGPSSGAPPPS |
| P265 SEQ ID NO:226 | C6-HSDAVFTDNYTRLRAibQVAALKYLQSIKNKRYGGPSSGAPPPS |
| P269 SEQ ID NO:227 | C6-HSDAVFTDNYTRLAibKQVAAVKYLQSIKNKRYGGPSSGAPPPS |
| P270 SEQ ID NO:228 | C6-HSDAVFTDNYTRLRKQVAAKKYLQSIKQKGGPSSGAPPPS |
| P271 SEQ ID NO:229 | C6-HSDAVFTDNYTRLRKQVAAKKYLQSIKNGRPSSGAPPPS |
| P275 SEQ ID NO:230 | C6-HSDAVFTDNYTRLRKOVAGKKYLOSIKNKRYGGPSSGAPPPS |
| P282 SEQ ID NO:231 | |
| P284 SEQID NO:232 | C6-HSDAVFTDNYTRLRAibQLAAKAibYLQSIKNKRYGGPSSGAPPPS |
| P285 SEQ ID NO:233 | |
| P289 SEQ ID NO:234 | C6-HSDALFTDNYTRLRKQVAAKKYLQSIKNKRYGGPSSGAPPPS |
| P290 SEQ ID NO:235 | C6-HSDAVFTDNYTRLRKQLAAKKYLQSIKNKRYGGPSSGAPPPS |
| P291 SEQ ID NO:236 | C6-HSDAVFTDNYTRLRAibQLAAAibKYLQSIKNKRYGGPSSGAPPPS |
| P292 SEQ ID NO:237 | C6-HSDAVFTDNYTRLRAibQLAAAibKYLQSIKNKGGPSSGAPPPS |
| P293 SEQ ID NO:238 | C6-HSDAVFTDNYTRLAibKQVAAAibKYLQSIKQKGGPSSGAPPPS |
| P294 SEQ ID NO:239 | C6-HSDAVFTDNYTRLRAibQVAAAibKYLQSIKQKGGPSSGAPPPS |
| P295 SEQ ID NO:240 | C6-HSDAVFTDNYTRLRAibQVAAKAibYLQSIKQKGGPSSGAPPPS |
| P296 SEQ ID NO:241 | C6-HSDAVFTDNYTRLAibKQVAAAibKYLQSIKQGGPSSGAPPPS |
| P297 SEQ ID NO:242 | C6-HSDAVFTDNYTRLRAibQVAAAibKYLQSIKQGGPSSGAPPPS |
| P298 SEQ ID NO:243 | C6-HSDAVFTDNYTRLRAibQVAAKAibYLQSIKQGGPSSGAPPPS |
| P299 SEQ ID NO:244 | |
| P301 SEQ ID NO:245 | C6-HSDAVFTDNYTRLAAibQVAAAibKYLQSIKNKRYGGPSSGAPPPS |
| | |
| P302 SEQ ID NO:246 | C6-HSDAVFTDNYTRLQAibQVAAAibKYLQSIKNKRYGGPSSGAPPPS |
| P305 SEQ ID NO:247 | C6-HSDAVFTDNYTRLhRKQVAAKKYLQSIKNKRYGGPSSGAPPPS |
| P307 SEQ ID NO:248 | C6-HSDAVFTDNYTRLROrnQVAAKKYLQSIKNKRYGGPSSGAPPPS |
| P308 SEQ ID NO:249 | C6-HSDAVFIDNYTRLhROrnQVAAKKYLQSIKNKRYGGPSSGAPPPS |
| P314 SEQ ID NO:250 | C6-HSEAVFTENYTRLRAibQVAAAibKYLQSIKNKRYGGPSSGAPPPS |
| P315 SEQ ID NO:251 | C6-HSDAVFTDQYTRLRAibQVAAAibKYLQSIKQKRYGGPSSGAPPPS |
| P316 SEQ ID NO:252 | C6-HSDAVFTDNYTRLhRAibQVAAAibKYLQSIKNKRYGGPSSGAPPPS |
| P317 SEQ ID NO:253 | C6-HSDAVFTDNYTRLLAibQVAAAibKYLQSIKNKRYGGPSSGAPPPS |
| P318 SEQ ID NO:254 | C6-HSDAVFTDNYTRLKAibQVAAAibKYLQSIKNKRYGGPSSGAPPPS |
| P319 SEQ ID NO:255 | C6-HSDAVFTDNYTRLOrnAibQVAAAibKYLQSIKNKRYGGPSSGAPPPS |
| P320 SEQ ID NO:256 | C6-HSDAVFTDNYTRLCitAibQVAAAibKYLQSIKNKRYGGPSSGAPPPS |
| P321 SEQ ID NO:257 | C6-HSDAVFTDNYTRLRAibKVAAAibKYLQSIKNKRYGGPSSGAPPPS |
| P322 SEQ ID NO:258 | C6-HSDAVFTDNYTRLRAibQIAAAibKYLQSIKNKRYGGPSSGAPPPS |
| P323 SEQ ID NO:259 | C6-HSDAVFTDNYTRLRAibQKAAAibKYLQSIKNKRYGGPSSGAPPPS |
| P324 SEQ ID NO:260 | C6-HSDAVFTDNYTRLRAibQAAAAibKYLQSIKNKRYGGPSSGAPPPS |
| P325 SEQ ID NO:261 | C6-HSDAVFTDNYTRLRAibQNleAAAibKYLQSIKNKRYGGPSSGAPPPS |
| P326 SEQ ID NO:262 | |
| P327 SEQ ID NO:263 | |
| P329 SEQ ID NO:264 | C6-HSDAVFTDNYThRLRAibQVAAAibKYLQSIhRNhRGGPSSGAPPPS |
| P330 SEQ ID NO:265 | |
| P332 SEQ ID NO:266 | HSDAVFTDNYThRLRAibQVAAAibKYLQSIhRhRGGPSSGAPPPS |
| P333 SEQ ID NO:267 | |
| P335 SEQ ID NO:268 | C6-HSDAVFTDNYTOrnLRAibQVAAAibKYLQSIhRhRGGPSSGAPPPS |
| P336 SEQ ID NO:269 | |
| P338 SEQ ID NO:270 | |
| P339 SEQ ID NO:271 | |
| P341 SEQ ID NO:272 | C6-HSDAVFTDNYTRLRAibQVAAAibAYLQSIKNKRYGGPSSGAPPPS |
| P342 SEQ ID NO:273 | C6-HSDAVFTDNYTRLRAibQVAAAibOrnYLQSIKNKRYGGPSSGAPPPS |
| P344 SEQ ID NO:274 | C6-HSDAVFTDNYTOrnLRAibQVAAAibKYLQSIKNKRYGGPSSGAPPPS |
| | |
| P345 SEQ ID NO:275 | C6-HSDAVFTDNYTAibLRAibQVAAAibKYLQSIKNKRYGGPSSGAPPPS |
| P346 SEQ ID NO:276 | C6-HSDAVFTDNYTRAibRAibQVAAAibKYLQSIKNKRYGGPSSGAPPPS |
| P349 SEQ ID NO:277 | C6-HSDAVFTDNYTRLRAibQVAAAi bKYLQSIKAibKGGPSSGAPPPS |
| P350 SEQ ID NO:278 | C6-HSDAVFTDNYTRLRAibQVAAAibKYLQSIKPKGGPSSGAPPPS |
| P351 SEQ ID NO:279 | C6-HSDAVFTDNYTRLRAibQVAAAibKYLQSIKKGGPSSGAPPPS |
| P352 SEQ ID NO:280 | C6-HSDAVFTDNYTRLRAibQVAAAibKYLQSIOrnOrnGGPSSGAPPPS |
| P353 SEQ ID NO:281 | C6-HSDAVFTDNYTRLRAibQVAAAibKYLQSIdKdKGGPSSGAPPPS |
| P354 SEQ ID NO:282 | C6-HSDAVFTDNYTRLRAibQVAAAibKYLQSIKhRGGPSSGAPPPS |
| P355 SEQ ID NO:283 | C6-HSDAVFTDNYTRLRAibQVAAAibKYLQSIKAibGGPSSGAPPPS |
| P356 SEQ ID NO:284 | C6-HSDAVFTDNYTRLRAibQVAAAibKYLQSIOrnQOrnGGPSSGAPPPS |
| P364 SEQ ID NO:285 | C6-HSDAVFTDNYTOrnLRAibQLAAAibKYLQSIOrnNOrnGGPSSGAPPPS |
| P365 SEQ ID NO:286 | C6-HSDAVFTDNYTOrnLRAibQIAAAibKYLQSIOrnNOrnGGPSSGAPPPS |
| P366 SEQ ID NO:287 | C6-HSDAVFTDNYTALRAibQVAAAibKYLQSIOrnNOrnGGPSSGAPPPS |

More preferred VPAC2 peptide receptor agonists of the present invention comprise an amino acid sequence selected from:

| **Agonist #** | **Sequence** |
|---|---|
| P18 | HSDAVFTDNYTRLRKQVAAhRKYLQSIKNKRYGGPSSGAPPPS |
| P20 | HSDAVFTDNYTRLRKQVAARKYLQSIKNKRYGGPSSGAPPPS |
| P21 | HSDAVFTDNYTRLRKQVA ASKYLQSIKNKRYGGPSSGAPPPS |
| P22 | HSDAVFTDNYTRLRKQVAAKKYLQSIhRNKRYGGPSSGAPPPS |
| P23 | HSDAVFTDNYTRLRKQVAAKKYLQSIRNKRYGGPSSGAPPPS |
| P31 | C6-HSDAVFTDNYTRLRKQVAAKKYLQSIKNKRYGGPSSGAPPPS |
| P33 | Ac-HSDAVFTDNYTRLRKQVAAKKYLQSIKNKRYGGPSSGAPPPS |
| P44 | HVDAVFTDNYTRLRKQVAAKKYLQSIKNKRYGGPSSGAPPPS |
| P47 | HdADAVFTDNYTRLRKQVAAKKYLQSIKNKRYGGPSSGAPPPS |
| P48 | HdSDAVFTDNYTRLRKQVAAKKYLQSIKNKRYGGPSSGAPPPS |
| P61 | HSDALFTDNYTRLRKQVAAKKYLQSIKNKRYGGPSSGAPPPS |
| P72 | HSDAVFTDNYTRLRRQVAAKKYLQSIKNKRYGGPSSGAPPPS |
| P89 | HSDAVFTDNYTRLLAKVAAKKYLQSIKNKRYGGPSSG APPPS |
| P98 | C6-HSDAVFTDNYTRLRRQVAARRYLQSIRNRRYGGPSSGAPPPS |
| P99 | C6-HVDAVFTDNYTRLRKQVAAKKYLQSIKNKRYGGPSSGAPPPS |
| P100 | M-HSDAVFTDQYTRLRKQVAAKKYLQSIKQKRYGGPSSGAPPPS |
| P101 | C6-HdADAVFTDNYTRLRKQVAAKKYLQSIKNKRYGGPSSGAPPPS |
| P103 | C6-HSDAVFTDNYTKLKKQVAAKKYLQSIKNKKYGGP SSGAPPPS |
| P104 | M-HSDAVFTDNYTRLRKQVAAKKYLQSIKNKRYGGPSSGAPPPS |
| P106 | C6-HSDAVFTDNYTRLRKQVAAKKYLQSFKNKRYGGPSSGAPPPS |
| P107 | C6-HSDAVFTDNYTRLRKQVAAKKYLQSLKNKRYGGPSSGAPPPS |
| P108 | C6-HSDAVFTDNYTRLRKQVAAKKYLQSTKNKRYGGPSSGAPPPS |
| P109 | C6-HSDAVFTDNYTRLRKQVAAKKYLQSVKNKR YGGPSSGAPPPS |
| P110 | |
| P111 | C6-HSDAVFTDNYTRLRKQVAAKKYLQSYKNKRYGGPSSGAPPPS |
| P112 | C6-HSDAVFTDNYTRLRKQVAAKKYLQFIKNKRYGGPSSGAPPPS |
| P113 | C6-HSDAVFTDNYTRLRKQVAAKKYLQIIKNKRYGGPSSGAPPPS |
| P114 | C6-HSDAVFTDNYTRLRKQVAAKKYLQLIKNKRYGGPSSGAPPPS |
| P115 | C6-HSDAVFTDNYTRLRKQVAAKKYLQTIKNKRYGGPSSGAPPPS |
| P116 | C6-HSDAVFTDNYTRLRKQVAAKKYLQVIKNKRYGGPSSGAPPPS |
| P117 | C6-HSDAVFTDNYTRLRKQVAAKKYLQWIKNKRYGGPSSGAPPPS |
| P119 | C6-HSDAVFTDNYTRLLAKLALQKYLQSIKNKRYGGPSSGAPPPS |
| P120 | C6-HSDAVFTDNYTRLRKQVAAKKYLQSIKNKRYGGPSSGAPPPC |
| P121 | C6-HSDAVFTDNYTRLRKQVAAKKYLQSIKNKKGGPSSGAPPPS |
| P122 | C6-HSDAVFTDNYTRLRKQVAAKKYLQSIKNSRGGPSSGAPPPS |
| P123 | C6-HSDAVFTDNYTRLRKQVAAKKYLQSIKNKRGGPSSGAPPPS |
| P124 | C6-HSDAVFTDNYTRLRKQVAAKKYLQQIKNKRYGGPSSGAPPPS |
| P125 | C6-HSDAVFTDNYTRLRKQVAAKKYLQNIKNKRYGGPSSGAPPPS |
| P127 | C6-HSDAVFTDNYTRLRKQVAAKKYLQYIKNKRYGGPSSGAPPPS |
| P129 | C6-HSDAVFTDNYTRLRKQVAAKKWLQSIKNKRYGGPSSGAPPPS |
| P130 | C6-HSDAVFTDNYTRLRKQVAAKKFLQSIKNKRYGGPSSGAPPPS |
| P132 | C6-HSDAVFTDNYTRLRKQVAAKKLLQSIKNKRYGGPSSGAPPPS |
| P133 | C6-HSDAVFTDNYTRLRKQVAAKKILQSIKNKRYGGPSSGAPPPS |
| P134 | C6-HSDAVFTDNYTRLRKQVAAKKVLQSIKNKRYGGPSSGAPPPS |
| P135 | C6-HSDAVFTDNYTRLLAKVAAKKYLQSIKNKRYGGPSSGAPPPS |
| P138 | C6-HSDAVFTDNYTRLRAQVAAQKYLQSIKNKRYGGPSSGAPPPS |
| P139 | C6-HSDAVFTDNYTRLRAQVAAKKYLQSIKNKRYGGPSSGAPPPS |
| P142 | C6-HSDAVFTDNYTRLRAQLAAQKYLQSIKNKRYGGPSSGAPPPS |
| P143 | C6-HSDAVFTDNYTRLRKQMAAQKYLNQLKKGGPSSGAPPPS |
| P144 | C6-HSDAVFTDNYTRLRKQVAAQKYLNQLKKGGPSSGAPPPS |
| P146 | C6-HSDAVFTDNYTRLRKQVAAVKYLQSIKNKRYGGPSSGAPPPS |
| P147 | C6-HSDAVFTDNYTRLRKQVAAYKYLQSIKNKRYGGPSSGAPPPS |
| P148 | C6-HSDAVFTDNYTRLRKQVAAFKYLQSIKNKRYGGPSSGAPPPS |
| P149 | C6-HSDAVFTDNYTRLRKQVAAIKYLQSIKNKRYGGPSSGAPPPS |
| P150 | C6-HSDAVFTDNYTRLRKQVAAQKYLQSIKNKRYGGPSSGAPPPS |
| P151 | C6-HSDAVFTDNYTRLRKQVAALKYLQSIKNKRYGGPSSGAPPPS |
| P152 | C6-HSDAVFTDNYTRLRKQVAATKYLQSIKNKRYGGPSSGAPPPS |
| P153 | C6-HSDAVFTDNYTRLRKQVAAWKYLQSIKNKRYGGPSSGAPPPS |
| P154 | C6-HSDAVFTDNYTRLRKQVAAKKYLQSIKNGGPSSGAPPPS |
| P155 | C6-HSDAVFTDNYTRLRKQVALKKYLQSIKNKRYGGPSSGAPPPS |
| P158 | C6-HSDAVFTANYTRLRKQVAAKKYLQSIKNKRYGGPSSGAPPPS |
| P159 | C6-HSDAVFTENYTRLRKQVAAKKYLQSIKNKRYGGPSSGAPPPS |
| P164 | C6-HSDAVFTDNYTRLLAKLALQKYLQSIKNKRYGGPSSGAPPPC |
| P165 | C6-HSDAVFTEEYTRLQKQVAAKQYLQSIKNKRYGGPSSGAPPPS |
| P166 | C6-HAibDAVFTDNYTRLRKQVAAKKYLQSIKNKRYGGPSSGAPPPS |
| P167 | C6-HSDAVFTDNYTRLAibKQVAAKKYLQSIKNKRYGGPSSGAPPPS |
| P168 | C6-HSDAVFTDNYTRLRAibQVAAKKYLQSIKNKRYGGPSSGAPPPS |
| P169 | C6-HSDAVFTDNYTRLRKQVAAAibKYLQSIKNKRYGGPSSGAPPPS |
| P170 | C6-HSDAVFTDNYTRLRKQVAAKAibYLQSIKNKRYGGPSSGAPPPS |
| P171 | C6-HSDAVFTDNYTRLKKQVAAKKYLQSIKNKRYGGPSSGAPPPS |
| P172 | C6-HSDAVFTDNYTRLQKQVAAKKYLQSIKNKRYGGPSSGAPPPS |
| P173 | C6-HSDAVFTDNYTRLAKQVAAKKYLQSIKNKRYGGPSSGAPPPS |
| P175 | C6-HSDAVFTDNYTRLFKQVAAKKYLQSIKNKRYGGPSSGAPPPS |
| P176 | C6-HSDAVFTDNYTRLRRQVAAKKYLQSIKNKRYGGPSSGAPPPS |
| P177 | C6-HSDAVFTDNYTRLRQQVAAKKYLQSIKNKRYGGPSSGAPPPS |
| P179 | C6-HSDAVFTDNYTRLRLQVAAKKYLQSIKNKRYGGPSSGAPPPS |
| P180 | C6-HSDAVFTDNYTRLRFQVAAKKYLQSIKNKRYGGPSSGAPPPS |
| P181 | C6-HSDAVFTDNYTRLRKQVAAAKYLQSIKNKRYGGPSSGAPPPS |
| P182 | C6-HSDAVFTDNYTRLRKQVAAKRYLQSIKNKRYGGPSSGAPPPS |
| P183 | C6-HSDAVFTDNYTRLRKQVAAKQYLQSIKNKRYGGPSSGAPPPS |
| P184 | C6-HSDAVFTDNYTRLRKQVAAKAYLQSIKNKRYGGPSSGAPPPS |
| P185 | C6-HSDAVFTDNYTRLRKQVAAKLYLQSIKNKRYGGPSSGAPPPS |
| P186 | C6-HSDAVFTDNYTRLRKQVAAKFYLQSIKNKRYGGPSSGAPPPS |
| P187 | C6-HSDAVFTDNYTRLRKQVAAKKYLQAibIKNKRYGGPSSGAPPPS |
| P188 | C6-HSDAVFTDNYTRLRKQVAAKKYLQSAibKNKRYGGPSSGAPPPS |
| P192 | C6-HSDAVFTDQYTRLLAKLALQKYLQSIKQKRYGGPSSGAPPPS |
| P193 | |
| P194 | |
| P195 | C6-HSDAVFTDNYTRLLAQLALQKYLQSIKNKRYGGPSSGAPPPS |
| P196 | C6-HSDAVFTDNYTRLLAKVALQKYLQSIKNKRYGGPSSGAPPPS |
| P197 | C6-HSDAVFTDNYTRLLAKLAAQKYLQSIKNKRYGGPSSGAPPPS |
| P207 | C6-HSDdAVFTDNYTRLRKQVAAKKYLQSIKNKRYGGPSSGAPPPS |
| P209 | C6-HSDAibVFTDNYTRLRKQVAAKKYLQSIKNKRYGGPSSGAPPPS |
| P210 | C6-HSDAdVFTDNYTRLRKQVAAKKYLQSIKNKRYGGPSSGAPPPS |
| P212 | C6-HSDAAibFTDNYTRLRKQVAAKKYLQSIKNKRYGGPSSGAPPPS |
| P213 | C6-HSDAVFTDNYTRLRKQVAAKRYLQSIRNGGPSSGAPPPS |
| P214 | C6-HSDAVFTDNYTRLRKQVAARRYLQSIRNGGPSSGAPPPS |
| P215 | C6-HSDAVFTDNYTRLRRQVAAKRYLQSIRNGGPSSGAPPPS |
| P216 | C6-HSDAVFTDNYTRLRRQVAARKYLQSIRNGGPSSGAPPPS |
| P240 | C6-HSDAVFTDNYTRLAibKQLAAAibKYLQSIKNKRYGGPSSGAPPPS |
| P241 | C6-HSDAVFTDNYTRLAibKQLAAKAibYLQSIKNKRYGGPSSGAPPPS |
| P242 | C6-HSDAVFTDNYTRLAibKQVAAAibKYLQSIKNKRYGGPSSGAPPPS |
| P243 | C6-HSDAVFTDNYTRLAibKQVAAQKYLQSIKNKRYGGPSSGAPPPS |
| P244 | C6-HSDAVFTDNYTRLAibKQVAAKAibYLQSIKNKRYGGPSSGAPPPS |
| P249 | C6-HSDAVFTDNYTRLAibKQVAAKQYLQSIKNKRYGGPSSGAPPPS |
| P250 | C6-HSDAVFTDNYTRLQKQVAAAibKYLQSIKNKRYGGPSSGAPPPS |
| P251 | C6-HSDAVFTDNYTRLQKQVAAKAibYLQSIKNKRYGGPSSGAPPPS |
| P258 | C6-HSDAVFTDNYTRLQAibQVAAKKYLQSIKNKRYGGPSSGAPPPS |
| P259 | C6-HSDAVFTDNYTRLAibKQVAALKYLQSIKNKRYGGPSSGAPPPS |
| P260 | C6-HSDAVFTDNYTRLAibKQVAAAKYLQSIKNKRYGGPSSGAPPPS |
| P261 | C6-HSDAVFTDNYTRLRAibQVAAAibKYLQSIKNKRYGGPSSGAPPPS |
| P262 | C6-HSDAVFTDNYTRLRAibQVAAVKYLQSIKNKRYGGPSSGAPPPS |
| P263 | C6-HSDAVFTDNYTRLRAibQVAAAKYLQSIKNKRYGGPSSGAPPPS |
| P264 | C6-HSDAVFTDNYTRLRAibQVAAKAibYLQSIKNKRYGGPSSGAPPPS |
| P265 | C6-HSDAVFTDNYTRLRAibQVAALKYLQSIKNKRYGGPSSGAPPPS |
| P269 | C6-HSDAVFTDNYTRLAibKQVAAVKYLQSIKNKRYGGPSSGAPPPS |
| P284 | C6-HSDAVFTDNYTRLRAibQLAAKAibYLQSIKNKRYGGPSSGAPPPS |
| P291 | C6-HSDAVFTDNYTRLRAibQLAAAibKYLQSIKNKRYGGPSSGAPPPS |
| P292 | C6-HSDAVFTDNYTRLRAibQLAAAibKYLQSIKNKGGPSSGAPPPS |
| P293 | C6-HSDAVFTDNYTRLAibKQVAAAibKYLQSIKQKGGPSSGAPPPS |
| P294 | C6-HSDAVFTDNYTRLRAibQVAAAibKYLQSIKQKGGPSSGAPPPS |
| P295 | C6-HSDAVFTDNYTRLRAibQVAAKAibYLQSIKQKGGPSSGAPPPS |
| P296 | C6-HSDAVFTDNYTRLAibKQVAAAibKYLQSIKQGGPSSGAPPPS |
| P297 | C6-HSDAVFTDNYTRLRAibQVAAAibKYLQSIKQGGPSSGAPPPS |
| P298 | C6-HSDAVFTDNYTRLRAibQVAAKAibYLQSIKQGGPSSGAPPPS |
| P301 | C6-HSDAVFTDNYTRLAAibQVAAAibKYLQSIKNKRYGGPSSGAPPPS |
| P302 | C6-HSDAVFTDNYTRLQAibQVAAAibKYLQSIKNKRYGGPSSGAPPPS |
| P305 | C6-HSDAVFTDNYTRLhRKQVAAKKYLQSIKNKRYGGPSSGAPPPS |
| P307 | C6-HSDAVFTDNYTRLROrnQVAAKKYLQSIKNKRYGGPSSGAPPPS |
| P314 | C6-HSEAVFTENYTRLRAibQVAAAibKYLQSIKNKRYGGPSSGAPPPS |
| P317 | C6-HSDAVFTDNYTRLLAibQVAAAibKYLQSIKNKRYGGPSSGAPPPS |
| P318 | C6-HSDAVFTDNYTRLKAibQVAAAibKYLQSIKNKRYGGPSSGAPPPS |
| P319 | C6-HSDAVFTDNYTRLOrnAibQVAAAibKYLQSIKNKRYGGPSSGAPPPS |
| P321 | C6-HSDAVFTDNYTRLRAibKVAAAibKYLQSIKNKRYGGPSSGAPPPS |
| P322 | C6-HSDAVFTDNYTRLRAibQIAAAibKYLQSIKNKRYGGPSSGAPPPS |
| P323 | C6-HSDAVFTDNYTRLRAibQKAAAibKYLQSIKNKRYGGPSSGAPPPS |
| P324 | C6-HSDAVFTDNYTRLRAibQAAAAibKYLQSIKNKRYGGPSSGAPPPS |
| P325 | C6-HSDAVFTDNYTRLRAibQNleAAAibKYLQSIKNKRYGGPSSGAPPPS |
| P326 | C6-HSDAVFTDNYTOrnLRAibQVAAAibKYLQSIOrnNOrnGGPSSGAPPPS |
| P329 | C6-HSDAVFTDNYThRLRAibQVAAAibKYLQSIhRNhRGGPSSGAPPPS |
| P349 | C6-HSDAVFTDNYTRLRAibQVAAAibKYLQSIKAibKGGPSSGAPPPS |
| P350 | C6-HSDAVFTDNYTRLRAibQVAAAibKYLQSIKPKGGPSSGAPPPS |
| P351 | C6-HSDAVFTDNYTRLRAibQVAAAibK YLQSIKKGGPSSGAPPPS |
| P353 | C6-HSDAVFTDNYTRLRAibQVAAAibKYLQSIdKdKGGPSSGAPPPS |
| P354 | C6-HSDAVFTDNYTRLRAibQVAAAibKYLQSIKhRGGPSSGAPPPS |
| P355 | C6-HSDAVFTDNYTRLRAibQVAAAibKYLQSIKAibGGPSSGAPPPS |
| P364 | C6-HSDAVFTDNYTOrnLRAibQLAAAibKYLQSIOrnNOrnGGPSSGAPPPS |
| P365 | C6-HSDAVFTDNYTOrnLRAibQIAAAibKYLQSIOrnNOrnGGPSSGAPPPS |
| P366 | C6-HSDAVFTDNYTALRAibQVAAAibKYLQSIOrnNOrnGGPSSGAPPPS |

Even more preferred VPAC2 peptide receptor agonists of the present invention comprise an amino acid sequence selected from:

| Peptide | Sequence |
|---|---|
| P167 | C6-HSDAVFTDNYTRLAibKQVAAKKYLQSIKNKRYGGPSSGAPPPS |
| P168 | C6-HSDAVFTDNYTRLRAibQVAAKKYLQSIKNKRYGGPSSGAPPPS |
| P169 | C6-HSDAVFTDNYTRLRKQVAAAibKYLQSIKNKRYGGPSSGAPPPS |
| P 170 | C6-HSDAVFTDNYTRLRKQVAAKAibYLQSIKNKRYGGPSSGAPPPS |
| P240 | C6-HSDAVFTDNYTRLAibKQLAAAibKYLQSIKNKRYG GPSSGAPPPS |
| P241 | C6-HSDAVFTDNYTRLAibKQLAAKAibYLQSIKNKRYGGPSSGAPPPS |
| P242 | C6-HSDAVFTDNYTRLAibKQVAAAibKYLQSIKNKRYGGPSSGAPPPS |
| P243 | C6-HSDAVFTDNYTRLAibKQVAAQKYLQSIKNKRYGGPSSGAPPPS |
| P244 | C6-HSDAVFTDNYTRLAibKQVAAKAibYLQSIKNKRYGGPSSGAPPPS |
| P249 | C6-HSDAVFTDNYTRLAibKQVAAKQYLQSIKNKRYGGPSSGAPPPS |
| P250 | C6-HSDAVFTDNYTRLQKQVAAAibKYLQSIKNKRYGGPSSGAPPPS |
| P251 | C6-HSDAVFTDNYTRLQKQVAAKAibYLQSIKNKRYGGPSSGAPPPS |
| P258 | C6-HSDAVFTDNYTRLQAibQVAAKKYLQSIKNKRYGGPSSGAPPPS |
| P259 | C6-HSDAVFTDNYTRLAibKQVAALKYLQSIKNKRYGGPSSGAPPPS |
| P260 | C6-HSDAVFTDNYTRLAibKQVAAAKYLQSIKNKRYGGPSSGAPPPS |
| P261 | C6-HSDAVFTDNYTRLRAibQVAAAibKYLQSIKNKRYGGPSSGAPPPS |
| P262 | C6-HSDAVFTDNYTRLRAibQVAAVKYLQSIKNKRYGGPSSGAPPPS |
| P263 | C6-HSDAVFTDNYTRLRAibQVAAAKYLQSIKNKRYGGPSSGAPPPS |
| P264 | C6-HSDAVFTDNYTRLRAibQVAAKAibYLQSIKNKRYGGPSSGAPPPS |
| P265 | C6-HSDAVFTDNYTRLRAibQVAALKYLQSIKNKRYGGPSSGAPPPS |
| P269 | C6-HSDAVFTDNYTRLAibKQVAAVKYLQSIKNKRYGGPSSGAPPPS |
| P284 | C6-HSDAVFTDNYTRLRAibQLAAKAibYLQSIKNKRYGGPSSGAPPPS |
| P291 | C6-HSDAVFTDNYTRLRAibQLAAAibKYLQSIKNKRYGGPSSGAPPPS |
| P292 | C6-HSDAVFTDNYTRLRAibQLAAAibKYLQSIKNKGGPSSGAPPPS |
| P293 | C6-HSDAVFTDNYTRLAibKQVAAAibKYLQSIKQKGGPSSGAPPPS |
| P294 | C6-HSDAVFTDNYTRLRAibQVAAAibKYLQSIKQKGGPSSGAPPPS |
| P295 | C6-HSDAVFTDNYTRLRAibQVAAKAibYLQSIKQKGGPSSGAPPPS |
| P296 | C6-HSDAVFTDNYTRLAibKQVAAAibKYLQSIKQGGPSSGAPPPS |
| P297 | C6-HSDAVFTDNYTRLRAibQVAAAibKYLQSIKQGGPSSGAPPPS |
| P298 | C6-HSDAVFTDNYTRLRAibQVAAKAibYLQSIKQGGPSSGAPPPS |
| P301 | C6-HSDAVFTDNYTRLAAibQVAAAibKYLQSIKNKRYGGPSSGAPPPS |
| P302 | C6-HSDAVFTDNYTRLQAibQVAAAibKYLQSIKNKRYGGPSSGAPPPS |
| P314 | C6-HSEAVFTENYTRLRAibQVAAAibKYLQSIKNKRYGGPSSGAPPPS |
| P317 | C6-HSDAVFTDNYTRLLAibQVAAAibKYLQSIKNKRYGGPSSGAPPPS |
| P318 | C6-HSDAVFTDNYTRLKAibQVAAAibKYLQSIKNKRYGGPSSGAPPPS |
| P319 | C6-HSDAVFTDNYTRLOrnAibQVAAAibKYLQSIKNKRYGGPSSGAPPPS |
| P321 | C6-HSDAVFTDNYTRLRAibKVAAAibKYLQSIKNKRYGGPSSGAPPPS |
| P322 | C6-HSDAVFTDNYTRLRAibQIAAAibKYLQSIKNKRYGGPSSGAPPPS |
| P323 | C6-HSDAVFTDNYTRLRAibQKAAAibKYLQSIKNKRYGGPSSGAPPPS |
| P324 | C6-HSDAVFTDNYTRLRAibQAAAAibKYLQSIKNKRYGGPSSGAPPPS |
| P325 | C6-HSDAVFTDNYTRLRAibQNleAAAibKYLQSIKNKRYGGPSSGAPPPS |
| P326 | C6-HSDAVFTDNYTOrnLRAibQVAAAibKYLQSIOrnNOrnGGPSSGAPPPS |
| P329 | C6-HSDAVFTDNYThRLRAibQVAAAibKYLQSIhRNhRGGPSSGAPPPS |
| P349 | C6-HSDAVFTDNYTRLRAibQVAAAibKYLQSIKAibKGGPSSGAPPPS |
| P350 | C6-HSDAVFTDNYTRLRAibQVAAAibKYLQSIKPKGGPSSGAPPPS |
| P351 | C6-HSDAVFTDNYTRLRAibQVAAAibKYLQSIKKGGPSSGAPPPS |
| P353 | C6-HSDAVFTDNYTRLRAibQVAAAibKYLQSIdKdKGGPSSGAPPPS |
| P354 | C6-HSDAVFTDNYTRLRAibQVAAAibKYLQSIKhRGGPSSGAPPPS |
| P355 | C6-HSDAVFTDNYTRLRAibQVAAAibKYLQSIKAibGGPSSGAPPPS |
| P364 | C6-HSDAVFTDNYTOrnLRAibQLAAAibKYLQSIOrnNOrnGGPSSGAPPPS |
| P365 | C6-HSDAVFTDNYTOrnLRAibQIAAAibKYLQSIOrnNOrnGGPSSGAPPPS |
| P366 | C6-HSDAVFTDNYTALRAibQVAAAibKYLQSIOrnNOrnGGPSSGAPPPS |

According to a second aspect of the present invention, there is provided a VPAC2 receptor peptide agonist of the present invention for use as a medicament.

According to a third aspect of the present invent ion there is provided a VPAC2 receptor peptide agonist for use in the treatment of non -insulin dependent diabetes or insulin - dependent diabetes.

According to a further aspect of the present invention, there is provided the use of a VPAC2 receptor peptide agonist of the present invention in the manufacture of a medicament for the treatment of non-insulin-dependent diabetes.

According to yet a further aspect of the present invention, there is provided the use of a VPAC2 receptor peptide agonist of the pre sent invention in the manufacture of a medicament for the treatment of insulin -dependent diabetes.

Alternative embodiments of the present invention include a VPAC2 receptor peptide agonist further comprising a N-terminal modification linked to the N -terminus of the peptide sequence wherein the N -terminal modification involves acylation, alkylation, acetylation, a carbobenzoyl group, a succinimide group, a sulfonamide group, a carbamate group, or a urea group. N-terminal modification includes, but is not limited to eighteen carbons (C-18), ten carbons (C-10), and six carbons (C -6). N-terminal modification also includes HS(CH₂)₂CO.

Other alternative embodiments of the present invention include a VPAC2 receptor peptide agonist further comprising a N -terminal modification linked to the N -terminus of the peptide sequence wherein the N -terminal modification is selected from D-histidine and isoleucine.

Alternative embodiments of the present invention also include a VPAC2 receptor peptide agonist further comprising a N-terminal modification linked to the N -terminus of the peptide sequence wherein the N -terminal modification is selected from a cetyl, propionyl, butyryl, pentanoyl, hexanoyl, Met, 3 -phenylpropionyl, phenylacetyl, benzoyl, and norleucine.

The VPAC2 receptor peptide agonists of the present invention, therefore, have the advantage that they have enhanced selectivity, potency and/or stability over known VPAC2 receptor peptide agonists. In particular, the addition of the extension sequence of exendi n-4 as the c-capping sequence surprisingly increased the VPAC2 receptor selectivity as well as increasing proteolytic stability.

A "selective VPAC2 receptor peptide agonist" of the present invention is a peptide that selectively activates the VPAC2 recepto r to induce insulin secretion. Preferably, the sequence for a selective VPAC2 receptor peptide agonist of the present invention has from about twenty-five to about thirty-five naturally occurring and/or non -naturally occurring amino acids. More preferably, this sequence has from twenty -eight to thirty-one naturally occurring and/or non -naturally occurring amino acids.

Optionally, the selective VPAC2 receptor peptide agonist can also have an N -terminal modification. Examples include adding one or more nat urally occurring or non -naturally occurring amino acids or acylation of the N -terminus.

The N-terminal modification for the peptides of the present invention may comprise the addition of one or more naturally occurring or non -naturally occurring amino ac ids to the VPAC2 receptor peptide agonist sequence, preferably not more than ten amino acids, with one amino acid being more preferred. Naturally occurring amino acids which may be added to the N-terminus include methionine and isoleucine. A modified a mino acid added to the N-terminus may be D-histidine. Alternatively, the following amino acids may be added to the N-terminus: SEQ ID NO: 618 Ser-Trp-Cys-Glu-Pro-Gly-Trp-Cys-Arg, wherein the Arg is linked to the N-terminus of the peptide ag onist. Preferably, any amino acids added to the N - terminus are linked to the N -terminus by a peptide bond.

The term "linked to" as used herein, with reference to the term N -terminal modification, includes the addition or attachment of amino acids or chemical groups directly to the N-terminus of the VPAC2 receptor agonist. The addition of the above N -terminal modifications is usually achieved under normal coupling conditions for peptide bond formation.

The N-terminus of the peptide agonist may also be mo dified by the addition of an alkyl group (R), preferably a C ₁-C₁₆ alkyl group, to form (R)NH -.

Alternatively, the N-terminus of the peptide agonist may be modified by the addition of a group of the formula -C(O)R¹ to form an amide of the formula R¹C(O)NH-. The addition of a group of the formula -C(O)R¹ may be achieved by reaction with an organic acid of the formula R ¹COOH. Modification of the N -terminus of an amino acid sequence using acylation is demonstrated in the art (e.g. Gozes et al., J. Pharmacol Exp Ther, 273:161-167 (1995)). Addition of a group of the formula -C(O)R¹ may result in the formation of a urea group (see WO 01/23240, WO 2004/006839) or a carbamate group at the N -terminus.

The N-terminus of the peptide agonist may be modified by th e addition of a group of the formula -SO₂R⁵, to form a sulfonamide group at the N -terminus.

The N-terminus of the peptide agonist may also be modified by reacting with succinic anhydride to form a succinimide group at the N -terminus. The succinimide group incorporates the nitrogen at the N -terminal of the peptide.

The N-terminus may alternatively be modified by the addition of methionine sulfoxide.

Selective VPAC2 receptor peptide agonists of the present invention have a C-terminal extension. The "C -terminal extension" of the present invention comprises a sequence having from one to eleven naturally occurring or non -naturally occurring amino acids linked to the C-terminus of the sequence at the N -terminus of the C-terminal extension via a peptide bond.

As used herein, the term "linked to" with reference to the term C -terminal extension, includes the addition or attachment of amino acids or chemical groups directly to the C - terminus of the peptide of the Formula 12 or 13.

Most of the sequences of the prese nt invention, including the N - terminal modifications and the C - terminal extensions contain the standard single letter codes for the twenty naturally occurring amino acids. The other codes used are defined as follows:
Ac = Acetyl
C6 = hexanoyl
d = the D isoform (nonnaturally occurring) of the respective amino acid, e.g., dA = D-alanine, dS = D-serine, dK = D-lysine
hR = homoarginine
_ = position not occupied
Aib = amino isobutyric acid
CH2 = ethylene
Met(O) = methionine sulfoxide
OMe = methoxy
Nle = Nor-leucine
NMe = N-methyl attached to the alpha amino group of an amino acid, e.g., NMeA = N-methyl alanine, NMeV = N -methyl valine
Orn = ornithine
Cit = citrulline
K (Ac) = ε-acetyl lysine.
M = methionine
I = isoleucine

The term "VPAC2" is used to refer to and in conjunction with the particular receptor (see Lutz, 1999; Adamou, 1995) that the agonists of the present invention activate. This term also is used to refer to and in conjunction with the agonists of the present invention.

VIP naturally occurs as a single sequence having 28 amino acids. However, PACAP exists as either a 38 amino acid peptide (PACAP-38) or as a 27 amino acid peptide (PACAP-27) with an amidated carboxyl (Miyata, et al., Biochem Biophys Res Commun, 170:643-648 (1990)). The sequences for VIP, PACAP-27, and PACAP-38 are as follows:

| **Peptide** | **Seq.ID** # | **Sequence** |
|---|---|---|
| VIP | SEQ ID NO: 1 | HSDAVFTDNYTRLRKQMAVKKYLNSILN |
| PACAP-27 | SEQ ID NO: 2 | HSDGIFTDSYSRYRKQMAVKKYLAAVL-NH₂ |
| PACAP-38 | SEQ ID NO: 3 | |

The term "naturally occurring amino acid" as used herein means the twenty amino acids coded for by the human genetic code (i.e. the twenty standard amino acids). These twenty amino acids are: Alanine, Arginine, Asparagine, Aspartic Acid, Cysteine, Glutamine, Glutamic Acid, Glycine, Histidine, Isoleucine, Leucine, Lysine, Methionine, Phenylalanine, Proline, Serine, Threonine, Tryptophan, Tyrosine and Valine.

Examples of "non-naturally occurring amino acids" include both synthetic amino acids and those modified by the body. These include D-amino acids, arginine-like amino acids (e.g., homoarginine), and other amino acids having an extra methylene in the side chain ("homo" amino acids), and modified amino acids (e.g norleucine, lysine (isopropyl) - wherein the side chain amine of lysine is modified by an isopropyl group). Also included are amino acids such as ornithine and amino isobutyric acid. Preferably, however, the selective VPAC2 receptor peptide agonists of the present invention most frequently comprise naturally occurring amino acids except as otherwise specifically provided herein.

"Selective" as used herein refers to a VPAC2 receptor peptide agonist with increased selectivity for the VPAC2 receptor compared to other known receptors. The degree of selectivity is determined by a ratio of VPAC2 receptor binding affinity to VPAC1 receptor binding affinity and by a ratio of VPAC2 receptor binding affinity to PAC1 receptor binding affinity. Preferably, the agonists of the present invention have a selectivity ratio where the affinity for the VPAC2 receptor is at least 50 times greater than for the VPAC1 and/or for PAC1 receptors. More preferably, the affinity is at least 100 times greater for VPAC2 than for VPAC1 and/or for PAC1. Even more preferably, the affinity is at least 200 times greater for VPAC2 than for VPAC1 and/or for PAC1. Still more preferably, the affinity is at least 500 times greater for VPAC2 than for VPAC1 and/or for PAC1. Yet more preferably, the affinity is at least 1000 times greater for VPAC2 than for VPAC1 and/or for PAC1. Binding affinity is determined as described below in Example 4.

"Percent (%) sequence identity" as used herein is used to denote sequences which when aligned have similar (identical or conservatively replaced) amino acids in like positions or regions, where identical or conservatively replaced amino acids are those which do not alter the activity or function of the protein as compared to the starting protein. For example, two amino acid sequences with at least 85% identity to each other have at least 85% similar (identical or conservatively replaced residues) in a like position when aligned optimally allowing for up to 3 gaps, with the proviso that in respect of the gaps a total of not more than 15 amino acid residues is affected. Percent sequence identity may be calculated by determining the number of residues that differ between a peptide encompassed by the present invention and a reference peptide such as VIP, taking that number and dividing it by the number of amino acids in the reference peptide (e.g.. 28 amino acids for VIP), multiplying the result by 100, and subtracting that resulting number from 100. For example, a sequence having 28 amino acids with four amino acids that are different from VIP would have a percent (%) sequence identity of 86% (e.g. 100 - ((4 / 28) x 100)). For a sequence that is longer than 28 amino acids, the number of residues that differ from the VIP sequence will include the additional amino acids over 28 for purposes of the aforementioned calculation. For example, a sequence having 31 amino acids, with four amino acids different from the 28 amino acids in the VIP sequence and with three additional amino acids at the carboxy terminus which are not present in the VIP sequence, would have a total of seven amino acids that differ from VIP. Thus, this sequence would have a percent (%) sequence identity of 75% (e.g. 100 - ((7 / 28) x 100)). The degree of sequence identity may be determined using methods well known in the art (see, for example, Wilbur, W.J. and Lipman, D.J. "Rapid Similarity Searches of Nucleic Acid and Protein Data Banks", "Proceedings of the National Academy of Sciences USA 80, 726-730 (1983)" and Myers E. and Miller W. "Optimal Alignments in Linear Space" Comput. Appl. Biosci. 4:11-17 (1988)). One program which may be used in determining the degree of similarity is the MegAlign Lipman-Pearson one pair method (using default parameters) which can be obtained from DNAstar Inc, 1128, Selfpark Street, Madison, Wisconsin, 53715, USA as part of the Lasergene system. Another program, which may be used, is Clustal W. This is a multiple sequence alignment package developed by Thompson et al (Nucleic Acids Research, 1994, Vol. 22, No. 22, 4673-4680) for DNA or protein sequences. This tool is useful for performing cross-species comparisons of related sequences and viewing sequence conservation. Clustal W is a general purpose multiple sequence alignment program for DNA or proteins. It produces biologically meaningful multiple sequence alignments of divergent sequences. It calculates the best match for the selected sequences, and lines them up so that the identities, similarities and differences can be seen. Evolutionary relationships can be seen via viewing Cladograms or Phylograms.

The sequence for selective VPAC2 receptor peptide agonists of the present invention are selective for the VPAC2 receptor and preferably has a sequence identity in the range of 60% to 70%, 60% to 65%, 65% to 70%, 70% to 80%, 70% to 75%, 75% to 80%, 80% to 90%, 80% to 85%, 85% to 90%, 90% to 97%, 90% to 95%, or 95% to 97%, with VIP (SEQ ID NO: 1). More preferably, the sequence has about 61%, 64%, 68%, 71%, 75%, 79%, 82%, 86%, 89%, 93%, or 96% sequence identity with VIP.

The term "C₁-C₁₆ alkyl" as used herein means a monovalent saturated straight, branched or cyclic chain hydrocarbon radical having from 1 to 16 carbon atoms. Thus the term "C₁-C₁₆ alkyl" includes, for example, methyl, ethyl, n-propyl, isopropyl, n-butyl, isobutyl, sec-butyl, tert-butyl, n-heptyl, n-octyl, cyclopropyl, cyclobutyl, cyclopentyl and cyclohexyl. The C₁-C₁₆ alkyl group may be optionally substituted with one or more substituents.

The term "C₁- C₆ alkyl" as used herein means a monovalent saturated straight-chain, branched or cyclic chain hydrocarbon radical having from 1 to 6 carbon atoms. Thus the term "C₁-C₆ alkyl" includes, for example, methyl, ethyl, n-propyl, isopropyl, n-butyl, isobutyl, sec-butyl, tert-butyl, cyclopropyl, cyclobutyl, cyclopentyl and cyclohexyl. The C₁-C₆ alkyl group may be optionally substituted with one or more substituents.

The term "C₂-C₆ alkenyl" as used herein means a monovalent straight, branched or cyclic chain hydrocarbon radical having at least one double bond and having from 2 to 6 carbon atoms. Thus the term "C₂-C₆ alkenyl" includes vinyl, prop-2-enyl, but-3-enyl, pent-4-enyl and isopropenyl. The C₂-C₆ alkenyl group may be optionally substituted with one or more substituents.

The term "C₂-C₆ alkynyl" as used herein means a monovalent straight or branched chain hydrocarbon radical having at least one triple bond and having from 2 to 6 carbon atoms. Thus the term "C₂-C₆ alkynyl" includes prop-2-ynyl, but-3-ynyl and pent-4-ynyl. The C₂-C₆ alkynyl may be optionally substituted with one or more substituents.

The term "halo" or "halogen" means fluorine, chlorine, bromine or iodine.

The term "aryl" when used alone or as part of a group is a 5 to 10 membered aromatic or heteroaromatic group including a phenyl group, a 5 or 6- membered monocyclic heteroaromatic group, each member of which may be optionally substituted with 1, 2, 3, 4 or 5 substituents (depending upon the number of available substitution positions), a naphthyl group or an 8-, 9- or 10- membered bicyclic heteroaromatic group, each member of which may be optionally substituted with 1, 2, 3, 4, 5 or 6 substituents (depending on the number of available substitution positions). Within this definition of aryl, suitable substitutions include C₁-C₆ alkyl, C₂-C₆ alkenyl, C₂-C₆ alkynyl, amino, hydroxy, halogen, -SH and CF₃.

The term "aryl C₁-C₄ alkyl" as used herein means a C₁-C₄ alkyl group substituted with an aryl. Thus the term "aryl C₁-C₄ alkyl" includes benzyl, 1-phenylethyl (α-methylbenzyl), 2-phenylethyl, 1-naphthalenemethyl or 2-naphthalenemethyl.

The term "naphthyl" includes 1-naphthyl, and 2-naphthyl. 1-naphthyl is preferred.

The term "benzyl" as used herein means a monovalent unsubstituted phenyl radical linked to the point of substitution by a -CH₂- group.

The term "5- or 6-membered monocyclic heteroaromatic group" as used herein means a monocyclic aromatic group with a total of 5 or 6 atoms in the ring wherein from 1 to 4 of those atoms are each independently selected from N, O and S. Preferred groups have 1 or 2 atoms in the ring which are each independently selected from N, O and S. Examples of 5-membered monocyclic heteroaromatic groups include pyrrolyl (also called azolyl), furanyl, thienyl, pyrazolyl (also called 1H-pyrazolyl and 1,2-diazolyl), imidazolyl, oxazolyl (also called 1,3-oxazolyl), isoxazolyl (also called 1,2-oxazolyl), thiazolyl (also called 1,3-thiazolyl), isothiazolyl (also called 1,2-thiazolyl), triazolyl, oxadiazolyl, thiadiazolyl, tetrazolyl, oxatriazolyl and thiatriazolyl. Examples of 6-membered monocyclic heteroaromatic groups include pyridinyl, pyrimidyl, pyrazinyl, pyridazinyl and triazinyl.

The term "8-, 9- or 10-membered bicyclic heteroaromatic group" as used herein means a fused bicyclic aromatic group with a total of 8, 9 or 10 atoms in the ring system wherein from 1 to 4 of those atoms are each independently selected from N, O and S. Preferred groups have from 1 to 3 atoms in the ring system which are each independently selected from N, O and S. Suitable 8-membered bicyclic heteroaromatic groups include imidazo[2,1-b][1,3]thiazolyl, thieno[3,2-b]thienyl, thieno[2,3-d][1,3]thiazolyl and thieno[2,3-d]imidazolyl. Suitable 9-membered bicyclic heteroaromatic groups include indolyl, isoindolyl, benzofuranyl (also called benzo[b]furanyl), isobenzofuranyl (also called benzo[c]furanyl), benzothienyl (also called benzo[b]thienyl), isobenzothienyl (also called benzo[c]thienyl), indazolyl, benzimidazolyl, 1,3-benzoxazolyl, 1,2-benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1,2,3-benzothiadiazolyl, 2,1,3-benzothiadiazolyl, thienopyridinyl, purinyl and imidazo[1,2-a]pyridine. Suitable 10-membered bicyclic heteroaromatic groups include quinolinyl, isoquinolinyl, cinnolinyl, quinazolinyl, quinoxalinyl, 1,5-naphthyridyl, 1,6-naphthyridyl, 1,7-naphthyridyl and 1,8-naphthyridyl.

The term "C₁-C₆ alkoxy" as used herein means a monovalent unsubstituted saturated straight-chain or branched-chain hydrocarbon radical having from 1 to 6 carbon atoms linked to the point of substitution by a divalent O radical. Thus the term "C₁-C₆ alkoxy" includes, for example, methoxy, ethoxy, n-propoxy, isopropoxy, n -butoxy, isobutoxy, sec -butoxy and tert-butoxy. The C₁-C₆ alkoxy group may be optionally substituted with one or more substituents.

The term "N-terminal modification" as used herein includes the addition or attachment of a mino acids or chemical groups directly to the N -terminal of a peptide and the formation of chemical groups, which incorporate the nitrogen at the N -terminal of a peptide.

In a preferred embodiment, the VPAC2 receptor peptide agonist comprises a sequence of Formula 12 (SEQ ID NO: 20) or Formula 13 (SEQ ID NO: 21) wherein there is at least one amino acid substitution selected from:
Xaa₃ is: Glu;
Xaa₈ is: Glu;
Xaa₁₂ is: hR, Orn or Lys;
Xaa₁₄ is: Aib, Gln, Ala, Leu, Lys, Orn, Cit, or hR;
Xaa₁₅ is: Aib, or Orn;
Xaa₁₆ is: Lys;
Xaa₁₇ is: Leu, Ala, Ile, Lys, or Nle;
Xaa₂₀ is: Aib, Gln, Leu, Ala, or Val;
Xaa₂₁ is: Aib, Orn, Ala, or Gln;
Xaa₂₇ is: Orn or hR;
Xaa₂₈ is: Gln, Lys, hR, Aib, Pro, or Orn; and
Xaa₂₉ is: Orn or hR.
and a C-terminal extension wherein the N -terminus of the C-terminal extension is linked to the C-terminus of the peptide of Formula 12 (SEQ ID NO: 20) or Formula 13 (SEQ ID NO: 21) and wherein the C -terminal extension comprises an amino acid sequence of Formula 7 (SEQ ID NO: 15).

It is more preferred that the VPAC2 receptor peptide agonist comprises at least two of the above amino acid substitutions.

According to another embodiment of the present invention, the VPAC2 receptor peptide agonist comprises a sequence of Formula 12 (SEQ ID NO: 20) or Formula 13 (SEQ ID NO: 21) wherein Xaa ₁₄ is Leu, Xaa₁₅ is Ala, Xaa₁₆ is Lys, Xaa₁₇ is Leu, and Xaag₂₀ is Gln.

According to a preferred embodiment of the present invention, there is provided a VPAC2 receptor peptide agonist comprising an amino acid sequence of Formula 12 (SEQ ID NO: 20) or Formula 13 (SEQ ID NO: 21) wherein Xaa ₃ is Asp or Glu, Xaa₈ is Asp or Glu, Xaa₁₂ is Arg, hR, Lys, or Orn, Xaa₁₄ is Arg, Gln, Aib, hR, Orn, Cit, Lys, Ala, or Leu, Xaa ₁₅ is Lys, Aib, or Orn, Xaa ₁₆ is Gln or Lys, Xaa ₁₇ is Val, Leu, Ala, Ile, Lys, or Nle, Xaa ₂₀ is Lys, Val, Leu, Aib, Ala, or Gln, Xaa ₂₁ is Lys, Aib, Orn, Ala, or Gln, Xaa ₂₇ is Lys, Orn, or hR, Xaa₂₈ is Asn, Gln, Lys, hR, Aib, Pro, or Orn and Xaa ₂₉ is Lys, Orn, hR, or absent, and a C - terminal extension comprising a n amino acid sequence of Formula 7 (SEQ ID NO: 15).

It is more preferred that the C -terminal extension is selected from: GGPSSGAPPPS (SEQ ID NO: 10), GGPSSGAPPPS -NH₂ (SEQ ID NO: 11), GGPSSGAPPPC (SEQ ID NO: 22), GGPSSGAPPPC -NH₂ (SEQ ID NO: 23), GRPSSGAPPPS (SEQ ID NO: 24) and GRPSSGAPPPS-NH₂ (SEQ ID NO: 25).

According to another preferred embodiment of the present invention, there is provided a VPAC2 receptor peptide agonist comprising an amino acid sequence of Formula 12 (SEQ ID NO: 20) or Formula 13 (SEQ ID NO: 21) wherein Xaa₃₀ and Xaa₃₁ are absent, and a C-terminal extension comprising an amino acid sequence of Formula 7 (SEQ ID NO: 15).

Alternatively, in yet another preferred embodiment of the present invention, the VPAC2 receptor peptide agonist comprises an amino acid sequence of Formula 12 (SEQ ID NO: 20) or Formula 13 (SEQ ID NO: 21) wherein Xaa ₂₉, Xaa₃₀ and Xaa₃₁ are absent, and a C-terminal extension comprising an amino acid sequence of Formula 7 (SEQ ID NO: 15).

It is more preferred that the C -terminal extension is selected from: GGPSSGAPPPS (SEQ ID NO: 10) or GGPSSGAPPPS -NH₂ (SEQ ID NO: 11).

According to another preferred embodiment of the present invention, there is provided a VPAC receptor peptide agonist comprising an amino acid sequence of Formula 12 (SEQ ID NO: 20) or Formula 13 (SEQ ID NO: 21) and a C -terminal extension wherein the N-terminus of the C -terminal extension is linked to the C -terminus of the peptide of Formula 12 (SEQ ID NO: 20) or Formula 13 (SEQ ID NO: 21) wherein either Xaa₁₄ or Xaa₁₅ is Aib and either Xaa ₂₀ or Xaa₂₁ is Aib and wherein the C -terminal extension comprises an amino acid sequence of Formula 7 (SEQ ID NO: 15).

According to yet another preferred embodiment of the present invention, there is provided a VPAC receptor peptide agonist comprising an amino acid sequence of Formula 12 (SEQ ID NO: 20) or Formula 13 (SEQ ID NO: 21) and a C -terminal extension wherein the N-terminus of the C -terminal extension is linked to the C -terminus of the peptide of Formula 12 (SEQ ID NO: 20) or Formula 13 (SEQ ID NO: 21) wherein either Xaa ₁₄ or Xaa₁₅ is Aib and either Xaa ₂₀ or Xaag₂₁ is Aib and Xaa₂₈ is Gln and Xaa₂₉ is Lys or absent, and wherein the C -terminal extension comprises an amino acid sequence of Formula 7 (SEQ ID NO: 15).

In a further preferred embodiment of the present invention, there is provided a VPAC receptor peptide agonist comprising an amino acid sequence of Formula 12 (SEQ ID NO: 20) or Formula 13 (SEQ ID NO: 21) and a C -terminal extension wherein the N -terminus of th e C-terminal extension is linked to the C -terminus of the peptide of Formula 12 (SEQ ID NO: 20) or Formula 13 (SEQ ID NO: 21) wherein either Xaa ₁₄ or Xaa₁₅ is Aib and either Xaa₂₀ or Xaa₂₁ is Aib and Xaa₁₂ of the peptide sequence is hR or Orn, Xaa ₂₇ is hR or Orn and Xaa₂₉ is hR or Orn and wherein the C -terminal extension comprises an amino acid sequence of Formula 7 (SEQ ID NO: 15).

In the above preferred embodiments of the present invention, it is especially preferred that the VPAC2 receptor peptide agonis t further comprises a N-terminal modification, wherein the N-terminal modification is the addition of a group selected from: acetyl, propionyl, butyryl, pentanoyl, hexanoyl, methionine, methionine sulfoxide, 3 - phenylpropionyl, phenylacetyl, benzoyl, norleu cine, D-histidine, isoleucine and 3 - mercaptopropionyl and more preferably is the addition of acetyl or hexanoyl.

In a preferred embodiment, there is provided a VPAC2 receptor peptide agonist comprising an amino acid sequence of Formula 12 (SEQ ID NO: 20) or Formula 13 (SEQ ID NO: 21) wherein either Xaa ₁₄ or Xaa ₁₅ is Aib and either Xaa ₂₀ or Xaag₂₁ is Aib, and a C - terminal extension selected from: GGPSSGAPPPS (SEQ ID NO: 10), GGPSSGAPPPS -NH₂ (SEQ ID NO: 11), GGPSSGAPPPC (SEQ ID NO: 22), GGPSSGAPPPC -NH₂ (SEQ ID NO: 23), GRPSSGAPPPS (SEQ ID NO: 24) and GRPSSGAPPPS -NH₂ (SEQ ID NO: 25) and wherein the VPAC2 receptor peptide agonist further comprises a N -terminal modification which modification is the addition of hexanoyl or acetyl.

In another preferred embodiment, there is provided a VPAC2 receptor peptide agonist comprising an amino acid sequence of Formula 12 (SEQ ID NO: 20) or Formula 13 (SEQ ID NO: 21) wherein either Xaa ₁₄ or Xaa₁₅ is Aib and either Xaa₂₀ or Xaa₂₁ is Aib, Xaa₂₈ is Gln and Xaa₂₉ is Lys or absent, and a C-terminal extension selected from: GGPSSGAPPPS (SEQ ID NO: 10), GGPSSGAPPPS -NH₂ (SEQ ID NO: 11), GGPSSGAPPPC (SEQ ID NO: 22), GGPSSGAPPPC-NH₂ (SEQ ID NO: 23), GRPSSGAPPPS (SEQ ID NO: 24) and GRPSSGAPPPS-NH₂ (SEQ ID NO: 25) wherein the VPAC2 recept or peptide agonist further comprises a N-terminal modification which modification is the addition of hexanoyl or acetyl.

In yet another preferred embodiment, there is provided a VPAC2 receptor peptide agonist comprising an amino acid sequence of Formula 12 (SEQ ID NO: 20) or Formula 13 (SEQ ID NO: 21) wherein either Xaa ₁₄ or Xaa₁₅ is Aib and either Xaa ₂₀ or Xaa₂₁ is Aib, Xaa₁₂ is hR or Orn, Xaa₂₇ is hR or Orn and Xaa₂₉ is hR or Orn, and a C -terminal extension selected from: GGPSSGAPPPS (SEQ ID NO: 10), GG PSSGAPPPS-NH₂ (SEQ ID NO: 11), GGPSSGAPPPC (SEQ ID NO: 22), GGPSSGAPPPC -NH₂ (SEQ ID NO: 23), GRPSSGAPPPS (SEQ ID NO: 24) and GRPSSGAPPPS -NH₂ (SEQ ID NO: 25) wherein the VPAC2 receptor peptide agonist further comprises a N -terminal modification which modification is the addition of hexanoyl or acetyl.

Preferably, the agonists of the present invention have a selectivity ratio where the affinity for the VPAC2 receptor is at least 50 times greater than for the VPAC1 and/or for PAC1 receptors. More preferably, this affinity is at least 100 times greater for VPAC2 than for VPAC 1 and/or for PAC1. Even more preferably, the affinity is at least 200 times greater for VPAC2 than for VPAC1 and/or for PAC1. Still more preferably, the affinity is at least 500 times greater for VPAC2 than for VPAC 1 and/or for PAC1. Yet more preferably, the affinity is at least 1000 times greater for VPAC2 than for VPAC1 and/or for PAC1. Preferably, these agonists have a sequence identity in the range of 60% to 70%, 60% to 65%, 65% to 70%, 70% to 80%, 70% to 75%, 75% to 80%, 80% to 90%, 80% to 85%, 85% to 90%, 90% to 97%, 90% to 95%, or 95% to 97%, with VIP (SEQ ID NO: 1). More preferably, the sequence has 61%, 64%, 68%, 71%, 75%, 79%, 82%, 86%, 89%, 93%, or 96% sequence identity wi th VIP.

Preferred C-terminal extension s of the present invention include the following sequences:

| **SEQ ID #** | **Sequence** |
|---|---|
| SEQ ID NO: 10 | GGPSSGAPPPS |
| SEQ ID NO: 11 | GGPSSGAPPPS-NH₂ |

Preferably, the C-terminal extension differs from SEQ ID NO: 10 or SEQ ID NO: 11 by no more than eight amino acids, still preferably by no more than seven amino acids, yet still preferably by no more than six amino acids, more preferably by no more than five amino acids, even more preferably by no more than four amino acids, still more preferably by no more than three amino acids, yet more preferably by no more than two amino acids, and most preferably by no more than one amino acid.

Alternative C-terminal extensions of the present invention also include variants of these sequences, including:

| **SEQ ID #** | **Sequence** |
|---|---|
| SEQ ID NO: 12 | GGPSSGAPPS-NH2 |
| SEQ ID NO: 13 | GGPSSGAPPPS-OH |
| SEQ ID NO: 14 | GGPSSGAPPS |

These sequences contain the standard single letter codes for the twenty naturally occurring amino acids. SEQ ID NO: 11 and SEQ ID NO: 12 contain sequences that are amidated at the C -terminus of the sequence.

Preferably, the C-terminal extension differs from SEQ ID NO:12, or SEQ ID NO: 14 by no more than eight amino acids, still preferably by no more than seven amino acids, yet still preferably by no more than six amino acids, more preferably by no more than five amino acids, even more preferably by no more than four amino acids, still more preferably by no more than three amino acids, yet more preferably by no more than two amino acids, an d most preferably by no more than one amino acid.

The C-terminal extension of the VPAC2 receptor peptide agonists of the present invention comprises an amino acid sequence of the Formula 7 (SEQ ID NO: 15), provided that if Xaa₁, Xaa₂, Xaa₃, Xaa₄, Xaa₅, Xaa₆, Xaa₇, Xaa₈, Xaa₉, or Xaa₁₀ of Formula 7 is absent, the next amino acid present downstream is the next amino acid in the C -terminal extension and wherein the C-terminal amino acid may be amidated. For example, if Xaa ₁ is Gly and Xaa₂ is absent, the next amino acid bonded to Gly at position 1 is an amino acid listed for position 3 or, if position 3 is also absent, an amino acid listed for position 4 is bonded to Gly at position 1, and so forth.

The present invention includes the following alternative selective VPAC2 receptor peptide agonists:

| **Agonist #** | **Sequence** |
|---|---|
| P226 | |
| P227 | |

"Insulinotropic activity" refers to the ability to stimulate insulin secretion in response to elevated glucose levels, thereby causing glucose uptake by cells and decreased plasma glucose levels. Insulinotropic activity can be assessed by methods known in the art, including using experiments that measure VPAC2 receptor binding activity or receptor activation (e.g. insulin secretion by insulinoma cell lines or islets, intravenous glucose tolerance test (IVGTT), intraperitoneal glucose tolerance test (IPGTT), and oral glucose tolerance test (OGTT)). Insulinotropic activity is routinely measured in humans by measuring insulin levels or C -peptide levels. Selective VPAC2 receptor peptide agon ists of the present invention have insulinotropic activity.

"*In vitro* potency" as used herein is the measure of the ability of a peptide to activate the VPAC2 receptor in a cell -based assay. *In vitro* potency is expressed as the "EC ₅₀" which is the effective concentration of compound that results in a 50% of maximum increase in activity in a single dose -response experiment. For the purposes of the present invention, *in vitro* potency is determined using two different assays: DiscoveRx and Alpha Screen. See Example 3 for further details of these assays. Whilst these assays are performed in different ways, the results demonstrate a general correlation between the two assays.

The present invention encompasses the discovery that acylation of or specific amino acids added to the N-terminus of a peptide sequence for a selective VPAC2 receptor peptide agonist provide features that may enhance potency and/or provide stability against DPP-IV cleavage.

The present invention encompasses the discovery that specific amino acids added to the C-terminus of a peptide sequence for a VPAC2 receptor peptide agonist provide features that may protect the peptide as well as may enhance activity, selectivity, and/or potency. For example, these C -terminal extensions may stabilize the helical structure of the peptide and sites within the peptide prone to enzymatic cleavage that are located near the C -terminus. Further, many of the C -terminally extended peptides disclosed herein may be more selective for the VPAC2 receptor and c an be more potent than VIP, PACAP, and other known VPAC2 receptor peptide agonists. An example of a preferred C -terminal extension is the extension peptide of exendin-4 as the C-capping sequence. Exendin -4 is found in the salivary excretions from the Gil a Monster, *Heloderma Suspectum* , (J.Biol.Chem., Vol. 267, No. 11, April 15, pp. 7402 -7405, 1992).

VIP and some known VPAC2 receptor peptide agonists are susceptible to cleavage by various enzymes and, thus, have a short *in vivo* half-life. Five regions, identified below, correspond to the same positions in VIP (SEQ ID NO: 1), are discussed relative to the amino acid position in VIP, and are applicable to the sequences noted herein.

Region 1 contains a cleavage site at amino acid position 2 of Formula e 12 and 13 for the enzyme dipeptidyl-peptidase IV (DPP-IV). Cleavage of the peptide occurs between position 2 (serine) and position 3 (aspartic acid). The compounds of the present invention are stable against DPP-IV cleavage due to various substitutions at position 2 of Formulae 12 and 13 and/or the addition of a N -terminal modification as discussed previously. Examples of amino acids at position 2 that may improve stability against DPP -IV inactivation preferably include valine, D-alanine, or D-serine. More preferably, position 2 is valine or D -alanine. Examples of N-terminal modifications that may improve stability against DPP -IV inactivation include the addition of acetyl, propionyl, butyryl, pentanoyl, hexanoyl, methionine, methionine sulfoxide, 3 -phenylpropionyl, phenylacetyl, benzoyl, norleucine, D - histidine, isoleucine and 3-mercaptopropionyl. Preferably, the N-terminal modification is the addition of acetyl or hexanoyl. For these examples and preferred examples of N-terminal modifications, preferred amino acids at position 2 include serine as well as valine, D-alanine, or D-serine, with more preference for position 2 being substituted with valine or D-alanine Example 8 illustrates the stability of various selective VPAC2 receptor peptide agonists against DPP-IV inactivation encompassed by the present invention.

Regions 2 and 3, which encompass basic amino acids at positions 14 and 15 and positions 20 and 21 respectively in wild-type VIP as well as numerous VPAC2 receptor agonists known in the art, are also susceptible to enzymatic cleavage. The selective VPAC2 receptor agonists of the present invention generally have improved proteolytic stability *in vivo* due to substitutions in these two regions. These substitutions can render the peptide resistant to cleavage by trypsin-like enzymes, including trypsin. Examples of amino acids at position 14 that confer some resistance to cleavage by trypsin-like enzymes alone or in combination with the amino acids specified for position 15 below include glutamine, amino isobutyric acid, homoarginine, ornithine, citrulline, lysine, alanine and leucine. Also, position 14 may be arginine when position 15 is an amino acid other than lysine. Also, position 14 can be arginine when position 15 is lysine, but this specific combination does not address enzymatic cleavage. Examples of amino acids at position 15 that confer some resistance to cleavage by trypsin-like enzymes alone or in combination with amino acids specified above for position 14 include amino isobutyric acid and ornithine. Also, position 15 may be lysine when position 14 is an amino acid other than arginine. Also, position 15 can be lysine when position 14 is arginine, but this specific combination does not address enzymatic cleavage. Examples of amino acids at position 20 that confer some resistance to cleavage by trypsin-like enzymes alone or in combination with amino acids specified for position 21 include valine, leucine, amino isobutyric acid, alanine and glutamine. Also, position 20 may be lysine when position 21 is an amino acid other than lysine. Also, position 20 can be lysine when position 21 is lysine, but this specific combination does not address enzymatic cleavage. An example of an amino acid at position 21 that confers some resistance to cleavage by trypsin-like peptides alone or in combination with amino acids specified for position 20 include amino isobutyric acid, ornithine, alanine, or glutamine. Also, position 21 may be lysine when position 20 is an amino acid other than lysine. Also, position 21 can be lysi ne when position 20 is lysine, but this specific combination does not address enzymatic cleavage. The improved stability of a representative number of selective VPAC2 receptor peptide agonists with resistance to peptidase cleavage and encompassed by the present invention is demonstrated in Example 6.

Region 4 encompasses the amino acids at positions 25 and 26 of Formula e 12 and 13. Region 4 is another area that is susceptible to enzymatic cleavage. This cleavage site can be completely or partially eliminated through substitution of the amino acid at position 25 and/or the amino acid at position 26. Examples of amino acids at position 25 that confer at least some resistance to enzymatic cleavage include phenylalanine, isoleucine, leucine, threonine, valine, tryptophan, glutamine, asparagine, tyrosine, or amino isobutyric acid . Also, position 25 may be serine when position 26 is an amino acid other than isoleucine. Also, position 25 can be serine when position 26 is isoleucine, but this specific combination does not address enzymatic cleavage. Examples of amino acids at position 26 that confer at least some resistance to enzymatic cleavage alone or in combination with the amino acids specified above for position 25 include leucine, threonine, valine, tr yptophan, tyrosine, phenylalanine, or amino isobutyric acid. Also, position 26 may be isoleucine when position 25 is an amino acid other than serine. Also, position 26 can be isoleucine when position 25 is serine, but this specific combination does not address enzymatic cleavage.

Region 4 also encompasses the amino acids at positions 27, 28, 29, 30 and 31 respectively in wild -type VIP as well as in many VPAC2 receptor peptide agonists known in the art. This area is also susceptible to enzymatic cleaving. The addition of a C -terminal extension peptide may render the peptide agonist more stable against neutroendopeptidase (NEP). The addition of the extension peptide may also increase selectivity for the VPAC2 receptor. Trypsin-like enzymes may also attack these positions. If that occurs, the peptide agonist may lose its C -terminal extension with the additional carboxypeptidase activity leading to an inactive form of the peptide.

In addition to selective VPAC2 receptor peptide agonists with resistance to cleavage by various peptidases, the selective VPAC2 peptide receptor agonists of the present invention may also encompass peptides with enhanced selectivity for the VPAC2 receptor, increased potency, and/or increased stability compared with some peptide s known in the art. Examples of amino acid positions that may affect such properties include positions: 3, 8, 12, 14, 15, 16, 17, 20, 21, 27, 28, and 29 of Formula 12, or 13. For example, the amino acid at position 3 is preferably aspartic acid or glutam ic acid; the amino acid at position 8 is preferably aspartic acid or glutamic acid; the amino acid at position 12 is preferably arginine, homoarginine, ornithine, or lysine; the amino acid at position 14 is preferably arginine, glutamine, amino isobutyric acid, homoarginine, ornithine, citrulline, lysine, alanine, or leucine; the amino acid at position 15 is preferably lysine, amino isobutyric acid, or ornithine; the amino acid at position 16 is preferably glutamine or lysine; the amino acid at position 17 is preferably valine, alanine, leucine, isoleucine, lysine, or norleucine; the amino acid at position 20 is preferably lysine, valine, leucine, amino isobutyric acid, alanine, or glutamine; the amino acid at position 21 is preferably lysine, amino isobutyr ic acid, ornithine, alanine, or glutamine; the amino acid at position 27 is preferably lysine, ornithine, or homoarginine; the amino acid at position 28 is preferably asparagine, glutamine, lysine, homoarginine, amino isobutyric acid, proline, or ornithine ; and, if present, the amino acid at position 29 is preferably lysine, ornithine, or homoarginine. Preferably, these amino acid substitutions may be combined with substitutions at positions that affect the five aforementioned regions susceptible to cleavage by various enzymes.

The increased potency and selectivity for various VPAC2 receptor peptide agonists of the present invention is demonstrated in Examples 3 and 4. For example, Table 1 in Example 3 provides a list of selective VPAC2 receptor peptide agonists and their corresponding *in vitro* potency results. Preferably, the selective VPAC2 receptor peptide agonists of the present invention have an EC ₅₀ value less than 2 nM. More preferably, the EC ₅₀ value is less than 1 nM. Even more preferably, the EC₅₀ value is less than 0.5 nM. Still more preferably, the EC₅₀ value is less than 0.1 nM.

Table 2 in Example 4 provides a list of VPAC2 receptor peptide agonists and their corresponding selectivity results for VPAC2, VPAC1, and PAC1. See Example 4 for further details of these assays. These results are provided as a ratio of VPAC2 binding affinity to VPAC1 binding affinity and as a ratio of VPAC2 binding affinity to PAC1 binding affinity. Preferably, the agonists of the present invention have a selectivity ratio where the affinity for the VPAC2 receptor is at least 50 times greater than for the VPAC1 and/or for PAC1 receptors. More preferably, this affinity is at least 100 times greater for VPAC2 than for VPAC1 and/or for PAC1. Even more preferably, the affinity is at least 200 times greater for VPAC2 than for VPAC1 and/or for PAC1. Still more preferably, the affinity is at least 500 times greater for VPAC2 than for VPAC1 and/or for PAC1. Yet more preferably, the ratio is at least 1000 times greater for VPAC2 than for VPAC1 and/or for PAC1.

As used herein, "selective VPAC2 receptor peptide agonists" also include pharmaceutically acceptable salts of the compounds described herein. A selective VPAC2 receptor peptide agonist of this invention can possess a sufficiently acidic, a sufficiently basic, or both functional groups, and accordingly react with any of a number of inorganic bases, and inorganic and organic acids, to form a salt. Acids commonly employed to form acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, *p*-bromophenyl-sulfonic acid, carbonic acid, succinic acid, citric acid, benzoic acid, acetic acid, trifluoroacetic acid, and the like. Examples of such salts include the sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, gammahydroxybutyrate, glycolate, tartrate, methanesulfonate, propanesulfonate, naphthalene-1-sulfonate, naphthalene-2-sulfonate, mandelate, and the like.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, and the like.

The selective VPAC2 receptor peptide agonists of the present invention can be administered parenterally. Parenteral administration can include, for example, systemic administration, such as by intramuscular, intravenous, subcutaneous, intradermal, or intraperitoneal injection. These agonists can be administered to the subject in conjunction with an acceptable pharmaceutical carrier, diluent, or excipient as part of a pharmaceutical composition for treating NIDDM. Standard pharmaceutical formulation techniques may be employed such as those described in Remington's Pharmaceutical Sciences, Mack Publishing Company; Easton, PA. The selective VPAC2 receptor peptide agonists of the present invention may be formulated for administration through the buccal, topical, oral, transdermal, nasal, or pulmonary route.

The selective VPAC2 receptor peptide agonists described herein can be used to treat subjects with a wide variety of diseases and conditions. Agonists encompassed by the present invention exert their biological effects by acting at a receptor referred to as the VPAC2 receptor. Subjects with diseases and/or conditions that respond favorably to VPAC2 receptor stimulation or to the administration of VPAC2 receptor peptide agonists can therefore be treated with the VPAC2 agonists of the present invention. These subjects are said to "be in need of treatment with VPAC2 agonists" or "in need of VPAC2 receptor stimulation".

The selective VPAC2 receptor peptide agonists of the present invention may be employed to treat diabetes, including both type 1 and type 2 diabetes (non-insulin dependent diabetes mellitus). Also included are subjects requiring prophylactic treatment with a VPAC2 receptor agonist, e.g., subjects at risk for developing NIDDM. Such treatment may also delay the onset of diabetes and diabetic complications. Additional subjects include those with impaired glucose tolerance or impaired fasting glucose, subjects whose body weight is about 25% above normal body weight for the subject's height and body build, subjects having one or more parents with NIDDM, subjects who have had gestational diabetes, and subjects with metabolic disorders such as those resulting from decreased endogeno us insulin secretion. The selective VPAC2 receptor peptide agonists may be used to prevent subjects with impaired glucose tolerance from proceeding to develop type 2 diabetes, prevent pancreatic β-cell deterioration, induce β-cell proliferation, improve β-cell function, activate dormant β-cells, differentiate cells into β-cells, stimulate β-cell replication, and inhibit β-cell apoptosis. Other diseases and conditions that may be treated or prevented using compounds of the invention include: Maturity -Onset Diabetes of the Young (MODY) (Herman, et al., Diabetes 43:40, 1994); Latent Autoimmune Diabetes Adult (LADA) (Zimmet, et al., Diabetes Med. 11:299, 1994); impaired glucose tolerance (IGT) (Expert Committee on Classification of Diabetes Mellitus, Diabetes Care 22 (Supp. 1):S5, 1999); impaired fasting glucose (IFG) (Charles, et al., Diabetes 40:796, 1991); gestational diabetes (Metzger, Diabetes, 40:197, 1991); metabolic syndrome X, dyslipidemia, hyperglycemia, hyperinsulinemia, hypertriglyceridemia, and in sulin resistance.

The selective VPAC2 receptor peptide agonists of the present invention may also be effective in the prevention or treatment of such disorders as obesity, atherosclerotic disease, hyperlipidemia, hypercholesteremia, low HDL levels, hyperte nsion, primary pulmonary hypertension, cardiovascular disease (including atherosclerosis, coronary heart disease, coronary artery disease, and hypertension), cerebrovascular disease and peripheral vessel disease; and for the treatment of lupus, polycystic ovary syndrome, carcinogenesis, and hyperplasia, asthma, male and female reproduction problems, sexual disorders, ulcers, sleep disorders, disorders of lipid and carbohydrate metabolism, circadian dysfunction, growth disorders, disorders of energy homeosta sis, immune diseases including autoimmune diseases (e.g., systemic lupus erythematosus), as well as acute and chronic inflammatory diseases, rheumatoid arthritis, and septic shock.

The selective VPAC2 receptor peptide agonists of the present invention ma y also be useful for treating physiological disorders related to, for example, cell differentiation to produce lipid accumulating cells, regulation of insulin sensitivity and blood glucose levels, which are involved in, for example, abnormal pancreatic β-cell function, insulin secreting tumors and/or autoimmune hypoglycemia due to autoantibodies to insulin, autoantibodies to the insulin receptor, or autoantibodies that are stimulatory to pancreatic β-cells, macrophage differentiation which leads to the formation of atherosclerotic plaques, inflammatory response, carcinogenesis, hyperplasia, adipocyte gene expression, adipocyte differentiation, reduction in the pancreatic β-cell mass, insulin secretion, tissue sensitivity to insulin, liposarcoma cell gro wth, polycystic ovarian disease, chronic anovulation, hyperandrogenism, progesterone production, steroidogenesis, redox potential and oxidative stress in cells, nitric oxide synthase (NOS) production, increased gamma glutamyl transpeptidase, catalase, plasma triglycerides, HDL, and LDL cholesterol levels, and the like.

The selective VPAC2 receptor peptide agonists of the invention may also be used to treat secondary causes of diabetes (Expert Committee on Classification of Diabetes Mellitus, Diabetes Care 22 (Supp. 1):S5, 1999). Such secondary causes include glucocorticoid excess, growth hormone excess, pheochromocytoma, and drug -induced diabetes. Drugs that may induce diabetes include, but are not limited to, pyriminil, nicotinic acid, glucocorticoids, phenytoin, thyroid hormone, β-adrenergic agents, α-interferon and drugs used to treat HIV infection.

In addition, the selective VPAC2 receptor peptide agonists of the invention may be used for treatment of asthma (Bolin, et al., Biopolymer 37:57 -66, 1995; U.S. Patent No. 5677,419; showing that polypeptide R3PO is active in relaxing guinea pig tracheal smooth muscle); hypotension induction (VIP induces hypotension, tachycardia, and facial flushing in asthmatic patients (Morice, et al., Peptides 7:279 -280, 1986; Morice, et al., Lancet 2:1225-1227, 1983); male reproduction problems (Siow, et al., Arch. Androl. 43(1):67 -71, 1999); as an anti-apoptosis/neuroprotective agent (Brenneman, et al., Ann. N. Y. Acad. Sci. 865:207 - 12, 1998); cardioprotection during ischemic events ( Kalfin, et al. , J. Pharmacol. Exp. Ther. 1268(2):952-8, 1994; Das, et al., Ann. N. Y. Acad. Sci. 865:297 -308, 1998), manipulation of the circadian clock and its associated disorders (Hamar, et al., Cell 109:497 -508, 2002; Shen, et al., Proc. Natl. Acad. Sci. 97:11575 -80, 2000), and as an anti -ulcer agent (Tuncel, et al., Ann. N. Y. Acad. Sci. 865:309 -22, 1998).

An "effective amount" of a selective VPAC2 receptor peptide agonist is the quantity that results in a desired therapeutic and/or prophylactic effect without causing unacceptable side effects when administered to a subject in need of VPAC2 receptor stimulation. A "desired therapeutic effect" includes one or more of the following: 1) an amelioration of the symptom(s) associated with the disease or condition; 2) a delay in the onset of symptoms associated with the disease or condition; 3) increased longevity compared with the absence of the treatment; and 4) greater quality of life compared with the absence of the treatment. For example, an "effective amount" of a VPAC2 agonist for the treatment of NIDDM is the quantity that would result in greater control of blood glucose concentration than in the absence of treatment, thereby resulting in a delay in the onset of diabetic complications such as retinopathy, neuropathy, or kidney disease. An "effective amount" of a selective VPAC2 receptor peptide agonist for the prevention of NIDDM is the quantity that would delay, compared with the absence of treatment, the onset of elevated blood glucose levels that require treatment with anti-hypoglycemic drugs such as sulfonylureas, thiazolidinediones, insulin, and/or bisguanidines.

An "effective amount" of the selective VPAC2 receptor peptide agonist administered to a subject will also depend on the type and severity of the disease and on the characteristics of the subject, such as general health, age, sex, body weight and tolerance to drugs. The dose of selective VPAC2 peptide receptor agonist effective to normalize a patient's blood glucose will depend on a number of factors, among which are included, without limitation, the subject's sex, weight and age, the severity of inability to regulate blood glucose, the route of administration and bioavailability, the pharmacokinetic profile of the peptide, the potency, and the formulation.

A typical dose range for the selective VPAC2 receptor peptide agonists of the present invention will range from about 1 µg per day to about 5000 µg per day. Preferably, the dose ranges from about 1 µg per day to about 2500 µg per day, more preferably from about 1 µg per day to about 1000 µg per day. Even more preferably, the dose ranges from about 5 µg per day to about 100 µg per day. A further preferred dose range is from about 10 µg per day to about 50 µg per day. Most preferably, the dose is about 20 µg per day.

A "subject" is a mammal, preferably a human, but can also be an animal, e.g., companion animals (e.g., dogs, cats, and the like), farm animals (e.g., cows, sheep, pigs, horses, and the like) and laboratory animals (e.g., rats, mice, guinea pigs, and the like).

The selective VPAC2 receptor peptide agonists of the present invention can be prepared by using standard methods of solid -phase peptide synthesis techniques. Peptide synthesizers are commercially available from, for example, Rainin -PTI Symphony Peptide Synthesizer (Tucson, AZ). Reagents for solid phase synthesis are commercially available, for example, from Glycopep (Chicago, IL). Solid phase peptide synthesizers can be used according to manufacturers instructions for blocking interfering groups, protecting the amino acid to be reacted, coupling, decoupling, and capping of unreacted amino acids.

Typically, an α-*N*-protected amino acid and the N-terminal amino acid on the growing peptide chain on a resin is coupled at room temperature in an inert solvent such as dimethylformamide, N-methylpyrrolidone or methylene chloride in the presence of coupling agents such as dicyclohexylcarbodiimide and 1 -hydroxybenzotri azole and a base such as diisopropylethylamine. The α-*N*-protecting group is removed from the resulting peptide resin using a reagent such as trifluoroacetic acid or piperidine, and the coupling reaction repeated with the next desired *N*-protected amino acid to be added to the peptide chain. Suitable amine protecting groups are well known in the art and are described, for example, in Green and Wuts, "Protecting Groups in Organic Synthesis" , John Wiley and Sons, 1991 . Examples include t-butyloxycarbonyl (tBoc) and fluorenylmethoxycarbonyl (Fmoc).

The selective VPAC2 receptor peptide agonists are also synthesized using standard automated solid-phase synthesis protocols using t-butoxycarbonyl- or fluorenylmethoxycarbonyl -alpha-amino acids with appropriate side -chain protection. After completion of synthesis, peptides are cleaved from the solid -phase support with simultaneous side-chain deprotection using standard hydrogen fluoride methods or trifluoroacetic acid (TFA). Crude peptides are then further purified using Reversed-Phase Chromatography on Vydac C18 columns using acetonitrile gradients in 0.1% trifluoroacetic acid (TFA). To remove acetonitrile, peptides are lyophilized from a solution containing 0.1 % TFA, acetonitrile and water. Purity can be verified by analytical reversed phase chromatography. Identity of peptides can be verified by mass spectrometry. Peptides can be solubilized in aqueous buffers at neutral pH.

The peptide agonists of the present invention may also be made by recombinant methods known in the art using both eukaryotic and prokaryotic cellular hosts.

Various preferred features and embodiments of the present invention will now be described with reference to the following non-limiting examples.

### Example 1

### Preparation of the Selective VPAC2 Receptor Peptide Agonists by Solid Phase t-Boc Chemistry:

Approximately 0.5-0.6 grams (0.38-0.45 mmole) Boc Ser(Bzl)-PAM resin is placed in a standard 60 mL reaction vessel. Double couplings are run on an Applied Biosystems ABI430A peptide synthesizer. The following side-chain protected amino acids (2 mmole cartridges of Boc amino acids) are obtained from Midwest Biotech (Fishers, IN) and are used in the synthesis:
Arg-Tosyl (TOS), Asp-δ-cyclohexyl ester(CHXL), Glu-δ-cycohexyl ester (CHXL), His-benzyloxymethyl(BOM), Lys-2-chlorobenzyloxycarbonyl (2Cl-Z), Ser-O-benzyl ether (OBzl), Thr-O-benzyl ether (OBzl), Trp-formyl (CHO) and Tyr-2-bromobenzyloxycarbonyl (2Br-Z) and Boc Gly PAM resin. Trifluoroacetic acid (TFA), di-isopropylethylamine (DIEA), 0.5 M hydroxybenzotriazole (HOBt) in DMF and 0.5 M dicyclohexylcarbodiimide (DCC) in dichloromethane are purchased from PE-Applied Biosystems (Foster City, CA). Dimethylformamide (DMF-Burdick and Jackson) and dichloromethane (DCM-Mallinkrodt) is purchased from Mays Chemical Co. (Indianapolis, IN).

Standard double couplings are run using either symmetric anhydride or HOBt esters, both formed using DCC. At the completion of the syntheses, the N-terminal Boc group is removed and the peptidyl resins are treated with 20% piperidine in DMF to deformylate the Trp side chain if Trp is present in the sequence. For the N-terminal acylation, four-fold excess of symmetric anhydride of the corresponding acid is added onto the peptide resin. The symmetric anhydride is prepared by diisopropylcarbodiimde (DIC) activation in DCM. The reaction is allowed to proceed for 4 hours and monitored by ninhydrin test. After washing with DCM, the resins are transferred to a TEFLON reaction vessel and are dried *in vacuo*.

Cleavages are done by attaching the reaction vessels to a HF (hydrofluoric acid) apparatus (Penninsula Laboratories). 1 mL m-cresol per gram/resin is added and 10 mL HF (purchased from AGA, Indianapolis, IN) is condensed into the pre-cooled vessel. 1 mL DMS per gram resin is added when methionine is present. The reactions are stirred one hour in an ice bath. The HF is removed *in vacuo*. The residues are suspended in ethyl ether. The solids are filtered and are washed with ether. Each peptide is extracted into aqueous acetic acid and either is freeze dried or is loaded directly onto a reverse-phase column.

Purifications are run on a 2.2 x 25cm VYDAC C18 column in buffer A (0.1 % Trifluoroacteic acid in water, B: 0.1% TFA in acetonitrile). A gradient of 20% to 90% B is run on an HPLC (Waters) over 120 minutes at 10 mL/minute while monitoring the UV at 280 nm (4.0 A) and collecting one minute fractions. Appropriate fractions are combined, frozen and lyophilized. Dried products are analyzed by HPLC (0.46 x 15 cm METASIL AQ C18) and MALDI mass spectrometry.

### Example 2

### Preparation of the Selective VPAC2 Receptor Peptide Agonists by Solid Phase FMoc Chemistry:

Approximately 114 mg (50 mMole) FMOC Ser(tBu) WANG resin (purchased from GlycoPep, Chicago, IL) is placed in each reaction vessel. The synthesis is conducted on a Rainin Symphony Peptide Synthesizer. Analogs with a C-terminal amide are prepared using 75 mg (50 µmole) Rink Amide AM resin (Rapp Polymere. Tuebingen, Germany).

The following FMOC amino acids are purchased from GlycoPep (Chicago, IL), and NovaBiochem (La Jolla, CA): Arg-2,2,4,6,7-pentamethyldihydrobenzofuran-5-sulfonyl (Pbf), Asn-trityl (Trt), Asp-β-t-Butyl ester (tBu), Glu-δ-t-butyl ester (tBu), Gln-trityl (Trt), His-trityl (Trt), Lys-t-butyloxycarbonyl (Boc), Ser-t-butyl ether (OtBu), Thr-t-butyl ether (OtBu), Trp-t-butyloxycarbonyl (Boc), Tyr-t-butyl ether (OtBu).

Solvents dimethylformamide (DMF-Burdick and Jackson), N-methyl pyrrolidone (NMP-Burdick and Jackson), dichloromethane (DCM-Mallinkrodt) are purchased from Mays Chemical Co. (Indianapolis, IN).

Hydroxybenzotrizole (HOBt), di-isopropylcarbodiimde (DIC), di-isopropylethylamine (DIEA), and piperidine (Pip) are purchased from Aldrich Chemical Co (Milwaukee, WI).

All amino acids are dissolved in 0.3 M in DMF. Three hour DIC/HOBt activated couplings are run after 20 minutes deprotection using 20% Pip/DMF. Each resin is washed with DMF after deprotections and couplings. After the last coupling and deprotection, the peptidyl resins are washed with DCM and are dried *in vacuo* in the reaction vessel. For the N-terminal acylation, four-fold excess of symmetric anhydride of the corresponding acid is added onto the peptide resin. The symmetric anhydride is prepared by diisopropylcarbodiimde (DIC) activation in DCM. The reaction is allowed to proceed for 4 hours and monitored by ninhydrin test. The peptide resin is then washed with DCM and dried *in vacuo.*

The cleavage reaction is mixed for 2 hours with a cleavage cocktail consisting of 0.2 mL thioanisole, 0.2 mL methanol, 0.4 mL triisopropylsilane, per 10 mL trifluoroacetic acid (TFA), all purchased from Aldrich Chemical Co., Milwaukee, WI. If Cys is present in the sequence, 2% of ethanedithiol is added. The TFA filtrates are added to 40 mL ethyl ether. The precipitants are centrifuged 2 minutes at 2000 rpm. The supernatants are decanted. The pellets are resuspended in 40 mL ether, re-centrifuged, re-decanted, dried under nitrogen and then *in vacuo*.

0.3-0.6 mg of each product is dissolved in 1 mL 0.1% TFA/acetonitrile(ACN), with 20 µL being analyzed on HPLC [0.46 x 15cm METASIL AQ C18, 1mL/min, 45C°, 214 nM (0.2A), A=0.1%TFA, B=0.1%TFA/50%ACN. Gradient = 50% B to 90% B over 30 minutes].

Purifications are run on a 2.2 x 25 cm VYDAC C18 column in buffer A (0.1% trifluoroacteic acid in water, B: 0.1% TFA in acetonitrile). A gradient of 20% to 90% B is run on an HPLC (Waters) over 120 minutes at 10 mL/minute while monitoring the UV at 280 nm (4.0A) and collecting 1 minute fractions. Appropriate fractions are combined, frozen and lyophilized. Dried products are analyzed by HPLC (0.46 x 15 cm METASIL AQ C18) and MALDI mass spectrometry.

### Example 3 In vitro potency:

*DiscoveRx:* A CHO-S cell line stably expressing human VPAC2 receptor in a 96-well microtiter plate is seeded with 50,000 cells/well the day before the assay. The cells are allowed to attach for 24 hours in 200 µL culture medium. On the day of the experiment, the medium is removed. Also, the cells are washed twice. The cells are incubated in assay buffer plus IBMX for 15 minutes at room temperature. Afterwards, the stimuli are added and are dissolved in assay buffer. The stimuli are present for 30 minutes. Then, the assay buffer is gently removed. The cell lysis reagent of the DiscoveRx cAMP kit is added. Thereafter, the standard protocol for developing the cAMP signal as described by the manufacturer is used (DiscoveRx Inc., USA). EC₅₀ values for cAMP generation are calculated from the raw signal or are based on absolute cAMP levels as determined by a standard curve performed on each plate. In the case of VPAC1 and PAC1 receptors, CHO-PO cells are transiently transfected with human VPAC1 or PAC1 receptor DNA using commercially available transfection reagents (Lipofectamine from Invitrogen). The cells are seeded at a density of 10,000/well in a 96-well plate and are allowed to grow for 3 days in 200 mL culture medium. At day 3, the assay described above for the VPAC2 receptor cell line is performed.

Results for each agonist are the mean of two independent runs. VPAC1 and PAC1 results are only generated using the DiscoveRx assay. The typically tested concentrations of peptide are: 1000, 300, 100, 10, 1, 0.3, 0.1, 0.01, 0.001, 0.0001 and 0 nM.

*Alpha screen:* Cells are washed in the culture flask once with PBS. Then, the cells are rinsed with enzyme free dissociation buffer. The dissociated cells are removed. The cells are then spun down and washed in stimulation buffer. For each data point, 50,000 cells suspended in stimulation buffer are used. To this buffer, Alpha screen acceptor beads are added along with the stimuli. This mixture is incubated for 60 minutes. Lysis buffer and Alpha screen donor beads are added and are incubated for 60 to 120 minutes. The Alpha screen signal (indicative of intracellular cAMP levels) is read in a suitable instrument (e.g. AlphaQuest from Perkin-Elmer). Steps including Alpha screen donor and acceptor beads are performed in reduced light. The EC₅₀ for cAMP generation is calculated from the raw signal or is based on absolute cAMP levels as determined by a standard curve performed on each plate.

Results for each agonist are, at minimum, from two analyses performed in a single run. For some agonists, the results are the mean of more than one run. The tested peptide concentrations are: 10000, 1000, 100, 10, 3, 1, 0.1, 0.01, 0.003, 0.001, 0.0001 and 0.00001 nM. The activity (EC₅₀ (nM)) for the human VPAC2, VPAC1, and PAC1 receptors is reported in Table 1

**Table 1**

| Agonist # | Human VPAC2 Receptor: DiscoveRx¹ | Human VPAC2 Receptor: Alpha Screen² | Human VPAC1 Receptor: DiscoveRx¹ | Human PAC1 Receptor: DiscoveRx¹ |
|---|---|---|---|---|
| PACAP-27 | 0.84 | 2.33 | 0.05 | 0.06 |
| VIP (SEQ ID NO: 1) | 0.70 | 1.00 | 0.02 | 15.4 |
| VPAC1-P1 | 179.29 | | | |
| P100 | | 0.28 | | |
| P101 | | 0.21 | | |
| P102 | | 27.38 | | |
| P103 | | 0.12 | | |
| P104 | | 0.27 | | |
| P105 | | 8.33 | | |
| P106 | | 0.16 | | |
| P107 | | 0.12 | | |
| P108 | | 0.27 | | |
| P109 | | 0.22 | | |
| P110 | | 0.13 | | |
| P111 | | 0.34 | | |
| P112 | | 0.16 | | |
| P113 | | 0.26 | | |
| P114 | | 0.14 | | |
| P115 | | 0.20 | | |
| P116 | | 0.12 | | |
| P117 | | 0.20 | | |
| P119 | 0.34 | 0.42 | | |
| P120 | | 0.06 | | |
| P121 | | 0.10 | | |
| P122 | | 0.14 | | |
| P123 | | 0.12 | | |
| P124 | | 0.09 | | |
| P125 | | 0.14 | | |
| P127 | | 0.22 | | |
| P129 | | 0.49 | | |
| P 130 | | 0.77 | | |
| P131 | | 22.28 | | |
| P132 | | 3.10 | | |
| P133 | | 2.70 | | |
| P134 | | 5.47 | | |
| P135 | | 0.18 | | |
| P138 | | 0.45 | | |
| P139 | | 0.20 | | |
| P140 | | 0.07 | | |
| P141 | | 0.72 | | |
| P142 | | 0.15 | | |
| P143 | | 0.10 | | |
| P 144 | | 0.32 | | |
| P146 | | 0.17 | | |
| P147 | | 0.13 | | |
| P148 | | 0.10 | | |
| P149 | | 0.41 | | |
| P150 | | 0.18 | | |
| P151 | | 0.07 | | |
| P152 | | 0.17 | | |
| P153 | | 0.11 | | |
| P154 | | 0.18 | | |
| P155 | | 0.24 | | |
| P158 | | 0.11 | | |
| P159 | | 0.17 | | |
| P160 | | 13.16 | | |
| P161 | | 4.00 | | |
| P162 | | 11.07 | | |
| P163 | | 5.53 | | |
| P164 | | 0.11 | | |
| P165 | | 0.94 | | |
| P166 | | 0.15 | | |
| P167 | | 0.17 | | |
| P168 | | 0.27 | | |
| P169 | | 0.14 | | |
| P170 | | 0.18 | | |
| P171 | | 0.82 | | |
| P172 | | 0.29 | | |
| P173 | | 0.25 | | |
| P174 | | 0.26 | | |
| P175 | | 0.68 | | |
| P176 | | 0.28 | | |
| P 177 | | 0.21 | | |
| P179 | | 0.24 | | |
| P18 | 2.88 | 3.31³ | 0.31 | 435.0 |
| P180 | | 1.04 | | |
| P181 | | 0.13 | | |
| P 182 | | 0.29 | | |
| P183 | | 0.14 | | |
| P184 | | 0.22 | | |
| P185 | | 0.18 | | |
| P186 | | 0.15 | | |
| P187 | | 0.40 | | |
| P188 | | 0.32 | | |
| P189 | | 0.31 | | |
| P19 | 9.03 | | 2.9 | |
| P192 | | 0.18 | | |
| P193 | | 0.18 | | |
| P194 | | 0.13 | | |
| P 195 | | 0.25 | | |
| P 196 | | 0.23 | | |
| P197 | | 0.09 | | |
| P199 | | 29.65 | | |
| P20 | 2.13 | 2.77 | 2.0 | >25 |
| P203 | | 0.14 | | |
| P205 | | 1.14 | | |
| P207 | | 0.86 | | |
| P208 | | 50.15 | | |
| P209 | | 0.10 | | |
| P21 | 6.87 | 11.60 | 10.70 | >100 |
| P210 | | 0.91 | | |
| P211 | | 988.66 | | |
| P212 | | 0.12 | | |
| P213 | | 0.24 | | |
| P214 | | 0.16 | | |
| P215 | | 0.09 | | |
| P216 | | 0.11 | | |
| P22 | 1.18 | 2.23 | 0.9 | 73.5 |
| P220 | | 0.12 | | |
| P221 | | 1.98 | | |
| P222 | | 0.51 | | |
| P223 | | 5.82 | | |
| P224 | | 0.14 | | |
| P228 | | 3.48 | | |
| P229 | | 2.60 | | |
| P23 | 1.41 | 2.30 | 0.9 | 98.3 |
| P230 | | 0.30 | | |
| P231 | | 0.26 | | |
| P232 | | 1.94 | | |
| P233 | | 0.11 | | |
| P234 | | 0.16 | | |
| P235 | | 0.82 | | |
| P236 | | 0.12 | | |
| P24 | 5.78 | | 2.5 | >100 |
| P240 | | 0.51 | | |
| P241 | | 2.17 | | |
| P242 | | 0.80 | | |
| P243 | | 0.28 | | |
| P244 | | 2.03 | | |
| P249 | | 0.93 | | |
| P25 | 3.02 | | 2.7 | 136.8 |
| P250 | | 0.18 | | |
| P251 | | 0.92 | | |
| P252 | | 0.26 | | |
| P253 | | 0.46 | | |
| P258 | | 0.17 | | |
| P259 | | 1.08 | | |
| P26 | 4.81 | | 0.9 | 84.0 |
| P260 | | 0.83 | | |
| P261 | | 0.09 | | |
| P262 | | 0.24 | | |
| P263 | | 0.07 | | |
| P264 | | 0.10 | | |
| P265 | | 0.21 | | |
| P269 | | 1.02 | | |
| P27 | 27.65 | | 5.2 | >100 |
| P270 | | 0.13 | | |
| P271 | | 0.12 | | |
| P275 | | 0.32 | | |
| P279 | | 5.37 | | |
| P28 | 3.92 | | 2.6 | 53.6 |
| P284 | | 0.18 | | |
| P289 | | 0.16 | | |
| P290 | | 0.22 | | |
| P291 | | 0.36 | | |
| P292 | | 0.23 | | |
| P293 | | 1.89 | | |
| P294 | | 0.24 | | |
| P295 | | 0.39 | | |
| P296 | | 6.69 | | |
| P297 | | 0.36 | | |
| P298 | | 0.50 | | |
| P301 | | 0.89 | | |
| P302 | | 0.45 | | |
| P305 | | 0.17 | | |
| P307 | | 0.32 | | |
| P308 | | 0.26 | | |
| P31 | 0.11 | 0.13 | 6.0 | 115.8 |
| P314 | | 0.22 | | |
| P315 | | 0.19 | | |
| P316 | | 0.30 | | |
| P317 | | 0.36 | | |
| P318 | | 0.32 | | |
| P319 | | 0.26 | | |
| P320 | | 0.49 | | |
| P321 | | 0.38 | | |
| P322 | | 0.22 | | |
| P323 | | 0.23 | | |
| P324 | | 0.23 | | |
| P325 | | 0.24 | | |
| P326 | | 0.21 | | |
| P329 | | 0.31 | | |
| P33 | 0.86 | 0.73 | 4.7 | 111.5 |
| P333 | | 0.75 | | |
| P335 | | 0.22 | | |
| P338 | | 0.46 | | |
| P341 | | 0.38 | | |
| P342 | | 0.21 | | |
| P344 | | 0.13 | | |
| P345 | | 1.17 | | |
| P346 | | 0.23 | | |
| P349 | | 0.08 | | |
| P350 | | 0.13 | | |
| P351 | | 0.11 | | |
| P352 | | 0.14 | | |
| P353 | | 0.18 | | |
| P354 | | 0.14 | | |
| P355 | | 0.10 | | |
| P364 | | 0.11 | | |
| P365 | | 0.13 | | |
| P366 | | 2.60 | | |
| P43 | 5.37 | | 75.6 | 104.0 |
| P44 | 0.92 | | 5.5 | 36.6 |
| P45 | 11.64 | | 1.75 | 315.5 |
| P46 | 121.01 | | 57.5 | >1000 |
| P47 | 1.07 | 1.39 | 3.6 | 1362 |
| P48 | 9.55 | | 9.93 | 95.9 |
| P49 | 13.41 | | 22.8 | 44.2 |
| P50 | 19.25 | | 110.6 | |
| P51 | 91.09 | | 3.61 | >1000 |
| P52 | 140.05 | | 37.52 | >1000 |
| P53 | 50.47 | | 117.9 | >1000 |
| P54 | 32.78 | | 84.1 | >1000 |
| P55 | 15.32 | | 133.5 | >1000 |
| P56 | 108.54 | | 9.96 | 404.7 |
| P58 | 11.79 | | 44.1 | 35.6 |
| P6 | 2.56 | 3.19 | 5.0 | >40 |
| P61 | 2.14 | | 2.58 | 11.6 |
| P62 | 13.23 | | 17 | 137.2 |
| P63 | >1000 | | >1000 | 0.9 |
| P64 | >1000 | | 221.3 | 0.1 |
| P65 | >1000 | | >1000 | 0.7 |
| P66 | >1000 | | <1000 | 1.4 |
| P67 | | 94.14 | | |
| P68 | 20.33 | | 41 | 94.7 |
| P69 | 6.39 | | 8.48 | 103.6 |
| P70 | 4.89 | | 27.4 | 20.7 |
| P71 | 3.14 | | 0.2 | 14.6 |
| P72 | 1.79 | | 0.3 | 35.3 |
| P74 | 2.72 | | 0.4 | 145.3 |
| P75 | 3.44 | | 0.65 | 13.4 |
| P76 | 3.88 | | 0.8 | 15.9 |
| P82 | | 15.24 | | |
| P84 | | 185 | | |
| P85 | | 22.2 | | |
| P87 | | >10000 | | |
| P88 | >1000 | 1718.93 | | |
| P89 | 0.7 | 1.23 | | |
| P9 | 2.41 | | | |
| P92 | 8.2 | 10.18 | | |
| P94 | >1000 | 696 | | |
| P98 | | 0.15 | | |
| P99 | | 0.14 | | |

| | | | | |
|---|---|---|---|---|
| ¹EC₅₀ (nM); Mean of two independent runs ²EC₅₀ (nM); Single result from two analyses performed in a single run ³Mean of two separate results for the given assay ⁴NA = Not assayed (for all NA entries) | | | | |

### Example 4 Selectivity:

*Binding assays:* Membrane prepared from a stable VPAC2 cell line (see Example 3) or from cells transiently transfected with human VPAC1 or PAC1 are used. A filter binding assay is performed using 125I-labeled VIP for VPAC1 and VPAC2 and 125I-labeled PACAP-27 for PAC1 as the tracers.

For this assay, the solutions and equipment include:
Presoak solution: 0.5 % Polyethyleneamine in Aqua dest.
Buffer for flushing filter plates: 25 mM HEPES pH 7.4
Blocking buffer: 25 mM HEPES pH 7.4; 0.2 % protease free BSA
Assay buffer: 25 mM HEPES pH 7.4; 0.5 % protease free BSA
Dilution and assay plate: PS-Microplate, U form
Filtration Plate: Multiscreen FB Opaque Plate; 1.0 µM Type B Glasfiber filter

In order to prepare the filter plates, aspirate the presoak solution by vacuum filtration. Flush the plates twice with 200 µL flush buffer. Add 200 µL blocking buffer to the filter plate. The filter plate is then incubated with 200 µL presoak solution for 1 hour at room temperature.

Fill the assay plate with 25 µL assay buffer, 25 µL membranes (2.5 µg) suspended in assay buffer, 25 µL compound (agonist) in assay buffer, and 25 µL tracer (about 40000 cpm) in assay buffer. Incubate the filled plate for 1 hour with shaking.

Conduct the transfer from assay plate to filter plate. Aspirate the blocking buffer by vacuum filtration and wash two times with flush buffer. Transfer 90 µL from the assay plate to the filter plate. Aspirate the 90 µL transferred from assay plate and wash three times with 200 µL flush buffer. Remove the plastic support. Dry for 1 hour at 60 °C. Add 30 µL Microscint. Perform the count.

The selectivity (IC₅₀) for human VPAC2, VPAC1, and PAC1 is reported in Table 2.

**Table 2**

| Agonist # | Human VPAC2 Receptor Binding | Human VPAC1 Receptor Binding | Human PAC1 Receptor Binding |
|---|---|---|---|
| PACAP-27 | 2.76 | 3.63 | 9.1 |
| VIP (SEQ ID NO: 1) | 5.06 | 3.28 | >25000 |
| P100 | 0.40 | 321.05 | 186.7 |
| P101 | 0.18 | 93.57 | 274.3 |
| P102 | 15.42 | 183.51 | 55.0 |
| P103 | 0.08 | 228.28 | 333.1 |
| P104 | 0.68 | 301.08 | 117.2 |
| P105 | 5.41 | 263.0 | 127.1 |
| P106 | 0.22 | 117.79 | 1817.2 |
| P107 | 0.16 | 121.9 | 225.2 |
| P108 | 0.45 | 331.9 | 485.8 |
| P109 | 0.11 | 274.3 | 257.3 |
| P110 | 0.10 | 424.6 | 320.9 |
| P111 | 0.19 | 300.0 | 258.3 |
| P112 | 0.49 | 192.65 | 164.0 |
| P113 | 0.69 | 293.29 | 189.3³ |
| P114 | 0.56 | 170.85 | 188.1³ |
| P115 | 0.10 | 266.82 | 189.6 |
| P116 | 0.74 | 369.05 | 145.3 |
| P117 | 0.51 | 442.36 | 243.2 |
| P119 | 0.48 | >3000 | >25000 |
| P120 | 0.44 | | 577.0 |
| P121 | 0.19 | 169.3 | 252.8³ |
| P122 | 0.17 | 77.6 | 303.9³ |
| P123 | 0.26 | 166.0 | 265.4³ |
| P124 | 0.2 | 211.2 | 264.2³ |
| P125 | 0.19 | 178.9 | 299.7³ |
| P127 | 0.23 | 289.84 | 209.2 |
| P129 | 0.38 | 461.64 | 678.2 |
| P130 | 0.43 | 232.66 | 444.3 |
| P131 | 5.67 | 1372.89 | 631.5 |
| P132 | 1.51 | 616.90 | 1022.7 |
| P133 | 5.30 | 469.91 | 1938.0 |
| P134 | 2.13 | 832.58 | 479.1 |
| P135 | 0.43 | 251.24 | 747.8 |
| P138 | 1.01 | 5.28 | 1113.2 |
| P139 | 0.34 | 234.71 | >25000 |
| P140 | 1.94 | 1136.71 | 485.3 |
| P141 | 0.64 | 25.91 | 658.0 |
| P142 | 0.58 | 363.55 | 1191.4 |
| P143 | 0.50 | 605.08 | 1082.0 |
| P144 | 0.57 | 4.43 | 1183.7 |
| P146 | 0.52 | 400.85 | 1540.8 |
| P147 | 0.24 | 106.79 | 464.0 |
| P148 | 0.77 | 247.37 | 1003.5 |
| P149 | 0.61 | 212.32 | 773.5 |
| P150 | 0.25 | 444.11 | 916.2 |
| P151 | 0.72 | 203.65 | 720.4 |
| P152 | 0.37 | 514.09 | 671.6 |
| P153 | 0.70 | 180.58 | 1127.1 |
| P154 | 0.31 | 140.90 | 1960.1 |
| P155 | 0.71 | 231.22 | 148.5 |
| P158 | 0.21 | 25.71 | |
| P159 | 0.17 | 131.23 | |
| P160 | 4.77 | 50.31 | 262.0 |
| P161 | 6.61 | 118.51 | 4259.0 |
| P162 | 3.72 | 5.14 | 186.1 |
| P163 | 2.00 | 35.68 | 237.9 |
| P164 | 2.46 | >3000 | 9368.8 |
| P165 | 18.38 | 486.02 | 2862.2 |
| P166 | 0.46 | 248.03 | 126.8 |
| P167 | 1.14 | 1153.0 | 1010.0 |
| P168 | 0.21 | 233.64 | 537.7 |
| P169 | 0.26 | 240.0 | 482.0 |
| P170 | 0.34 | 275.20 | 199.8 |
| P171 | 5.81 | 174.32 | 335.1 |
| P172 | 3.58 | 411.67 | 500.3 |
| P173 | 0.55 | 1787.03 | 755.6 |
| P174 | 55.07 | 302.47 | 620.8 |
| P175 | 13.31 | >3000 | >25000 |
| P176 | 6.88 | 93.05 | 226.8 |
| P177 | 0.70 | 127.68 | 276.0 |
| P179 | 0.42 | 390.05 | |
| P180 | 5.63 | >3000 | 589.3 |
| P181 | 0.29 | 325.19 | 521.5 |
| P182 | 0.17 | 205.03 | 428.8 |
| P183 | 0.26 | 423.64 | 505.5 |
| P184 | 0.23 | 324.43 | 338.9 |
| P185 | 0.41 | 358.01 | |
| P186 | 0.87 | 835.09 | 318.2 |
| P187 | 0.17 | 134.34 | 295.8 |
| P188 | 0.29 | 6.19 | 700.4 |
| P189 | 0.20 | 6.87 | 303.4 |
| P191 | 60.61 | 1277.23 | >25000 |
| P192 | 0.53 | 1432.29 | 14311.8 |
| P193 | 0.37 | 249.93 | 541.7 |
| P194 | 0.28 | 172.86 | 625.5 |
| P195 | 2.34 | >3000 | 139.3 |
| P196 | 0.85 | 453.46 | >25000 |
| P197 | 0.31 | 446.72 | |
| P199 | 8.26 | 429.11 | 244.3 |
| P20 | 2.50³ | 98.7³ | |
| P203 | 0.18 | | |
| P207 | 0.81 | 282.27 | |
| P209 | 0.20 | 130.14 | 342.7 |
| P21 | 4.94³ | 384.7³ | |
| P210 | 0.68 | 395.02 | |
| P212 | 0.19 | 197.91 | |
| P213 | 0.27 | 32.25 | |
| P214 | 0.19 | 14.51 | |
| P22 | 0.87³ | 123.0³ | |
| P220 | 0.25 | 5.86 | 848.8 |
| P222 | 1.05 | 176.98 | 465.7 |
| P224 | 0.35 | 207.86 | 209.7 |
| P23 | 0.92³ | 149.9³ | |
| P230 | 0.75 | 660.65 | 462.6 |
| P231 | 0.55 | 457.81 | 300.0 |
| P233 | 0.31 | 323.46 | 319.9 |
| P234 | 0.33 | 640.85 | 248.3 |
| P235 | 1.47 | 1335.57 | 1113.1 |
| P236 | 0.37 | 588.91 | 225.2 |
| P24 | 1.97³ | 198.4³ | |
| P240 | 6.08 | >3000 | >25000 |
| P241 | 18.09 | >3000 | >25000 |
| P242 | 6.78 | >3000 | >25000 |
| P243 | 5.51 | >3000 | >25000 |
| P244 | 26.86 | >3000 | >25000 |
| P249 | 6.59 | >3000 | >25000 |
| P25 | 1.65³ | 233.4³ | |
| P250 | 0.63 | 854.93 | >25000 |
| P251 | 2.33 | >3000 | >25000 |
| P252 | 0.98 | 1180.24 | >25000 |
| P253 | 1.78 | 2162.63 | >25000 |
| P258 | 0.63 | >3000 | >25000 |
| P259 | 13.21 | 3462.0 | >3000 |
| P260 | 4.79 | 3318.0 | >3000 |
| P261 | 0.2 | 1004.0 | >25000 |
| P262 | 0.61 | >3000 | >25000 |
| P263 | 0.38 | 2682.0 | 636 |
| P264 | 0.88 | >3000 | >25000 |
| P265 | 0.68 | >3000 | >25000 |
| P269 | 10.09 | >3000 | >25000 |
| P270 | 0.15 | 110.06 | 436.2 |
| P271 | 0.14 | 67.57 | 352.3 |
| P275 | 0.38 | 557.68 | 629.8 |
| P284 | 0.38 | >3000 | >25000 |
| P289 | 0.38 | 259.58 | 323.9 |
| P290 | 0.27 | 220.62 | 209.1 |
| P291 | 0.45 | >3000 | >25000 |
| P292 | 0.3 | >3000 | >25000 |
| P293 | 16.71 | >3000 | >25000 |
| P294 | 0.45 | >3000 | >25000 |
| P295 | 0.75 | >3000 | >25000 |
| P296 | >100 | >3000 | >25000 |
| P297 | 1.95 | >3000 | >25000 |
| P298 | 8.20 | >3000 | >25000 |
| P301 | 7.27 | >3000 | >25000 |
| P302 | 1.62 | >3000 | >25000 |
| P305 | 0.17 | 268.13 | 171.2 |
| P307 | 0.28 | 274.09 | 293.4 |
| P308 | 0.34 | 261.59 | |
| P31 | 0.23 | 145.13 | 405.7 |
| P314 | 0.57 | >3000 | >25000 |
| P315 | 0.29 | >3000 | |
| P316 | 0.38 | >3000 | |
| P317 | 1.23 | >3000 | >25000 |
| P318 | 0.63 | >3000 | >25000 |
| P319 | 0.69 | >3000 | >25000 |
| P320 | 1.20 | >3000 | |
| P321 | 0.29 | 429.44 | |
| P322 | 0.40 | >3000 | >25000 |
| P323 | 0.32 | >3000 | |
| P324 | 0.43 | >3000 | |
| P325 | 0.40 | >3000 | >25000 |
| P326 | 0.33 | >3000 | >25000 |
| P329 | 1.41 | >3000 | >25000 |
| P33 | 2.31³ | 120.0³ | |
| P333 | 9.71 | >3000 | |
| P335 | 0.39 | >3000 | |
| P338 | 0.76 | >3000 | |
| P341 | 2.39 | >3000 | |
| P342 | 0.35 | >3000 | |
| P344 | 0.25 | >3000 | |
| P345 | 19.26 | >3000 | |
| P346 | 0.25 | >3000 | |
| P352 | 0.60 | >3000 | |
| P47 | 0.84 | 123.71 | |
| P6 | 2.58³ | 194.8³ | 334.4 |
| P71 | 1.52 | 73.07 | |
| P72 | 1.52 | 158.95 | |
| P82 | 1.9 | 195.0 | |
| P84 | 20 | 469.0 | |
| P85 | 1.7 | 226.0 | |
| P87 | 1106 | 636.0 | |
| P88 | 93.9 | 979.0 | |
| P89 | 0.15 | 162.0 | |
| P92 | 0.86 | 74.6 | |
| P94 | 71.5 | 271.0 | |
| P98 | 0.17 | 69.0 | 96.3 |
| P99 | 1.72 | 394.45 | 2056.7 |

| | | | |
|---|---|---|---|
| ¹No affinity for the human PAC 1 receptor ²NA = Not assayed (for all NA entries) ³Mean of separate results for the given assay | | | |

### Comparison of the interaction of VIP, P31, P104, and P119 with the recombinant rat VPAC1, VPAC2 and PAC1 receptors expressed in CHO cells:

The peptide samples are stored frozen and thawed prior to the assay. Reference compounds (Eg. VIP and the tracers) are not stored fro zen. All peptide sample and reference compound dilutions are performed in PBS. Peptides solutions are kept in the cold room for four days. Stock solutions are stored at -80°C. New dilution curves are prepared every week.

All studies are performed on crude membranes prepared from three different cell cultures expressing the different recombinant receptors, using methodology that is known in the literature. Duplicate values are obtained for each assay.

The selectivity of the VPAC2 receptor peptide agonists of the present invention are tested on the rat VPAC1 and VPAC2 receptors recombinantly expressed in CHO cells. The compound of the invention were evaluated in receptor binding & adenylate cyclase activation assays.

Competion binding curves from 10⁻¹¹ to 10⁻⁵ M (two concentrations per log) of unlabelled peptide using ¹²⁵I-VIP (VPAC1-R) and ¹²⁵I-RO 25-1553 (VPAC2-R) as tracers; incubations performed at 25°C for 30 minutes. In each series of assays, unlabelled VIP and RO 25-1553 are used as standards. Each assay is done in duplicate and performed on two different membrane preparations.

**Table 3: Binding data are expressed as the IC₅₀ values of tracer inhibition. A different reference compound is used as the tracer for the VPAC1 receptor and for the VPAC2 receptor. The last column represents the ratio between the IC₅₀ values on VPAC1 and VPAC2 receptor and is, thus, an index of selectivity for a receptor subtype. VIP has a threefold preference for the VPAC1 receptor whereas all the other molecules tested indicated a clear preference for the VPAC2 receptor. The values are given with the standard error.**

| **Peptide** | **RAT VPAC1** | **RAT VPAC2** | **VPAC1/VPAC2** | |
|---|---|---|---|---|
| | IC50 ( log M) | IC50 (log M) | | N |
| VIP | 8.526 ± 0.190 | 8.108 ± 0.200 | 0.38 | 3 |
| P 31 | 6.678 ± 0.089 | 9.415 ± 0.090 | 546 | 3 |
| P 104 | 5.754 ± 0.236 | 8.744 ± 0.101 | 977 | 3 |
| P 119 | 7.534 ± 0.115 | 9.209 ± 0.040 | 47 | 3 |
| VIP, 2^{nd} run | 9.26 ± 0.40 | 8.54 ± 0.062 | 0.19 | 2 |
| P 261 | 6.89 ± 0.04 | 9.45 ± 0.16 | 363 | 3 |
| P 292 | 6.69 ± 0.22 | 9.12 ± 0.04 | 269 | 3 |

| | | | | |
|---|---|---|---|---|
| II Adenylate cyclase activation) | | | | |

Dose-effect curves of adenylate cyclase activation were generated using the VPAC2 receptor peptide agonists (10⁻¹¹ to 10⁻⁶ M, two concentrations per log) of the present invention.

Adenylate cyclase activity was determined by the procedure of Salomon et al. (1974), A highly sensitive adenylate cyclase assay. Analytical Biochemistry 58 (1974. Membrane proteins (3-15 g) are incubated in a total volume of 60 l containing 0.5 mM [³²P]-ATP, 10 M GTP, 5 mM MgCl₂, 0.5 mM EGTA, 1 mM cAMP, 1 mM theophylline, 10 mM phospho(enol)pyruvate, 30 g/ml pyruvate kinase and 30 mM Tris-HCl at a final pH of 7.8. The reaction is initiated by membrane addition and is terminated after 15 min incubation at 37 °C by addition of 0.5 ml of 0.5 % sodium dodecyl-sulfate solution containing 0.5 mM ATP, 0.5 mM cAMP and 20,000g [³H]-cAMP. cAMP was separated from ATP by two successive chromatographies on Dowex 50Wx8 and neutral alumina.

**Table 4: Functional data are obtained by measuring adenylate cyclase activation on membrane preparations expressing the rat VPAC1 and VPAC2 receptors. The values are provided as the EC₅₀ result (the ratio between the EC₅₀ and the maximal increase in cyclic AMP production (over basal) per minute and per mg protein). The values are given with the standard error.**

| **Peptide** | **VPAC1** | **VPAC2** | **VPAC1/VPAC2** | **VPAC1** | **VPAC2** |
|---|---|---|---|---|---|
| | **EC₅₀ (log M)** | **EC₅₀ (log M)** | | **cAMP max** | **cAMP max** |
| VIP | 8.831 ± 0.140 | 8.102 ± 0.080 | 0.19 | 224 ± 18 | 152 ± 15 |
| P 31 | 7.599 ± 0.091 | 9.840 ± 0.083 | 174 | 200 ± 13 | 134 ± 12 |
| P 104 | 6.731 ± 0.187 | 8.996 ± 0.098 | 184 | 195 ± 18 | 129 ± 19 |
| P 119 | 8.538 ± 0.317 | 10.040 ± 0.229 | 32 | 207 ± 13 | 133 ± 18 |
| VIP, 2^{nd} run | 9.23 ± 0.252 | 8.456 ± 0.178 | 0.17 | 135 | 80.7 |
| P 261 | 7.90 ± 0.43 | 10.20 ± 0.35 | 200 | 126 | 78.5 |
| P 292 | 7.94 ± 0.39 | 9.94 ± 0.48 | 100 | 124 | 79.1 |

VIP, PACAP, P31, P104 and P119 were also evaluated in binding and adenylate cyclase assays. For VIP, P31, P104 and P119 binding could not be reliably determined due to incomplete tracer displacement at the maximal concentration tested (10 µM peptide). In the adenylate cyclase assay the potency ratio compared to PACAP -27 was >200 for VIP, P31 and P119 and >1000 for P104.

**Table 5: In vitro potency using DiscoveRx (See Example 3). CHO -PO cells are transiently transfected with rat VPAC 1 or VPAC 2 receptor DNA. The activity (EC ₅₀ (nm)) for these receptors is reported in the table below.**

| Agonist # | Rat VPAC 2 Receptor DiscoveRx | Rat VPAC 1 Receptor DiscoveRx |
|---|---|---|
| P31 | | 0.44 |
| P89 | | 0.02 |
| PACAP-27 | | 0.07 |
| VIP | 0.50 | 0.02 |
| P 104 | 0.51 | 6.75 |
| P115 | | 0.95 |
| P119 | | 0.07 |
| P120 | | 0.42 |
| P121 | | 0.66 |
| P135 | 0.05 | 0.02 |
| P 140 | | 5.81 |
| P143 | | 3.81 |
| P150 | 0.10 | |
| P154 | | 0.85 |
| P167 | 0.24 | 6.88 |
| P168 | 0.05 | |
| P169 | 0.07 | 1.32 |
| P170 | 0.15 | |
| P172 | 0.11 | |
| P177 | 0.10 | |
| P222 | 34.92 | 43.89 |
| P240 | 0.36 | 24.54 |
| P241 | | 61.06 |
| P242 | 0.46 | 72.21 |
| P243 | 0.79 | 156.85 |
| P244 | 1.85 | 127.40 |
| P251 | | 212.12 |
| P261 | 0.04 | 0.35 |
| P264 | 0.10 | 4.01 |
| P270 | | 0.67 |
| P284 | 0.07 | 0.44 |
| P291 | 0.06 | 1.12 |
| P292 | 0.07 | 1.91 |
| P305 | 0.04 | 3.71 |
| P308 | 0.09 | 9.74 |
| P314 | 0.12 | 29.25 |
| P315 | 0.04 | 1.18 |
| P318 | 0.09 | 9.96 |
| P321 | 0.04 | 0.11 |
| P322 | 0.04 | 0.50 |
| P323 | 0.07 | 4.41 |
| P325 | 0.04 | 1.46 |
| P326 | 0.09 | 4.53 |
| P329 | 0.15 | 4.87 |
| P333 | 0.13 | 1.37 |
| P335 | 0.05 | 0.94 |
| P338 | 0.07 | 5.16 |
| P342 | 0.06 | 2.39 |
| P344 | 0.06 | 1.56 |
| P346 | 0.06 | 3.02 |
| P352 | 0.09 | 4.35 |
| P356 | NA | NA |

### Example 5 In vivo assays:

*Intravenous glucose tolerance test (IVGTT):* Normal Wistar rats are fasted overnight and are anesthetized prior to the experiment. A blood sampling catheter is inserted into the rats. The compound is given in the jugular vein. Blood samples are taken from the carotid artery. A blood sample is drawn immediately prior to the injection of glucose along with the compound. After the initial blood sample, glucose mixed with compound is injected intravenously (i.v.). A glucose challenge of 0.5 g/kg body weight is given, injecting a total of 1.5 mL vehicle with glucose and agonist per kg body weight. The peptide concentration vary to produce the desired dose in µg/kg. Blood samples are drawn at 2, 4, 6 and 10 minutes after giving glucose. The control group of animals receives the same vehicle along with glucose, but with no compound added. In some instances, a 30 minute post-glucose blood sample is drawn. Aprotinin is added to the blood sample (250 kIU/ml blood). The serum is then analyzed for glucose and insulin using standard methodologies.

The assay uses a formulated and calibrated peptide stock in PBS. Normally, this stock is a prediluted 100 µM stock. However, a more concentrated stock with approximately 1 mg agonist per mL is used. The specific concentration is always known. Variability in the maximal response is mostly due to variability in the vehicle dose.

Protocol details are as follows:

| | |
|---|---|
| SPECIES/STRAIN/WEIGHT | Rat/Wistar Unilever/approximately 275 - 300 g |
| TREATMENT DURATION | Single dose |
| DOSE VOLUME/ROUTE | 1.5 mL/kg/iv |
| VEHICLE | 8% PEG300, 0.1% BSA in water |
| FOOD/WATER REGIMEN | Rats are fasted overnight prior to surgery. |
| LIVE-PHASE PARAMETERS | Animals are sacrificed at the end of the test. |
| IVGTT: Performed on rats (with two catheters, jugular vein and carotid | Glucose IV bolus: 500 mg/kg as 10% solution (5 mL/kg) at time = 0. |
| artery) of each group, under | Compound iv: Just after glucose. |
| pentobarbital anesthesia. | Blood samplings (300 µL from carotid artery; EDTA as anticoagulant; aprotinin and PMSF as antiproteolytics; kept on ice): 0, 2, 4, 6, and 10 minutes. |
| | Parameter determined: Insulin. |
| TOXICOKINETICS | Plasma samples remaining after insulin measurements are kept at -20°C and are sent to Hamburg for determination of compound levels. |
| NUMBER OF SAMPLES | 150 |

**Table 6**

| Peptide | % increase AUC: Dose = 0.1 µg/kg | % increase AUC: Dose = 0.5 µg/kg | % increase AUC: Dose = 10 µg/kg | IVGTT (ED50; µg/kg) |
|---|---|---|---|---|
| P31 | NA | 250 | NA | NA |
| P44 | 19 | NA | 194 | NA |
| P89 | NA | 110 | 280 | NA |
| P104 | NA | 142, 205² | 198, 370² | 0.09 |
| P119 | NA | 118 | 240 | 0.3 |
| P261 | NA | 136 | 320 | NA |
| P264 | NA | 10 | 95 | NA |
| P292 | NA | 292 | 370 | NA |

| | | | | |
|---|---|---|---|---|
| ¹NA = Not assayed (for all NA entries) ²Analysis from two independent experiments. AUC = Area under curve | | | | |

*Delayed IVGTT:* Perform IVGTT as described above, making the following changes. After the initial blood sample, compound or vehicle is injected i.v. Glucose is injected i.v. 30 minutes later in a separate injection. Blood samples are taken immediately prior to administration of the compound, at 15 minutes after administration of the compound, and at 30 minutes after administration of the compound. The sample at 30 minutes after administration of the compound is taken immediately prior to glucose administration. Blood samples are drawn 2, 4, 6, 10, and 30 minutes after giving glucose (i.e. 32, 34, 36, 40 and 60 minutes after compound administration). The blood samples at 15 and 60 minutes are not essential to the study and not always taken. Aprotinin is added to the blood sample (250 kIU/ml blood). The serum is then analyzed for glucose and insulin using standard methodologies.

The assay uses a formulated and calibrated peptide stock in PBS. Normally, this stock is a prediluted 100 µM stock. However, a more concentrated stock with approximately' 1 mg agonist per mL is used. The specific concentration is always known.

**Table 7**

| Peptide | Dose (µg/kg) | Route | Effects on insulin | Effects on glucose | Exposure |
|---|---|---|---|---|---|
| P31 (2212924) | 10 | iv | + 91 % AUC₍₀₋ 10min) | None | Not measured |

### Oral Glucose Tolerance Test (OGTT):

The effect of a selective VPAC2 receptor peptide agonist (P31) on plasma insulin and glucose is evaluated during OGTT in conscious Wistar rats. The maximal dose of agonist is 10 µg/kg. Since the peptide is given intravenously and has a very short half-life, a delay between glucose and compound administrations is applied.

Protocol details are as follows:

| | | | | | |
|---|---|---|---|---|---|
| SPECIES/STRAIN/ WEIGHT | Rat/Wistar Unilever/approximately 275 - 300 g | | | | |
| TREATMENT DURATION | Single dose | | | | |
| GROUP/COMPOUND /DOSE/NUMBER/ SEX | Group | Compound | Dose (µg/kg) | Injection Time (min) | Number/Sex |
| | 1 | vehicle | 0 | 15 | 6M |
| | 2 | vehicle | 0 | 30 | 6M |
| | 5 | P31 | 10 | 15 | 6M |
| | 6 | P31 | 10 | 30 | 6M |
| DOSE VOLUME/ROUTE | 1.5 mL/kg/iv | | | | |
| VEHICLE | 8% PEG300, 0.1% | | BSA in water. | | |
| FOOD/WATER REGIMEN | Fasted overnight prior to the test. | | | | |
| LIVE-PHASE PARAMETERS | Animals will be trained for contention, gavage, and tail massage 2 days before the experiment. Animals will be sacrificed at the end of the test. Two animals of each group are tested on each day. | | | | |
| OGTT: Performed on conscious, non-cannulated rats of each group. | Glucose orally: 2.5 g/kg as 50% solution (5 mL/kg) at time = 0. | | | | |
| | Compound IV: 15 or 30 min after glucose. | | | | |
| | Blood samplings: (300 µL from tail tip; EDTA as anticoagulant; aprotinin and PMSF as antiproteolytics; kept on ice): Groups 1, 3, and 5: before glucose (time 0), at 15 min (just before compound), and at 20, 30, 45, 75, 105, and 135 | | | | |
| | minutes. | | | | |
| | Groups 2, 4, and 6: before glucose (time 0), at 30 min (just before compound), and at 35, 45, 60, 90, 120, and 150 minutes. | | | | |
| | Parameters determined: Insulin, glucose | | | | |
| TOXICOKINETICS | Plasma samples remaining after insulin measurements will be kept at -20°C and sent to Hamburg for determination of compound levels. | | | | |

Results are as follows:

**Table 8**

| Study | Peptide | Dose (µg/kg) | Route | Effects on insulin | Effects on glucose |
|---|---|---|---|---|---|
| OGTT (15 min), #1 | P31 (2212924) | 10 | iv | + 71% AUC₍₀₋ 75min) | -22% AUC₍₀₋ 135min) |
| OGTT (15 min), #2 | P31 (2212924) | 0.5 | iv | + 16% AUC₍₀₋ 75min) | -5% AUC₍₀₋₁₃₅ₘᵢₙ₎ |
| OGTT (15 min), #2 | P31 (2212924) | 10 | iv | + 17% AUC₍₀₋ 75min) | -2% AUC₍₀₋₁₃₅ₘᵢₙ₎ |
| OGTT (30 min) | P31 (2212924) | 10 | iv | + 85% AUC₍₀₋ 90min) | -1% AVC₍₀₋₁₅₀ₘᵢₙ₎ (NS) |

### Example 6 Rat Serum Stability Studies:

In order to determine the stability of VPAC2 receptor peptide agonists in rat serum, obtain CHO-VPAC2 cells clone #6 (96 well plates/50,000 cells/well and 1 day culture), PBS 1X (Gibco), the peptides for the analysis in a 100 µM stock solution, rat serum from a sacrificed normal Wistar rat, aprotinin, and a DiscoveRx assay kit. The rat serum is stored at 4°C until use and is used within two weeks.

On Day 0, prepare two 100 µL aliquots of 10 µM peptide in rat serum by adding 10 µL peptide stock to 90 µL rat serum for each aliquot. Add 250 kIU aprotinin / mL to one of these aliquots. Store the aliquot with aprotinin at 4°C. Store the aliquot without aprotinin at 37°C. Allow the aliquots to incubate for 18 hours.

On Day 1, after incubation of the aliquots prepared on day 0 for 18 hours, prepare an incubation buffer containing PBS + 1.3 mM CaCl₂, 1.2 mM MgCl₂, 2 mM glucose, and 0.25 mM IBMX. Prepare a plate with 11 serial 5X dilutions of peptide for the 4°C and 37°C aliquot for each peptide studied. Use 2000 nM as the maximal concentration if the peptide has an EC₅₀ above 1 nM and 1000 nM as maximal concentration if the peptide has an EC ₅₀ below 1 nM from the primary screen (see Example 3). Wash the plate(s) with cells twice in incubation buffer. Allow the plates to hold 50 µL incubation media per well for 15 minutes. Transfer 50 µL solution per well to the cells from the plate prepared with 11 serial 5X dilutions of peptide for the 4°C and 37°C aliquot for each peptide studied, usi ng the maximal concentrations that are indicated by the primary screen, in duplicate. This step dilutes the peptide concentration by a factor of two. Incubate in room temperature for 30 minutes. Remove the supernatant. Add 40 µL/well of the DiscoveRx a ntibody/extraction buffer. Incubate on the shaker (300 rpm) for 1 hour. Proceed as normal with the DiscoveRx kit. Include cAMP standards in column 12. Determine EC ₅₀ values from the cAMP assay data. The remaining amount of active peptide is estimated by the formula EC _{50, 4C}/EC_{50, 37C} for each condition.

**Table 9**

| Peptide | Rat Serum Stability (estimated purity in % after 18 hours) |
|---|---|
| P104 | 1.01 |
| P106 | 0.11 |
| P119 | 37.23 |
| P120 | 5 |
| P133 | <2.00 |
| P 138 | 0.48 |
| P 139 | 0.33 |
| P 142 | 0.51 |
| P146 | 0.53 |
| P147 | 0.07 |
| P148 | <0.10 |
| P149 | 0.29 |
| P150 | 0.21 |
| P151 | 0.35 |
| P152 | 0.06 |
| P153 | <0.16 |
| P155 | 0.25 |
| P158 | 0.32 |
| P159 | 0.15 |
| P160 | <4.25 |
| P163 | <1.25 |
| P165 | 7.28 |
| P167 | 14.98 |
| P168 | 10.47 |
| P169 | 0.40 |
| P170 | 0.31 |
| P171 | 1.78 |
| P172 | 5.95 |
| P173 | 1.04 |
| P174 | 1.91 |
| P175 | <0.11 |
| P176 | <0.14 |
| P177 | 0.49 |
| P179 | 1.66 |
| P180 | 1.14 |
| P181 | <0.53 |
| P182 | 0.15 |
| P183 | 0.18 |
| P184 | 0.29 |
| P185 | 0.27 |
| P186 | 0.25 |
| P193 | 0.25 |
| P194 | 1.11 |
| P197 | 5.68 |
| P203 | <0.09 |
| P205 | 35.02 |
| P207 | <0.29 |
| P209 | 0.13 |
| P210 | <0.91 |
| P212 | 0.21 |
| P213 | 0.04 |
| P214 | 0.07 |
| P215 | 0.08 |
| P216 | 0.06 |
| P220 | 0.08 |
| P221 | 0.87 |
| P222 | 0.23 |
| P223 | 1.52 |
| P228 | <5.00 |
| P229 | <5.00 |
| P230 . | 0.53 |
| P231 | 0.57 |
| P232 | <5.00 |
| P233 | 1.27 |
| P234 | 0.69 |
| P235 | 2.62 |
| P236 | 0.79 |
| P240 | 43.87 |
| P241 | 62.55 |
| P242 | 40.28 |
| P243 | 12.12 |
| P244 | 30.64 |
| P249 | 12.9 |
| P250 | 7.11 |
| P251 | 6.63 |
| P252 | 1.19 |
| P253 | 1.27 |
| P258 | 9.47 |
| P259 | 17.65 |
| P260 | 28.57 |
| P261 | 27.06 |
| P262 | 10.14 |
| P263 | 11.72 |
| P264 | 19.22 |
| P265 | 8.70 |
| P269 | 23.85 |
| P284 | 27.89 |
| P289 | 0.04 |
| P290 | 0.11 |
| P291 | 28.09 |
| P292 | 33.43 |
| P293 | 32.70 |
| P294 | 24.25 |
| P295 | 22.22 |
| P296 | 42.26 |
| P297 | 21.17 |
| P298 | 30.97 |
| P301 | 6.86 |
| P302 | 21.43 |
| P305 | 0.23 |
| P307' | 0.32 |
| P308 | 0.97 |
| P31 | 0.88 |
| P314 | 8.44 |
| P315 | 24.00 |
| P316 | 15.06 |
| P317 | 33.34 |
| P318 | 29.95 |
| P319 | 13.53 |
| P320 | 28.59 |
| P322 | 42.18 |
| P323 | 16.81 |
| P324 | 13.64 |
| P325 | 13.13 |
| P326 | 90.10 |
| P329 | 47.98 |
| P333 | 40.90 |
| P335 | 98.66 |
| P338 | 36.61 |
| P341 | 12.82 |
| P342 | 25.96 |
| P344 | 50.19 |
| P345 | 20.23 |
| P346 | 43.44 |
| P349 | 41.22 |
| P350 | 25.83 |
| P351 | 18.52 |
| P352 | 45.09 |
| P353 | 31.23 |
| P354 | 15.10 |
| P355 | 35.94 |
| P356 | 45.17 |
| P364 | 86.23 |
| P365 | 91.13 |
| P366 | 92.00 |
| P99 | 0.61 |

**Table 10**

| Peptide | Rat Serum Stability (Estimated purity in % after 72 hours) |
|---|---|
| P89 | <0.4 |
| P240 | 10.4 |
| P242 | 3.8 |
| P244 | 6.2 |
| P264 | 0.9 |
| P322 | 1.3 |
| P326 | 49.6 |
| P329 | 11.2 |
| P333 | 61.2 |
| P335 | 22.0 |
| P344 | 43.0 |
| P346 | 27.6 |
| P352 | 2.2 |
| P317 | 21.5 |

### Example 7 Pharmacokinetic Assay:

An analysis of active peptide levels in rat plasma is conducted after IV injection of 10 µg/kg of each peptide. An IVGTT with glucose is given immediately after T = 0 to 6 animals per condition. Samples are taken at 0, 2, 4, 6, and 10 minutes after injection.

For cell handling, Plate 50,000 cells / well and keep in culture over night. Wash cell twice in PBS and add 50 µl/well stimulation medium consisting of PBS + 1.2 MgC12, 1.3 CaCl2, 2 glucose and 0.5 IBMX. Incubate 15 minutes and add 50 µl/well of the plasma samples (see layout below). Incubate 30 minutes, remove the supernatant and proceed as normal with the DiscoveRx assay. Prepare plates in duplicate. Protease and peptidase inhibitor are present in all plasma samples.

**Table 11**

| Peptide | Exposure (10 µg/kg i.v., Cmax; nM) | Exposure (10 µg/kg; t1/2, min) | Exposure (10 µg/kg; clearance in µg/(min*mM*kg) | Exposure (10 µg/kg, Vdist in µg/(nM*kg) | Exposure (AUC last, nM*min) |
|---|---|---|---|---|---|
| P31 | 4.37 | 12 | 0.65 | 10 | 18.5 |
| P89 | 5.45 | 1.32 | 0.33 | 0.64 | 31.41 |
| P104 | 3.19 | 2.35 | 0.87 | 2.64 | 21.3 |
| P264 | 0.96 | 1.3 | 2.8 | 5.1 | 3.6 |
| P261 | 12.1 | 3.2 | 0.18 | 0.8 | 53.9 |
| P292 | 5.37 | 5.5 | 0.2 | 1.9 | 41.3 |

**Table 12**

| Peptide | Exposure (0.5 µg/kg i.v., Cmax; nM) | Exposure (0.5 µg/kg; tl/2, min) | Exposure (0.5 µg/kg; clearance in µg/(min*nM*kg) | Exposure (0.5 µg/kg, Vdist in µg/(nM*kg) | Exposure (AUC last, nM*min) |
|---|---|---|---|---|---|
| P292 | 0.12 | 15.0 | 0.4 | 9.5 | 1.0 |

### Example 8 DPP-IV HPLC Assays:

### Part 1: Formulation of selective VPAC2 receptor peptide agonists:

Approximately 2 mg of lyophilized peptide is weighed and dissolved in approximately 1.6 mL de-ionized water. If the peptide does not dissolve, the pH is adjusted with 1M NaOH to between pH 10.0 and 10.5. After incubation at room temperature for 30 minutes, 1/10^{th} of the original volume 10 x PBS is added. The pH is adjusted to between pH 7.2 and 7.6. The peptide solution is filtered through a 0.22 µm Millex-GV syringe filter (Millipore, Bedford MA, USA). The peptide concentration is determined through absorption at 280 mm. The peptide concentration is then adjusted to 100 µM. The peptides are frozen at -20°C for further use.

### Part 2: In vitro incubation of selective VPAC2 receptor peptide agonists with purified Dipeptidyl-peptidase IV (DPP-IV):

The stability of selective VPAC2 receptor peptide agonists against proteolysis by DPP-IV is determined using 100 µL of a 100 µM peptide solution in 1 x PBS. A 10 µL solution is removed and quenched with 40 µL of 0.1 % trifluoroacetic acid (TFA)/ 20% acetonitrile (ACN). This solution (20 µL) is analyzed by reversed-phase HPLC. The reversed-phase analysis consists of a Zorbax 300SB-C8 column (3.5 micron, 4.6x50mm, Alltech Associates, Inc., Deerfield IL, USA) running a 15-40%B gradient over 15 minutes at 60°C where A-buffer is 0.1%(v/v) TFA in water and B-buffer is 0.085%(v/v) TFA in ACN. The peak area is integrated. This peak area serves as an internal control as 100% intact peptide.

A 10 µL aliquot of a 1.12 mU/µL solution of DPP-IV (Sigma, St. Louis, LO, USA) is added to 90 µL of a 100 µM solution of peptide, resulting in a substrate concentration of 90 µM peptide. The reaction mixture is then stored at 37°C. At various time-points, 10 µL of solution is removed, quenched with 40 µL 0.1% TFA/ 20% ACN, and analyzed by reversed-phase HPLC as described above. The remaining full length peptide concentration (nM) at each timepoint, except time = 0, is calculated using following formula: $\frac{peak area \left[time x\right] * concentration \left[t⁢0\right]}{peak area \left[time 0\right] * 0.9}$

For the time = 0 timepoint, the concentration (nM) is calculated using the following formula: $\frac{peak area \left[time x\right] * initial substrate concentration \left[9 nM\right]}{peak area \left[time 0\right]}$

**Table 13: Concentration (nM) of Remaining Main Peak by RP-HPLC after Incubation of Peptide with Purified Porcine DPP-IV**

| Study Number | Sample | Time¹ = 0 | Time¹ = 2 | Time¹ = 6 | Time¹ = 24 |
|---|---|---|---|---|---|
| 1 | VPAC2-P31 | 9.0 | 6.7 | 5.6 | 3.9 |
| 1 | VPAC2-P44 | 9.0 | 6.5 | 3.5 | 0.8 |
| 1 | VPAC2-P47 | 9.0 | 7.9 | 6.1 | 2.8 |
| 2 | VPAC2-P44, 1 | 9.0 | 6.4 | 5.5 | 1.5 |
| 2 | VPAC2-P44,2 | 9.0 | 7.9 | 4.8 | 1.4 |
| 3 | VPAC2-P31 | 9.0 | 8.0 | 6.7 | 2.2 |
| 4 | VPAC2-P31 | 9.0 | 7.0 | 6.1 | 2.6 |
| 4 | VPAC2-P44 | 9.0 | 4.5 | 2.3 | 0.4 |
| 4 | VPAC2-P104 | 9.0 | 7.8 | 6.6 | 4.1 |
| 4 | VPAC2-P119 | 9.0 | 8.0 | 6.6 | 4.2 |

| | | | | | |
|---|---|---|---|---|---|
| ¹Time = Hours at 37°C | | | | | |

Other modifications of the present invention will be apparent to those skilled in the art without departing from the scope of the invention.

## Claims

1. A VPAC2 receptor peptide agonist comprising a sequence of the formula: wherein:
Xaa₁ is: His, dH, or is absent;
Xaa₂ is: dA, Ser, Val, Gly, Thr, Leu, dS, Pro, or Aib;
Xaa₃ is: Asp or Glu;
Xaa₄ is: Ala, Ile, Tyr, Phe, Val, Thr, Leu, Trp, Gly, dA, Aib, or NMeA;
Xaa₅ is: Val, Leu, Phe, Ile, Thr, Trp, Tyr, dV, Aib, or NMeV;
Xaa₆ is: Phe, Ile, Leu, Thr, Val, Trp, or Tyr,
Xaa₈ is: Asp, Glu, Ala, Lys, Leu, Arg, or Tyr;
Xaa₉ is: Asn, Gln, or Glu;
Xaa₁₀ is: Tyr, Trp, or Tyr(OMe);
Xaa₁₂ is: Arg, Lys, hR, Orn, Aib, Cit, or Ala;
Xaa₁₃ is: Leu, Phe, Glu, Ala, or Aib;
Xaa₁₄ is: Arg, Leu, Lys, Ala, hR, Orn, Phe, Gln, Aib, or Cit;
Xaa₁₅ is: Lys, Ala, Arg, Glu, Leu, hR, Orn, Phe, Gln, Aib, K(Ac), or Cit;
Xaa₁₆ is: Gln, Lys, Ala, hR, Orn, or Cit;
Xaa₁₇ is: Val, Ala, Leu, Ile, Met, Nle, Lys, or Aib;
Xaa₁₉ is: Ala, Gly, or Leu;
Xaa₂₀ is: Lys, Gln, hR, Arg, Ser, Orn, Ala, Aib, Trp, Thr, Leu, Ile, Phe, Tyr, Val, K(Ac), or Cit;
Xaa₂₁ is: Lys, Arg, Ala, Phe, Aib, Leu, Gln, Orn, hR, K(Ac) or Cit;
Xaa₂₂ is: Tyr, Trp, Phe, Thr, Leu, Ile, Val, Tyr(OMe), Ala, or Aib;
Xaa₂₃ is: Leu, Phe, Ile, Ala, Trp, Thr, Val, or Aib;
Xaa₂₄ is: Gln, or Asn;
Xaa₂₅ is: Ser, Asp, Phe, Ile, Leu, Thr, Val, Trp, Gln, Asn, Tyr, Aib, or Glu;
Xaa₂₆ is: Ile, Leu, Thr, Val, Trp, Tyr, Phe or Aib;
Xaa₂₇ is: Lys, hR, Arg, Gln, Orn, or dK;
Xaa₂₈ is: Asn, Gln, Lys, Arg, Aib, Orn, hR, Cit, Pro, or dK;
Xaa₂₉ is: Lys, Ser, Arg, Asn, hR, Orn, Cit, Aib or is absent;
Xaa₃₀ is: Arg, Lys, Ile, hR, Cit, Aib, Orn, or is absent;
Xaa₃₁ is: Tyr, His, Phe, or is absent; and
Xaa₃₂ is: Cys, or is absent;
provided that if Xaa₂₉, Xaa₃₀, or Xaa₃₁ is absent, the next amino acid present downstream is the next amino acid in the peptide agonist sequence,
and a C-terminal extension wherein the N-terminus of the C-terminal extension is linked to the C-terminus of the peptide of Formula 12 and wherein the C-terminal extension comprises an amino acid sequence of the formula:
Xaa₁-Xaa₂-Xaa₃-Xaa₄-Xaa₅-Xaa₆-Xaa₇-Xaa₈-Xaa₉-Xaa₁₀-Xaa₁₁ Formula 7 (SEQ ID NO:15)
wherein:
Xaa₁ is: Gly, Cys, or absent;
Xaa₂ is: Gly, Arg, or absent;
Xaa₃ is: Pro, Thr, or absent;
Xaa₄ is: Ser, or absent;
Xaa₅ is: Ser, or absent;
Xaa₆ is: Gly, or absent;
Xaa₇ is: Ala, or absent;
Xaa₈ is: Pro, or absent;
Xaa₉ is: Pro, or absent;
Xaa₁₀ is: Pro, or absent; and
Xaa₁₁ is: Ser, Cys, or absent;
provided that at least five of Xaa₁ to Xaa₁₁ of the C-terminal extension are present and provided that if Xaa₁, Xaa₂, Xaa₃, Xaa₄, Xaa₅, Xaa₆, Xaa₇, Xaa₈, Xaa₉, or Xaa₁₀ is absent, the next amino acid present downstream is the next amino acid in the C-terminal extension and wherein the C-terminal amino acid may be amidated.

2. The VPAC2 receptor peptide agonist according to claim 1 comprising a sequence of the formula: wherein:
Xaa₂ is: dA, Ser, Val, dS, or Aib;
Xaa₃ is: Asp or Glu;
Xaa₄ is: Ala, dA, or Aib;
Xaa₅ is: Val, Leu, dV, or Aib;
Xaa₈ is: Asp, Glu, or Ala;
Xaa₉ is: Asn, Gln, or Glu;
Xaa₁₂ is: Ala, Arg, Lys, hR, or Orn;
Xaa₁₄ is: Arg, Leu, Lys, Ala, hR, Orn, Phe, Gln, Aib, or Cit;
Xaa₁₅ is: Lys, Ala, Arg, Leu, Orn, Phe, Gln, Aib, or K(Ac);
Xaa₁₆ is: Gln, or Lys;
Xaa₁₇ is: Val, Ala, Leu, Ile, Met, Nle, or Lys;
Xaa₁₉ is: Ala, or Leu;
Xaa₂₀ is: Lys, Gln, hR, Arg, Ser, Ala, Aib, Trp, Thr, Leu, Ile, Phe, Tyr, Val, or K(Ac);
Xaa₂₁ is: Lys, Arg, Ala, Phe, Aib, Leu, Gln, K(Ac), or Orn;
Xaa₂₂ is: Tyr, Trp, Phe, Leu, Ile, or Val;
Xaa₂₄ is: Gln, or Asn;
Xaa₂₅ is: Ser, Phe, Ile, Leu, Thr, Val, Trp, Gln, Asn, Tyr, or Aib;
Xaa₂₆ is: Ile, Leu, Thr, Val, Trp, Tyr, Phe or Aib;
Xaa₂₇ is: Lys, hR, Arg, dK, or Orn;
Xaa₂₈ is: Asn, Gln, Lys, hR, Aib, Orn, dK, or Pro;
Xaa₂₉ is: Lys, Ser, Arg, hR, Orn, or is absent;
Xaa₃₀ is: Arg, Lys, or is absent; and
Xaa₃₁ is: Tyr, Phe, or is absent;
provided that if Xaa₂₉, or Xaa₃₀ is absent, the next amino acid present downstream is the next amino acid in the peptide agonist sequence,
and a C-terminal extension wherein the N-terminus of the C-terminal extension is linked to the C-terminus of the peptide of Formula 13 and wherein the C-terminal extension comprises an amino acid sequence of the formula:
Xaa₁-Xaa₂-Xaa₃-Xaa₄-Xaa₅-Xaa₆-Xaa₇-Xaa₈-Xaa₉-Xaa₁₀-Xaa₁₁ Formula 7 (SEQ ID NO:15)
wherein:
Xaa₁ is: Gly, Cys, or absent;
Xaa₂ is: Gly, Arg, or absent;
Xaa₃ is: Pro, Thr, or absent;
Xaa₄ is: Ser, or absent;
Xaa₅ is: Ser, or absent;
Xaa₆ is: Gly, or absent;
Xaa₇ is: Ala, or absent;
Xaa₈ is: Pro, or absent;
Xaa₉ is: Pro, or absent;
Xaa₁₀ is: Pro, or absent; and
Xaa₁₁ is: Ser, Cys, or absent;
provided that at least five of Xaa₁ to Xaa₁₁ of the C-terminal extension are present and provided that if Xaa₁, Xaa₂, Xaa₃, Xaa₄, Xaa₅, Xaa₆, Xaa₇, Xaa₈, Xaa₉, or Xaa₁₀ is absent, the next amino acid present downstream is the next amino acid in the C-terminal extension and wherein the C-terminal amino acid may be amidated.

3. The VPAC2 receptor peptide agonist according to any one of the preceding claims wherein the C-terminal extension is selected from:
| | |
|---|---|
| SEQ ID NO: 10 | GGPSSGAPPPS |
| SEQ ID NO: 11 | GGPSSGAPPPS-NH₂ |
| SEQ ID NO: 22 | GGPSSGAPPPC |
| SEQ ID NO: 23 | GGPSSGAPPPC-NH₂ |
| SEQ ID NO: 24 | GRPSSGAPPPS |
| SEQ ID NO: 25 | GRPSSGAPPPS-NH₂ |

4. The VPAC2 receptor peptide agonist according to any one of the preceding claims further comprising a N-terminal modification at the N-terminus of the peptide agonist wherein the N-terminal modification is selected from:
a) addition of D-histidine, isoleucine, methionine, or norleucine;
b) addition of a peptide comprising the sequence Ser-Trp-Cys-Glu-Pro-Gly-Trp-Cys-Arg wherein the Arg is linked to the N-terminus of the peptide agonist;
c) addition of C₁-C₁₆ alkyl optionally substituted with one or more substituents independently selected from aryl, C₁-C₆alkoxy, -NH₂, -OH, halogen and -CF₃;
d) addition of -C(O)R¹ wherein R¹ is a C₁-C₁₆ alkyl optionally substituted with one or more substituents independently selected from aryl, C₁-C₆alkoxy,-NH₂, -OH, halogen, -SH and -CF_{3;} a aryl or aryl C₁-C₄alkyl optionally substituted with one or more substituents independently selected from C₁-C₆ alkyl, C₂-C₆ alkenyl, C₂-C₆ alkynyl, C₁-C₆ alkoxy, -NH₂, -OH, halogen and - CF₃; -NR²R³ wherein R² and R³ are independently hydrogen, C₁-C₆ alkyl, aryl or aryl C₁-C₄ alkyl; or -OR⁴ wherein R⁴ is C₁-C₁₆ alkyl optionally substituted with one or more substituents independently selected from aryl, C₁-C₆ alkoxy, -NH₂, -OH, halogen and -CF₃, aryl or aryl C₁-C₄ alkyl optionally substituted with one or more substituents independently selected from C₁-C₆ alkyl, C₂-C₆ alkenyl, C₂-C₆ alkynyl, C₁-C₆ alkoxy, -NH₂, -OH, halogen and -CF₃;
e) addition of -SO₂R⁵ wherein R⁵ is aryl, aryl C₁-C₄ alkyl or C₁-C₁₆ alkyl;
f) formation of a succinimide group optionally substituted with C₁-C ₆ alkyl or - SR⁶, wherein R⁶ is hydrogen or C₁-C₆ alkyl; and
g) addition of methionine sulfoxide.

5. The VPAC2 receptor peptide agonist according to claim 4 wherein the N-terminal modification is the addition of a group selected from: acetyl, propionyl, butyryl, pentanoyl, hexanoyl, methionine, methionine sulfoxide, 3-phenylpropionyl, phenylacetyl, benzoyl, norleucine, D-histidine, isoleucine and 3-mercaptopropionyl.

6. The VPAC2 receptor peptide agonist according to claim 4 or claim 5 wherein the N-terminal modification is the addition of acetyl or hexanoyl.

7. The VPAC2 peptide receptor agonist according to claim 1 comprising an amino acid sequence selected from:
| **Agonist** # | **Sequence** |
|---|---|
| P6 | HSDAVFTDNYTRLRKQVAAKKYLQSIKNKRYGGPSSGAPPPS |
| P9 | HSDAVFTDNYTRLRKQVAAKKYLQSIKNKKGGPSSGAPPPS |
| P18 | HSDAVFTDNYTRLRKQVAAhRKYLQSIKNKRYGGPSSGAPPPS |
| P19 | HSDAVFTDNYTRLRKQVAAIKYLQSIKNKRYGGPSSGAPPPS |
| P20 | HSDAVFTDNYTRLRKQVAARKYLQSIKNKRYGGPSSGAPPPS |
| P21 | HSDAVFTDNYTRLRKQVAASKYLQSIKNKRYGGPSSGAPPPS |
| P22 | HSDAVFTDNYTRLRKQVAAKKYLQSIhRNKRYGGPSSGAPPPS |
| P23 | HSDAVFTDNYTRLRKQVAAKKYLQSIRNKRYGGPSSGAPPPS |
| P24 | HSDAVFTDNYTRLRKQVAAKKYLQSIKNhRRYGGPSSGAPPPS |
| P25 | HSDAVFTDNYTRLRKQVAAKKYLQSIKNRRYGGPSSGAPPPS |
| P26 | HSDAVFTDNYTRFRKQVAAKKYLQSIKNKRYGGPSSGAPPPS |
| P27 | HSDAVFTDNWTRLRKQVAAKKYLQSIKNKRYGGPSSGAPPPS |
| P28 | HSDAVFTDNYTRLRKQVAAKKYLQSIKNKRHGGPSSGAPPPS |
| P31 | C6-HSDAVFTDNYTRLRKQVAAKKYLQSIKNKRYGGPSSGAPPPS |
| P33 | Ac-HSDAVFTDNYTRLRKQVAAKKYLQSIKNKRYGGPSSGAPPPS |
| P43 | HGDAVFTDNYTRLRKQVAAKKYLQSIKNKRYGGPSSGAPPPS |
| P44 | HVDAVFTDNYTRLRKQVAAKKYLQSIKNKRYGGPSSGAPPPS |
| P45 | HTDAVFTDNYTRLRKQVAAKKYLQSIKNKRYGGPSSGAPPPS |
| P46 | HLDAVFTDNYTRLRKQVAAKKYLQSIKNKRYGGPSSGAPPPS |
| P47 | HdADAVFTDNYTRLRKQVAAKKYLQSIKNKRYGGPSSGAPPPS |
| P48 | HdSDAVFTDNYTRLRKQVAAKKYLQSIKNKRYGGPSSGAPPPS |
| P49 | HPDAVFTDNYTRLRKQVAAKKYLQSIKNKRYGGPSSGAPPPS |
| P50 | HSDIVFTDNYTRLRKQVAAKKYLQSIKNKRYGGPSSGAPPPS |
| P51 | HSDYVFTDNYTRLRKQVAAKKYLQSIKNKRYGGPSSGAPPPS |
| P52 | HSDFVFTDNYTRLRKQVAAKKYLQSIKNKRYGGPSSGAPPPS |
| P53 | HSDVVFTDNYTRLRKQVAAKKYLQSIKNKRYGGPSSGAPPPS |
| P54 | HSDTVFTDNYTRLRKQVAAKKYLQSIKNKRYGGPSSGAPPPS |
| P55 | HSDLVFTDNYTRLRKQVAAKKYLQSIKNKRYGGPSSGAPPPS |
| P56 | HSDWVFTDNYTRLRKQVAAKKYLQSIKNKRYGGPSSGAPPPS |
| P58 | HSDAFFTDNYTRLRKQVAAKKYLQSIKNKRYGGPSSGAPPPS |
| P60 | HSDAIFTDNYTRLRKQVAAKKYLQSIKNKRYGGPSSGAPPPS |
| P61 | HSDALFTDNYTRLRKQVAAKKYLQSIKNKRYGGPSSGAPPPS |
| P62 | HSDATFTDNYTRLRKQVAAKKYLQSIKNKRYGGPSSGAPPPS |
| P63 | HSDAVITDNYTRLRKQVAAKKYLQSIKNKRYGGPSSGAPPPS |
| P64 | HSDAVLTDNYTRLRKQVAAKKYLQSIKNKRYGGPSSGAPPPS |
| P65 | HSDAVTTDNYTRLRKQVAAKKYLQSIKNKRYGGPSSGAPPPS |
| P66 | HSDAVVTDNYTRLRKQVAAKKYLQSIKNKRYGGPSSGAPPPS |
| P67 | HSDAVWTDNYTRLRKQVAAKKYLQSIKNKRYGGPSSGAPPPS |
| P68 | HSDAVYTDNYTRLRKQVAAKKYLQSIKNKRYGGPSSGAPPPS |
| P69 | HSDAWFTDNYTRLRKQVAAKKYLQSIKNKRYGGPSSGAPPPS |
| P70 | HSDAYFTDNYTRLRKQVAAKKYLQSIKNKRYGGPSSGAPPPS |
| P71 | HSDAVFTDNYTRLRRQVAARRYLQSIRNRRYGGPSSGAPPPS |
| P72 | HSDAVFTDNYTRLRRQVAAKKYLQSIKNKRYGGPSSGAPPPS |
| P73 | HSDAVFTDNYTRLRKQVAARKYLQSIKNKRYGGPSSGAPPPS |
| P74 | HSDAVFTDNYTRLRKQVAAKKYLQSIQNKRYGGPSSGAPPPS |
| P75 | HSDAVFTDNYTRLRKQVAAKKYLQSIKNNRYGGPSSGAPPPS |
| P76 | HSDAVFTDNYTRLRKQVAAKKYLQSIKNKIYGGPSSGAPPPS |
| P82 | HSDAVFTDNYTRLRKQVAAKKYLQSIKRGGPSSGAPPPS |
| P83 | HSDAVFTDNYTRLRKQVAAKKYLQSIKNGGPSSGAPPPS |
| P84 | dHSDAVFTDNYTRLRKQVAAKKYLQSIKNKRYGGPSSGAPPPS |
| P85 | HSDAVFTDNYTRLRKQVAAKKYLQSIKKGGPSSGAPPPS |
| P87 | HSDAVFTDNYTREKEKVAAKKYLQSIKNKRYGGPSSGAPPPS |
| P88 | HSDAVFTDNYTRAAAKVAAKKYLQSIKNKRYGGPSSGAPPPS |
| P89 | HSDAVFTDNYTRLLAKVAAKKYLQSIKNKRYGGPSSGAPPPS |
| P92 | HSDAVFTDNYTRLRKQVAAKKYLQSIKNGRPSSGAPPPS |
| P93 | HSDAVFTDNYTRLLLKVAAKKYLQSIKNKRYGGPSSGAPPPS |
| P94 | HSDAVFTDNYTRAKAKVAAKKYLQSIKNKRYGGPSSGAPPPS |
| P98 | C6-HSDAVFTDNYTRLRRQVAARRYLQSIRNRRYGGPSSGAPPPS |
| P99 | C6-HVDAVFTDNYTRLRKQVAAKKYLQSIKNKRYGGPSSGAPPPS |
| P100 | M-HSDAVFTDQYTRLRKQVAAKKYLQSIKQKRYGGPSSGAPPPS |
| P101 | C6-HdADAVFTDNYTRLRKQVAAKKYLQSIKNKRYGGPSSGAPPPS |
| P102 | HSDGVFTDNYTRLRKQVAAKKYLQSIKNKRYGGPSSGAPPPS |
| P103 | C6-HSDAVFTDNYTKLKKQVAAKKYLQSIKNKKYGGPSSGAPPPS |
| P104 | M-HSDAVFTDNYTRLRKQVAAKKYLQSIKNKRYGGPSSGAPPPS |
| P105 | I-HSDAVFTDNYTRLRKQVAAKKYLQSIKNKRYGGPSSGAPPPS |
| P106 | C6-HSDAVFTDNYTRLRKQVAAKKYLQSFKNKRYGGPSSGAPPPS |
| P107 | C6-HSDAVFTDNYTRLRKQVAAKKYLQSLKNKRYGGPSSGAPPPS |
| P108 | C6-HSDAVFTDNYTRLRKQVAAKKYLQSTKNKRYGGPSSGAPPPS |
| P109 | C6-HSDAVFTDNYTRLRKQVAAKKYLQSVKNKRYGGPSSGAPPPS |
| P110 | C6-HSDAVFTDNYTRLRKQVAAKKYLQSWKNKRYGGPSSGAPPPS |
| P111 | C6-HSDAVFTDNYTRLRKQVAAKKYLQSYKNKRYGGPSSGAPPPS |
| P112 | C6-HSDAVFTDNYTRLRKQVAAKKYLQFIKNKRYGGPSSGAPPPS |
| P113 | C6-HSDAVFTDNYTRLRKQVAAKKYLQIIKNKRYGGPSSGAPPPS |
| P114 | C6-HSDAVFTDNYTRLRKQVAAKKYLQLIKNKRYGGPSSGAPPPS |
| P115 | C6-HSDAVFTDNYTRLRKQVAAKKYLQTIKNKRYGGPSSGAPPPS |
| P116 | C6-HSDAVFTDNYTRLRKQVAAKKYLQVIKNKRYGGPSSGAPPPS |
| P117 | C6-HSDAVFTDNYTRLRKQVAAKKYLQWIKNKRYGGPSSGAPPPS |
| P119 | C6-HSDAVFTDNYTRLLAKLALQKYLQSIKNKRYGGPSSGAPPPS |
| P120 | C6-HSDAVFTDNYTRLRKQVAAKKYLQSIKNKRYGGPSSGAPPPC |
| P121 | C6-HSDAVFTDNYTRLRKQVAAKKYLQSIKNKKGGPSSGAPPPS |
| P122 | C6-HSDAVFTDNYTRLRKQVAAKKYLQSIKNSRGGPSSGAPPPS |
| P123 | C6-HSDAVFTDNYTRLRKQVAAKKYLQSIKNKRGGPSSGAPPPS |
| P124 | C6-HSDAVFTDNYTRLRKQVAAKKYLQQIKNKRYGGPSSGAPPPS |
| P125 | C6-HSDAVFTDNYTRLRKQVAAKKYLQNIKNKRYGGPSSGAPPPS |
| P126 | HSDAVFTDNYTRLRKQVAAKKYLQSIKRGRPSSGAPPPS |
| P127 | C6-HSDAVFTDNYTRLRKQVAAKKYLQYIKNKRYGGPSSGAPPPS |
| P129 | C6-HSDAVFTDNYTRLRKQVAAKKWLQSIKNKRYGGPSSGAPPPS |
| P130 | C6-HSDAVFTDNYTRLRKQVAAKKFLQSIKNKRYGGPSSGAPPPS |
| P131 | C6-HSDAVFTDNYTRLRKQVAAKKTLQSIKNKRYGGPSSGAPPPS |
| P132 | C6-HSDAVFTDNYTRLRKQVAAKKLLQSIKNKRYGGPSSGAPPPS |
| P133 | C6-HSDAVFTDNYTRLRKQVAAKKILQSIKNKRYGGPSSGAPPPS |
| P134 | C6-HSDAVFTDNYTRLRKQVAAKKVLQSIKNKRYGGPSSGAPPPS |
| P135 | C6-HSDAVFTDNYTRLLAKVAAKKYLQSIKNKRYGGPSSGAPPPS |
| P138 | C6-HSDAVFTDNYTRLRAQVAAQKYLQSIKNKRYGGPSSGAPPPS |
| P139 | C6-HSDAVFTDNYTRLRAQVAAKKYLQSIKNKRYGGPSSGAPPPS |
| P140 | M-HSDAVFTDNYTRLLAKLALQKYLQSIKNKRYGGPSSGAPPPS |
| P141 | C6-HSDAVFTDNYTRLKAQVAAKKYLQSIKNKRYGGPSSGAPPPS |
| P142 | C6-HSDAVFTDNYTRLRAQLAAQKYLQSIKNKRYGGPSSGAPPPS |
| P143 | C6-HSDAVFTDNYTRLRKQMAAQKYLNQLKKGGPSSGAPPPS |
| P144 | C6-HSDAVFTDNYTRLRKQVAAQKYLNQLKKGGPSSGAPPPS |
| P146 | C6-HSDAVFTDNYTRLRKQVAAVKYLQSIKNKRYGGPSSGAPPPS |
| P147 | C6-HSDAVFTDNYTRLRKQVAAYKYLQSIKNKRYGGPSSGAPPPS |
| P 148 | C6-HSDAVFTDNYTRLRKQVAAFKYLQSIKNKRYGGPSSGAPPPS |
| P149 | C6-HSDAVFTDNYTRLRKQVAAIKYLQSIKNKRYGGPSSGAPPPS |
| P 150 | C6-HSDAVFTDNYTRLRKQVAAQKYLQSIKNKRYGGPSSGAPPPS |
| P151 | C6-HSDAVFTDNYTRLRKQVAALKYLQSIKNKRYGGPSSGAPPPS |
| P152 | C6-HSDAVFTDNYTRLRKQVAATKYLQSIKNKRYGGPSSGAPPPS |
| P153 | C6-HSDAVFTDNYTRLRKQVAAWKYLQSIKNKRYGGPSSGAPPPS |
| P154 | C6-HSDAVFTDNYTRLRKQVAAKKYLQSIKNGGPSSGAPPPS |
| P155 | C6-HSDAVFTDNYTRLRKQVALKKYLQSIKNKRYGGPSSGAPPPS |
| P158 | C6-HSDAVFTANYTRLRKQVAAKKYLQSIKNKRYGGPSSGAPPPS |
| P159 | C6-HSDAVFTENYTRLRKQVAAKKYLQSIKNKRYGGPSSGAPPPS |
| P160 | C6-HSDAVFTKNYTRLRKQVAAKKYLQSIKNKRYGGPSSGAPPPS |
| P161 | C6-HSDAVFTLNYTRLRKQVAAKKYLQSIKNKRYGGPSSGAPPPS |
| P162 | C6-HSDAVFTRNYTRLRKQVAAKKYLQSIKNKRYGGPSSGAPPPS |
| P163 | C6-HSDAVFTYNYTRLRKQVAAKKYLQSIKNKRYGGPSSGAPPPS |
| P164 | C6-HSDAVFTDNYTRLLAKLALQKYLQSIKNKRYGGPSSGAPPPC |
| P165 | C6-HSDAVFTEEYTRLQKQVAAKQYLQSIKNKRYGGPSSGAPPPS |
| P166 | C6-HAibDAVFTDNYTRLRKQVAAKKYLQSIKNKRYGGPSSGAPPPS |
| P167 | C6-HSDAVFTDNYTRLAibKQVAAKKYLQSIKNKRYGGPSSGAPPPS |
| P168 | C6-HSDAVFTDNYTRLRAibQVAAKKYLQSIKNKRYGGPSSGAPPPS |
| P169 | C6-HSDAVFTDNYTRLRKQVAAAibKYLQSIKNKRYGGPSSGAPPPS |
| P170 | C6-HSDAVFTDNYTRLRKQVAAKAibYLQSIKNKRYGGPSSGAPPPS |
| P171 | C6-HSDAVFTDNYTRLKKQVAAKKYLQSIKNKRYGGPSSGAPPPS |
| P172 | C6-HSDAVFTDNYTRLQKQVAAKKYLQSIKNKRYGGPSSGAPPPS |
| P173 | C6-HSDAVFTDNYTRLAKQVAAKKYLQSIKNKRYGGPSSGAPPPS |
| P174 | C6-HSDAVFTDNYTRLLKQVAAKKYLQSIKNKRYGGPSSGAPPPS |
| P175 | C6-HSDAVFTDNYTRLFKQVAAKKYLQSIKNKRYGGPSSGAPPPS |
| P176 | C6-HSDAVFTDNYTRLRRQVAAKKYLQSIKNKRYGGPSSGAPPPS |
| P177 | C6-HSDAVFTDNYTRLRQQVAAKKYLQSIKNKRYGGPSSGAPPPS |
| P179 | C6-HSDAVFTDNYTRLRLQVAAKKYLQSIKNKRYGGPSSGAPPPS |
| P180 | C6-HSDAVFTDNYTRLRFQVAAKKYLQSIKNKRYGGPSSGAPPPS |
| P181 | C6-HSDAVFTDNYTRLRKQVAAAKYLQSIKNKRYGGPSSGAPPPS |
| P182 | C6-HSDAVFTDNYTRLRKQVAAKRYLQSIKNKRYGGPSSGAPPPS |
| P183 | C6-HSDAVFTDNYTRLRKQVAAKQYLQSIKNKRYGGPSSGAPPPS |
| P184 | C6-HSDAVFTDNYTRLRKQVAAKAYLQSIKNKRYGGPSSGAPPPS |
| P185 | C6-HSDAVFTDNYTRLRKQVAAKLYLQSIKNKRYGGPSSGAPPPS |
| P186 | C6-HSDAVFTDNYTRLRKQVAAKFYLQSIKNKRYGGPSSGAPPPS |
| P187 | C6-HSDAVFTDNYTRLRKQVAAKKYLQAibIKNKRYGGPSSGAPPPS |
| P188 | C6-HSDAVFTDNYTRLRKQVAAKKYLQSAibKNKRYGGPSSGAPPPS |
| P189 | C6-HSDAVFTDNYTRLRKQAibAAKKYLQSIKNKRYGGPSSGAPPPS |
| P191 | C6-HHSDAVFTDNYTRLLAKLALQKYLQSIKNKRYGGPSSGAPPPS |
| P192 | C6-HSDAVFTDQYTRLLAKLALQKYLQSIKQKRYGGPSSGAPPPS |
| P193 | |
| P194 | |
| P195 | C6-HSDAVFTDNYTRLLAQLALQKYLQSIKNKRYGGPSSGAPPPS |
| P196 | C6-HSDAVFTDNYTRLLAKVALQKYLQSIKNKRYGGPSSGAPPPS |
| P197 | C6-HSDAVFTDNYTRLLAKLAAQKYLQSIKNKRYGGPSSGAPPPS |
| P198 | C6-HSDAVFTDNYTRLLAKLALQKYLQSIKNKGGPSSGAPPPC |
| P199 | Met(O)-HSDAVFTDNYTRLRKQVAAKKYLQSIKNKRYGGPSSGAPPPS |
| P203 | C6-HSDA VFTDNYTRLRLRKOVAAKKYLOSIKNKRFGGPSSAPPPS |
| P205 | |
| P206 | |
| P207 | C6-HSDdAVFTDNYTRLRKQVAAKKYLQSIKNKRYGGPSSGAPPPS |
| P208 | C6-HSDNMeAVFTDNYTRLRKQVAAKKYLQSIKNKRYGGPSSGAPPPS |
| P209 | C6-HSDAibVFTDNYTRLRKQVAAKKYLQSIKNKRYGGPSSGAPPPS |
| P210 | C6-HSDAdVFTDNYTRLRKQVAAKKYLQSIKNKRYGGPSSGAPPPS |
| P211 | C6-HSDANMeVFTDNYTRLRKQVAAKKYLQSIKNKRYGGPSSGAPPPS |
| P212 | C6-HSDAAibFTDNYTRLRKQVAAKKYLQSIKNKRYGGPSSGAPPPS |
| P213 | C6-HSDAVFTDNYTRLRKQVAAKRYLQSIRNGGPSSGAPPPS |
| P214 | C6-HSDAVFTDNYTRLRKQVAARRYLQSIRNGGPSSGAPPPS |
| P215 | C6-HSDAVFTDNYTRLRRQVAAKRYLQSIRNGGPSSGAPPPS |
| P216 | C6-HSDAVFTDNYTRLRRQVAARKYLQSIRNGGPSSGAPPPS |
| P220 | C6-HSDAVFTDQYTRLRRQVAARKYLQSIRQGGPSSGAPPPS |
| P221 | C6-HSDIVFTDNYTRLRKQVAAKKYLQSIKNKRYGGPSSGAPPPS |
| P222 | C6-HGEGTFTDNYTRLRKQVAAKKYLQSIKNKRYGGPSSGAPPPS |
| P223 | C6-HSDLVFTDNYTRLRKQVAAKKYLQSIKNKRYGGPSSGAPPPS |
| P224 | C6-HSEAVFTDNYTRLRKQVAAKKYLQSIKNKRYGGPSSGAPPPS |
| P228 | C6-HSDAVFTDNYTRLRKQVAAKKAibLQSIKNKRYGGPSSGAPPPS |
| P229 | C6-HSDAVFTDNYTRLRKQVAAKKALQSIKNKRYGGPSSGAPPPS |
| P230 | C6-HSDAVFTDNYTRLRKQVAAKKYWQSIKNKRYGGPSSGAPPPS |
| P231 | C6-HSDAVFTDNYTRLRKQVAAKKYFQSIKNKRYGGPSSGAPPPS |
| P232 | C6-HSDAVFTDNYTRLRKQVAAKKYTQSIKNKRYGGPSSGAPPPS |
| P233 | C6-HSDAVFTDNYTRLRKQVAAKKYIQSIKNKRYGGPSSGAPPPS |
| P234 | C6-HSDAVFTDNYTRLRKQVAAKKYVQSIKNKRYGGPSSGAPPPS |
| P235 | C6-HSDAVFTDNYTRLRKQVAAKKYAQSIKNKRYGGPSSGAPPPS |
| P236 | C6-HSDAVFTDNYTRLRKQVAAKKYAibQSIKNKRYGGPSSGAPPPS |
| P240 | C6-HSDAVFTDNYTRLAibKQLAAAibKYLQSIKNKRYGGPSSGAPPPS |
| P241 | C6-HSDAVFTDNYTRLAibKQLAAKAibYLQSIKNKRYGGPSSGAPPPS |
| P242 | C6-HSDAVFTDNYTRLAibKQVAAAibKYLQSIKNKRYGGPSSGAPPPS |
| P243 | C6-HSDAVFTDNYTRLAibKQVAAQKYLQSIKNKRYGGPSSGAPPPS |
| P244 | C6-HSDAVFTDNYTRLAibKQVAAKAibYLQSIKNKRYGGPSSGAPPPS |
| P249 | C6-HSDAVFTDNYTRLAibKQVAAKQYLQSIKNKRYGGPSSGAPPPS |
| P250 | C6-HSDAVFTDNYTRLQKQVAAAibKYLQSIKNKRYGGPSSGAPPPS |
| P251 | C6-HSDAVFTDNYTRLQKQVAAKAibYLQSIKNKRYGGPSSGAPPPS |
| P252 | C6-HSDAVFTDNYTRLQKQVAAQKYLQSIKNKRYGGPSSGAPPPS |
| P253 | C6-HSDAVFTDNYTRLQKQVAAKQYLQSIKNKRYGGPSSGAPPPS |
| P258 | C6-HSDAVFTDNYTRLQAibQVAAKKYLQSIKNKRYGGPSSGAPPPS |
| P259 | C6-HSDAVFTDNYTRLAibKQVAALKYLQSIKNKRYGGPSSGAPPPS |
| P260 | C6-HSDAVFTDNYTRLAibKQVAAAKYLQSIKNKRYGGPSSGAPPPS |
| P261 | C6-HSDAVFTDNYTRLRAibQVAAAibKYLQSIKNKRYGGPSSGAPPPS |
| P262 | C6-HSDAVFTDNYTRLRAibQVAAVKYLQSIKNKRYGGPSSGAPPPS |
| P263 | C6-HSDAVFTDNYTRLRAibQVAAAKYLQSIKNKRYGGPSSGAPPPS |
| P264 | C6-HSDAVFTDNYTRLRAibQVAAKAibYLQSIKNKRYGGPSSGAPPPS |
| P265 | C6-HSDAVFTDNYTRLRAibQVAALKYLQSIKNKRYGGPSSGAPPPS |
| P269 | C6-HSDAVFTDNYTRLAibKQVAAVKYLQSIKNKRYGGPSSGAPPPS |
| P270 | C6-HSDAVFTDNYTRLRKQVAAKKYLQSIKQKGGPSSGAPPPS |
| P271 | C6-HSDAVFTDNYTRLRKQVAAKKYLQSIKNGRPSSGAPPPS |
| P275 | C6-HSDAVFTDNYTRLRKQVAGKKYLQSIKNKRYGGPSSGAPPPS |
| P282 | |
| P284 | C6-HSDAVFTDNYTRLRAibQLAAKAibYLQSIKNKRYGGPSSGAPPPS |
| P285 | |
| P289 | C6-HSDALFTDNYTRLRKQVAAKKYLQSIKNKRYGGPSSGAPPPS |
| P290 | C6-HSDAVFTDNYTRLRKQLAAKKYLQSIKNKRYGGPSSGAPPPS |
| P291 | C6-HSDAVFTDNYTRLRAibQLAAAibKYLQSIKNKRYGGPSSGAPPPS |
| P292 | C6-HSDAVFTDNYTRLRAibQLAAAibKYLQSIKNKGGPSSGAPPPS |
| P293 | C6-HSDAVFTDNYTRLAibKQVAAAibKYLQSIKQKGGPSSGAPPPS |
| P294 | C6-HSDAVFTDNYTRLRAibQVAAAibKYLQSIKQKGGPSSGAPPPS |
| P295 | C6-HSDAVFTDNYTRLRAibQVAAKAibYLQSIKQKGGPSSGAPPPS |
| P296 | C6-HSDAVFTDNYTRLAibKQVAAAibKYLQSIKQGGPSSGAPPPS |
| P297 | C6-HSDAVFTDNYTRLRAibQVAAAibKYLQSIKQGGPSSGAPPPS |
| P298 | C6-HSDAVFTDNYTRLRAibQVAAKAibYLQSIKQGGPSSGAPPPS |
| P299 | |
| P301 | C6-HSDAVFTDNYTRLAAibQVAAAibKYLQSIKNKRYGGPSSGAPPPS |
| P302 | C6-HSDAVFTDNYTRLQAibQVAAAibKYLQSIKNKRYGGPSSGAPPPS |
| P305 | C6-HSDAVFTDNYTRLhRKQVAAKKYLQSIKNKRYGGPSSGAPPPS |
| P307 | C6-HSDAVFTDNYTRLROrnQVAAKKYLQSIKNKRYGGPSSGAPPPS |
| P308 | C6-HSDAVFTDNYTRLhROrnQVAAKKYLQSIKNKRYGGPSSGAPPPS |
| P314 | C6-HSEAVFTENYTRLRAibQVAAAibKYLQSIKNKRYGGPSSGAPPPS |
| P315 | C6-HSDAVFTDQYTRLRAibQVAAAibKYLQSIKQKRYGGPSSGAPPPS |
| P316 | C6-HSDAVFTDNYTRLhRAibQVAAAibKYLQSIKNKRYGGPSSGAPPPS |
| P317 | C6-HSDAVFTDNYTRLLAibQVAAAibKYLQSIKNKRYGGPSSGAPPPS |
| P318 | C6-HSDAVFTDNYTRLKAibQVAAAibKYLQSIKNKRYGGPSSGAPPPS |
| P319 | |
| P320 | C6-HSDAVFTDNYTRLCitAibQVAAAibKYLQSIKNKRYGGPSSGAPPPS |
| P321 | C6-HSDAVFTDNYTRLRAibKVAAAibKYLQSIKNKRYGGPSSGAPPPS |
| P322 | C6-HSDAVFTDNYTRLRAibQIAAAibKYLQSIKNKRYGGPSSGAPPPS |
| P323 | C6-HSDAVFTDNYTRLRAibQKAAAibKYLQSIKNKRYGGPSSGAPPPS |
| P324 | C6-HSDAVFTDNYTRLRAibQAAAAibKYLQSIKNKRYGGPSSGAPPPS |
| P325 | C6-HSDAVFTDNYTRLRAibQNleAAAibKYLQSIKNKRYGGPSSGAPPPS |
| P326 | |
| P327 | |
| P329 | C6-HSDAVFTDNYThRLRAibQVAAAibKYLQSIhRNhRGGPSSGAPPPS |
| P330 | |
| P332 | HSDAVFTDNYThRLRAibOVAAAibKYLOSIhRhRGGPSSGAPPPS |
| P333 | |
| P335 | C6-HSDAVFTDNYTOrnLRAibQVAAAibKYLQSIhRhRGGPSSGAPPPS |
| P336 | |
| P338 | |
| P339 | |
| P341 | C6-HSDAVFTDNYTRLRAibQVAAAibAYLQSIKNKRYGGPSSGAPPPS |
| P342 | C6-HSDAVFTDNYTRLRAibQVAAAibOrnYLQSIKNKRYGGPSSGAPPPS |
| P344 | |
| P345 | C6-HSDAVFTDNYTAibLRAibQVAAAibKYLQSIKNKRYGGPSSGAPPPS |
| P346 | |
| P349 | C6-HSDAVFTDNYTRLRAibQVAAAibKYLQSIKAibKGGPSSGAPPPS |
| P350 | C6-HSDAVFTDNYTRLRAibQVAAAibKYLQSIKPKGGPSSGAPPPS |
| P351 | C6-HSDAVFTDNYTRLRAibQVAAAibKYLQSIKKGGPSSGAPPPS |
| P352 | C6-HSDAVFTDNYTRLRAibQVAAAibKYLQSIOrnOrnGGPSSGAPPPS |
| P353 | C6-HSDAVFTDNYTRLRAibQVAAAibKYLQSIdKdKGGPSSGAPPPS |
| P354 | C6-HSDAVFTDNYTRLRAibQVAAAibKYLQSIKhRGGPSSGAPPPS |
| P355 | C6-HSDAVFTDNYTRLRAibQVAAAibKYLQSIKAibGGPSSGAPPPS |
| P356 | C6-HSDAVFTDNYTRLRAibQVAAAibKYLQSIOrnQOrnGGPSSGAPPPS |
| P364 | |
| P365 | C6-HSDAVFTDNYTOrnLRAibQIAAAibKYLQSIOrnNOrnGGPSSGAPPPS |
| P366 | C6-HSDAVFTDNYTALRAibQVAAAibKYLQSIOrnNOrnGGPSSGAPPPS |

8. The VPAC2 receptor peptide agonist according to any one of claims 1 to 7 for use as a medicament.

9. The VPAC2 receptor peptide agonist according to any one of claims 1 to 7 for use in the treatment of non-insulin dependent diabetes or insulin-dependent diabetes.

10. The use of a VPAC2 receptor peptide agonist according to any one of claims 1 to 7 in the manufacture of a medicament for the treatment of non-insulin-dependent diabetes or insulin-dependent diabetes.'

## Patentansprüche

1. VPAC2 Rezeptorpeptidagonist, umfassend eine Sequenz der folgenden Formel worin
Xaa₁ für His oder dH steht oder fehlt,
Xaa₂ für dA, Ser, Val, Gly, Thr, Leu, dS, Pro oder Aib steht,
Xaa₃ für Asp oder Glu steht,
Xaa₄ für Ala, Ile, Tyr, Phe, Val, Thr, Leu, Trp, Gly, dA, Aib or NMeA steht,
Xaa₅ für Val, Leu, Phe, Ile, Thr, Trp, Tyr, dV, Aib oder NMeV steht,
Xaa₆ für Phe, Ile, Leu, Thr, Val, Trp oder Tyr steht,
Xaa₈ für Asp, Glu, Ala, Lys, Leu, Arg oder Tyr steht,
Xaa₉ für Asn, Gln oder Glu steht,
Xaa₁₀ für Tyr, Trp oder Tyr(OMe) steht,
Xaa₁₂ für Arg, Lys, hR, Orn Aib, Cit oder Ala steht,
Xaa₁₃ für Leu, Phe, Glu, Ala oder Aib steht,
Xaa₁₄ für Arg, Leu, Lys, Ala, hR, Orn, Phe, Gln, Aib oder Cit steht,
Xaa₁₅ für Lys, Ala, Arg, Glu, Leu, hR, Orn, Phe, Gln, Aib, K(Ac) oder Cit steht,
Xaa₁₆ für Gln, Lys, Ala, hR, Orn oder Cit steht,
Xaa₁₇ für Val, Ala, Leu, Ile, Met, Nle, Lys oder Aib steht,
Xaa₁₉ für Ala, Gly oder Leu steht,
Xaa₂₀ für Lys, Gln, hR, Arg, Ser, Orn, Ala, Aib, Trp, Thr, Leu, Ile, Phe, Tyr, Val, K(Ac) der Cit steht,
Xaa₂₁ für Lys, Arg, Ala, Phe, Aib, Leu, Gln, Orn, hR, K(Ac) oder Cit steht,
Xaa₂₂ für Tyr, Trp, Phe, Thr, Leu, Ile, Val, Tyr(OMe) oder Aib steht,
Xaa₂₃ für Leu, Phe, Ile, Ala, Trp, Thr, Val oder Aib steht,
Xaa₂₄ für Gln oder Asn steht,
Xaa₂₅ für Ser, Asp, Phe, Ile, Leu, Thr, Val, Trp, Gln, Asn, Tyr, Aib oder Glu steht,
Xaa₂₆ für Ile, Leu, Thr, Val, Trp, Tyr, Phe oder Aib steht,
Xaa₂₇ für Lys, hR, Arg, Gln, Orn oder dK steht,
Xaa₂₈ für Asn, Gln, Lys, Arg, Aib, Orn, hR, Cit, Pro oder dK steht,
Xaa₂₉ für Lys, Ser, Arg, Asn, hR, Orn, Cit oder Aib steht oder fehlt,
Xaa₃₀ für Arg, Lys, Ile, hR, Cit, Aib oder Orn steht oder fehlt,
Xaa₃₁ für Tyr, His oder Phe steht oder fehlt, und
Xaa₃₂ für Cys steht oder fehlt,
mit der Maßgabe, dass, falls Xaa₂₉, Xaa₃₀ oder Xaa₃₁ fehlt, die nächste stromabwärts vorhandene Aminosäure die nächste Aminosäure in der Peptidagonistensequenz ist,
und eine C-terminale Extension worin der N-Terminus der C-terminalen Extension an den C-Terminus des Peptids der Formel 12 gebunden ist und worin die C-terminale Extension eine Aminosäuresequenz der folgenden Formel umfasst:
Xaa₁-Xaa₂-Xaa₃-Xaa₄-Xaa₅-Xaa₆-Xaa₇-Xaa₈-Xaa₉-Xaa₁₀-Xaa₁₁ Formel 7 (SEQ ID NR:15)
worin
Xaa₁ für Gly oder Cys steht oder fehlt,
Xaa₂ für Gly oder Arg steht oder fehlt,
Xaa₃ für Pro oder Thr steht oder fehlt,
Xaa₄ für Ser steht oder fehlt,
Xaa₅ für Ser steht oder fehlt,
Xaa₆ für Gly steht oder fehlt,
Xaa₇ für Ala steht oder fehlt,
Xaa₈ für Pro steht oder fehlt,
Xaa₉ für Pro steht oder fehlt,
Xaa₁₀ für Pro steht oder fehlt, und
Xaa₁₁ für Ser oder Cys steht oder fehlt,
mit der Maßgabe, dass wenigstens fünf der Gruppen Xaa₁ bis Xaa₁₁ der C-terminalen Extension vorhanden sind und
mit der Maßgabe, dass, falls Xaa₁, Xaa₂, Xaa₃, Xaa₄, Xaa₅, Xaa₆, Xaa₇, Xaa₈, Xaa₉ oder Xaa₁₀ fehlt, die nächste stromabwärts vorhandene Aminosäure dann die nächste Aminosäure in der C-terminalen Extension ist und worin die C-terminale Aminosäure amidiert sein kann.

2. VPAC2 Rezeptorpeptidagonist nach Anspruch 1, umfassend eine Sequenz der folgenden Formel worin
Xaa₂ für dA, Ser, Val, dS oder Aib steht,
Xaa₃ für Asp oder Glu steht,
Xaa₄ für Ala, dA oder Aib steht,
Xaa₅ für Val, Leu, dV oder Aib steht,
Xaa₈ für Asp, Glu oder Ala steht,
Xaa₉ für Asn, Gln oder Glu steht,
Xaa₁₂ für Ala, Arg, Lys, hR oder Orn steht,
Xaa₁₄ für Arg, Leu, Lys, Ala, hR, Orn, Phe, Gln, Aib oder Cit steht,
Xaa₁₅ für Lys, Ala, Arg, Leu, Orn, Phe, Gln, Aib oder K(Ac) steht,
Xaa₁₆ für Gln oder Lys steht,
Xaa₁₇ für Val, Ala, Leu, Ile, Met, Nle oder Lys steht,
Xaa₁₉ für Ala oder Leu steht,
Xaa₂₀ für Lys, Gln, hR, Arg, Ser, Ala, Aib, Trp, Thr, Leu, Ile, Phe, Tyr, Val oder K(Ac) steht,
Xaa₂₁ für Lys, Arg, Ala, Phe, Aib, Leu, Gln, K(Ac) oder Orn steht,
Xaa₂₂ für Tyr, Trp, Phe, Leu, Ile oder Val steht,
Xaa₂₄ für Gln oder Asn steht,
Xaa₂₅ für Ser, Phe, Ile, Leu, Thr, Val, Trp, Gln, Asn, Tyr oder Aib steht,
Xaa₂₆ für Ile, Leu, Thr, Val, Trp, Tyr, Phe oder Aib steht,
Xaa₂₇ für Lys, hR, Arg, dK oder Orn steht,
Xaa₂₈ für Asn, Gln, Lys, hR, Aib, Orn, dK oder Pro steht,
Xaa₂₉ für Lys, Ser, Arg, hR oder Orn steht oder fehlt,
Xaa₃₀ für Arg oder Lys steht oder fehlt, und
Xaa₃₁ für Tyr oder Phe steht oder fehlt,
mit der Maßgabe, dass, falls Xaa₂₉ oder Xaa₃₀ fehlt, die nächste stromabwärts vorhandene Aminosäure an die nächste Aminosäure in der Peptidagonistensequenz ist,
und eine C-terminale Extension, worin der N-Terminus der C-terminalen Extension an den C-Terminus des Peptids der Formel 13 gebunden ist und worin die C-terminale Extension eine Aminosäure der folgenden Sequenz umfasst:
Xaa₁-Xaa₂-Xaa₃-Xaa₄-Xaa₅-Xaa₆-Xaa₇Xaa₈-Xaa₉-Xaa₁₀-Xaa₁₁ Formel 7 (SEQ ID NR:15)
worin
Xaa₁ für Gly oder Cys steht oder fehlt,
Xaa₂ für Gly oder Arg steht oder fehlt,
Xaa₃ für Pro oder Thr steht oder fehlt,
Xaa₄ für Ser steht oder fehlt,
Xaa₅ für Ser steht oder fehlt,
Xaa₆ für Gly steht oder fehlt,
Xaa₇ für Ala steht oder fehlt,
Xaa₈ für Pro steht oder fehlt,
Xaa₉ für Pro steht oder fehlt,
Xaa₁₀ für Pro steht oder fehlt, und
Xaa₁₁ für Ser oder Cys steht oder fehlt,
mit der Maßgabe, dass wenigstens fünf der Gruppen Xaa₁ bis Xaa₁₁ der C-terminalen Extension vorhanden sind und
mit der Maßgabe, dass, falls Xaa₁, Xaa₂, Xaa₃, Xaa₄, Xaa₅, Xaa₆, Xaa₇, Xaa₈, Xaa₉ oder Xaa₁₀ fehlt, die nächste stromabwärts vorhandene Aminosäure dann die nächste Aminosäure in der C-terminalen Extension ist und worin die C-terminale Aminosäure amidiert sein kann.

3. VPAC2 Rezeptorpeptidagonist nach einem der vorhergehenden Ansprüche, worin die C-terminale Extension ausgewählt ist aus:
SEQ ID NR: 10 GGPSSGAPPPS
SEQ ID NR: 11 GGPSSGAPPPS-NH₂
SEQ ID NR: 22 GGPSSGAPPPC
SEQ ID NR: 23 GGPSSGAPPPC-NH₂
SEQ ID NR: 24 GRPSSGAPPPS
SEQ ID NR: 25 GRPSSGAPPPS-NH₂.

4. VPAC2 Rezeptorpeptidagonist nach einem der vorhergehenden Ansprüche, weiter umfassend eine N-terminale Modifikation am N-Terminus des Peptidagonisten, worin die N-terminale Modifikation ausgewählt ist aus
a) einer Addition von D-Histidin, Isoleucin, Methionin oder Norleucin,
b) einer Addition eines Peptids, umfassend die Sequenz Ser-Trp-Cys-Glu-Pro-Gly-Trp-Cys-Arg, worin das Arg an den N-Terminus des Peptidagonisten gebunden ist,
c) einer Addition von C₁-C₁₆-Alkyl, das optional substituiert ist mit ein oder mehr Substituenten, die unabhängig ausgewählt sind aus Aryl, C₁-C₆-Alkoxy, -NH₂, -OH, Halogen und -CF₃,
d) einer Addition von -C(O)R¹, worin R¹ für ein C₁-C₁₆-Alkyl steht, das optional substituiert ist mit ein oder mehr Substituenten, die unabhängig ausgewählt sind aus Aryl, C₁-C₆-Alkoxy, -NH₂, -OH, Halogen, -SH und -CF₃, für Aryl oder Aryl-C₁-C₄-alkyl, das optional substituiert ist mit ein oder mehr Substituenten, die unabhängig ausgewählt sind aus C₁-C₆-Alkyl, C₂-C₆-Alkenyl, C₂-C₆-Alkinyl, C₁-C₆-Alkoxy, -NH₂, -OH, Halogen und -CF₃, oder -NR²R³, worin R² und R³ unabhängig für Wasserstoff, C₁-C₆-Alkyl, Aryl oder Aryl-C₁-C₄-alkyl stehen, oder für -OR⁴, worin R⁴ für C₁-C₆-Alkyl steht, das optional substituiert ist mit ein oder mehr Substituenten, die unabhängig ausgewählt sind aus Aryl, C₁-C₆-Alkoxy, -NH₂, -OH, Halogen und -CF₃, oder für Aryl oder Aryl-C₁-C₄-alkyl, das optional substituiert ist mit ein oder mehr Substituenten, die unabhängig ausgewählt sind aus C₁-C₆-Alkyl, C₂-C₆-Alkenyl, C₂-C₆-Alkinyl, C₁-C₆-Alkoxy, -NH₂, -OH, Halogen und -CF₃,
e) einer Addition von -SO₂R⁵, worin R⁵ für Aryl, Aryl-C₁-C₄-alkyl oder C₁-C₁₆-Alkyl steht,
f) einer Bildung einer Succinimidgruppe, die optional substituiert ist mit C₁-C₆-Alkyl oder -SR₆, worin R⁶ für Wasserstoff oder C₁-C₆-Alkyl steht, und
g) einer Addition von Methioninsulfoxid.

5. VPAC2 Rezeptorpeptidagonist nach Anspruch 4, worin die N-terminale Modifikation die Addition einer Gruppe ist, die ausgewählt ist aus Acetyl, Propionyl, Butyryl, Pentanoyl, Hexanoyl, Methionin, Methioninsulfoxid, 3-Phenylpropionyl, Phenylacetyl, Benzoyl, Norleucin, D-Histidin, Isoleucin und 3-Mercaptopropionyl.

6. VPAC2 Rezeptorpeptidagonist nach Anspruch 4 oder 5, worin die N-terminale Modifikation die Addition von Acetyl oder Hexanoyl ist.

7. VPAC2 Peptidrezeptoragonist nach Anspruch 1, umfassend eine Aminosäuresequenz, die ausgewählt ist aus:
| Agonist Nr. | Sequenz |
|---|---|
| P6 | HSDAVFTDNYTRLRKQVAAKKYLQSIKNKRYGGPSSGAPPPS |
| P9 | HSDAVFTDNYTRLRKQVAAKKYLQSIKNKKGGPSSGAPPPS |
| P18 | HSDAVFTDNYTRLRKQVAAhRKYLQSIKNKRYGGPSSGAPPPS |
| P19 | HSDAVFTDNYTRLRKQVAAIKYLQSIKNKRYGGPSSGAPPPS |
| P20 | HSDAVFTDNYTRLRKQVAARKYLQSIKNKRYGGPSSGAPPPS |
| P21 | HSDAVFTDNYTRLRKQVAASKYLQSIKNKRYGGPSSGAPPPS |
| P22 | HSDAVFTDNYTRLRKQVAAKKYLQSIhRNKRYGGPSSGAPPPS |
| P23 | HSDAVFTDNYTRLRKQVAAKKYLQSIRNKRYGGPSSGAPPPS |
| P24 | HSDAVFTDNYTRLRKQVAAKKYLQSIKNhRRYGGPSSGAPPPS |
| P25 | HSDAVFTDNYTRLRKQVAAKKYLQSIKNRRYGGPSSGAPPPS |
| P26 | HSDAVFTDNYTRFRKQVAAKKYLQSIKNKRYGGPSSGAPPPS |
| P27 | HSDAVFTDNWTRLRKQVAAKKYLQSIKNKRYGGPSSGAPPPS |
| P28 | HSDAVFTDNYTRLRKQVAAKKYLQSIKNKRHGGPSSGAPPPS |
| P31 | C6-HSDAVFTDNYTRLRKQVAAKKYLQSIKNKRYGGPSSGAPPPS |
| P33 | Ac-HSDAVFTDNYTRLRKQVAAKKYLQSIKNKRYGGPSSGAPPPS |
| P43 | HGDAVFTDNYTRLRKQVAAKKYLQSIKNKRYGGPSSGAPPPS |
| P44 | HVDAVFTDNYTRLRKQVAAKKYLQSIKNKRYGGPSSGAPPPS |
| P45 | HTDAVFTDNYTRLRKQVAAKKYLQSIKNKRYGGPSSGAPPPS |
| P46 | HLDAVFTDNYTRLRKQVAAKKYLQSIKNKRYGGPSSGAPPPS |
| P47 | HdADAVFTDNYTRLRKQVAAKKYLQSIKNKRYGGPSSGAPPPS |
| P48 | HdSDAVFTDNYTRLRKQVAAKKYLQSIKNKRYGGPSSGAPPPS |
| P49 | HPDAVFTDNYTRLRKQVAAKKYLQSIKNKRYGGPSSGAPPPS |
| P50 | HSDIVFTDNYTRLRKQVAAKKYLQSIKNKRYGGPSSGAPPPS |
| P51 | HSDYVFTDNYTRLRKQVAAKKYLQSIKNKRYGGPSSGAPPPS |
| P52 | HSDFVFTDNYTRLRKQVAAKKYLQSIKNKRYGGPSSGAPPPS |
| P53 | HSDVVFTDNYTRLRKQVAAKKYLQSIKNKRYGGPSSGAPPPS |
| P54 | HSDTVFTDNYTRLRKQVAAKKYLQSIKNKRYGGPSSGAPPPS |
| P55 | HSDLVFTDNYTRLRKQVAAKKYLQSIKNKRYGGPSSGAPPPS |
| P56 | HSDWVFTDNYTRLRKQVAAKKYLQSIKNKRYGGPSSGAPPPS |
| P58 | HSDAFFTDNYTRLRKQVAAKKYLQSIKNKRYGGPSSGAPPPS |
| P60 | HSDAIFTDNYTRLRKQVAAKKYLQSIKNKRYGGPSSGAPPPS |
| P61 | HSDALFTDNYTRLRKQVAAKKYLQSIKNKRYGGPSSGAPPPS |
| P62 | HSDATFTDNYTRLRKQVAAKKYLQSIKNKRYGGPSSGAPPPS |
| P63 | HSDAVITDNYTRLRKQVAAKKYLQSIKNKRYGGPSSGAPPPS |
| P64 | HSDAVLTDNYTRLRKQVAAKKYLQSIKNKRYGGPSSGAPPPS |
| P65 | HSDAVTTDNYTRLRKQVAAKKYLQSIKNKRYGGPSSGAPPPS |
| P66 | HSDAVVTDNYTRLRKQVAAKKYLQSIKNKRYGGPSSGAPPPS |
| P67 | HSDAVWTDNYTRLRKQVAAKKYLQSIKNKRYGGPSSGAPPPS |
| P68 | HSDAVYTDNYTRLRKQVAAKKYLQSIKNKRYGGPSSGAPPPS |
| P69 | HSDAWFTDNYTRLRKQVAAKKYLQSIKNKRYGGPSSGAPPPS |
| P70 | HSDAYFTDNYTRLRKQVAAKKYLQSIKNKRYGGPSSGAPPPS |
| P71 | HSDAVFTDNYTRLRRQVAARRYLQSIRNRRYGGPSSGAPPPS |
| P72 | HSDAVFTDNYTRLRRQVAAKKYLQSIKNKRYGGPSSGAPPPS |
| P73 | HSDAVFTDNYTRLRKQVAARKKYLQSIKNKRYGGPSSGAPPPS |
| P74 | HSDAVFTDNYTRLRKQVAAKKYLQSIQNKRYGGPSSGAPPPS |
| P75 | HSDAVFTDNYTRLRKQVAAKKYLQSIKNNRYGGPSSGAPPPS |
| P76 | HSDAVFTDNYTRLRKQVAAKKYLQSIKNKIYGGPSSGAPPPS |
| P82 | HSDAVFTDNYTRLRKQVAAKKYLQSIKRGGPSSGAPPPS |
| P83 | HSDAVFTDNYTRLRKQVAAKKYLQSIKNGGPSSGAPPPS |
| P84 | dHSDAVFTDNYTRLRKQVAAKKYLQSIKNKRYGGPSSGAPPPS |
| P85 | HSDAVFTDNYTRLRKQVAAKKYLQSIKKGGPSSGAPPPS |
| P87 | HSDAVFTDNYTREKEKVAAKKYLQSIKNKRYGGPSSGAPPPS |
| P88 | HSDAVFTDNYTRAAAKVAAKKYLQSIKNKRYGGPSSGAPPPS |
| P89 | HSDAVFTDNYTRLLAKVAAKKYLQSIKNKRYGGPSSGAPPPS |
| P92 | HSDAVFTDNYTRLRKQVAAKKYLQSIKNGRPSSGAPPPS |
| P93 | HSDAVFTDNYTRLLLKVAAKKYLQSIKNKRYGGPSSGAPPPS |
| P94 | HSDAVFTDNYTRAKAKVAAKKYLQSIKNKRYGGPSSGAPPPS |
| P98 | C6-HSDAVFTDNYTRLRRQVAARRYLQSIRNRRYGGPSSGAPPPS |
| P99 | C6-HVDAVFTDNYTRLRKQVAAKKYLQSIKNKRYGGPSSGAPPPS |
| P100 | M-HSDAVFTDQYTRLRKQVAAKKYLQSIKQKRYGGPSSGAPPPS |
| P101 | C6-HdADAVFTDNYTRLRKQVAAKKYLQSIKNKRYGGPSSGAPPPS |
| P102 | HSDGVFTDNYTRLRKQVAAKKYLQSIKNKRYGGPSSGAPPPS |
| P103 | C6-HSDAVFTDNYTKLKKQVAAKKYLQSIKNKKYGGPSSGAPPPS |
| P104 | M-HSDAVFTDNYTRLRKQVAAKKYLQSIKNKRYGGPSSGAPPPS |
| P105 | 1-HSDAVFTDNYTRLRKQVAAKKYLQSIKNKRYGGPSSGAPPPS |
| P106 | C6-HSDAVFTDNYTRLRKQVAAKKYLQSFKNKRYGGPSSGAPPPS |
| P107 | C6-HSDAVFTDNYTRLRKQVAAKKYLQSLKNKRYGGPSSGAPPPS |
| P108 | C6-HSDAVFTDNYTRLRKQVAAKKYLQSTKNKRYGGPSSGAPPPS |
| P 109 | C6-HSDAVFTDNYTRLRKQVAAKKYLQSVKNKRYGGPSSGAPPPS |
| P110 | C6-HSDAVFTDNYTRLRKQVAAKKYLQSWKNKRYGGPSSGAPPPS |
| P111 | C6-HSDAVFTDNYTRLRKQVAAKKYLQSYKNKRYGGPSSGAPPPS |
| P112 | C6-HSDAVFTDNYTRLRKQVAAKKYLQFIKNKRYGGPSSCAPPPS |
| P113 | C6-HSDAVFTDNYTRLRKQVAAKKYLQIIKNKRYGGPSSGAPPPS |
| P114 | C6-HSDAVFTDNYTRLRKQVAAKKYLQLIKNKRYGGPSSGAPPPS |
| P115 | C6-HSDAVFTDNYTRLRKQVAAKKYLQTIKNKRYGGPSSGAPPPS |
| P116 | C6-HSDAVFTDNYTRLRKQVAAKKYLQVIKNKRYGGPSSGAPPPS |
| P117 | C6-HSDAVFTDNYTRLRKQVAAKKYLQWIKNKRYGGPSSGAPPPS |
| P119 | C6-HSDAVFTDNYTRLLAKLALQKYLQSIKNKRYGGPSSGAPPPS |
| P120 | C6-HSDAVFTDNYTRLRKQVAAKKYLQSIKNKRYGGPSSGAPPPC |
| P121 | C6-HSDAVFTDNYTRLRKQVAAKKYLQSIKNKKGGPSSGAPPPS |
| P122 | C6-HSDAVFTDNYTRLRKQVAAKKYLQSIKNSRGGPSSGAPPPS |
| P123 | C6-HSDAVFTDNYTRLRKQVAAKKYLQSIKNKRGGPSSGAPPPS |
| P124 | C6-HSDAVFTDNYTRLRKQVAAKKYLQQIKNKRYGGPSSGAPPPS |
| P125 | C6-HSDAVFTDNYTRLRKQVAAKKYLQNIKNKRYGGPSSGAPPPS |
| P126 | HSDAVFTDNYTRLRKQVAAKKYLQSIKRGRPSSGAPPPS |
| P127 | C6-HSDAVFTDNYTRLRKQVAAKKYLQYIKNKRYGGPSSGAPPPS |
| P129 | C6-HSDAVFTDNYTRLRKQVAAKKWLQSIKNKRYGGPSSGAPPPS |
| P130 | C6-HSDAVFTDNYTRLRKQVAAKKFLQSIKNKRYGGPSSGAPPPS |
| P131 | C6-HSDAVFTDNYTRLRKQVAAKKTLQSIKNKRYGGPSSGAPPPS |
| P132 | C6-HSDAVFTDNYTRLRKQVAAKKLLQSIKNKRYGGPSSGAPPPS |
| P133 | C6-HSDAVFTDNYTRLRKQVAAKKILQSIKNKRYGGPSSGAPPPS |
| P134 | C6-HSDAVFTDNYTRLRKQVAAKKVLQSIKNKRYGGPSSGAPPPS |
| P135 | C6-HSDAVFTDNYTRLLAKVAAKKYLQSIKNKRYGGPSSGAPPPS |
| P138 | C6-HSDAVFTDNYTRLRAQVAAQKYLQSIKNKRYGGPSSGAPPPS |
| P139 | C6-HSDAVFTDNYTRLRAQVAAKKYLQSIKNKRYGGPSSGAPPPS |
| P140 | M-HSDAVFTDNYTRLLAKLALQKYLQSIKNKRYGGPSSGAPPPS |
| P141 | C6-HSDAVFTDNYTRLKAQVAAKKYLQSIKNKRYGGPSSGAPPPS |
| P142 | C6-HSDAVFTDNYTRLRAQLAAQKYLQSIKNKRYGGPSSGAPPPS |
| P143 | C6-HSDAVFTDNYTRLRKQMAAQKYLNQLKKGGPSSGAPPPS |
| P144 | C6-HSDAVFTDNYTRLRKQVAAQKYLNQLKKGGPSSGAPPPS |
| P146 | C6-HSDAVFTDNYTRLRKQVAAVKYLQSIKNKRYGGPSSGAPPPS |
| P147 | C6-HSDAVFTDNYTRLRKQVAAYKYLQSIKNKRYGGPSSGAPPPS |
| P148 | C6-HSDAVFTDNYTRLRKQVAAFKYLQSIKNKRYGGPSSGAPPPS |
| P149 | C6-HSDAVFTDNYTRLRKQVAAIKYLQSIKNKRYGGPSSGAPPPS |
| P150 | C6-HSDAVFTDNYTRLRKQVAAQKYLQSIKNKRYGGPSSGAPPPS |
| P151 | C6-HSDAVFTDNYTRLRKQVAALKYLQSIKNKRYGGPSSGAPPPS |
| P152 | C6-HSDAVFTDNYTRLRKQVAATKYLQSIKNKRYGGPSSGAPPPS |
| P153 | C6-HSDAVFTDNYTRLRKQVAAWKYLQSIKNKRYGGPSSGAPPPS |
| P154 | C6-HSDAVFTDNYTRLRKQVAAKKYLQSIKNGGPSSGAPPPS |
| P155 | C6-HSDAVFTDNYTRLRKQVALKKYLQSIKNKRYGGPSSGAPPPS |
| P158 | C6-HSDAVFTANYTRLRKQVAAKKYLQSIKNKRYGGPSSGAPPPS |
| P159 | C6-HSDAVFTENYTRLRKQVAAKKYLQSIKNKRYGGPSSGAPPPS |
| P160 | C6-HSDAVFTKNYTRLRKQVAAKKYLQSIKNKRYGGPSSGAPPPS |
| P161 | C6-HSDAVFTLNYTRLRKQVAAKKYLQSIKNKRYGGPSSGAPPPS |
| P162 | C6-HSDAVFTRNYTRLRKQVAAKKYLQSIKNKRYGGPSSGAPPPS |
| P163 | C6-HSDAVFTYNYTRLRKQVAAKKYLQSIKNKRYGGPSSGAPPPS |
| P164 | C6-HSDAVFTDNYTRLLAKLALQKYLQSIKNKRYGGPSSGAPPPC |
| P165 | C6-HSDAVFTEEYTRLQKQVAAKQYLQSIKNKRYGGPSSGAPPPS |
| P166 | C6-HAibDAVFTDNYTRLRKQVAAKKYLQSIKNKRYGGPSSGAPPPS |
| P167 | C6-HSDAVFTDNYTRLAibKQVAAKKYLQSIKNKRYGGPSSGAPPPS |
| P168 | C6-HSDAVFTDNYTRLRAibQVAAKKYLQSIKNKRYGGPSSGAPPPS |
| P169 | C6-HSDAVFTDNYTRLRKQVAAAibKYLQSIKNKRYGGPSSGAPPPS |
| P170 | C6-HSDAVFTDNYTRLRKQVAAKAibYLQSIKNKRYGGPSSGAPPPS |
| P171 | C6-HSDAVFTDNYTRLKKQVAAKKYLQSIKNKRYGGPSSGAPPPS |
| P172 | C6-HSDAVFTDNYTRLQKQVAAKKYLQSIKNKRYGGPSSGAPPPS |
| P173 | C6-HSDAVFTDNYTRLAKQVAAKKYLQSIKNKRYGGPSSGAPPPS |
| P174 | C6-HSDAVFTDNYTRLLKQVAAKKYLQSIKNKRYGGPSSGAPPPS |
| P175 | C6-HSDAVFTDNYTRLFKQVAAKKYLQSIKNKRYGGPSSGAPPPS |
| P176 | C6-HSDAVFTDNYTRLRRQVAAKKYLQSIKNKRYGGPSSGAPPPS |
| P177 | C6-HSDAVFTDNYTRLRQQVAAKKYLQSIKNKRYGGPSSGAPPPS |
| P179 | C6-HSDAVFTDNYTRLRLQVAAKKYLQSIKNKRYGGPSSGAPPPS |
| P180 | C6-HSDAVFTDNYTRLRFQVAAKKYLQSIKNKRYGGPSSGAPPPS |
| P181 | C6-HSDAVFTDNYTRLRKQVAAAKYLQSIKNKRYGGPSSGAPPPS |
| P182 | C6-HSDAVFTDNYTRLRKQVAAKRYLQSIKNKRYGGPSSGAPPPS |
| P183 | C6-HSDAVFTDNYTRLRKQVAAKQYLQSIKNKRYGGPSSGAPPPS |
| P184 | C6-HSDAVFTDNYTRLRKQVAAKAYLQSIKNKRYGGPSSGAPPPS |
| P185 | C6-HSDAVFTDNYTRLRKQVAAKLYLQSIKNKRYGGPSSGAPPPS |
| P186 | C6-HSDAVFTDNYTRLRKQVAAKFYLQSIKNKRYGGPSSGAPPPS |
| P187 | C6-HSDAVFTDNYTRLRKQVAAKKYLQAibIKNKRYGGPSSGAPPPS |
| P188 | C6-HSDAVFTDNYTRLRKQVAAKKYLQSAibKNKRYGGPSSGAPPPS |
| P189 | C6-HSDAVFTDNYTRLRKQAibAAKKYLQSIKNKRYGGPSSGAPPPS |
| P191 | C6-HHSDAVFTDNYTRLLAKLALQKYLQSIKNKRYGGPSSGAPPPS |
| P192 | C6-HSDAVFTDQYTRLLAKLALQKYLQSIKQKRYGGPSSGAPPPS |
| P193 | |
| P194 | |
| P195 | C6-HSDAVFTDNYTRLLAQLALQKYLQSIKNKRYGGPSSGAPPPS |
| P196 | C6-HSDAVFTDNYTRLLAKVALQKYLQSIKNKRYGGPSSGAPPPS |
| P197 | C6-HSDAVFTDNYTRLLAKLAAQKYLQSIKNKRYGGPSSGAPPPS |
| P198 | C6-HSDAVFTDNYTRLLAKLALQKYLQSIKNKGGPSSGAPPPC |
| P199 | Met(O)-HSDAVFTDNYTRLRKQVAAKKYLQSIKNKRYGGPSSGAPPPS |
| P203 | C6-HSDAVFTDNYTRLRKQVAAKKYLQSIKNKRFGGPSSGAPPPS |
| P205 | |
| P206 | |
| P207 | C6-HSDdAVFTDNYTRLRKQVAAKKYLQSIKNKRYGGPSSGAPPPS |
| P208 | C6-HSDNMeAVFTDNYTRLRKQVAAKKYLQSIKNKRYGGPSSGAPPPS |
| P209 | C6-HSDAibVFTDNYTRLRKQVAAKKYLQSIKNKRYGGPSSGAPPPS |
| P210 | C6-HSDAdVFTDNYTRLRKQVAAKKYLQSIKNKRYGGPSSGAPPPS |
| P211 | C6-HSDANMcVFTDNYTRLRKQVAAKKYLQSIKNKRYGGPSSGAPPPS |
| P212 | C6-HSDAAibFTDNYTRLRKQVAAKKYLQSIKNKRYGGPSSGAPPPS |
| P213 | C6-HSDAVFTDNYTRLRKQVAAKRYLQSIRNGGPSSGAPPPS |
| P214 | C6-HSDAVFTDNYTRLRKQVAARRYLQSIRNGGPSSGAPPPS |
| P215 | C6-HSDAVFTDNYTRLRRQVAAKRYLQSIRNGGPSSGAPPPS |
| P216 | C6-HSDAVFTDNYTRLRRQVAARKYLQSIRNGGPSSGAPPPS |
| P220 | C6-HSDAVFTDQYTRLRRQVAARKYLQSIRQGGPSSGAPPPS |
| P221 | C6-HSDIVFTDNYTRLRKQVAAKKYLQSIKNKRYGGPSSGAPPPS |
| P222 | C6-HGEGTFTDNYTRLRKQVAAKKYLQSIKNKRYGGPSSGAPPPS |
| P223 | C6-HSDLVFTDNYTRLRKQVAAKKYLQSIKNKRYGGPSSGAPPPS |
| P224 | C6-HSEAVFTDNYTRLRKQVAAKKYLQSIKNKRYGGPSSGAPPPS |
| P228 | C6-HSDAVFTDNYTRLRKQVAAKKAibLQSIKNKRYGGPSSGAPPPS |
| P229 | C6-HSDAVFTDNYTRLRKQVAAKKALQSIKNKRYGGPSSGAPPPS |
| P230 | C6-HSDAVFTDNYTRLRKQVAAKKYWQSIKNKRYGGPSSGAPPPS |
| P231 | C6-HSDAVFTDNYTRLRKQVAAKKYFQSIKNKRYGGPSSGAPPPS |
| P232 | C6-HSDAVFTDNYTRLRKQVAAKKYTQSIKNKRYGGPSSGAPPPS |
| P233 | C6-HSDAVFTDNYTRLRKQVAAKKYIQSIKNKRYGGPSSGAPPPS |
| P234 | C6-HSDAVFTDNYTRLRKQVAAKKYVQSIKNKRYGGPSSGAPPPS |
| P235 | C6-HSDAVFTDNYTRLRKQVAAKKYAQSIKNKRYGGPSSGAPPPS |
| P236 | C6-HSDAVFTDNYTRLRKQVAAKKYAibQSIKNKRYGGPSSGAPPPS |
| P240 | C6-HSDAVFTDNYTRLAibKQLAAAibKYLQSIKNKRYGGPSSGAPPPS |
| P241 | C6-HSDAVFTDNYTRLAibKQLAAKAibYLQSIKNKRYGGPSSGAPPPS |
| P242 | C6-HSDAVFTDNYTRLAibKQVAAAibKYLQSIKNKRYGGPSSGAPPPS |
| P243 | C6-HSDAVFTDNYTRLAibKQVAAQKYLQSIKNKRYGGPSSGAPPPS |
| P244 | C6-HSDAVFTDNYTRLAibKQVAAKAibYLQSIKNKRYGGPSSGAPPPS |
| P249 | C6-HSDAVFTDNYTRLAibKQVAAKQYLQSIKNKRYGGPSSGAPPPS |
| P250 | C6-HSDAVFTDNYTRLQKQVAAAibKYLQSIKNKRYGGPSSGAPPPS |
| P251 | C6-HSDAVFTDNYTRLQKQVAAKAibYLQSIKNKRYGGPSSGAPPPS |
| P252 | C6-HSDAVFTDNYTRLQKQVAAQKYLQSIKNKRYGGPSSGAPPPS |
| P253 | C6-HSDAVFTDNYTRLQKQVAAKQYLQSIKNKRYGGPSSGAPPPS |
| P258 | C6-HSDAVFTDNYTRLQAibQVAAKKYLQSIKNKRYGGPSSGAPPPS |
| P259 | C6-HSDAVFTDNYTRLAibKQVAALKYLQSIKNKRYGGPSSGAPPPS |
| P260 | C6-HSDAVFTDNYTRLAibKQVAAAKYLQSIKNKRYGGPSSGAPPPS |
| P261 | C6-HSDAVFTDNYTRLRAibQVAAAibKYLQSIKNKRYGGPSSGAPPPS |
| P262 | C6-HSDAVFTDNYTRLRAibQVAAVKYLQSIKNKRYGGPSSGAPPPS |
| P263 | C6-HSDAVFTDNYTRLRAibQVAAAKYLQSIKNKRYGGPSSGAPPPS |
| P264 | C6-HSDAVFTDNYTRLRAibQVAAKAibYLQSIKNKRYGGPSSGAPPPS |
| P265 | C6-HSDAVFTDNYTRLRAibQVAALKYLQSIKNKRYGGPSSGAPPPS |
| P269 | C6-HSDAVFTDNYTRLAibKQVAAVKYLQSIKNKRYGGPSSGAPPPS |
| P270 | C6-HSDAVFTDNYTRLRKQVAAKKYLQSIKQKGGPSSGAPPPS |
| P271 | C6-HSDAVFTDNYTRLRKQVAAKKYLQSIKNGRPSSGAPPPS |
| P275 | C6-HSDAVFTDNYTRLRKQVAGKKYLQSIKNKRYGGPSSGAPPPS |
| P282 | |
| P284 | C6-HSDAVFTDNYTRLRAibQLAAKAibYLQSIKNKRYGGPSSGAPPPS |
| P285 | |
| P289 | C6-HSDALFTDNYTRLRKQVAAKKYLQSIKNKRYGGPSSGAPPPS |
| P290 | C6-HSDAVFTDNYTRLRKQLAAKKYLQSIKNKRYGGPSSGAPPPS |
| P291 | C6-HSDAVFTDNYTRLRAibQLAAAibKYLQSIKNKRYGGPSSGAPPPS |
| P292 | C6-HSDAVFTDNYTRLRAibQLAAAibKYLQSIKNKGGPSSGAPPPS |
| P293 | C6-HSDAVFTDNYTRLAibKQVAAAibKYLQSIKQKGGPSSGAPPPS |
| P294 | C6-HSDAVFTDNYTRLRAibQVAAAibKYLQSIKQKGGPSSGAPPPS |
| P295 | C6-HSDAVFTDNYTRLRAibQVAAKAibYLQSIKQKGCPSSGAPPPS |
| P296 | C6-HSDAVFTDNYTRLAibKQVAAAibKYLQSIKQGGPSSGAPPPS |
| P297 | C6-HSDAVFTDNYTRLRAibQVAAAibKYLQSIKQGGPSSGAPPPS |
| P298 | C6-HSDAVFTDNYTRLRAibQVAAKAibYLQSIKQGGPSSGAPPPS |
| P299 | |
| P301 | C6-HSDAVFTDNYTRLAAibQVAAAibKYLQSIKNKRYGGPSSGAPPPS |
| P302 | C6-HSDAVFTDNYTRLQAibQVAAAibKYLQSIKNKRYGGPSSGAPPPS |
| P305 | C6-HSDAVFTDNYTRLhRKQVAAKKYLQSIKNKRYGGPSSGAPPPS |
| P307 | C6-HSDAVFTDNYTRLROmQVAAKKYLQSIKNKRYGGPSSGAPPPS |
| P308 | C6-HSDAVFTDNYTRLhROrnQVAAKKYLQSIKNKRYGGPSSGAPPPS |
| P314 | C6-HSEAVFTENYTRLRAibQVAAAibKYLQSIKNKRYGGPSSGAPPPS |
| P315 | C6-HSDAVFTDQYTRLRAibQVAAAibKYLQSIKQKRYGGPSSGAPPPS |
| P316 | C6-HSDAVFTDNYTRLhRAibQVAAAibKYLQSIKNKRYGGPSSGAPPPS |
| P317 | C6-HSDAVFTDNYTRLLAibQVAAAibKYLQSIKNKRYGGPSSGAPPPS |
| P318 | C6-HSDAVFTDNYTRLKAibQVAAAibKYLQSIKNKRYGGPSSGAPPPS |
| P319 | |
| P320 | C6-HSDAVFTDNYTRLCitAibQVAAAibKYLQSIKNKRYGGPSSGAPPPS |
| P321 | C6-HSDAVFTDNYTRLRAibKVAAAibKYLQSIKNKRYGGPSSGAPPPS |
| P322 | C6-HSDAVFTDNYTRLRAibQIAAAibKYLQSIKNKRYGGPSSGAPPPS |
| P323 | C6-HSDAVFTDNYTRLRAibQKAAAibKYLQSIKNKRYGGPSSGAPPPS |
| P324 | C6-HSDAVFTDNYTRLRAibQAAAAibKYLQSIKNKRYGGPSSGAPPPS |
| P325 | C6-HSDAVFTDNYTRLRAibQNleAAAibKYLQSIKNKRYGGPSSGAPPPS |
| P326 | |
| P327 | |
| P329 | C6-HSDAVFTDNYThRLRAibQVAAAibKYLQSIhRNhRGGPSSGAPPPS |
| P330 | |
| P332 | HSDAVFTDNYThRLRAibOVAAAibKLYLOSIhRhRGGPSSGAPPPS |
| P333 | |
| P335 | C6-HSDAVFTDNYTOrnLRAibQVAAAibKYLQSIhRhRGGPSSGAPPPS |
| P336 | |
| P338 | |
| P339 | |
| P341 | C6-HSDAVFTDNYTRLRAibQVAAAibAYLQSIKNKRYGGPSSGAPPPS |
| P342 | C6-HSDAVFTDNYTRLRAibQVAAAibOrnYLQSIKNKRYGGPSSGAPPPS |
| P344 | |
| P345 | C6-HSDAVFTDNYTAibLRAibQVAAAibKYLQSIKNKRYGGPSSGAPPPS |
| P346 | |
| P349 | C6-HSDAVFTDNYTRLRAibQVAAAibKYLQSIKAibKGGPSSGAPPPS |
| P350 | C6-HSDAVFTDNYTRLRAibQVAAAibKYLQSIKPKGGPSSGAPPPS |
| P351 | C6-HSDAVFTDNYTRLRAibQVAAAibKYLQSIKKGGPSSGAPPPS |
| P352 | C6-HSDAVFTDNYTRLRAibQVAAAibKYLQSIOrnOrnGGPSSGAPPPS |
| P353 | C6-HSDAVFTDNYTRLRAibQVAAAibKYLQSIdKdKGGPSSGAPPPS |
| P354 | C6-HSDAVFTDNYTRLRAibQVAAAibKYLQSIKhRGGPSSGAPPPS |
| P355 | C6-HSDAVFTDNYTRLRAibQVAAAibKYLQSIKAibGGPSSGAPPPS |
| P356 | C6-HSDAVFTDNYTRLRAibQVAAAibKYLQSIQrnQOrnGGPSSGAPPPS |
| P364 | |
| P365 | C6-HSDAVFTDNYTOrnLRAibQIAAAibKYLQSIOrnNOrnGGPSSGAPPPS |
| P366 | C6-HSDAVFTDNYTALRAibQVAAAibKYLQSIOrnNOrnGGPSSGAPPPS |

8. VPAC2 Rezeptorpeptidagonist nach einem der Ansprüche 1 bis 7 für die Verwendung als ein Arzneimittel.

9. VPAC2 Rezeptorpeptidagonist nach einem der Ansprüche 1 bis 7 für die Verwendung zur Behandlung von nicht insulinabhängigem Diabetes oder von insulinabhängigem Diabetes.

10. Verwendung eines VPAC2 Rezeptorpeptidagonisten nach einem der Ansprüche 1 bis 7 zur Herstellung eines Arzneimittels für die Behandlung von nicht insulinabhängigem Diabetes oder von insulinabhängigem Diabetes.

## Revendications

1. Agoniste peptidique du récepteur VPAC2 comprenant une séquence de la formule : dans laquelle :
Xaa₁ est : His, dH, ou est absent ;
Xaa₂ est : dA, Ser, Val, Gly, Thr, Leu, dS, Pro, ou Aib ;
Xaa₃ est : Asp ou Glu ;
Xaa₄ est : Ala, Ile, Tyr, Phe, Val, Thr, Leu, Trp, Gly, dA, Aib, ou NMeA ;
Xaa₅ est : Val, Leu, Phe, Ile, Thr, Trp, Tyr, dV, Aib, ou NMeV ;
Xaa₆ est : Phe, Ile, Leu, Thr, Val, Trp, ou Tyr ;
Xaa₈ est : Asp, Glu, Ala, Lys, Leu, Arg, ou Tyr ;
Xaa₉ est : Asn, Gln, ou Glu ;
Xaa₁₀ est : Tyr, Trp, ou Tyr(OMe) ;
Xaa₁₂ est : Arg, Lys, hR, Orn, Aib, Cit, ou Ala ;
Xaa₁₃ est : Leu, Phe, Glu, Ala, ou Aib ;
Xaa₁₄ est : Arg, Leu, Lys, Ala, hR, Orn, Phe, Gln, Aib, ou Cit ;
Xaa₁₅ est : Lys, Ala, Arg, Glu, Leu, hR, Orn, Phe, Gln, Aib, K(Ac), ou Cit ;
Xaa₁₆ est : Gln, Lys, Ala, hR, Orn, ou Cit ;
Xaa₁₇ est : Val, Ala, Leu, Ile, Met, Nle, Lys, ou Aib ;
Xaa₁₉ est : Ala, Gly, ou Leu ;
Xaa₂₀ est : Lys, Gln, hR, Arg, Ser, Orn, Ala, Aib, Trp, Thr, Leu, Ile, Phe, Tyr, Val, K(Ac), ou Cit ;
Xaa₂₁ est : Lys, Arg, Ala, Phe, Aib, Leu, Gln, Orn, hR, K(Ac) ou Cit ;
Xaa₂₂ est : Tyr, Trp, Phe, Thr, Leu, Ile, Val, Tyr(OMe), Ala, ou Aib ;
Xaa₂₃ est : Leu, Phe, Ile, Ala, Trp, Thr, Val, ou Aib ;
Xaa₂₄ est : Gln, ou Asn ;
Xaa₂₅ est : Ser, Asp, Phe, Ile, Leu, Thr, Val, Trp, Gln, Asn, Tyr, Aib, ou Glu ;
Xaa₂₆ est : Ile, Leu, Thr, Val, Trp, Tyr, Phe ou Aib ;
Xaa₂₇ est : Lys, hR, Arg, Gln, Orn, ou dK ;
Xaa₂₈ est : Asn, Gln, Lys, Arg, Aib, Orn, hR, Cit, Pro, ou dK ;
Xaa₂₉ est : Lys, Ser, Arg, Asn, hR, Orn, Cit, Aib ou est absent ;
Xaa₃₀ est : Arg, Lys, Ile, hR, Cit, Aib, Orn, ou est absent ;
Xaa₃₁ est : Tyr, His, Phe, ou est absent ; et
Xaa₃₂ est : Cys, ou est absent ;
à condition que si Xaa₂₉, Xaa₃₀, ou Xaa₃₁ est absent, l'acide aminé suivant présent en aval soit l'acide aminé suivant dans la séquence de l'agoniste peptidique,
et une extension C-terminale dans laquelle l'extrémité N de l'extension C-terminale est liée à l'extrémité C du peptide de formule 12 et dans lequel l'extension C-terminale comprend une séquence d'acides aminés de la formule :
Xaa₁-Xaa₂-Xaa₃-Xaa₄-Xaa₅-Xaa₆-Xaa₇-Xaa₈-Xaa₉-Xaa₁₀-Xaa₁₁ Formule 7 (SEQ ID NO: 15)
dans laquelle :
Xaa₁ est : Gly, Cys, ou absent ;
Xaa₂ est : Gly, Arg, ou absent ;
Xaa₃ est : Pro, Thr, ou absent ;
Xaa₄ est : Ser, ou absent ;
Xaa₅ est : Ser, ou absent ;
Xaa₆ est : Gly, ou absent ;
Xaa₇, est : Ala, ou absent ;
Xaa₈ est : Pro, ou absent ;
Xaa₉ est : Pro, ou absent ;
Xaa₁₀ est : Pro, ou absent; et
Xaa₁₁ est : Ser, Cys, ou absent;
à condition qu'au moins cinq des acides aminés Xaa₁ à Xaa₁₁ de l'extension C-terminale soient présents et à condition que si Xaa₁, Xaa₂, Xaa₃, Xaa₄, Xaa₅, Xaa₆, Xaa₇, Xaa₈, Xaa₉, ou Xaa₁₀ est absent, l'acide aminé suivant présent en aval soit l'acide aminé suivant dans l'extension C-terminale et dans laquelle l'acide aminé C-terminal puisse être amidé.

2. Agoniste peptidique du récepteur VPAC2 selon la revendication 1 comprenant une séquence de la formule : dans laquelle :
Xaa₂ est : dA, Ser, Val, dS, ou Aib ;
Xaa₃ est : Asp ou Glu ;
Xaa₄ est : Ala, dA, ou Aib ;
Xaa₅ est : Val, Leu, dV, ou Aib ;
Xaa₈ est : Asp, Glu, ou Ala ;
Xaa₉ est : Asn, Gln, ou Glu ;
Xaa₁₂ est : Ala, Arg, Lys, hR, ou Orn ;
Xaa₁₄ est : Arg, Leu, Lys, Ala, hR, Orn, Phe, Gln, Aib, ou Cit ;
Xaa₁₅ est : Lys, Ala, Arg, Leu, Orn, Phe, Gln, Aib, ou K(Ac) ;
Xaa₁₆ est : Gln, ou Lys ;
Xaa₁₇ est : Val, Ala, Leu, Ile, Met, Nle, ou Lys ;
Xaa₁₉ est : Ala, ou Leu ;
Xaa₂₀ est : Lys, Gln, hR, Arg, Ser, Ala, Aib, Trp, Thr, Leu, Ile, Phe, Tyr, Val, ou K(Ac) ;
Xaa₂₁ est : Lys, Arg, Ala, Phe, Aib, Leu, Gln, K(Ac), ou Orn ;
Xaa₂₂ est : Tyr, Trp, Phe, Leu, Ile, ou Val ;
Xaa₂₄ est : Gln, ou Asn ;
Xaa₂₅ est : Ser, Phe, Ile, Leu, Thr, Val, Trp, Gln, Asn, Tyr, ou Aib ;
Xaa₂₆ est : Ile, Leu, Thr, Val, Trp, Tyr, Phe ou Aib ;
Xaa₂₇ est : Lys, hR, Arg, dK, ou Orn ;
Xaa₂₈ est : Asn, Gln, Lys, hR, Aib, Orn, dK, ou Pro ;
Xaa₂₉ est : Lys, Ser, Arg, hR, Orn, ou est absent ;
Xaa₃₀ est : Arg, Lys, ou est absent ; et
Xaa₃₁ est : Tyr, Phe, ou est absent ;
à condition que si Xaa₂₉, ou Xaa₃₀ est absent, l'acide aminé suivant présent en aval soit l'acide aminé suivant dans la séquence de l'agoniste peptidique,
et une extension C-terminale dans laquelle l'extrémité N de l'extension C-terminale est liée à l'extrémité C du peptide de formule 13 et dans lequel l'extension C-terminale comprenne une séquence d'acides aminés de la formule :
Xaa₁-Xaa₂-Xaa₃-Xaa₄-Xaa₅-Xaa₆-Xaa₇-Xaa₈-Xaa₉-Xaa₁₀-Xaa₁₁ Formule 7 (SEQ ID NO: 15)
dans laquelle :
Xaa₁ est : Gly, Cys, ou absent ;
Xaa₂ est : Gly, Arg, ou absent ;
Xaa₃ est : Pro, Thr, ou absent ;
Xaa₄ est : Ser, ou absent ;
Xaa₅ est : Ser, ou absent ;
Xaa₆ est : Gly, ou absent ;
Xaa₇ est : Ala, ou absent ;
Xaa₈ est : Pro, ou absent ;
Xaa₉ est : Pro, ou absent ;
Xaa₁₀ est : Pro, ou absent ; et
Xaa₁₁ est : Ser, Cys, ou absent ;
à condition qu'au moins cinq des acides aminés Xaa₁ à Xaa₁₁ de l'extension C-terminale soient présents et à condition que si Xaa₁, Xaa₂, Xaa₃, Xaa₄, Xaa₅, Xaa₆, Xaa₇, Xaa₈, Xaa₉, ou Xaa₁₀ est absent, l'acide aminé suivant présent en aval soit l'acide aminé suivant dans l'extension C-terminale et dans laquelle l'acide aminé C-terminal puisse être amidé.

3. Agoniste peptidique du récepteur VPAC2 selon l'une quelconque des revendications précédentes, dans lequel l'extension C-terminale est choisie parmi :
| | |
|---|---|
| SEQ ID NO: 10 | GGPSSGAPPPS |
| SEQ ID NO: 11 | GGPSSGAPPPS-NH₂ |
| SEQ ID NO: 22 | GGPSSGAPPPC |
| SEQ ID NO: 23 | GGPSSGAPPPC-NH₂ |
| SEQ ID NO: 24 | GRPSSGAPPPS |
| SEQ ID NO: 25 | GRPSSGAPPPS-NH₂ |

4. Agoniste peptidique du récepteur VPAC2 selon l'une quelconque des revendications précédentes, comprenant en outre une modification N-terminale au niveau de l'extrémité N de l'agoniste peptidique dans lequel la modification N-terminale est choisie parmi :
a) l'ajout de D-histidine, d'isoleucine, de méthionine, ou de norleucine ;
b) l'ajout d'un peptide comprenant la séquence Ser-Trp-Cys-Glu-Pro-Gly-Trp-Cys-Arg dans laquelle l'Arg est liée à l'extrémité N de l'agoniste peptidique ;
c) l'ajout d'un groupe alkyle en C₁-C₁₆ éventuellement substitué par un ou plusieurs substituant(s) choisi(s), de manière indépendante, parmi un groupe aryle, alcoxy en C₁-C₆, -NH₂, -OH, un atome d'halogène et -CF₃ ;
d) l'ajout de -C(O)R¹ dans lequel R¹ représente un groupe alkyle en C₁-C₁₆ éventuellement substitué par un ou plusieurs substituant(s) choisi(s), de manière indépendante, parmi un groupe aryle, alcoxy en C₁-C₆, -NH₂, -OH, un atome d'halogène, un groupe -SH et -CF₃ ; un groupe aryle ou arylalkyle en C₁-C₄ éventuellement substitué par un ou plusieurs substituant(s) choisi(s), de manière indépendante, parmi un groupe alkyle en C₁-C₆, alcényle en C₂-C₆, alcynyle en C₂-C₆, alcoxy en C₁-C₆, -NH₂, -OH, un atome d'halogène et un groupe -CF₃ ; -NR²R³ dans lequel R² et R³ représentent, de manière indépendante, un atome d'hydrogène, un groupe alkyle en C₁-C₆, aryle ou arylalkyle en C₁-C₄ ou -OR⁴ dans lequel R⁴ représente un groupe alkyle en C₁-C₁₆ éventuellement substitué par un ou plusieurs substituant(s) choisi(s), de manière indépendante, parmi un groupe aryle, alcoxy en C₁-C₆, -NH₂, -OH, un atome d'halogène et un groupe -CF₃, aryle ou arylalkyle en C₁-C₄ éventuellement substitué par un ou plusieurs substituant(s) choisi(s), de manière indépendante, parmi un groupe alkyle en C₁-C₆, alcényle en C₂-C₆, alcynyle en C₂-C₆, alcoxy en C₁-C₆, -NH₂, -OH, un atome d'halogène et un groupe -CF₃ ;
e) l'ajout de -SO₂R⁵ dans lequel R⁵ représente un groupe aryle, arylalkyle en C₁-C₄ ou alkyle en C₁-C₁₆ ;
f) la formation d'un groupe succinimide éventuellement substitué par un groupe alkyle en C₁-C₆, ou -SR⁶, dans lequel R⁶ représente un atome d'hydrogène ou un groupe alkyle en C₁-C₆ ; et
g) l'ajout de méthionine sulfoxyde.

5. Agoniste peptidique du récepteur VPAC2 selon la revendication 4, dans lequel la modification N-terminale est l'ajout d'un groupe choisi parmi : l'acétyle, le propionyle, le butyryle, le pentanoyle, l'hexanoyle, la méthionine, la méthionine sulfoxyde, le 3-phénylpropionyle, le phénylacétyle, le benzoyle, la norleucine, la D-histidine, l'isoleucine et le 3-mercaptopropionyle.

6. Agoniste peptidique du récepteur VPAC2 selon la revendication 4 ou la revendication 5, dans lequel la modification N-terminale est l'ajout d'un groupe acétyle ou hexanoyle.

7. Agoniste peptidique du récepteur VPAC2 selon la revendication 1, comprenant une séquence d'acides aminés choisie parmi :
| | |
|---|---|
| Agoniste n° | Séquence |
| P6 | HSDAVFTDNYTRLRKQVAAKKYLQSIKNKRYGGPSSGAPPPS |
| P9 | HSDAVFTDNYTRLRKQVAAKKYLQSIKNKKGGPSSGAPPPS |
| P18 | HSDAVFTDNYTRLRKQVAAhRKYLQSIKNKRYGGPSSGAPPPS |
| P19 | HSDAVFTDNYTRLRKQVAAIKYLQSIKNKRYGGPSSGAPPPS |
| P20 | HSDAVTDNYTRLRKQVAARKYLQSIKNKRYGGPSSGAPPPS |
| P21 | HSDAVFTDNYTRLRKQVAASKYLQSIKNKRYGGPSSGAPPPS |
| P22 | HSDAVFTDNYTRLRKQVAAKKYLQSIhRNKRYGGPSSGAPPPS |
| P23 | HSDAVFTDNYTRLRKQVAAKKYLQSIRNKRYGGPSSGAPPPS |
| P24 | HSDAVFTDNYTRLRKQVAAKKYLQSIKNhRRYGGPSSGAPPPS |
| P25 | HSDAVFTDNYTRLRKQVAAKKYLQSIKNRRYGGPSSGAPPPS |
| P26 | HSDAVFTDNYTRLRKQVAAKKYLQSIKNRRYGGPSSGAPPPS |
| P27 | HSDAVFTDNWTRLRKQVAAKKYLQSIKNRRYGGPSSGAPPPS |
| P28 | HSDAVFTDNYTRLRKQVAAKKYLQSIKNRRYGGPSSGAPPPS |
| P31 | C6-HSDAVFTDNYTRLRKQVAAKKYLQSIKNKRYGGPSSGAPPPS |
| P33 | Ac-HSDAVFTDNYTRLRKQVAAKKYLQSIKNKRYGGPSSGAPPPS |
| P43 | HGDAVFTDNYTRLRKQVAAKKYLQSIKNKRYGGPSSGAPPPS |
| P44 | HVDAVFTDNYTRLRKQVAAKKYLQSIKNKRYGGPSSGAPPPS |
| P45 | HTDAVFTDNYTRLRKQVAAKKYLQSIKNKRYGGPSSGAPPPS |
| P46 | HLDAVFTDNYTRLRKQVAAKKYLQSIKNKRYGGPSSGAPPPS |
| P47 | HdADAVFTDNYTRLRKQVAAKKYLQSIKNKRYGGPSSGAPPPS |
| P48 | HdSDAVFTDNYTRLRKQVAAKKYLQSIKNKRYGGPSSGAPPPS |
| P49 | HPDAVFTDNYTRLRKQVAAKKYLQSIKNKRYGGPSSGAPPPS |
| P50 | HSDIVFTDNYTRLRKQVAAKKYLQSIKNKRYGGPSSGAPPPS |
| P51 | HSDYVFTDNYTRLRKQVAAKKYLQSIKNKRYGGPSSGAPPPS |
| P52 | HSDFVFTDNYTRLRKQVAAKKYLQSIKNKRYGGPSSGAPPPS |
| P53 | HSDVVFTDNYTRLRKQVAAKKYLQSIKNKRYGGPSSGAPPPS |
| P54 | HSDTVFTDNYTRLRKQVAAKKYLQSIKNKRYGGPSSGAPPPS |
| P55 | HSDLVFTDNYTRLRKQVAAKKYLQSIKNKRYGGPSSGAPPPS |
| P56 | HSDWVFTDNYTRLRKQVAAKKYLQSIKNKRYGGPSSGAPPPS |
| P58 | HSDAFFTDNYTRLRKQVAAKKYLQSIKNKRYGGPSSGAPPPS |
| P60 | HSDAIFTDNYTRLRKQVAAKKYLQSIKNKRYGGPSSGAPPPS |
| P61 | HSDALFTDNYTRLRKQVAAKKYLQSIKNKRYGGPSSGAPPPS |
| P62 | HSDATFTDNYTRLRKQVAAKKYLQSIKNKRYGGPSSGAPPPS |
| P63 | HSDAVITDNYTRLRKQVAAKKYLQSIKNKRYGGPSSGAPPPS |
| P64 | HSDAVLTDNYTRLRKQVAAKKYLQSIKNKRYGGPSSGAPPPS |
| P65 | HSDAVTTDNYTRLRKQVAAKKYLQSIKNKRYGGPSSGAPPPS |
| P66 | HSDAVVTDNYTRLRKQVAAKKYLQSIKNKRYGGPSSGAPPPS |
| P67 | HSDAVWTDNYTRLRKQVAAKKYLQSIKNKRYGGPSSGAPPPS |
| P68 | HSDAVYTDNYTRLRKQVAAKKYLQSIKNKRYGGPSSGAPPPS |
| P69 | HSDAWFTDNYTRLRKQVAAKKYLQSIKNKRYGGPSSGAPPPS |
| P70 | HSDAYFTDNYTRLRKQVAAKKYLQSIKNKRYGGPSSGAPPPS |
| P71 | HSDAVFTDNYTRLRRQVAARRYLQSIRNRRYGGPSSGAPPPS |
| P72 | HSDAVFTDNYTRLRRAVAAKKYLQSIKNKRYGGPSSGAPPPS |
| P73 | HSDAVFTDNYTRLRKQVAARKYLQSIKNKRYGGPSSGAPPPS |
| P74 | HSDAVFTDNYTRLRKQVAAKKYLQSIQNKRYGGPSSGAPPPS |
| P75 | HSDAVFTDNYTRLRKQVAAKKYLQSIKNNRYGGPSSGAPPPS |
| P76 | HSDAVFTDNYTRLRKQVAAKKYLQSIKNKIYGGPSSGAPPPS |
| P82 | HSDAVFTDNYTRLRKQVAAKKYLQSIKRGGPSSGAPPPS |
| P83 | HSDAVFTDNYTRLRKQVAAKKYLQSIKNGGPSSGAPPPS |
| P84 | dHSDAVFTDNYTRLRKQVAAKKYLQSIKNKRYGGPSSGAPPPS |
| P85 | HSDAVFTDNYTRLRKQVAAKKYLQSIKKGGPSSGAPPPS |
| P87 | HSDAVFTDNYTREKEKVAAKKYLQSIKNKRYGGPSSGAPPPS |
| P88 | HSDAVFTDNYTRAAKVAAKKYLQSIKNKRYGGPSSGAPPPS |
| P89 | HSDAVFTDNYTRLLAKVAAKKYLQSIKNKRYGGPSSGAPPPS |
| P92 | HSDAVFTDNYTRLRKQVAAKKYLQSIKNGPPSSGAPPPS |
| P93 | HSDAVFTDNYTRLLLKVAAKKYLQSIKNKRYGGPSSGAPPPS |
| P94 | HSDAVFTDNYTRAKAKVAAKKYLQSIKNKRYGGPSSGAPPPS |
| P98 | C6-HSDAVFTYDNYRLRRQVAARRYLQSIRNRRYGGPSSGAPPPS |
| P99 | C6-HVDAVFTDNYTRLRKQVAAKKYLQSIKNKRYGGPSSGAPPPS |
| P100 | M-HSDAVFTDQYTRLRKQVAAKKYLQSIKQKRYGGPSSGAPPPS |
| P101 | C6-HdADAVFTDNYTRLRKQVAAKKYLQSIKNKRYGGPSSGAPPPS |
| P102 | HSDGVFTDNYTRLRKQVAAKKYLQSIKNKRYGGPSSGAPPPS |
| P103 | C6-HSDAVFTDNYTKLKKQVAAKKYLQSIKNKKYGGPSSGAPPPS |
| P104 | M-HSDAVFTDNYTRLRKQVAAKKYLQSIKNKRYGGPSSGAPPPS |
| P105 | 1-HSDAVFTDNYTRIRKQVAAKKYLQSFKNKRYGGPSSGAPPPS |
| P106 | C6-HSDAVFTDNYTRLRKQVAAKKYLQSFKNKRYGGPSSGAPPPS |
| P107 | C6-HSDAVFTDNYTRLRKQVAAKKYLQSLKNKRYGGPSSGAPPPS |
| P108 | C6-HSDAVFTDNYTRLRKQVAAKKYLQSTKNKRYGGPSSGAPPPS |
| P109 | C6-HSDAVFTDNYTRLRKQVAAKKYLQSVKNKRYGGPSSGAPPPS |
| P110 | C6-HSDAVFTDNYTRLRKQVAAKKYLQSWKNKRYGGPSSGAPPPS |
| P111 | C6-HSDAVFTDNYTRLRKQVAAKKYLQSYKNKRYGGPSSGAPPPS |
| P112 | C6-HSDAVFTDNYTRLRKQVAAKKYLQFIKNKRYGGPSSGAPPPS |
| P113 | C6-HSDAVFTDNYTRLRKQVAAKKYLQIIKNKRYGGPSSGAPPPS |
| P114 | C6-HSDAVFTDNYTRLRKQVAAKKYLQLIKNKRYGGPSSGAPPPS |
| P115 | C6-HSDAVFTDNYTRLRKQVAAKKYLQTINKKRYGGPSSGAPPPS |
| P116 | C6-HSDAVFTDNYTRLRKQVAAKKYLQVIKNKRYGGPSSGAPPPS |
| P117 | C6-HSDAVFTDNYTRLRKQVAAKKYLQWIKNKRYGGPSSGAPPPS |
| P119 | C6-HSDAVFTDNYTRLLAKLALQKYLQSIKNKRYGGPSSGAPPPS |
| P120 | C6-HSDAVFTDNYTRLRKQVAAKKYLQSIKNKRYGGPSSGAPPPS |
| P121 | C6-HSDAVFTDNYTTRLRKQVAAKKYLQSIKNKKGGPSSGAPPPS |
| P122 | C6-HSDAVFTDNYTRLRKQVAAKKYLQSIKNSRGGPSSGAPPPS |
| P123 | C6-HSDAVFTDNYTRLRKQVAAKKYLQSIKNKRGGPSSGAPPPS |
| P124 | C6-HSDAVFTDNYTRLRKQVAAKKYLQQIKNKRYGGSSGAPPPS |
| P125 | C6-HSDAVFTDNYTRLRKQVAAKKYLQNIKNKRYGGPSSGAPPPS |
| P126 | HSDAVFTDNYTRLRKQVAAKKYLQSIKRGRPSSGAPPPS |
| P127 | C6-HSDAVFTDNYTRLRKQVAAKKYLQYLKNKRYGGPSSGAPPPS |
| P129 | C6-HSDAVFTDNYTRLRKQVAAKKWLQSIKNKRYGGPSSGAPPPS |
| P130 | C6-HSDAVFTDNYTRLRKQVAAKKFLQSIKNKRYGGPSSGAPPPS |
| P131 | C6-HSDAVFTDNYTRLRKQVAAKKTLQSIKNKRYGGPSSGAPPPS |
| P132 | C6-HSDAVFTDNYTRLRKQVAAKKLLQSIKNKRYGGPSSGAPPPS |
| P133 | C6-HSDAVFTDNYTRLRKQVAAKKILQSIKNKRYGGPSSGAPPPS |
| P134 | C6-HSDAVFTDNYTRLRKQVAAKKVLQSIKNKRYGGPSSGAPPPS |
| P135 | C6-HSDAVFTDNYTRLLAKVAAKKYLQSIKNKRYGGPSSGAPPPS |
| P138 | C6-HSDAVFTDNYTRLRAQVAAQKYLQSIKNKRYGGPSSGAPPPS |
| P139 | C6-HSDAVFTDNYTRLRAQVAAKKYLQSIKNKRYGGPSSGAPPPS |
| P140 | M-HSDAVFTDNYTRLLAKLALQKYLQSIKNKRYGGPSSGAPPPS |
| P141 | C6-HSDAVFTDNYTRLKAQVAAKKYLQSIKNKRYGGPSSGAPPPS |
| P142 | C6-HSDAVFTDNYTRLRAQLAAQKYLQSIKNKRYGGPSSGAPPPS |
| P143 | C6-HSDAVFTDNYTRLRKQMAAQKYLNQLKKGGPSSGAPPPS |
| P144 | C6-HSDAVFTDNYTRLRKQVAAQKYLNQLKKGGPSSGAPPPS |
| P146 | C6-HSDAVFTDNYTRLRKQVAAVKYLQSIKNKRYGGPSSGAPPPS |
| P147 | C6-HSDAVFTDNYTRLRKQVAAYKYLQSIKNKRYGGPSSGAPPPS |
| P148 | C6-HSDAVFTDNYTRLRKQVAAFKYLQSIKNKRYGGPSSGAPPPS |
| P149 | C6-HSDAVFTDNYTRLRKQVAAIKYLQSIKNKRYGGPSSGAPPPS |
| P150 | C6-HSDAVFTDNYTRLRKQVAALKYLQSIKNKRYGGPSSGAPPPS |
| P151 | C6-HSDAVFTDNYTRLRKQVAALKYLQSIKNKRYGGPSSGAPPPS |
| P152 | C6-HSDAVFTDNYTRLRKQVAATKYLQSIKNKRYGGPSSGAPPPS |
| P153 | C6-HSDAVFTDNYTRLRKQVAAWKYLQSIKNKRYGGPSSGAPPPS |
| P154 | C6-HSDAVFTDNYTRLRKQVAAKKYLQSIKNGGPSSGAPPPS |
| P155 | C6-HSDAVFTDNYTRLRKQVALKKYLQSIKNKRYGGPSSGAPPPS |
| P158 | C6-HSDAVFTANYTRLRKQVAAKKYLQSIKNKRYGGPSSGAPPPS |
| P159 | C6-HSDAVFTENYTRLRKQVAAKKYLQSIKNKRYGGPSSGAPPPS |
| P160 | C6-HSDAVFTKNYTRLRKQVAAKKYLQSIKNKRYGGPSSGAPPPS |
| P161 | C6-HSDAVFTLNYTRLRKQVAAKKYLQSIKNKRYGGPSSGAPPPS |
| P162 | C6-HSDAVFTRNYTRLRKQVAAKKYLQSIKNKRYGGPSSGAPPPS |
| P163 | C6-HSDAVFTYNYTRLRKQVAAKKYLQSIKNKRYGGPSSGAPPPS |
| P164 | C6-HSDAVFTDNYTRLLAKLALQKYLQSIKNKRYGGPSSGAPPPS |
| P165 | C6-HSDAVFTEEYTRLQKQVAAKQYLQSIKNKRYGGPSSGAPPPS |
| P166 | C6-HAibDAVFTDNYTRLRKQVAAKKYLQSIKNKRYGGPSSGAPPPS |
| P167 | C6-HSDAVFTDNYTRLAibKQVAAKKYLQSIKNKRYGGPSSGAPPPS |
| P168 | C6-HSDAVFTDNYTRLRAibQVAAKKYLQSIKNKRYGGPSSGAPPPS |
| P169 | C6-HSDAVFTDNYTRLRKQVAAAibKYLQSIKNKRYGGPSSGAPPPS |
| P170 | C6-HSDAVFTDNYTRLRKQVAAKAibYLQSIKNKRYGGPSSGAPPPS |
| P171 | C6-HSDAVFTDNYTRLKKQVAAKKYLQSIKNKRYGGPSSGAPPPS |
| P172 | C6-HSDAVFTDNYTRLQKQVAAKKYLQSIKNKRYGGPSSGAPPPS |
| P173 | C6-HSDAVFTDNYTRLAKQVAAKKYLQSIKNKRYGGPSSGAPPPS |
| P174 | C6-HSDAVFTDNYTRLLKQVAAKKYLQSIKNKRYGGPSSGAPPPS |
| P175 | C6-HSDAVFTDNYTRLFKQVAAKKYLQSIKNKRYGGPSSGAPPPS |
| P176 | C6-HSDAVFTDNYTRLRRQVAAKKYLQSIKNKRYGGPSSGAPPPS |
| P177 | C6-HSDAVFTDNYTRLRQQVAAKKYLQSIKNKRYGGPSSGAPPPS |
| P179 | C6-HSDAVFTDNYTRLRLQVAAKKYLQSIKNKRYGGPSSGAPPPS |
| P180 | C6-HSDAVFTDNYTRLRFQVAAKKYLQSIKNKRYGGPSSGAPPPS |
| P181 | C6-HSDAVFTDNYTRLRKQVAAAKYLQSIKNKRYGGPSSGAPPPS |
| P182 | C6-HSDAVFTDNYTRLRKQVAAKRYLQSIKNKRYGGPSSGAPPPS |
| P183 | C6-HSDAVFTDNYTRLRKQVAAKQYLQSIKNKRYGGPSSGAPPPS |
| P184 | C6-HSDAVFTDNYTRLRKQVAAKAYLQSIKNKRYGGPSSGAPPPS |
| P185 | C6-HSDAVFTDNYTRLRKQVAAKLYLQSIKNKRYGGPSSGAPPPS |
| P186 | C6-HSDAVFTDNYTRLRKQVAAKFYLQSIKNKRYGGPSSGAPPPS |
| P187 | C6-HSDAVFTDNYTRLRKQVAAKKYLQAibIKNKRYGGPSSGAPPPS |
| P188 | C6-HSDAVFTDNYTRLRKQVAAKKYLQSAibKNKRYGGPSSGAPPPS |
| P189 | C6-HSDAVFTDNYTRLRKQAibAAKKYLQSIKNKRYGGPSSGAPPPS |
| P191 | C6-HHSDAVFTDNYTRLLAKLALQKYLQSIKNKRYGGPSSGAPPPS |
| P192 | C6-HSDAVFTDQYTRLLAKLALQKYLQSIKQKRYGGPSSGAPPPS |
| P193 | |
| P194 | |
| P195 | C6-HSDAVFTDNYTRLLAQLALQKYLQSIKNKRYGGPSSGAPPPS |
| P196 | C6-HSDAVFTDNYTRLLAKVALQKYLQSIKNKRYGGPSSGAPPPS |
| P197 | C6-HSDAVFTDNYTRLLAKLAAQKYLQSIKNKRYGGPSSGAPPPS |
| P198 | C6-HSDAVFTDNYTRLLAKLALQKYLQSIKNKGGPSSGAPPPC |
| P199 | Met(O)-HSDAVFTDNYTRLRKQVAAKKYLQSIKNKRYGGPSSGAPPPS |
| P203 | C6-HSDAVFTDNYTRLRKQVAAKKYLQSIKNKRFGGPSSGAPPPS |
| P205 | |
| P206 | |
| P207 | C6-HSDdAVFTDNYTRLRKQVAAKKYLQSIKNKRYGGPSSGAPPPS |
| P208 | C6-HSDNMeAVFTDNYTRLRKQVAAKKYLQSIKNKRYGGPSSGAPPPS |
| P209 | C6-HSDAibVFTDNYTRLRKQVAAKKYLQSIKNKRYGGPSSGAPPPS |
| P210 | C6-HSDAdVFTDNYTRLRKQVAAKKYLQSIKNKRYGGPSSGAPPPS |
| P211 | C6-HSDANMeVFTDNYTRLRKQVAAKKYLQSIKNKRYGGPSSGAPPPS |
| P212 | C6-HSDAAibFTDNYTRLRKQVAAKKYLQSIKNKRYGGPSSGAPPPS |
| P213 | C6-HSDAVFTDNYTRLRKQVAAKRYLQSIRNGGPSSGAPPPS |
| P214 | C6-HSDAVFTDNYTRLRKQVAARRYLQSIRNGGPSSGAPPPS |
| P215 | C6-HSDAVFTDNYTRLRRQVAAKRYLQSIRNGGPSSGAPPPS |
| P216 | C6-HSDAVFTDNYTRLRRQVAARKYLQSIRNGGPSSGAPPPS |
| P220 | C6-HSDAVFTDQYTRLRRQVAARKYLQSIRQGGPSSGAPPPS |
| P221 | C6-HSDIVFTDNYTRLRKQVAAKKYLQSIKNKRYGGPSSGAPPPS |
| P222 | C6-HGEGTFTDNYTRLRKQVAAKKYLQSIKNKRYGGPSSGAPPPS |
| P223 | C6-HSDLVFTDNYTRLRKQVAAKKYLQSIKNKRYGGPSSGAPPPS |
| P224 | C6-HSEAVFTDNYTRLRKQVAAKKYLQSIKNKRYGGPSSGAPPPS |
| P228 | C6-HSDAVFTDNYTRLRKQVAAKKAZibLQSIKNKRYGGPSSGAPPPS |
| P229 | C6-HSDAVFTDNYTRLRKQVAAKKALQSIKNKRYGGPSSGAPPPS |
| P230 | C6-HSDAVFTDNYTRLRKQVAAKKYWQSIKNKRYGGPSSGAPPPS |
| P231 | C6-HSDAVFTDNYTRLRKQVAAKKYFQSIKNKRYGGPSSGAPPPS |
| P232 | C6-HSDAVFTDNYTRLRKQVAAKKYTQSIKNKRYGGPSSGAPPPS |
| P233 | C6-HSDAVFTDNYTRLRKQVAAKKYIQSIKNKRYGGPSSGAPPPS |
| P234 | C6-HSDAVFTDNYTRLRKQVAAKKYVQSIKNKRYGGPSSGAPPPS |
| P235 | C6-HSDAVFTDNYTRLRKQVAAKKYAQSIKNKRYGGPSSGAPPPS |
| P236 | C6-HSDAVFTDNYTRLRKQVAAKKYAibQSIKNKRYGGPSSGAPPPS |
| P240 | C6-HSDAVFTDNYTRLAibKQLAAAibKYLQSIKNKRYGGPSSGAPPPS |
| P241 | C6-HSDAVFTDNYTRLAibKQLAAKAibYLQSIKNKRYGGPSSGAPPPS |
| P242 | C6-HSDAVFTDNYTRLAibKQVAAAibKYLQSIKNKRYGGPSSGAPPPS |
| P243 | C6-HSDAVFTDNYTRLAibKQVAAQKYLQSIKNKRYGGPSSGAPPPS |
| P244 | C6-HSDAVFTDNYTRLAibKQVAAKAibYLQSIKNKRYGGPSSGAPPPS |
| P249 | C6-HSDAVFTDNYTRLAibKQVAAKQYLQSIKNKRYGGPSSGAPPPS |
| P250 | C6-HSDAVFTDNYTRLQKQVAAAibKYLQSIKNKRYGGPSSGAPPPS |
| P251 | C6-HSDAVFTDNYTRLQKQVAAKAibYLQSIKNKRYGGPSSGAPPPS |
| P252 | C6-HSDAVFTDNYTRLQKQVAAQKYLQSIKNKRYGGPSSGAPPPS |
| P253 | C6-HSDAVFTDNYTRLQKQVAAKQYLQSIKNKRYGGPSSGAPPPS |
| P258 | C6-HSDAVFTDNYTRLQAibQVAAKKYLQSIKNKRYGGPSSGAPPPS |
| P259 | C6-HSDAVFTDNYTRLAibKQVAALKYLQSIKNKRYGGPSSGAPPPS |
| P260 | C6-HSDAVFTDNYTRLAibKQVAAAKYLQSIKNKRYGGPSSGAPPPS |
| P261 | C6-HSDAVFTDNYTRLRAibQVAAAibKYLQSIKNKRYGGPSSGAPPPS |
| P262 | C6-HSDAVFTDNYTRLRAIBQVAAVKYLQSIKNKRYGGPSSGAPPPS |
| P263 | C6-HSDAVFTDNYTRLRibQVAAAKYLQSIKNKRYGGPSSGAPPPS |
| P264 | C6-HSDAVFTDNYTRLRAibQVAAKAibYLQSIKNKRYGGPSSGAPPPS |
| P265 | C6-HSDAVFTDNYTRLRAibQVAALKYLQSIKNKRYGGPSSGAPPPS |
| P269 | C6-HSDAVFTDNYTRLAibKQVAAVKYLQSIKNKRYGGPSSGAPPPS |
| P270 | C6-HSDAVFTDNYTRLRKQVAAKKYLQSIKQKGGPSSGAPPPS |
| P271 | C6-HSDAVFTDNYTRLRKQVAAKKYLQSIKNGRPSSGAPPPS |
| P275 | C6-HSDAVFTDNYTRLRKQVAGKKYLQSIKNKRYGGPSSGAPPPS |
| P282 | |
| P284 | C6-HSDAVFTDNYTRLRAibQLAAKAibYLQSINKNRYGGPSSGAPPPS |
| P285 | |
| P289 | C6-HSDALFTDNYTRLRKQVAAKKYLQSIKNKRYGGPSSGAPPPS |
| P290 | C6-HSDAVFTDNYTRLRKQLAAKKYLQSIKNKRYGGPSSGAPPPS |
| P291 | C6-HSDAVFTDNYTRLRAibQLAAAibKYLQSIKNKRYGGPSSGAPPPS |
| P292 | C6-HSDAVFTDNYTRLRAibQLAAAibKYLQSIKNKGGPSSGAPPPS |
| P293 | C6-HSDAVFTDNYTRLAibKQVAAAibKYLQSIKQKGGPSSGAPPPS |
| P294 | C6-HSDAVFTDNYTRLRAibQVAAAibKYLQSIKQKGGPSSGAPPPS |
| P295 | C6-HSDAVFTDNYTRLRAibQVAAKAibYLQSIKQKGGPSSGAPPPS |
| P296 | C6-HSDAVFTDNYTRLAibKQVAAAibKYLQSIKQGGPSSGAPPPS |
| P297 | C6-HSDAVFTDNYTRLRAibQVAAAibKYLQSIKQGGPSSGAPPPS |
| P298 | C6-HSDAVFTDNYTRLRAibQVAAKAibYLQSIKQGGPSSGAPPPS |
| P299 | |
| P301 | C6-HSDAVFTDNYTRLAAibQVAAAibKYLQSIKNKRYGGPSSGAPPPS |
| P302 | C6-HSDAVFTDNYTRLQAibQVAAAibKYLQSIKNKRYGGPSSGAPPPS |
| P305 | C6-HSDAVFTDNYTRLhRKQVAAKKYLQSIKNKRYGGPSSGAPPPS |
| P307 | C6-HSDAVFTDNYTRLROrnQVAAKKYLQSIKNKRYGGPSSGAPPPS |
| P308 | C6-HSDAVFTDNYTRLhROmQVAAKKYLQSIKNKRYGGPSSGAPPPS |
| P314 | C6-HSEAVFTENYTRLRAibQVAAAibKYLQSIKNKRYGGPSSGAPPPS |
| P315 | C6-HSDAVFTDQYTRLRAibQVAAAibKYLQSIKQKRYGGPSSGAPPPS |
| P316 | C6-HSDAVFTDNYTRLhRAibQVAAAibKYLQSIKNKRYGGPSSGAPPPS |
| P317 | C6-HSDAVFTDNYTRLLAibQVAAAibKYLQSIKNKRYGGPSSGAPPPS |
| P318 | C6-HSDAVFTDNYTRLKAIBQVAAAibKYLLQSIKNKRYGGPSSGAPPPS |
| P319 | |
| P320 | C6-HSDAVFTDNYTRLCitAibQVAAAibKYLQSIKNKRYGGPSSGAPPPS |
| P321 | C6-HSDAVFTDNYTRLRAibKVAAAibKYLQSIKNKRYGGPSSGAPPPS |
| P322 | C6-HSDAVFTDNYTRLRAibQIAAAibKYLQSIKNKRYGGPSSGAPPPS |
| P323 | C6-HSDAVFTDNYTRLRAibQKAAAibKYLQSIKNKRYGGPSSGAPPPS |
| P324 | C6-HSDAVFTDNYTRLRAibQAAAAibKYLQSIKNKRYGGPSSGAPPPS |
| P325 | C6-HSDAVFTDNYTRLRAibQNleAAAibKYLQSIKNKRYGGPSSGAPPPS |
| P326 | |
| P327 | |
| P329 | C6-HSDAVFTDNYThRLRAibQVAAAibKYLQSlhRNhRGGPSSGAPPPS |
| P330 | |
| P332 | HSDAVFTDNYThRLRAibQVAAAibKYLQSIhRhRGGPSSGAPPPS |
| P333 | |
| P335 | C6-HSDAVFTDNYTRmLRAibQVAAAibKYLQSlhRhRGGPSSGAPPPS |
| P336 | |
| P338 | |
| P339 | |
| P341 | C6-HSDAVFTDNYTRLRAibQVAAAibQYLQSIKNKRYGGPSSGAPPPS |
| P342 | C6-HSDAVFTDNYTRLRAibQVAAAibOrnYLQSIKNKRYGGPSSGAPPPS |
| P344 | |
| P345 | C6-HSDAVFTDNYTAibLRAibQVAAAibKYLQSIKNKRYGGPSSGAPPPS |
| P346 | |
| P349 | C6-HSDAVFTDNYTRLRAibQVAAAIBKYLQSIKAibKGGPSSGAPPPS |
| P350 | C6-HSDAVFTDNYTRLRAibQVAAAibKYLQSIKPKGGPSSGAPPPS |
| P352 | C6-HSDAVFTDNYTRLRAibQVAAAibKYLQSIKKGGPSSGAPPPS |
| P352 | C6-HSDAVFTDNYTRLRAibQVAAAIBKYLQSIOrnOrnGGPSSGAPPPS |
| P353 | C6-HSDAVFTDNYTRLRAibQVAAAibKYLQSIdKdKGGPSSGAPPPS |
| P354 | C6-HSDAVFTDNYTRLRAibQVAAAibKYLQSIKhRGGPSSGAPPPS |
| P355 | C6-HSDAVFTDNYTRLRAibQVAAAibKYLQSIKAibGGPSSGAPPPS |
| P356 | C6-HSDAVFTDNYTRLRAibQVAAAIbKYLQSIOrnQOrnGGPSSGAPPPS |
| P364 | |
| P365 | C6-HSDAVFTDNYTOrnLRAibQIAAAibKYLQSIOrnNOrnGGPSSGAPPPS |
| P366 | C6-HSDAVFTDNYTRARAibQVAAAibKYLQSIOrnNOrnGGPSSGAPPPS |

8. Agoniste peptidique du récepteur VPAC2 selon l'une quelconque des revendications 1 à 7 pour une utilisation en tant que médicament.

9. Agoniste peptidique du récepteur VPAC2 selon l'une quelconque des revendications 1 à 7 pour une utilisation dans le traitement du diabète non insulino-dépendant ou du diabète insulino-dépendant.

10. Utilisation d'un agoniste peptidique du récepteur VPAC2 selon l'une quelconque des revendications 1 à 7 dans la fabrication d'un médicament destiné au traitement du diabète non insulino-dépendant ou du diabète insulino-dépendant.


### SEQUENCE LISTING

<110> Eli Lilly and company
<120> Selective VPAC2 Receptor Peptide Agonists
<130> x16803
<160> 620
<170> PatentIn version 3.3
<210> 1
   <211> 28
   <212> PRT
   <213> Artificial
<220>
   <223> Synthetic construct
<400> 1
   His Ser Asp Ala Val Phe Thr Asp Asn Tyr Thr Arg Leu Arg Lys Gln
   1               5                   10                  15
   Met Ala Val Lys Lys Tyr Leu Asn Ser Ile Leu Asn
               20                  25
<210> 2
   <211> 27
   <212> PRT
   <213> Artificial
<220>
   <223> Synthetic construct
<220>
   <221> MOD_RES
   <222> (27)..(27)
   <223> AMIDATION
<400> 2
   His Ser Asp Gly Ile Phe Thr Asp Ser Tyr Ser Arg Tyr Arg Lys Gln
   1               5                   10                  15
   Met Ala Val Lys Lys Tyr Leu Ala Ala Val Leu
               20                  25
<210> 3
   <211> 37
   <212> PRT
   <213> Artificial
<220>
   <223> synthetic construct
<220>
   <221> MOD_RES
   <222> (37)..(37)
   <223> AMIDATION
<400> 3
   His Ser Asp Gly Ile Phe Thr Asp Ser Tyr Ser Arg Tyr Arg Lys Gln
   1               5                   10                  15
   Met Ala Val Lys Lys Tyr Leu Ala Ala Val Leu Gly Lys Arg Tyr Gln
               20                  25                  30
   Arg Val Lys Asn Lys
           35
<210> 10
   <211> 11
   <212> PRT
   <213> Artificial
<220>
   <223> synthetic construct
<400> 10
   Gly Gly Pro Ser Ser Gly Ala Pro Pro Pro Ser
   1               5                   10
<210> 11
   <211> 11
   <212> PRT
   <213> Artificial
<220>
   <223> Synthetic construct

<220>
   <221> MOD_RES
   <222> (11)..(11)
   <223> AMIDATION
<400> 11
   Gly Gly Pro Ser Ser Gly Ala Pro Pro Pro Ser
   1               5                   10
<210> 12
   <211> 10
   <212> PRT
   <213> Artificial
<220>
   <223> Synthetic construct
<220>
   <221> MOD_RES
   <222> (10)..(10)
   <223> AMIDATION
<400> 12
   Gly Gly Pro Ser Ser Gly Ala Pro Pro Ser
   1 5 10
<210> 13
   <211> 11
   <212> PRT
   <213> Artificial
<220>
   <223> synthetic construct
<220>
   <221> MISC_FEATURE
   <222> (11)..(11)
   <223> Unmodified acid
<400> 13
   Gly Gly Pro Ser Ser Gly Ala Pro Pro Pro Ser
   1 5 10
<210> 14
   <211> 10
   <212> PRT
   <213> Artificial
<220>
   <223> synthetic construct
<400> 14
   Gly Gly Pro Ser Ser Gly Ala Pro Pro Ser
   1 5 10
<210> 15
   <211> 11
   <212> PRT
   <213> Artificial
<220>
   <223> Synthetic construct
<220>
   <221> MISC_FEATURE
   <222> (1)..(1)
   <223> Xaa at position 1 is Gly, Cys, or absent
<220>
   <221> MISC_FEATURE
   <222> (2)..(2)
   <223> Xaa at position 2 is Gly, Arg, or absent
<220>
   <221> MISC_FEATURE
   <222> (3)..(3)
   <223> Xaa at position 3 is Pro, Thr, or absent
<220>
   <221> MISC_FEATURE
   <222> (4)..(4)
   <223> Xaa at position 4 is Ser or absent
<220>
   <221> MISC_FEATURE
   <222> (5)..(5)
   <223> Xaa at position 5 is Ser or absent
<220>
   <221> MISC_FEATURE
   <222> (6)..(6)
   <223> Xaa at position 6 is Gly or absent
<220>
   <221> MISC_FEATURE
   <222> (7)..(7)
   <223> Xaa at position 7 is Ala or absent
<220>
   <221> MISC_FEATURE
   <222> (8)..(8)
   <223> Xaa at position 8 is Pro or absent
<220>
   <221> MISC_FEATURE
   <222> (9)..(9)
   <223> xaa at position 9 is Pro or absent
<220>
   <221> MISC_FEATURE
   <222> (10)..(10)
   <223> Xaa at position 10 is Pro or absent
<220>
   <221> MISC_FEATURE
   <222> (11)..(11)
   <223> Xaa at position 11 is Ser, Cys, or absent
<400> 15
   Xaa Xaa Xaa Xaa Xaa Xaa Xaa Xaa Xaa Xaa Xaa
   1               5                   10

<210> 20
   <211> 32
   <212> PRT
   <213> Artificial
<220>
   <223> Synthetic construct
<220>
   <221> MISC_FEATURE
   <222> (1)..(1)
   <223> Xaa at position 1 is His, dH, or is absent
<220>
   <221> MISC_FEATURE
   <222> (2)..(2)
   <223> Xaa at position 2 is dA, Ser, Val, Gly, Thr, Leu, dS, Pro, or Aib
<220>
   <221> MISC_FEATURE
   <222> (3)..(3)
   <223> Xaa at position 3 is Asp or Glu
<220>
   <221> MISC_FEATURE
   <222> (4)..(4)
   <223> Xaa at position 4 is Ala, Ile, Tyr, Phe, Val, Thr, Leu, Trp, Gly, dA, Aib, or NMeA
<220>
   <221> MISC_FEATURE
   <222> (5)..(5)
   <223> Xaa at position 5 is Val, Leu, Phe, Ile, Thr, Trp, Tyr, dv, Aib, or NMeV
<220>
   <221> MISC_FEATURE
   <222> (6)..(6)
   <223> Xaa at position 6 is Phe, Ile, Leu, Thr, Val, Trp, or Tyr
<220>
   <221> MISC_FEATURE
   <222> (8)..(8)
   <223> Xaa at position 8 is Asp, Glu, Ala, Lys, Leu, Arg, or Tyr
<220>
   <221> MISC_FEATURE
   <222> (9)..(9)
   <223> Xaa at position 9 is Asn, Gln, or Glu
<220>
   <221> MISC_FEATURE
   <222> (10)..(10)
   <223> Xaa at position 10 is Tyr, Trp, or Tyr(OMe)
<220>
   <221> MISC_FEATURE
   <222> (12)..(12)
   <223> Xaa at position 12 is Arg, Lys, hR, orn, Aib, Cit, or Ala
<220>
   <221> MISC_FEATURE
   <222> (13)..(13)
   <223> xaa at position 13 is Leu, phe, Glu, Ala, or Aib
<220>
   <221> MISC_FEATURE
   <222> (14)..(14)
   <223> Xaa at position 14 is Arg, Leu, Lys, Ala, hR, Orn, Phe, Gln, Aib, or Cit
<220>
   <221> MISC_FEATURE
   <222> (15)..(15)
   <223> Xaa at position 15 is Lys, Ala, Arg, Glu, Leu, hR, Orn, Phe, Gln, Aib, K(AC), or Cit
<220>
   <221> MISC_FEATURE
   <222> (16)..(16)
   <223> Xaa at position 16 is Gln, Lys, Ala, hR, Orn, or Cit
<220>
   <221> MISC_FEATURE
   <222> (17)..(17)
   <223> Xaa at position 17 is Val, Ala, Leu, Ile, Met, Nle, Lys, or Aib
<220>
   <221> MISC_FEATURE
   <222> (19)..(19)
   <223> Xaa at position 19 is Ala, Gly, or Leu
<220>
   <221> MISC_FEATURE
   <222> (20)..(20)
   <223> Xaa at position 20 is Lys, Gln, hR, Arg, Ser, Orn, Ala, Aib, Trp, Thr, Leu, Ile, Phe, Tyr, Val, K(AC), or Cit
<220>
   <221> MISC_FEATURE
   <222> (21)..(21)
   <223> Xaa at position 21 is Lys, Arg, Ala, Phe, Aib, Leu, Gln, Orn, hR, K(AC), or Cit
<220>
   <221> MISC_FEATURE
   <222> (22)..(22)
   <223> Xaa at position 22 is Tyr, Trp, Phe, Thr, Leu, Ile, Val, Tyr(OMe), Ala, or Aib
<220>
   <221> MISC_FEATURE
   <222> (23)..(23)
   <223> Xaa at position 23 is Leu, Phe, Ile, Ala, Trp, Thr, Val, or Aib
<220>
   <221> MISC_FEATURE
   <222> (24)..(24)
   <223> Xaa at position 24 is Gln or Asn
<220>
   <221> MISC_FEATURE
   <222> (25)..(25)
   <223> Xaa at position 25 is Ser, Asp, Phe, Ile, Leu, Thr, Val, Trp, Gln, Asn, Tyr, Aib, or Glu
<220>
   <221> MISC_FEATURE
   <222> (26)..(26)
   <223> Xaa at position 26 is Ile, Leu, Thr, Val, Trp, Tyr, Phe or Aib
<220>
   <221> MISC_FEATURE
   <222> (27)..(27)
   <223> Xaa at position 27 is Lys, hR, Arg, Gln, Orn, or dK
<220>
   <221> MISC_FEATURE
   <222> (28)..(28)
   <223> Xaa at position 28 is Asn, Gln, Lys, Arg, Aib, orn, hR, Cit, Pro, or dK
<220>
   <221> MISC_FEATURE
   <222> (29)..(29)
   <223> Xaa at position 29 is Lys, Ser, Arg, Asn, hR, Orn, Cit, Aib or is absent
<220>
   <221> MISC_FEATURE
   <222> (30)..(30)
   <223> Xaa at position 30 is Arg, Lys, Ile, hR, Cit, Aib, orn, or is absent
<220>
   <221> MISC_FEATURE
   <222> (31)..(31)
   <223> xaa at position 31 is Tyr, His, Phe, or is absent
<220>
   <221> MISC_FEATURE
   <222> (32)..(32)
   <223> Xaa at position 32 is Cys or is absent
<400> 20
   Xaa Xaa Xaa Xaa Xaa Xaa Thr Xaa Xaa Xaa Thr Xaa Xaa Xaa Xaa Xaa
   1               5                   10                  15
   Xaa Ala Xaa Xaa Xaa Xaa Xaa Xaa Xaa Xaa Xaa Xaa Xaa Xaa Xaa Xaa
               20                  25                  30
<210> 21
   <211> 31
   <212> PRT
   <213> Artificial
<220>
   <223> Synthetic construct
<220>
   <221> MISC_FEATURE
   <222> (2)..(2)
   <223> Xaa at position 2 is dA, Ser, Val, dS, or Aib
<220>
   <221> MISC_FEATURE
   <222> (3)..(3)
   <223> Xaa at position 3 is Asp or Glu
<220>
   <221> MISC_FEATURE
   <222> (4)..(4)
   <223> Xaa at position 4 is Ala, dA, or Aib
<220>
   <221> MISC_FEATURE
   <222> (5)..(5)
   <223> Xaa at position 5 is Val, Leu, dv, or Aib

<220>
   <221> MISC_FEATURE
   <222> (8)..(8)
   <223> Xaa at position 8 is Asp, Glu, or ala
<220>
   <221> MISC_FEATURE
   <222> (9)..(9)
   <223> Xaa at position 9 is Asn, Gln, or Glu
<220>
   <221> MISC_FEATURE
   <222> (12)..(12)
   <223> Xaa at position 12 is Ala, Arg, Lys, hR, or Orn
<220>
   <221> MISC_FEATURE
   <222> (14)..(14)
   <223> Xaa at position 14 is Arg, Leu, Lys, ala, hR, orn, Phe, Gln, Aib, or Cit
<220>
   <221> MISC_FEATURE
   <222> (15)..(15)
   <223> Xaa at position 15 is Lys, Ala, Arg, Leu, Orn, Phe, Gln, Aib, or K(AC)
<220>
   <221> MISC_FEATURE
   <222> (16)..(16)
   <223> Xaa at position 16 is Gln or Lys
<220>
   <221> MISC_FEATURE
   <222> (17)..(17)
   <223> Xaa at position 17 is Val, Ala, Leu, Ile, Met, Nle, or Lys
<220>
   <221> MISC_FEATURE
   <222> (19)..(19)
   <223> Xaa at position 19 is Ala or Leu
<220>
   <221> MISC_FEATURE
   <222> (20)..(20)
   <223> Xaa at position 20 is Lys, Gln, hR, Arg, Ser, Ala, Aib, Trp, Thr, Leu, Ile, Phe, Tyr, Val, or K(AC)
<220>
   <221> MISC_FEATURE
   <222> (21)..(21)
   <223> Xaa at position 21 is Lys, Arg, Ala, Phe, Aib, Leu, Gln, K(AC) or Orn
<220>
   <221> MISC_FEATURE
   <222> (22)..(22)
   <223> Xaa at position 22 is Tyr, Trp, Phe, Leu, Ile, or Val
<220>
   <221> MISC_FEATURE
   <222> (24)..(24)
   <223> Xaa at position 24 is Gln or Asn
<220>
   <221> MISC_FEATURE
   <222> (25)..(25)
   <223> Xaa at position 25 is Ser, Phe, Ile, Leu, Thr, Val, Trp, Gln, Asn, Tyr, or Aib
<220>
   <221> MISC_FEATURE
   <222> (26)..(26)
   <223> Xaa at position 26 is Ile, Leu, Thr, Val, Trp, Tyr, Phe or Aib
<220>
   <221> MISC_FEATURE
   <222> (27)..(27)
   <223> Xaa at position 27 is Lys, hR, Arg, dK, or Orn
<220>
   <221> MISC_FEATURE
   <222> (28)..(28)
   <223> Xaa at position 28 is Asn, Gln, Lys, hR, Aib, Orn, dK, or Pro
<220>
   <221> MISC_FEATURE
   <222> (29)..(29)
   <223> Xaa at position 29 is Lys, Ser, Arg, hR, Orn, or is absent
<220>
   <221> MISC_FEATURE
   <222> (30)..(30)
   <223> Xaa at position 30 is Arg, Lys, or is absent
<220>
   <221> MISC_FEATURE
   <222> (31)..(31)
   <223> Xaa at position 31 is Tyr, Phe, or is absent
<400> 21
   His Xaa Xaa Xaa Xaa Phe Thr Xaa Xaa Tyr Thr Xaa Leu Xaa Xaa Xaa
   1               5                   10                  15
   Xaa Ala Xaa Xaa Xaa Xaa Leu Xaa Xaa Xaa Xaa Xaa Xaa Xaa Xaa
               20                  25                  30
<210> 22
   <211> 11
   <212> PRT
   <213> Artificial
<220>
   <223> Synthetic construct
<400> 22
   Gly Gly Pro Ser Ser Gly Ala Pro Pro Pro Cys
   1               5                   10
<210> 23
   <211> 11
   <212> PRT
   <213> Artificial
<220>
   <223> Synthetic construct
<220>
   <221> MOD_RES
   <222> (11)..(11)
   <223> AMIDATION
<400> 23
   Gly Gly Pro Ser Ser Gly Ala Pro Pro Pro Cys
   1               5                   10
<210> 24
   <211> 11
   <212> PRT
   <213> Artificial
<220>
   <223> Synthetic construct
<400> 24
   Gly Arg Pro Ser Ser Gly Ala Pro Pro Pro Ser
   1               5                   10
<210> 25
   <211> 11
   <212> PRT
   <213> Artificial
<220>
   <223> Synthetic construct
<220>
   <221> MOD_RES
   <222> (11)..(11)
   <223> AMIDATION
<400> 25
   Gly Arg Pro Ser Ser Gly Ala Pro Pro Pro Ser
   1               5                   10
<210> 28
   <211> 42
   <212> PRT
   <213> Artificial
<220>
   <223> Synthetic construct
<400> 28
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 31
   <211> 41
   <212> PRT
   <213> Artificial
<220>
   <223> Synthetic construct
<440> 31
<210> 32
   <211> 42
   <212> PRT
   <213> Artificial
<220>
   <223> Synthetic construct
<220>
   <221> MISC_FEATURE
   <222> (20)..(20)
   <223> X = homoarginine
<400> 32
   His Ser Asp Ala Val Phe Thr Asp Asn Tyr Thr Arg Leu Arg Lys Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 33
   <211> 42
   <212> PRT
   <213> Artificial
<220>
   <223> Synthetic construct
<400> 33
   His Ser Asp Ala Val Phe Thr Asp Asn Tyr Thr Arg Leu Arg Lys Gln
   1               5                   10                  15
   Val Ala Ala Ile Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 34
   <211> 42
   <212> PRT
   <213> Artificial
<220>
   <223> Synthetic construct
<400> 34
   His Ser Asp Ala Val Phe Thr Asp Asn Tyr Thr Arg Leu Arg Lys Gln
   1 5 10 15
   Val Ala Ala Arg Lys Tyr Leu Gln Ser Ile Lys Asn Lys Arg Tyr Gly
   20 25 30
   Gly Pro Ser Ser Gly Ala Pro Pro Pro Ser
   35 40
<210> 35
   <211> 42
   <212> PRT
   <213> Artificial
<220>
   <223> Synthetic construct
<400> 35
   His Ser Asp Ala Val Phe Thr Asp Asn Tyr Thr Arg Leu Arg Lys Gln
   1 5 10 15
   Val Ala Ala Ser Lys Tyr Leu Gln Ser Ile Lys Asn Lys Arg Tyr Gly
   20 25 30
   Gly Pro Ser Ser Gly Ala Pro Pro Pro Ser
   35 40
<210> 36
   <211> 42
   <212> PRT
   <213> Artificial
<220>
   <223> synthetic construct
<220>
   <221> MISC_FEATURE
   <222> (27)..(27)
   <223> Xaa at position 27 is homoarginine
<400> 36
   His Ser Asp Ala Val Phe Thr Asp Asn Tyr Thr Arg Leu Arg Lys Gln
   1 5 10 15
   Val Ala Ala Lys Lys Tyr Leu Gln Ser Ile Xaa Asn Lys Arg Tyr Gly
   20 25 30
   Gly Pro Ser Ser Gly Ala Pro Pro Pro Ser
   35 40
<210> 37
   <211> 42
   <212> PRT
   <213> Artificial
<220>
   <223> Synthetic construct
<400> 37
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Arg Asn Lys Arg Tyr Gly
               20                  25                  30
   Gly Pro Ser Ser Gly Ala Pro Pro Pro Ser
           35                  40
<210> 38
   <211> 42
   <212> PRT
   <213> Artificial
<220>
   <223> synthetic construct
<220>
   <221> MISC_FEATURE
   <222> (29)..(29)
   <223> X = homoarginine
<400> 38
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Xaa Arg Tyr Gly
               20                  25                  30
   Gly Pro Ser Ser Gly Ala Pro Pro Pro Ser
           35                  40
<210> 39
   <211> 42
   <212> PRT
   <213> Artificial
<220>
   <223> Synthetic construct
<400> 39
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Arg Arg Tyr Gly
               20                  25                  30
   Gly Pro Ser Ser Gly Ala Pro Pro Pro Ser
           35                  40
<210> 40
   <211> 42
   <212> PRT
   <213> Artificial
<220>
   <223> Synthetic construct
<400> 40
   His Ser Asp Ala Val Phe Thr Asp Asn Tyr Thr Arg Phe Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40

<210> 41
   <211> 42
   <212> PRT
   <213> Artificial
<220>
   <223> synthetic construct
<400> 41
   His Ser Asp Ala Val Phe Thr Asp Asn Trp Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 42
   <211> 42
   <212> PRT
   <213> Artificial
<220>
   <223> synthetic construct
<400> 42
   His Ser Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg His Gly
   20 25 30
   Gly Pro Ser Ser Gly Ala Pro Pro Pro Ser
   35 40
<210> 43
   <211> 42
   <212> PRT
   <213> Artificial
<220>
   <223> synthetic construct
<220>
   <221> MOD_RES
   <222> (1)..(1)
   <223> C6 acylation
<400> 43
   His Ser Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 44
   <211> 42
   <212> PRT
   <213> Artificial
<220>
   <223> Synthetic construct
<220>
   <221> MOD_RES
   <222> (1)..(1)
   <223> ACETYLATION
<400> 44
   His Ser Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 47
   <211> 42
   <212> PRT
   <213> Artificial
<220>
   <223> Synthetic construct
<400> 47
   His Gly Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 48
   <211> 42
   <212> PRT
   <213> Artificial
<220>
   <223> Synthetic construct
<400> 48
   His Val Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 49
   <211> 42
   <212> PRT
   <213> Artificial
<220>
   <223> synthetic construct
<400> 49
   His Thr Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 50
   <211> 42
   <212> PRT
   <213> Artificial
<220>
   <223> Synthetic construct
<400> 50
   His Leu Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 51
   <211> 42
   <212> PRT
   <213> Artificial
<220>
   <223> Synthetic construct
<220>
   <221> MISC_FEATURE
   <222> (2)..(2)
   <223> Ala is D isomer
<400> 51
   His Ala Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 52
   <211> 42
   <212> PRT
   <213> Artificial
<220>
   <223> Synthetic construct
<220>
   <221> MISC_FEATURE
   <222> (2)..(2)
   <223> Ser is D isomer
<400> 52
   His Ser Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 53
   <211> 42
   <212> PRT
   <213> Artificial
<220>
   <223> Synthetic construct
<400> 53
   His Pro Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 54
   <211> 42
   <212> PRT
   <213> Artificial
<220>
   <223> synthetic construct
<400> 54
   His Ser Asp Ile Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 55
   <211> 42
   <212> PRT
   <213> Artificial
<220>
   <223> Synthetic construct
<400> 55
   His Ser Asp Tyr Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 56
   <211> 42
   <212> PRT
   <213> Artificial
<220>
   <223> Synthetic construct
<400> 56
   His Ser Asp Phe Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 57
   <211> 42
   <212> PRT
   <213> Artificial
<220>
   <223> synthetic construct
<400> 57
   His Ser Asp Val Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 58
   <211> 42
   <212> PRT
   <213> Artificial
<220>
   <223> Synthetic construct
<400> 58
   His Ser Asp Thr Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 59
   <211> 42
   <212> PRT
   <213> Artificial
<220>
   <223> Synthetic construct
<400> 59
   His Ser Asp Leu Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 60
   <211> 42
   <212> PRT
   <213> Artificial
<220>
   <223> synthetic construct
<400> 60
   His Ser Asp Trp Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 61
   <211> 42
   <212> PRT
   <213> Artificial
<220>
   <223> Synthetic construct
<400> 61
   His Ser Asp Ala Phe Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 62
   <211> 42
   <212> PRT
   <213> Artificial
<220>
   <223> Synthetic construct
<400> 62
   His Ser Asp Ala Ile Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 63
   <211> 42
   <212> PRT
   <213> Artificial
<220>
   <223> Synthetic construct
<400> 63
   His Ser Asp Ala Leu Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 64
   <211> 42
   <212> PRT
   <213> Artificial
<220>
   <223> synthetic construct
<400> 64
   His Ser Asp Ala Thr Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 65
   <211> 42
   <212> PRT
   <213> Artificial
<220>
   <223> Synthetic construct
<400> 65
   His Ser Asp Ala Val Ile Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 66
   <211> 42
   <212> PRT
   <213> Artificial
<220>
   <223> synthetic construct
<400> 66
   His Ser Asp Ala Val Leu Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 67
   <211> 42
   <212> PRT
   <213> Artificial
<220>
   <223> Synthetic construct
<400> 67
   His Ser Asp Ala Val Thr Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 68
   <211> 42
   <212> PRT
   <213> Artificial
<220>
   <223> Synthetic construct
<400> 68
   His Ser Asp Ala Val Val Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 69
   <211> 42
   <212> PRT
   <213> Artificial
<220>
   <223> synthetic construct
<400> 69
   His Ser Asp Ala Val Trp Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 70
   <211> 42
   <212> PRT
   <213> Artificial
<220>
   <223> synthetic construct
<400> 70
   His Ser Asp Ala Val Tyr Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 71
   <211> 42
   <212> PRT
   <213> Artificial
<220>
   <223> Synthetic construct
<400> 71
   His Ser Asp Ala Trp Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 72
   <211> 42
   <212> PRT
   <213> Artificial
<220>
   <223> Synthetic construct
<400> 72
   His Ser Asp Ala Tyr Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 73
   <211> 42
   <212> PRT
   <213> Artificial
<220>
   <223> Synthetic construct
<400> 73
   His Ser Asp Ala Val Phe Thr Asp Asn Tyr Thr Arg Leu Arg Arg Gln
   1               5                   10                  15
   Val Ala Ala Arg Arg Tyr Leu Gln Ser Ile Arg Asn Arg Arg Tyr Gly
               20                  25                  30
   Gly Pro Ser Ser Gly Ala Pro Pro Pro Ser
           35                  40
<210> 74
   <211> 42
   <212> PRT
   <213> Artificial

<220>
   <223> synthetic construct
<400> 74
   His Ser Asp Ala Val Phe Thr Asp Asn Tyr Thr Arg Leu Arg Arg Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 75
   <211> 42
   <212> PRT
   <213> Artificial
<220>
   <223> synthetic construct
<400> 75
   His Ser Asp Ala Val Phe Thr Asp Asn Tyr Thr Arg Leu Arg Lys Gln
   1               5                   10                  15
   Val Ala Ala Arg Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 76
   <211> 42
   <212> PRT
   <213> Artificial
<220>
   <223> Synthetic construct
<400> 76
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Gln Asn Lys Arg Tyr Gly
               20                  25                  30
   Gly Pro Ser Ser Gly Ala Pro Pro Pro Ser
           35                  40
<210> 77
   <211> 42
   <212> PRT
   <213> Artificial
<220>
   <223> Synthetic construct
<400> 77
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Asn Arg Tyr Gly
               20                  25                  30
   Gly Pro Ser Ser Gly Ala Pro Pro Pro Ser
           35                  40
<210> 78
   <211> 42
   <212> PRT
   <213> Artificial
<220>
   <223> Synthetic construct
<400> 78
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Ile Tyr Gly
               20                  25                  30
   Gly Pro Ser Ser Gly Ala Pro Pro Pro Ser
           35                  40
<210> 79
   <211> 39
   <212> PRT
   <213> Artificial
<220>
   <223> Synthetic construct
<400> 79
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Arg Gly Gly Pro Ser
               20                  25                  30
   Ser Gly Ala Pro Pro Pro Ser
           35
<210> 80
   <211> 39
   <212> PRT
   <213> Artificial
<220>
   <223> Synthetic construct
<400> 80
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Gly Gly Pro Ser
               20                  25                  30
   Ser Gly Ala Pro Pro Pro Ser
           35
<210> 81
   <211> 42
   <212> PRT
   <213> Artificial
<220>
   <223> synthetic construct
<220>
   <221> MISC_FEATURE
   <222> (1)..(1)
   <223> His is D isomer
<400> 81
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 82
   <211> 39
   <212> PRT
   <213> Artificial
<220>
   <223> synthetic construct
<400> 82
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Lys Gly Gly Pro Ser
               20                  25                  30
   Ser Gly Ala Pro Pro Pro Ser
           35
<210> 83
   <211> 42
   <212> PRT
   <213> Artificial
<220>
   <223> Synthetic construct
<400> 83
   His Ser Asp Ala Val Phe Thr Asp Asn Tyr Thr Arg Glu Lys Glu Lys
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 84
   <211> 42
   <212> PRT
   <213> Artificial
<220>
   <223> Synthetic construct
<400> 84
   His Ser Asp Ala Val Phe Thr Asp Asn Tyr Thr Arg Ala Ala Ala Lys
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 85
   <211> 42
   <212> PRT
   <213> Artificial
<220>
   <223> synthetic construct
<400> 85
   His Ser Asp Ala Val Phe Thr Asp Asn Tyr Thr Arg Leu Leu Ala Lys
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 86
   <211> 39
   <212> PRT
   <213> Artificial
<220>
   <223> Synthetic construct
<400> 86
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Gly Arg Pro Ser
               20                  25                  30
   Ser Gly Ala Pro Pro Pro Ser
           35
<210> 87
   <211> 42
   <212> PRT
   <213> Artificial
<220>
   <223> Synthetic construct
<400> 87
   His Ser Asp Ala Val Phe Thr Asp Asn Tyr Thr Arg Leu Leu Leu Lys
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 88
   <211> 42
   <212> PRT
   <213> Artificial
<220>
   <223> Synthetic construct
<400> 88
   His Ser Asp Ala Val Phe Thr Asp Asn Tyr Thr Arg Ala Lys Ala Lys
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 89
   <211> 42
   <212> PRT
   <213> Artificial
<220>
   <223> synthetic construct
<220>
   <221> MOD_RES
   <222> (1).. (1)
   <223> c6 acylation
<400> 89
   His Ser Asp Ala Val Phe Thr Asp Asn Tyr Thr Arg Leu Arg Arg Gln
   1 5 10 15
   Val Ala Ala Arg Arg Tyr Leu Gln Ser Ile Arg Asn Arg Arg Tyr Gly
   20 25 30
   Gly Pro Ser Ser Gly Ala Pro Pro Pro Ser
   35 40
<210> 90
   <211> 42
   <212> PRT
   <213> Artificial
<220>
   <223> Synthetic construct
<220>
   <221> MOD_RES
   <222> (1)..(1)
   <223> C6 acylation
<400> 90
   His Val Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 91
   <211> 43
   <212> PRT
   <213> Artificial
<220>
   <223> Synthetic construct
<400> 91
   Met His Ser Asp Ala Val Phe Thr Asp Gln Tyr Thr Arg Leu Arg Lys
   1               5                   10                  15
   Gln Val Ala Ala Lys Lys Tyr Leu Gln Ser Ile Lys Gln Lys Arg Tyr
               20                  25                  30
   Gly Gly Pro Ser Ser Gly Ala Pro Pro Pro Ser
           35                  40
<210> 92
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (2)..(2)
   <223> Ala is D isomer
<400> 92
   His Ala Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 93
   <211> 42
   <212> PRT
   <213> Artificial
<220>
   <223> Synthetic construct
<400> 93
   His Ser Asp Gly Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 94
   <211> 42
   <212> PRT
   <213> Artificial
<220>
   <223> synthetic construct
<220>
   <221> MOD_RES
   <222> (1)..(1)
   <223> C6 acylation
<400> 94
   His Ser Asp Ala Val Phe Thr Asp Asn Tyr Thr Lys Leu Lys Lys Gln
   1 5 10 15
   Val Ala Ala Lys Lys Tyr Leu Gln Ser Ile Lys Asn Lys Lys Tyr Gly
   20 25 30
   Gly Pro Ser Ser Gly Ala Pro Pro Pro Ser
   35 40
<210> 95
   <211> 43
   <212> PRT
   <213> Artificial
<220>
   <223> Synthetic construct
<400> 95
   Met His Ser Asp Ala Val Phe Thr Asp Asn Tyr Thr Arg Leu Arg Lys
   1               5                   10                  15
   Gln Val Ala Ala Lys Lys Tyr Leu Gln Ser Ile Lys Asn Lys Arg Tyr
               20                  25                  30
   Gly Gly Pro Ser Ser Gly Ala Pro Pro Pro Ser
           35                  40
<210> 96
   <211> 43
   <212> PRT
   <213> Artificial
<220>
   <223> synthetic construct
<400> 96
   Ile His Ser Asp Ala Val Phe Thr Asp Asn Tyr Thr Arg Leu Arg Lys
   1               5                   10                  15
   Gln Val Ala Ala Lys Lys Tyr Leu Gln Ser Ile Lys Asn Lys Arg Tyr
               20                  25                  30
   Gly Gly Pro Ser Ser Gly Ala Pro Pro Pro Ser
           35                  40
<210> 97
   <211> 42
   <212> PRT
   <213> Artificial
<220>
   <223> Synthetic construct
<220>
   <221> MOD_RES
   <222> (1)..(1)
   <223> C6 acylation
<400> 97
   His Ser Asp Ala Val Phe Thr Asp Asn Tyr Thr Arg Leu Arg Lys Gln
   1 5 10 15
   Val Ala Ala Lys Lys Tyr Leu Gln Ser Phe Lys Asn Lys Arg Tyr Gly
   20 25 30
   Gly Pro Ser Ser Gly Ala Pro Pro Pro Ser
   35 40
<210> 98
   <211> 42
   <212> PRT
   <213> Artificial
<220>
   <223> Synthetic construct
<220>
   <221> MOD_RES
   <222> (1)..(1)
   <223> C6 acylation
<400> 98
   His Ser Asp Ala Val Phe Thr Asp Asn Tyr Thr Arg Leu Arg Lys Gln
   1 5 10 15
   Val Ala Ala Lys Lys Tyr Leu Gln Ser Leu Lys Asn Lys Arg Tyr Gly
   20 25 30
   Gly Pro Ser Ser Gly Ala Pro Pro Pro Ser
   35 40
<210> 99
   <211> 42
   <212> PRT
   <213> Artificial
<220>
   <223> Synthetic construct
<220>
   <221> MOD_RES
   <222> (1)..(1)
   <223> C6 acylation
<400> 99
   His Ser Asp Ala Val Phe Thr Asp Asn Tyr Thr Arg Leu Arg Lys Gln
   1 5 10 15
   Val Ala Ala Lys Lys Tyr Leu Gln Ser Thr Lys Asn Lys Arg Tyr Gly
   20 25 30
   Gly Pro Ser Ser Gly Ala Pro Pro Pro Ser
   35 40
<210> 100
   <211> 42
   <212> PRT
   <213> Artificial
<220>
   <223> synthetic construct
<220>
   <221> MOD_RES
   <222> (1)..(1)
   <223> C6 acylation
<400> 100
   His Ser Asp Ala Val Phe Thr Asp Asn Tyr Thr Arg Leu Arg Lys Gln
   1 5 10 15
   Val Ala Ala Lys Lys Tyr Leu Gln Ser Val Lys Asn Lys Arg Tyr Gly
   20 25 30
   Gly Pro Ser Ser Gly Ala Pro Pro Pro Ser
   35 40
<210> 101
   <211> 42
   <212> PRT
   <213> Artificial
<220>
   <223> Synthetic construct
<220>
   <221> MOD_RES
   <222> (1)..(1)
   <223> C6 acylation
<400> 101
   His Ser Asp Ala Val Phe Thr Asp Asn Tyr Thr Arg Leu Arg Lys Gln
   1 5 10 15
   Val Ala Ala Lys Lys Tyr Leu Gln Ser Trp Lys Asn Lys Arg Tyr Gly
   20 25 30
   Gly Pro Ser Ser Gly Ala Pro Pro Pro Ser
   35 40
<210> 102
   <211> 42
   <212> PRT
   <213> Artificial
<220>
   <223> Synthetic construct
<220>
   <221> MOD_RES
   <222> (1)..(1)
   <223> C6 acylation
<400> 102
   His Ser Asp Ala Val Phe Thr Asp Asn Tyr Thr Arg Leu Arg Lys Gln
   1 5 10 15
   Val Ala Ala Lys Lys Tyr Leu Gln Ser Tyr Lys Asn Lys Arg Tyr Gly
   20 25 30
   Gly Pro Ser Ser Gly Ala Pro Pro Pro Ser
   35 40
<210> 103
   <211> 42
   <212> PRT
   <213> Artificial
<220>
   <223> Synthetic construct
<220>
   <221> MOD_RES
   <222> (1)..(1)
   <223> C6 acylation
<400> 103
   His Ser Asp Ala Val Phe Thr Asp Asn Tyr Thr Arg Leu Arg Lys Gln
   1               5                   10                  15
   Val Ala Ala Lys Lys Tyr Leu Gln Phe Ile Lys Asn Lys Arg Tyr Gly
               20                  25                  30
   Gly Pro Ser Ser Gly Ala Pro Pro Pro Ser
           35                  40
<210> 104
   <211> 42
   <212> PRT
   <213> Artificial
<220>
   <223> Synthetic construct
<220>
   <221> MOD_RES
   <222> (1)..(1)
   <223> C6 acylation
<400> 104
   His Ser Asp Ala Val Phe Thr Asp Asn Tyr Thr Arg Leu Arg Lys Gln
   1               5                   10                  15
   Val Ala Ala Lys Lys Tyr Leu Gln Ile Ile Lys Asn Lys Arg Tyr Gly
               20                  25                  30
   Gly Pro Ser Ser Gly Ala Pro Pro Pro Ser
           35                  40
<210> 105
   <211> 42
   <212> PRT
   <213> Artificial
<220>
   <223> Synthetic construct
<220>
   <221> MOD_RES
   <222> (1)..(1)
   <223> C6 acylation
<400> 105
   His Ser Asp Ala Val Phe Thr Asp Asn Tyr Thr Arg Leu Arg Lys Gln
   1 5 10 15
   Val Ala Ala Lys Lys Tyr Leu Gln Leu Ile Lys Asn Lys Arg Tyr Gly
   20 25 30
   Gly Pro Ser Ser Gly Ala Pro Pro Pro Ser
   35 40
<210> 106
   <211> 42
   <212> PRT
   <213> Artificial
<220>
   <223> Synthetic construct
<220>
   <221> MOD_RES
   <222> (1)..(1)
   <223> C6 acylation
<400> 106
   His Ser Asp Ala Val Phe Thr Asp Asn Tyr Thr Arg Leu Arg Lys Gln
   1 5 10 15
   Val Ala Ala Lys Lys Tyr Leu Gln Thr Ile Lys Asn Lys Arg Tyr Gly
   20 25 30
   Gly Pro Ser Ser Gly Ala Pro Pro Pro Ser
   35 40

<210> 107
   <211> 42
   <212> PRT
   <213> Artificial
<220>
   <223> Synthetic construct
<220>
   <221> MOD_RES
   <222> (1)..(1)
   <223> C6 acylation
<400> 107
   His Ser Asp Ala Val Phe Thr Asp Asn Tyr Thr Arg Leu Arg Lys Gln
   1               5                   10                  15
   Val Ala Ala Lys Lys Tyr Leu Gln Val Ile Lys Asn Lys Arg Tyr Gly
               20                  25                  30
   Gly Pro Ser Ser Gly Ala Pro Pro Pro Ser
           35                  40
<210> 108
   <211> 42
   <212> PRT
   <213> Artificial
<220>
   <223> Synthetic construct
<220>
   <221> MOD_RES
   <222> (1)..(1)
   <223> C6 acylation
<400> 108
   His Ser Asp Ala Val Phe Thr Asp Asn Tyr Thr Arg Leu Arg Lys Gln
   1               5                   10                  15
   Val Ala Ala Lys Lys Tyr Leu Gln Trp Ile Lys Asn Lys Arg Tyr Gly
               20                  25                  30
   Gly Pro Ser Ser Gly Ala Pro Pro Pro Ser
           35                  40
<210> 109
   <211> 42
   <212> PRT
   <213> Artificial
<220>
   <223> Synthetic construct
<220>
   <221> MOD_RES
   <222> (1)..(1)
   <223> C6 acylation
<400> 109
   His Ser Asp Ala Val Phe Thr Asp Asn Tyr Thr Arg Leu Leu Ala Lys
   1 5 10 15
   Leu Ala Leu Gln Lys Tyr Leu Gln Ser Ile Lys Asn Lys Arg Tyr Gly
   20 25 30
   Gly Pro Ser Ser Gly Ala Pro Pro Pro Ser
   35 40
<210> 110
   <211> 42
   <212> PRT
   <213> Artificial
<220>
   <223> Synthetic construct
<220>
   <221> MOD_RES
   <222> (1)..(1)
   <223> C6 acylation
<400> 110
   His Ser Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Cys
   35 40
<210> 111
   <211> 41
   <212> PRT
   <213> Artificial
<220>
   <223> Synthetic construct
<220>
   <221> MOD_RES
   <222> (1)..(1)
   <223> C6 acylation
<400> 111
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Lys Gly Gly
               20                  25                  30
   Pro Ser Ser Gly Ala Pro Pro Pro Ser
           35                  40
<210> 112
   <211> 41
   <212> PRT
   <213> Artificial
<220>
   <223> Synthetic construct
<220>
   <221> MOD_RES
   <222> (1)..(1)
   <223> C6 acylation
<400> 112
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Ser Arg Gly Gly
               20                  25                  30
   Pro Ser Ser Gly Ala Pro Pro Pro Ser
           35                  40
<210> 113
   <211> 41
   <212> PRT
   <213> Artificial
<220>
   <223> Synthetic construct
<220>
   <221> MOD_RES
   <222> (1)..(1)
   <223> C6 acylation
<400> 113
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Gly Gly
               20                  25                  30
   Pro Ser Ser Gly Ala Pro Pro Pro Ser
           35                  40
<210> 114
   <211> 42
   <212> PRT
   <213> Artificial
<220>
   <223> synthetic construct
<220>
   <221> MOD_RES
   <222> (1)..(1)
   <223> C6 acylation
<400> 114
   His Ser Asp Ala Val Phe Thr Asp Asn Tyr Thr Arg Leu Arg Lys Gln
   1               5                   10                  15
   Val Ala Ala Lys Lys Tyr Leu Gln Gln Ile Lys Asn Lys Arg Tyr Gly
               20                  25                  30
   Gly Pro Ser Ser Gly Ala Pro Pro Pro Ser
           35                  40
<210> 115
   <211> 42
   <212> PRT
   <213> Artificial
<220>
   <223> Synthetic construct
<220>
   <221> MOD_RES
   <222> (1)..(1)
   <223> C6 acylation
<400> 115
   His Ser Asp Ala Val Phe Thr Asp Asn Tyr Thr Arg Leu Arg Lys Gln
   1               5                   10                  15
   Val Ala Ala Lys Lys Tyr Leu Gln Asn Ile Lys Asn Lys Arg Tyr Gly
               20                  25                  30
   Gly Pro Ser Ser Gly Ala Pro Pro Pro Ser
           35                  40
<210> 116
   <211> 39
   <212> PRT
   <213> Artificial
<220>
   <223> Synthetic construct
<400> 116
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Arg Gly Arg Pro Ser
               20                  25                  30
   Ser Gly Ala Pro Pro Pro Ser
           35
<210> 117
   <211> 42
   <212> PRT
   <213> Artificial
<220>
   <223> synthetic construct
<220>
   <221> MOD_RES
   <222> (1)..(1)
   <223> C6 acylation
<400> 117
   His Ser Asp Ala Val Phe Thr Asp Asn Tyr Thr Arg Leu Arg Lys Gln
   1               5                   10                  15
   Val Ala Ala Lys Lys Tyr Leu Gln Tyr Ile Lys Asn Lys Arg Tyr Gly
               20                  25                  30
   Gly Pro Ser Ser Gly Ala Pro Pro Pro Ser
           35                  40
<210> 118
   <211> 42
   <212> PRT
   <213> Artificial
<220>
   <223> Synthetic construct
<220>
   <221> MOD_RES
   <222> (1)..(1)
   <223> C6 acylation
<400> 118
   His Ser Asp Ala Val Phe Thr Asp Asn Tyr Thr Arg Leu Arg Lys Gln
   1               5                   10                  15
   Val Ala Ala Lys Lys Trp Leu Gln Ser Ile Lys Asn Lys Arg Tyr Gly
               20                  25                  30
   Gly Pro Ser Ser Gly Ala Pro Pro Pro Ser
           35                  40
<210> 119
   <211> 42
   <212> PRT
   <213> Artificial
<220>
   <223> Synthetic construct
<220>
   <221> MOD_RES
   <222> (1)..(1)
   <223> c6 acylation
<400> 119
   His Ser Asp Ala Val Phe Thr Asp Asn Tyr Thr Arg Leu Arg Lys Gln
   1 5 10 15
   Val Ala Ala Lys Lys Phe Leu Gln Ser Ile Lys Asn Lys Arg Tyr Gly
   20 25 30
   Gly Pro Ser Ser Gly Ala Pro Pro Pro Ser
   35 40
<210> 120
   <211> 42
   <212> PRT
   <213> Artificial
<220>
   <223> Synthetic construct
<220>
   <221> MOD_RES
   <222> (1)..(1)
   <223> C6 Acylation
<400> 120
   His Ser Asp Ala Val Phe Thr Asp Asn Tyr Thr Arg Leu Arg Lys Gln
   1 5 10 15
   Val Ala Ala Lys Lys Thr Leu Gln Ser Ile Lys Asn Lys Arg Tyr Gly
   20 25 30
   Gly Pro Ser Ser Gly Ala Pro Pro Pro Ser
   35 40
<210> 121
   <211> 42
   <212> PRT
   <213> Artificial
<220>
   <223> Synthetic construct
<220>
   <221> MOD_RES
   <222> (1)..(1)
   <223> C6 acylation
<400> 121
   His Ser Asp Ala Val Phe Thr Asp Asn Tyr Thr Arg Leu Arg Lys Gln
   1 5 10 15
   Val Ala Ala Lys Lys Leu Leu Gln Ser Ile Lys Asn Lys Arg Tyr Gly
   20 25 30
   Gly Pro Ser Ser Gly Ala Pro Pro Pro Ser
   35 40
<210> 122
   <211> 42
   <212> PRT
   <213> Artificial
<220>
   <223> synthetic construct
<220>
   <221> MOD_RES
   <222> (1)..(1)
   <223> C6 acylation
<400> 122
   His Ser Asp Ala Val Phe Thr Asp Asn Tyr Thr Arg Leu Arg Lys Gln
   1 5 10 15
   Val Ala Ala Lys Lys Ile Leu Gln Ser Ile Lys Asn Lys Arg Tyr Gly
   20 25 30
   Gly Pro Ser Ser Gly Ala Pro Pro Pro Ser
   35 40
<210> 123
   <211> 42
   <212> PRT
   <213> Artificial
<220>
   <223> synthetic construct
<220>
   <221> MOD_RES
   <222> (1)..(1)
   <223> C6 acylation
<400> 123
   His Ser Asp Ala Val Phe Thr Asp Asn Tyr Thr Arg Leu Arg Lys Gln
   1 5 10 15
   Val Ala Ala Lys Lys Val Leu Gln Ser Ile Lys Asn Lys Arg Tyr Gly
   20 25 30
   Gly Pro Ser Ser Gly Ala Pro Pro Pro Ser
   35 40
<210> 124
   <211> 42
   <212> PRT
   <213> Artificial
<220>
   <223> Synthetic construct
<220>
   <221> MOD_RES
   <222> (1)..(1)
   <223> C6 acylation
<400> 124
   His Ser Asp Ala Val Phe Thr Asp Asn Tyr Thr Arg Leu Leu Ala Lys
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 125
   <211> 42
   <212> PRT
   <213> Artificial
<220>
   <223> Synthetic construct
<220>
   <221> MOD_RES
   <222> (1)..(1)
   <223> C6 acylation
<400> 125
   His Ser Asp Ala Val Phe Thr Asp Asn Tyr Thr Arg Leu Arg Ala Gln
   1               5                   10                  15
   Val Ala Ala Gln Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 126
   <211> 42
   <212> PRT
   <213> Artificial
<220>
   <223> Synthetic construct
<220>
   <221> MOD_RES
   <222> (1)..(1)
   <223> C6 acylation
<400> 126
   His Ser Asp Ala Val Phe Thr Asp Asn Tyr Thr Arg Leu Arg Ala Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40

<210> 127
   <211> 43
   <212> PRT
   <213> Artificial
<220>
   <223> Synthetic construct
<400> 127
   Met His Ser Asp Ala Val Phe Thr Asp Asn Tyr Thr Arg Leu Leu Ala
   1 5 10 15
   Lys Leu Ala Leu Gln Lys Tyr Leu Gln Ser Ile Lys Asn Lys Arg Tyr
   20 25 30
   Gly Gly Pro Ser Ser Gly Ala Pro Pro Pro Ser
   35 40
<210> 128
   <211> 42
   <212> PRT
   <213> Artificial
<220>
   <223> synthetic construct
<220>
   <221> MOD_RES
   <222> (1)..(1)
   <223> C6 acylation
<400> 128
   His Ser Asp Ala Val Phe Thr Asp Asn Tyr Thr Arg Leu Lys Ala Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 129
   <211> 42
   <212> PRT
   <213> Artificial
<220>
   <223> Synthetic construct
<220>
   <221> MOD_RES
   <222> (1)..(1)
   <223> C6 acylation
<400> 129
   His Ser Asp Ala Val Phe Thr Asp Asn Tyr Thr Arg Leu Arg Ala Gln
   1               5                   10                  15
   Leu Ala Ala Gln Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 130
   <211> 39
   <212> PRT
   <213> Artificial
<220>
   <223> Synthetic construct
<220>
   <221> MOD_RES
   <222> (1)..(1)
   <223> C6 acylation
<400> 130
   His Ser Asp Ala Val Phe Thr Asp Asn Tyr Thr Arg Leu Arg Lys Gln
   1               5                   10                  15
   Met Ala Ala Gln Lys Tyr Leu Asn Gln Leu Lys Lys Gly Gly Pro Ser
               20                  25                  30
   Ser Gly Ala Pro Pro Pro Ser
           35
<210> 131
   <211> 39
   <212> PRT
   <213> Artificial
<220>
   <223> synthetic construct
<220>
   <221> MOD_RES
   <222> (1) .. (1)
   <223> C6 acylation
<400> 131
   His Ser Asp Ala Val Phe Thr Asp Asn Tyr Thr Arg Leu Arg Lys Gln
   1               5                   10                  15
   Val Ala Ala Gln Lys Tyr Leu Asn Gln Leu Lys Lys Gly Gly Pro Ser
   20 25 30
   Ser Gly Ala Pro Pro Pro Ser
   35
<210> 132
   <211> 42
   <212> PRT
   <213> Artificial
<220>
   <223> Synthetic construct
<220>
   <221> MOD_RES
   <222> (1)..(1)
   <223> C6 acylation
<400> 132
   His Ser Asp Ala Val Phe Thr Asp Asn Tyr Thr Arg Leu Arg Lys Gln
   1 5 10 15
   Val Ala Ala Val Lys Tyr Leu Gln Ser Ile Lys Asn Lys Arg Tyr Gly
   20 25 30
   Gly Pro Ser Ser Gly Ala Pro Pro Pro Ser
   35 40
<210> 133
   <211> 42
   <212> PRT
   <213> Artificial
<220>
   <223> Synthetic construct
<220>
   <221> MOD_RES
   <222> (1)..(1)
   <223> C6 acylation
<400> 133 -
<210> 134
   <211> 42
   <212> PRT
   <213> Artificial
<220>
   <223> Synthetic construct
<220>
   <221> MOD_RES
   <222> (1)..(1)
   <223> C6 acylation
<400> 134
   His Ser Asp Ala Val Phe Thr Asp Asn Tyr Thr Arg Leu Arg Lys Gln
   1 5 10 15
   Val Ala Ala Phe Lys Tyr Leu Gln Ser Ile Lys Asn Lys Arg Tyr Gly
   20 25 30
   Gly Pro Ser Ser Gly Ala Pro Pro Pro Ser
   35 40
<210> 135
   <211> 42
   <212> PRT
   <213> Artificial
<220>
   <223> Synthetic construct
<220>
   <221> MOD_RES
   <222> (1)..(1)
   <223> C6 acylation
<400> 135
   His Ser Asp Ala Val Phe Thr Asp Asn Tyr Thr Arg Leu Arg Lys Gln
   1 5 10 15
   Val Ala Ala Ile Lys Tyr Leu Gln Ser Ile Lys Asn Lys Arg Tyr Gly
   20 25 30
   Gly Pro Ser Ser Gly Ala Pro Pro Pro Ser
   35 40
<210> 136
   <211> 42
   <212> PRT
   <213> Artificial
<220>
   <223> Synthetic construct
<220>
   <221> MOD_RES
   <222> (1)..(1)
   <223> C6 acylation
<400> 136
   His Ser Asp Ala Val Phe Thr Asp Asn Tyr Thr Arg Leu Arg Lys Gln
   1               5                   10                  15
   Val Ala Ala Gln Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 137
   <211> 42
   <212> PRT
   <213> Artificial
<220>
   <223> Synthetic construct
<220>
   <221> MOD_RES
   <222> (1)..(1)
   <223> C6 acylation
<400> 137
   His Ser Asp Ala Val Phe Thr Asp Asn Tyr Thr Arg Leu Arg Lys Gln
   1               5                   10                  15
   Val Ala Ala Leu Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 138
   <211> 42
   <212> PRT
   <213> Artificial
<220>
   <223> Synthetic construct
<220>
   <221> MOD_RES
   <222> (1)..(1)
   <223> C6 acylation
<400> 138
   His Ser Asp Ala Val Phe Thr Asp Asn Tyr Thr Arg Leu Arg Lys Gln
   1               5                   10                  15
   Val Ala Ala Thr Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 139
   <211> 42
   <212> PRT
   <213> Artificial
<220>
   <223> synthetic construct
<220>
   <221> MOD_RES
   <222> (1)..(1)
   <223> C6 acylation
<400> 139
   His Ser Asp Ala Val Phe Thr Asp Asn Tyr Thr Arg Leu Arg Lys Gln
   1               5                   10                  15
   Val Ala Ala Trp Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 140
   <211> 39
   <212> PRT.
   <213> Artificial
<220>
   <223> Synthetic construct
<220>
   <221> MOD_RES
   <222> (1)..(1)
   <223> C6 acylation
<400> 140
   His Ser Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Gly Gly Pro Ser
   20 25 30
   Ser Gly Ala Pro Pro Pro Ser
   35
<210> 141
   <211> 42
   <212> PRT
   <213> Artificial
<220>
   <223> Synthetic construct
<220>
   <221> MOD_RES
   <222> (1)..(1)
   <223> C6 acylation
<400> 141
   His Ser Asp Ala Val Phe Thr Asp Asn Tyr Thr Arg Leu Arg Lys Gln
   1 5 10 15
   Val Ala Leu Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 142
   <211> 42
   <212> PRT
   <213> Artificial
<220>
   <223> Synthetic construct
<220>
   <221> MOD_RES
   <222> (1)..(1)
   <223> C6 acylation
<400> 142
   His Ser Asp Ala Val Phe Thr Ala Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 143
   <211> 42
   <212> PRT
   <213> Artificial
<220>
   <223> Synthetic construct
<220>
   <221> MOD_RES
   <222> (1)..(1)
   <223> C6 acylation
<400> 143
   His Ser Asp Ala Val Phe Thr Glu Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 144
   <211> 42
   <212> PRT
   <213> Artificial
<220>
   <223> Synthetic construct
<220>
   <221> MOD_RES
   <222> (1)..(1)
   <223> C6 acylation
<400> 144
   His Ser Asp Ala Val Phe Thr Lys Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 145
   <211> 42
   <212> PRT
   <213> Artificial
<220>
   <223> Synthetic construct
<220>
   <221> MOD_RES
   <222> (1)..(1)
   <223> C6 acylation
<400> 145
   His Ser Asp Ala Val Phe Thr Leu Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 146
   <211> 42
   <212> PRT
   <213> Artificial
<220>
   <223> Synthetic construct
<220>
   <221> MOD_RES
   <222> (1)..(1)
   <223> C6 acylation
<400> 146
   His Ser Asp Ala Val Phe Thr Arg Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 147
   <211> 42
   <212> PRT
   <213> Artificial
<220>
   <223> Synthetic construct
<220>
   <221> MOD_RES
   <222> (1)..(1)
   <223> C6 acylation
<400> 147
   His Ser Asp Ala Val Phe Thr Tyr Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 148
   <211> 42
   <212> PRT
   <213> Artificial
<220>
   <223> synthetic construct
<220>
   <221> MOD_RES
   <222> (1)..(1)
   <223> C6 acylation
<400> 148
   His Ser Asp Ala Val Phe Thr Asp Asn Tyr Thr Arg Leu Leu Ala Lys
   1 5 10 15
   Leu Ala Leu Gln Lys Tyr Leu Gln Ser Ile Lys Asn Lys Arg Tyr Gly
               20                  25                  30
   Gly Pro Ser Ser Gly Ala Pro Pro Pro Cys
           35                  40
<210> 149
   <211> 42
   <212> PRT
   <213> Artificial
<220>
   <223> Synthetic construct
<220>
   <221> MOD_RES
   <222> (1)..(1)
   <223> C6 acylation
<400> 149
   His Ser Asp Ala Val Phe Thr Glu Glu Tyr Thr Arg Leu Gln Lys Gln
   1               5                   10                  15
   Val Ala Ala Lys Gln Tyr Leu Gln Ser Ile Lys Asn Lys Arg Tyr Gly
               20                  25                  30
   Gly Pro Ser Ser Gly Ala Pro Pro Pro Ser
           35                  40
<210> 150
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (2)..(2)
   <223> X = Aib
<400> 150
   His Xaa Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 151
   <211> 42
   <212> PRT
   <213> Artificial
<220>
   <223> Synthetic construct
<220>
   <221> MOD_RES
   <222> (1)...(1)
   <223> C6 acylation
<220>
   <221> MISC_FEATURE
   <222> (14)..(14)
   <223> X = Aib
<400> 151
   His Ser Asp Ala Val Phe Thr Asp Asn Tyr Thr Arg Leu Xaa Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 152
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<400> 152
   His Ser Asp Ala Val Phe Thr Asp Asn Tyr Thr Arg Leu Arg Xaa Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 153
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (20)..(20)
   <223> X = Aib
<400> 153
   His Ser Asp Ala Val Phe Thr Asp Asn Tyr Thr Arg Leu Arg Lys Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 154
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (21)..(21)
   <223> X = Aib
<400> 154
   His Ser Asp Ala Val Phe Thr Asp Asn Tyr Thr Arg Leu Arg Lys Gln
   1               5                   10                  15
   Val Ala Ala Lys Xaa Tyr Leu Gln Ser Ile Lys Asn Lys Arg Tyr Gly
               20                  25                  30
   Gly Pro Ser Ser Gly Ala Pro Pro Pro Ser
           35                  40
<210> 155
   <211> 42
   <212> PRT
   <213> Artificial
<220>
   <223> Synthetic construct
<220>
   <221> MOD_RES
   <222> (1)..(1)
   <223> C6 acylation
<400> 155
   His Ser Asp Ala Val Phe Thr Asp Asn Tyr Thr Arg Leu Lys Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 156
   <211> 42
   <212> PRT
   <213> Artificial
<220>
   <223> Synthetic construct
<220>
   <221> MOD_RES
   <222> (1)..(1)
   <223> C6 acylation
<400> 156
   His Ser Asp Ala Val Phe Thr Asp Asn Tyr Thr Arg Leu Gln Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 157
   <211> 42
   <212> PRT
   <213> Artificial
<220>
   <223> Synthetic construct
<220>
   <221> MOD_RES
   <222> (1)..(1)
   <223> C6 acylation
<400> 157
   His Ser Asp Ala Val Phe Thr Asp Asn Tyr Thr Arg Leu Ala Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 158
   <211> 42
   <212> PRT
   <213> Artificial
<220>
   <223> Synthetic construct
<220>
   <221> MOD_RES
   <222> (1)..(1)
   <223> C6 acylation
<400> 158
   His Ser Asp Ala Val Phe Thr Asp Asn Tyr Thr Arg Leu Leu Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 159
   <211> 42
   <212> PRT
   <213> Artificial
<220>
   <223> Synthetic construct
<220>
   <221> MOD_RES
   <222> (1)..(1)
   <223> C6 acylation
<400> 159
   His Ser Asp Ala Val Phe Thr Asp Asn Tyr Thr Arg Leu Phe Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 160
   <211> 42
   <212> PRT
   <213> Artificial
<220>
   <223> Synthetic construct
<220>
   <221> MOD_RES
   <222> (1)..(1)
   <223> c6 acylation
<400> 160
   His Ser Asp Ala Val Phe Thr Asp Asn Tyr Thr Arg Leu Arg Arg Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 161
   <211> 42
   <212> PRT
   <213> Artificial
<220>
   <223> Synthetic construct
<220>
   <221> MOD_RES
   <222> (1)..(1)
   <223> C6 acylation
<400> 161
   His Ser Asp Ala Val Phe Thr Asp Asn Tyr Thr Arg Leu Arg Gln Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 162
   <211> 42
   <212> PRT
   <213> Artificial
<220>
   <223> Synthetic construct
<220>
   <221> MOD_RES
   <222> (1)..(1)
   <223> C6 acylation
<400> 162
   His Ser Asp Ala Val Phe Thr Asp Asn Tyr Thr Arg Leu Arg Leu Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 163
   <211> 42
   <212> PRT
   <213> Artificial
<220>
   <223> Synthetic construct
<220>
   <221> MOD_RES
   <222> (1)..(1)
   <223> c6 acylation
<400> 163
   His Ser Asp Ala Val Phe Thr Asp Asn Tyr Thr Arg Leu Arg Phe Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 164
   <211> 42
   <212> PRT
   <213> Artificial
<220>
   <223> Synthetic construct
<220>
   <221> MOD_RES
   <222> (1)..(1)
   <223> C6 acylation
<400> 164
   His Ser Asp Ala Val Phe Thr Asp Asn Tyr Thr Arg Leu Arg Lys Gln
   1 5 10 15
   Val Ala Ala Ala Lys Tyr Leu Gln Ser Ile Lys Asn Lys Arg Tyr Gly
   20 25 30
   Gly Pro Ser Ser Gly Ala Pro Pro Pro Ser
   35 40
<210> 165
   <211> 42
   <212> PRT
   <213> Artificial
<220>
   <223> Synthetic construct
<220>
   <221> MOD_RES
   <222> (1)..(1)
   <223> C6 acylation
<400> 165
   His Ser Asp Ala Val Phe Thr Asp Asn Tyr Thr Arg Leu Arg Lys Gln
   1 5 10 15
   Val Ala Ala Lys Arg Tyr Leu Gln Ser Ile Lys Asn Lys Arg Tyr Gly
   20 25 30
   Gly Pro Ser Ser Gly Ala Pro Pro Pro Ser
   35 40

<210> 166
   <211> 42
   <212> PRT
   <213> Artificial
<220>
   <223> Synthetic construct
<220>
   <221> MOD_RES
   <222> (1)..(1)
   <223> C6 acylation
<400> 166
   His Ser Asp Ala Val Phe Thr Asp Asn Tyr Thr Arg Leu Arg Lys Gln
   1               5                   10                  15
   Val Ala Ala Lys Gln Tyr Leu Gln Ser Ile Lys Asn Lys Arg Tyr Gly
               20                  25                  30
   Gly Pro Ser Ser Gly Ala Pro Pro Pro Ser
           35                  40
<210> 167
   <211> 42
   <212> PRT
   <213> Artificial
<220>
   <223> Synthetic construct
<220>
   <221> MOD_RES
   <222> (1)..(1)
   <223> C6 acylation
<400> 167
   His Ser Asp Ala Val Phe Thr Asp Asn Tyr Thr Arg Leu Arg Lys Gln
   1               5                   10                  15
   Val Ala Ala Lys Ala Tyr Leu Gln Ser Ile Lys Asn Lys Arg Tyr Gly
               20                  25                  30
   Gly Pro Ser Ser Gly Ala Pro Pro Pro Ser
           35                  40
<210> 168
   <211> 42
   <212> PRT
   <213> Artificial
<220>
   <223> Synthetic construct
<220>
   <221> MOD_RES
   <222> (1)..(1)
   <223> C6 acylation
<400> 168
   His Ser Asp Ala Val Phe Thr Asp Asn Tyr Thr Arg Leu Arg Lys Gln
   1               5                   10                  15
   Val Ala Ala Lys Leu Tyr Leu Gln Ser Ile Lys Asn Lys Arg Tyr Gly
               20                  25                  30
   Gly Pro Ser Ser Gly Ala Pro Pro Pro Ser
           35                  40
<210> 169
   <211> 42
   <212> PRT
   <213> Artificial
<220>
   <223> Synthetic construct
<220> .
   <221> MOD_RES
   <222> (1)..(1)
   <223> C6 acylation
<400> 169
   His Ser Asp Ala Val Phe Thr Asp Asn Tyr Thr Arg Leu Arg Lys Gln
   1               5                   10                  15
   Val Ala Ala Lys Phe Tyr Leu Gln Ser Ile Lys Asn Lys Arg Tyr Gly
               20                  25                  30
   Gly Pro Ser Ser Gly Ala Pro Pro Pro Ser
           35                  40
<210> 170
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (25)..(25)
   <223> X = Aib
<400> 170
   His Ser Asp Ala Val Phe Thr Asp Asn Tyr Thr Arg Leu Arg Lys Gln
   1               5                   10                  15
   Val Ala Ala Lys Lys Tyr Leu Gln Xaa Ile Lys Asn Lys Arg Tyr Gly
               20                  25                  30
   Gly Pro Ser Ser Gly Ala Pro Pro Pro Ser
           35                  40
<210> 171
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (26)..(26)
   <223> X = Aib
<400> 171
   His Ser Asp Ala Val Phe Thr Asp Asn Tyr Thr Arg Leu Arg Lys Gln
   1               5                   10                  15
   Val Ala Ala Lys Lys Tyr Leu Gln Ser Xaa Lys Asn Lys Arg Tyr Gly
               20                  25                  30
   Gly Pro Ser Ser Gly Ala Pro Pro Pro Ser
           35                  40
<210> 172
   <211> 42
   <212> PRT
   <213> Artificial
<220>
   <223> Synthetic construct
<220> .
   <221> MOD_RES
   <222> (1)..(1)
   <223> C6 acylation
<220>
   <221> MISC_FEATURE
   <222> (17)..(17)
   <223> X = Aib
<400> 172
   His Ser Asp Ala Val Phe Thr Asp Asn Tyr Thr Arg Leu Arg Lys Gln
   1 5 10 15
   Xaa Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 173
   <211> 43
   <212> PRT
   <213> Artificial
<220>
   <223> Synthetic construct
<220>
   <221> MOD_RES
   <222> (1)..(1)
   <223> C6 acylation
<400> 173
   His His Ser Asp Ala Val Phe Thr Asp Asn Tyr Thr Arg Leu Leu Ala
   1 5 10 15
   Lys Leu Ala Leu Gln Lys Tyr Leu Gln Ser Ile Lys Asn Lys Arg Tyr
   20 25 30
   Gly Gly Pro Ser Ser Gly Ala Pro Pro Pro Ser
   35 40
<210> 174
   <211> 42
   <212> PRT
   <213> Artificial
<220>
   <223> Synthetic construct
<220>
   <221> MOD_RES
   <222> (1)..(1)
   <223> C6 acylation
<400> 174
   His Ser Asp Ala Val Phe Thr Asp Gln Tyr Thr Arg Leu Leu Ala Lys
   1 5 10 15
   Leu Ala Leu Gln Lys Tyr Leu Gln Ser Ile Lys Gln Lys Arg Tyr Gly
   20 25 30
   Gly Pro Ser Ser Gly Ala Pro Pro Pro Ser
   35 40
<210> 175
   <211> 42
   <212> PRT
   <213> Artificial
<220>
   <223> Synthetic construct
<220>
   <221> MOD_RES
   <222> (1)..(1)
   <223> C6 acylation
<220>
   <221> MOD_RES
   <222> (15)..(15)
   <223> epsilon-acetyl lysine
<220>
   <221> MOD_RES
   <222> (20)..(20)
   <223> epsilon-acetyl lysine
<400> 175
   His Ser Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 176
   <211> 42
   <212> PRT
   <213> Artificial
<220>
   <223> Synthetic construct
<220>
   <221> MOD_RES
   <222> (1)..(1)
   <223> C6 acylation
<220>
   <221> MOD_RES
   <222> (15)..(15)
   <223> epsilon-acetyl lysine
<220>
   <221> MOD_RES
   <222> (21)..(21)
   <223> epsilon-acetyl lysine
<400> 176
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 177
   <211> 42
   <212> PRT
   <213> Artificial
<220>
   <223> synthetic construct
<220>
   <221> MOD_RES
   <222> (1)..(1)
   <223> C6 acylation
<400> 177
   His Ser Asp Ala Val Phe Thr Asp Asn Tyr Thr Arg Leu Leu Ala Gln
   1               5                   10                  15
   Leu Ala Leu Gln Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 178
   <211> 42
   <212> PRT
   <213> Artificial
<220>
   <223> Synthetic construct
<220>
   <221> MOD_RES
   <222> (1)..(1)
   <223> C6 acylation
<400> 178
   His Ser Asp Ala Val Phe Thr Asp Asn Tyr Thr Arg Leu Leu Ala Lys
   1               5                   10                  15
   Val Ala Leu Gln Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 179
   <211> 42
   <212> PRT
   <213> Artificial
<220>
   <223> Synthetic construct
<220>
   <221> MOD_RES
   <222> (1)..(1)
   <223> C6 acylation
<400> 179
   His Ser Asp Ala Val Phe Thr Asp Asn Tyr Thr Arg Leu Leu Ala Lys
   1               5                   10                  15
   Leu Ala Ala Gln Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 180
   <211> 40
   <212> PRT
   <213> Artificial
<220>
   <223> synthetic construct
<220>
   <221> MOD_RES
   <222> (1)..(1)
   <223> C6 acylation
<400> 180
   His Ser Asp Ala Val Phe Thr Asp Asn Tyr Thr Arg Leu Leu Ala Lys
   1               5                   10                  15
   Leu Ala Leu Gln Lys Tyr Leu Gln Ser Ile Lys Asn Lys Gly Gly Pro
               20                  25                  30
   Ser Ser Gly Ala Pro Pro Pro Cys
           35                  40
<210> 181
   <211> 43
   <212> PRT
   <213> Artificial
<220>
   <223> synthetic construct
<220>
   <221> MISC_FEATURE
   <222> (1)..(1)
   <223> x = methionine sulfoxide
<400> 181
   Xaa His Ser Asp Ala Val Phe Thr Asp Asn Tyr Thr Arg Leu Arg Lys
   1               5                   10                  15
   Gln Val Ala Ala Lys Lys Tyr Leu Gln Ser Ile Lys Asn Lys Arg Tyr
               20                  25                  30
   Gly Gly Pro Ser Ser Gly Ala Pro Pro Pro Ser
           35                  40
<210> 182
   <211> 42
   <212> PRT
   <213> Artificial
<220>
   <223> Synthetic construct
<220>
   <221> MOD_RES
   <222> (1)..(1)
   <223> C6 acylation
<400> 182
   His Ser Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Phe Gly
   20 25 30
   Gly Pro Ser Ser Gly Ala Pro Pro Pro Ser
   35 40
<210> 183
   <211> 42
   <212> PRT
   <213> Artificial
<220>
   <223> Synthetic construct
<220>
   <221> MOD_RES
   <222> (1)..(1)
   <223> HS(CH2)2CO substituent
<400> 183
   His Ser Asp Ala Val Phe Thr Asp Asn Tyr Thr Arg Leu Leu Ala Lys
   1 5 10 15
   Leu Ala Leu Gln Lys Tyr Leu Gln Ser Ile Lys Asn Lys Arg Tyr Gly
   20 25 30
   Gly Pro Ser Ser Gly Ala Pro Pro Pro Ser
   35 40
<210> 184
   <211> 42
   <212> PRT
   <213> Artificial
<220>
   <223> Synthetic construct
<220>
   <221> MOD_RES
   <222> (1)..(1)
   <223> HS(CH2)2SO substituent
<400> 184
   His Ser Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 185
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (4)..(4)
   <223> Ala is D-isomer
<400> 185
   His Ser Asp Ala Val Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 186
   <211> 42
   <212> PRT
   <213> Artificial
<220>
   <223> Synthetic construct
<220>
   <221> MOD_RES
   <222> (1)..(1)
   <223> C6 acylation
<220>
   <221> MOD_RES
   <222> (4)..(4)
   <223> N-methyl alanine
<400> 186
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 187
   <211> 42
   <212> PRT
   <213> Artificial
<220>
   <223> synthetic construct
<220>
   <221> MOD_RES
   <222> (1)..(1)
   <223> c6 acylation
<220>
   <221> MISC_FEATURE
   <222> (4)..(4)
   <223> X = Aib
<400> 187
   His Ser Asp Xaa Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 188
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (5)..(5)
   <223> Val is D-isomer
<400> 188
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 189
   <211> 42
   <212> PRT
   <213> Artificial
<220>
   <223> Synthetic construct
<220>
   <221> MOD_RES
   <222> (1)..(1)
   <223> C6 acylation
<220>
   <221> MOD_RES
   <222> (5)..(5)
   <223> N-methyl valine
<400> 189
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 190
   <211> 42
   <212> PRT
   <213> Artificial
<220>
   <223> Synthetic construct
<220>
   <221>. MOD_RES
   <222> (1)..(1)
   <223> C6 acylation
<220>
   <221> MISC_FEATURE
   <222> (5)..(5)
   <223> X = Aib
<400> 190
   His Ser Asp Ala Xaa Phe Thr Asp Asn Tyr Thr Arg Leu Arg Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 191
   <211> 39
   <212> PRT
   <213> Artificial
<220>
   <223> Synthetic construct
<220>
   <221> MOD_RES
   <222> (1)..(1)
   <223> C6 acylation
<400> 191
   His Ser Asp Ala Val Phe Thr Asp Asn Tyr Thr Arg Leu Arg Lys Gln
   1 5 10 15
   Val Ala Ala Lys Arg Tyr Leu Gln Ser Ile Arg Asn Gly Gly Pro Ser
   20 25 30
   Ser Gly Ala Pro Pro Pro Ser
   35

<210> 192
   <211> 39
   <212> PRT
   <213> Artificial
<220>
   <223> Synthetic construct
<220>
   <221> MOD_RES
   <222> (1)..(1)
   <223> C6 acylation
<400> 192
   His Ser Asp Ala Val Phe Thr Asp Asn Tyr Thr Arg Leu Arg Lys Gln
   1               5                   10                  15
   Val Ala Ala Arg Arg Tyr Leu Gln Ser Ile Arg Asn Gly Gly Pro Ser
               20                  25                  30
   Ser Gly Ala Pro Pro Pro Ser
           35
<210> 193
   <211> 39
   <212> PRT
   <213> Artificial
<220>
   <223> synthetic construct
<220>
   <221> MOD_RES
   <222> (1)..(1)
   <223> C6 acylation
<400> 193
   His Ser Asp Ala Val Phe Thr Asp Asn Tyr Thr Arg Leu Arg Arg Gln
   1               5                   10                  15
   Val Ala Ala Lys Arg Tyr Leu Gln Ser Ile Arg Asn Gly Gly Pro Ser
               20                  25                  30
   Ser Gly Ala Pro Pro Pro Ser
           35
<210> 194
   <211> 39
   <212> PRT
   <213> Artificial
<220>
   <223> Synthetic construct
<220>
   <221> MOD_RES
   <222> (1)..(1)
   <223> C6 acylation
<400> 194
   His Ser Asp Ala Val Phe Thr Asp Asn Tyr Thr Arg Leu Arg Arg Gln
   1               5                   10                  15
   Val Ala Ala Arg Lys Tyr Leu Gln Ser Ile Arg Asn Gly Gly Pro Ser
               20                  25                  30
   Ser Gly Ala Pro Pro Pro Ser
           35
<210> 195
   <211> 39
   <212> PRT
   <213> Artificial
<220>
   <223> Synthetic construct
<220>
   <221> MOD_RES
   <222> (1)..(1)
   <223> C6 acylation
<400> 195
   His Ser Asp Ala Val Phe Thr Asp Gln Tyr Thr Arg Leu Arg Arg Gln
   1               5                   10                  15
   Val Ala Ala Arg Lys Tyr Leu Gln Ser Ile Arg Gln Gly Gly Pro Ser
               20                  25                  30
   Ser Gly Ala Pro Pro Pro Ser
           35
<210> 196
   <211> 42
   <212> PRT
   <213> Artificial
<220>
   <223> Synthetic construct
<220>
   <221> MOD_RES
   <222> (1)..(1)
   <223> C6 acylation
<400> 196
   His Ser Asp Ile Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 197
   <211> 42
   <212> PRT
   <213> Artificial
<220>
   <223> Synthetic construct
<220>
   <221> MOD_RES
   <222> (1)..(1)
   <223> C6 acylation
<400> 197
   His Gly Glu Gly Thr Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 198
   <211> 42
   <212> PRT
   <213> Artificial
<220>
   <223> synthetic construct
<220>
   <221> MOD_RES
   <222> (1)..(1)
   <223> C6 acylation
<400> 198
   His Ser Asp Leu Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
   35                  40
<210> 199
   <211> 42
   <212> PRT
   <213> Artificial
<220>
   <223> synthetic construct
<220>
   <221> MOD_RES
   <222> (1)..(1)
   <223> C6 acylation
<400> 199
   His Ser Glu Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 200
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (22)..(22)
   <223> X = Aib
<400> 200
   His Ser Asp Ala Val Phe Thr Asp Asn Tyr Thr Arg Leu Arg Lys Gln
   1               5                   10                  15
   Val Ala Ala Lys Lys Xaa Leu Gln Ser Ile Lys Asn Lys Arg Tyr Gly
               20                  25                  30
   Gly Pro Ser Ser Gly Ala Pro Pro Pro Ser
           35                  40
<210> 201
   <211> 42
   <212> PRT
   <213> Artificial
<220>
   <223> Synthetic construct
<220>
   <221> MOD_RES
   <222> (1)..(1)
   <223> C6 acylation
<400> 201
   His Ser Asp Ala Val Phe Thr Asp Asn Tyr Thr Arg Leu Arg Lys Gln
   1               5                   10                  15
   Val Ala Ala Lys Lys Ala Leu Gln Ser Ile Lys Asn Lys Arg Tyr Gly
               20                  25                  30
   Gly Pro Ser Ser Gly Ala Pro Pro Pro Ser
           35                  40
<210> 202
   <211> 42
   <212> PRT
   <213> Artificial
<220>
   <223> Synthetic construct
<220>
   <221> MOD_RES
   <222> (1)..(1)
   <223> C6 acylation
<400> 202
   His Ser Asp Ala Val Phe Thr Asp Asn Tyr Thr Arg Leu Arg Lys Gln
   1 5 10 15
   Val Ala Ala Lys Lys Tyr Trp Gln Ser Ile Lys Asn Lys Arg Tyr Gly
   20 25 30
   Gly Pro Ser Ser Gly Ala Pro Pro Pro Ser
   35 40
<210> 203
   <211> 42
   <212> PRT
   <213> Artificial
<220>
   <223> Synthetic construct
<220>
   <221> MOD_RES
   <222> (1)..(1)
   <223> C6 acylation
<400> 203
   His Ser Asp Ala Val Phe Thr Asp Asn Tyr Thr Arg Leu Arg Lys Gln
   1 5 10 15
   Val Ala Ala Lys Lys Tyr Phe Gln Ser Ile Lys Asn Lys Arg Tyr Gly
   20 25 30
   Gly Pro Ser Ser Gly Ala Pro Pro Pro Ser
   35 40
<210> 204
   <211> 42
   <212> PRT
   <213> Artificial
<220>
   <223> Synthetic construct
<220>
   <221> MOD_RES
   <222> (1)..(1)
   <223> C6 acylation
<400> 204
   His Ser Asp Ala Val Phe Thr Asp Asn Tyr Thr Arg Leu Arg Lys Gln
   1 5 10 15
   Val Ala Ala Lys Lys Tyr Thr Gln Ser Ile Lys Asn Lys Arg Tyr Gly
   20 25 30
   Gly Pro Ser Ser Gly Ala Pro Pro Pro Ser
   35 40
<210> 205
   <211> 42
   <212> PRT
   <213> Artificial
<220>
   <223> synthetic construct
<220>
   <221> MOD_RES
   <222> (1)..(1)
   <223> C6 acylation
<400> 205
   His Ser Asp Ala Val Phe Thr Asp Asn Tyr Thr Arg Leu Arg Lys Gln
   1 5 10 15
   Val Ala Ala Lys Lys Tyr Ile Gln Ser Ile Lys Asn Lys Arg Tyr Gly
   20 25 30
   Gly Pro Ser Ser Gly Ala Pro Pro Pro Ser
   35 40
<210> 206
   <211> 42
   <212> PRT
   <213> Artificial
<220>
   <223> synthetic construct
<220>
   <221> MOD_RES
   <222> (1)..(1)
   <223> C6 acylation
<400> 206
   His Ser Asp Ala Val Phe Thr Asp Asn Tyr Thr Arg Leu Arg Lys Gln
   1               5                   10                  15
   Val Ala Ala Lys Lys Tyr Val Gln Ser Ile Lys Asn Lys Arg Tyr Gly
               20                  25                  30
   Gly Pro Ser Ser Gly Ala Pro Pro Pro Ser
           35                  40
<210> 207
   <211> 42
   <212> PRT
   <213> Artificial
<220>
   <223> Synthetic construct
<220>
   <221> MOD_RES
   <222> (1)..(1)
   <223> C6 acylation
<400> 207
   His Ser Asp Ala Val Phe Thr Asp Asn Tyr Thr Arg Leu Arg Lys Gln
   1               5                   10                  15
   Val Ala Ala Lys Lys Tyr Ala Gln Ser Ile Lys Asn Lys Arg Tyr Gly
               20                  25                  30
   Gly Pro Ser Ser Gly Ala Pro Pro Pro Ser
           35                  40
<210> 208
   <211> 42
   <212> PRT
   <213> Artificial
<220>
   <223> Synthetic construct
<220>
   <221> MOD_RES
   <222> (1)..(1)
   <223> c6 acylation
<220>
   <221> MISC_FEATURE
   <222> (23)..(23)
   <223> X = Aib
<400> 208
   His Ser Asp Ala Val Phe Thr Asp Asn Tyr Thr Arg Leu Arg Lys Gln
   1 5 10 15
   Val Ala Ala Lys Lys Tyr Xaa Gln Ser Ile Lys Asn Lys Arg Tyr Gly
   20 25 30
   Gly Pro Ser Ser Gly Ala Pro Pro Pro Ser
   35 40
<210> 209
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (14)..(14)
   <223> X = Aib
<220>
   <221> MISC_FEATURE
   <222> (20)..(20)
   <223> X = Aib
<400> 209
   His Ser Asp Ala Val Phe Thr Asp Asn Tyr Thr Arg Leu Xaa Lys Gln
   1 5 10 15
   Leu Ala Ala Xaa Lys Tyr Leu Gln Ser Ile Lys Asn Lys Arg Tyr Gly
   20 25 30
   Gly Pro Ser Ser Gly Ala Pro Pro Pro Ser
   35 40
<210> 210
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (14)..(14)
   <223> X = Aib
<220>
   <221> MISC_FEATURE
   <222> (21)..(21)
   <223> X = Aib
<400> 210
   His Ser Asp Ala Val Phe Thr Asp Asn Tyr Thr Arg Leu Xaa Lys Gln
   1               5                   10                  15
   Leu Ala Ala Lys Xaa Tyr Leu Gln Ser Ile Lys Asn Lys Arg Tyr Gly
               20                  25                  30
   Gly Pro Ser Ser Gly Ala Pro Pro Pro Ser
           35                  40
<210> 211
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (14)..(14)
   <223> X = Aib
<220>
   <221> MISC_FEATURE
   <222> (20)..(20)
   <223> X = Aib
<400> 211
   His Ser Asp Ala Val Phe Thr Asp Asn Tyr Thr Arg Leu Xaa Lys Gln
   1 5 10 15
   Val Ala Ala Xaa Lys Tyr Leu Gln Ser Ile Lys Asn Lys Arg Tyr Gly
   20 25 30
   Gly Pro Ser Ser Gly Ala Pro Pro Pro Ser
   35 40
<210> 212
   <211> 42
   <212> PRT
   <213> Artificial
<220>
   <223> synthetic construct
<220>
   <221> MOD_RES
   <222> (1)..(1)
   <223> c6 acylation
<220>
   <221> MISC_FEATURE
   <222> (14)..(14)
   <223> X = Aib
<400> 212
   His Ser Asp Ala Val Phe Thr Asp Asn Tyr Thr Arg Leu Xaa Lys Gln
   1 5 10 15
   Val Ala Ala Gln Lys Tyr Leu Gln Ser Ile Lys Asn Lys Arg Tyr Gly
   20 25 30
   Gly Pro Ser Ser Gly Ala Pro Pro Pro Ser
   35 40
<210> 213
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (14)..(14)
   <223> X = Aib
<220>
   <221> MISC_FEATURE
   <222> (21)..(21)
   <223> X = Aib
<400> 213
   His Ser Asp Ala Val Phe Thr Asp Asn Tyr Thr Arg Leu Xaa Lys Gln
   1               5                   10                  15
   Val Ala Ala Lys Xaa Tyr Leu Gln Ser Ile Lys Asn Lys Arg Tyr Gly
               20                  25                  30
   Gly Pro Ser Ser Gly Ala Pro Pro Pro Ser
           35                  40
<210> 214
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (14)..(14)
   <223> X = Aib
<400> 214
   His Ser Asp Ala Val Phe Thr Asp Asn Tyr Thr Arg Leu Xaa Lys Gln
   1               5                   10                  15
   Val Ala Ala Lys Gln Tyr Leu Gln Ser Ile Lys Asn Lys Arg Tyr Gly
               20                  25                  30
   Gly Pro Ser Ser Gly Ala Pro Pro Pro Ser
           35                  40
<210> 215
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (20)..(20)
   <223> X = Aib
<400> 215
   His Ser Asp Ala Val Phe Thr Asp Asn Tyr Thr Arg Leu Gln Lys Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 216
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (21)..(21)
   <223> X = Aib
<400> 216
   His Ser Asp Ala Val Phe Thr Asp Asn Tyr Thr Arg Leu Gln Lys Gln
   1               5                   10                  15
   Val Ala Ala Lys Xaa Tyr Leu Gln Ser Ile Lys Asn Lys Arg Tyr Gly
               20                  25                  30
   Gly Pro Ser Ser Gly Ala Pro Pro Pro Ser
           35                  40
<210> 217
   <211> 42
   <212> PRT
   <213> Artificial
<220>
   <223> Synthetic construct
<220>
   <221> MOD_RES
   <222> (1)..(1)
   <223> C6 acylation
<400> 217
   His Ser Asp Ala Val Phe Thr Asp Asn Tyr Thr Arg Leu Gln Lys Gln
   1               5                   10                  15
   Val Ala Ala Gln Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 218
   <211> 42
   <212> PRT
   <213> Artificial
<220>
   <223> Synthetic construct
<220>
   <221> MOD_RES
   <222> (1)..(1)
   <223> C6 acylation
<400> 218
   His Ser Asp Ala Val Phe Thr Asp Asn Tyr Thr Arg Leu Gln Lys Gln
   1               5                   10                  15
   Val Ala Ala Lys Gln Tyr Leu Gln Ser Ile Lys Asn Lys Arg Tyr Gly
               20                  25                  30
   Gly Pro Ser Ser Gly Ala Pro Pro Pro Ser
           35                  40
<210> 219
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<400> 219
   His Ser Asp Ala Val Phe Thr Asp Asn Tyr Thr Arg Leu Gln Xaa Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 220
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (14)..(14)
   <223> X =Aib
<400> 220
   His Ser Asp Ala Val Phe Thr Asp Asn Tyr Thr Arg Leu Xaa Lys Gln
   1 5 10 15
   Val Ala Ala Leu Lys Tyr Leu Gln Ser Ile Lys Asn Lys Arg Tyr Gly
   20 25 30
   Gly Pro Ser Ser Gly Ala Pro Pro Pro Ser
   35 40
<210> 221
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (14)..(14)
   <223> X = Aib
<400> 221
   His Ser Asp Ala Val Phe Thr Asp Asn Tyr Thr Arg Leu Xaa Lys Gln
   1               5                   10                  15
   Val Ala Ala Ala Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 222
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 222
   His Ser Asp Ala Val Phe Thr Asp Asn Tyr Thr Arg Leu Arg Xaa Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 223
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<400> 223
   His Ser Asp Ala Val Phe Thr Asp Asn Tyr Thr Arg Leu Arg Xaa Gln
   1               5                   10                  15
   Val Ala Ala Val Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 224
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<400> 224
   His Ser Asp Ala Val Phe Thr Asp Asn Tyr Thr Arg Leu Arg Xaa Gln
   1               5                   10                  15
   Val Ala Ala Ala Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 225
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (21)..(21)
   <223> X = Aib
<400> 225
   His Ser Asp Ala Val Phe Thr Asp Asn Tyr Thr Arg Leu Arg Xaa Gln
   1               5                   10                  15
   Val Ala Ala Lys Xaa Tyr Leu Gln Ser Ile Lys Asn Lys Arg Tyr Gly
               20                  25                  30
   Gly Pro Ser Ser Gly Ala Pro Pro Pro Ser
           35                  40
<210> 226
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<400> 226
   His Ser Asp Ala Val Phe Thr Asp Asn Tyr Thr Arg Leu Arg Xaa Gln
   1               5                   10                  15
   Val Ala Ala Leu Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 227
   <211> 42
   <212> PRT
   <213> Artificial
<220>
   <223> Synthetic construct
<220>
   <227> MOD_RES
   <222> (1)..(1)
   <223> C6 acylation
<220>
   <221> MISC_FEATURE
   <222> (14)..(14)
   <223> X = Aib
<400> 227
   His Ser Asp Ala Val Phe Thr Asp Asn Tyr Thr Arg Leu Xaa Lys Gln
   1               5                   10                  15
   Val Ala Ala Val Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 228
   <211> 40
   <212> PRT
   <213> Artificial
<220>
   <223> Synthetic construct
<220>
   <221> MOD_RES
   <222> (1)..(1)
   <223> c6 acylation
<400> 228
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Gln Lys Gly Gly Pro
               20                  25                  30
   Ser Ser Gly Ala Pro Pro Pro Ser
           35                  40
<210> 229
   <211> 39
   <212> PRT
   <213> Artificial
<220>
   <223> Synthetic construct
<220>
   <221> MOD_RES
   <222> (1)..(1)
   <223> C6 acylation
<400> 229
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Gly Arg Pro Ser
               20                  25                  30
   Ser Gly Ala Pro Pro Pro Ser
           35
<210> 230
   <211> 42
   <212> PRT
   <213> Artificial
<220>
   <223> Synthetic construct
<220>
   <221> MOD_RES
   <222> (1)..(1)
   <223> C6 acylation
<400> 230
   His Ser Asp Ala Val Phe Thr Asp Asn Tyr Thr Arg Leu Arg Lys Gln
   1               5                   10                  15
   Val Ala Gly Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 231
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (14)..(14)
   <223> X = Aib
<220>
   <221> MISC_FEATURE
   <222> (20)..(20)
   <223> X = Aib
<220>
   <221> MOD_RES
   <222> (42)..(42)
   <223> AMIDATION
<400> 231
   His Ser Asp Ala Val Phe Thr Asp Asn Tyr Thr Arg Leu Xaa Lys Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Cys
           35                  40
<210> 232
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (21)..(21)
   <223> X = Aib
<400> 232
   His Ser Asp Ala Val Phe Thr Asp Asn Tyr Thr Arg Leu Arg Xaa Gln
   1               5                   10                  15
   Leu Ala Ala Lys Xaa Tyr Leu Gln Ser Ile Lys Asn Lys Arg Tyr Gly
               20                  25                  30
   Gly Pro Ser Ser Gly Ala Pro Pro Pro Ser
           35                  40
<210> 233
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (21)..(21)
   <223> X = Aib
<220>
   <221> MOD_RES
   <222> (42)..(42)
   <223> AMIDATION
<400> 233
   His Ser Asp Ala Val Phe Thr Asp Asn Tyr Thr Arg Leu Arg Xaa Gln
   1               5                   10                  15
   Val Ala Ala Lys Xaa Tyr Leu Gln Ser Ile Lys Asn Lys Arg Tyr Gly
               20                  25                  30
   Gly Pro Ser Ser Gly Ala Pro Pro Pro Cys
           35                  40
<210> 234
   <211> 42
   <212> PRT
   <213> Artificial
<220>
   <223> Synthetic construct
<220>
   <221> MOD_RES
   <222> (1)..(1)
   <223> C6 acylation
<400> 234
   His Ser Asp Ala Leu Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 235
   <211> 42
   <212> PRT
   <213> Artificial
<220>
   <223> Synthetic construct
<220>
   <221> MOD_RES
   <222> (1)..(1)
   <223> c6 acylation
<400> 235
   His Ser Asp Ala Val Phe Thr Asp Asn Tyr Thr Arg Leu Arg Lys Gln
   1 5 10 15
   Leu Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 236
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 236
   His Ser Asp Ala Val Phe Thr Asp Asn Tyr Thr Arg Leu Arg Xaa Gln
   1 5 10 15
   Leu Ala Ala Xaa Lys Tyr Leu Gln Ser Ile Lys Asn Lys Arg Tyr Gly
   20 25 30
   Gly Pro Ser Ser Gly Ala Pro Pro Pro Ser
   35 40
<210> 237
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 237
   His Ser Asp Ala Val Phe Thr Asp Asn Tyr Thr Arg Leu Arg Xaa Gln
   1               5                   10                  15
   Leu Ala Ala Xaa Lys Tyr Leu Gln Ser Ile Lys Asn Lys Gly Gly Pro
               20                  25                  30
   Ser Ser Gly Ala Pro Pro Pro Ser
           35                  40
<210> 238
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (14)..(14)
   <223> Xaa at position 14 is Aib
<220>
   <221> MISC_FEATURE
   <222> (20)..(20)
   <223> X = Aib
<400> 238
   His Ser Asp Ala Val Phe Thr Asp Asn Tyr Thr Arg Leu Xaa Lys Gln
   1 5 10 15
   Val Ala Ala Xaa Lys Tyr Leu Gln Ser Ile Lys Gln Lys Gly Gly Pro
   20 25 30
   Ser Ser Gly Ala Pro Pro Pro Ser
   35 40
<210> 239
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 239
   His Ser Asp Ala Val Phe Thr Asp Asn Tyr Thr Arg Leu Arg Xaa Gln
   1 5 10 15
   Val Ala Ala Xaa Lys Tyr Leu Gln Ser Ile Lys Gln Lys Gly Gly Pro
   20 25 30
   Ser Ser Gly Ala Pro Pro Pro Ser
   35 40
<210> 240
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (21)..(21)
   <223> X = Aib
<400> 240
   His Ser Asp Ala Val Phe Thr Asp Asn Tyr Thr Arg Leu Arg Xaa Gln
   1               5                   10                  15
   Val Ala Ala Lys Xaa Tyr Leu Gln Ser Ile Lys Gln Lys Gly Gly Pro
               20                  25                  30
   Ser Ser Gly Ala Pro Pro Pro Ser
           35                  40
<210> 241
   <211> 39
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (14)..(14)
   <223> xaa at position 14 is Aib
<220>
   <221> MISC_FEATURE
   <222> (20)..(20)
   <223> X = Aib
<400> 241
   His Ser Asp Ala Val Phe Thr Asp Asn Tyr Thr Arg Leu Xaa Lys Gln
   1               5                   10                  15
   Val Ala Ala Xaa Lys Tyr Leu Gln Ser Ile Lys Gln Gly Gly Pro Ser
               20                  25                  30
   Ser Gly Ala Pro Pro Pro Ser
   35
<210> 242
   <211> 39
   <212> PRT
   <213> Artificial
<220>
   <223> Synthetic construct
<220>
   <221> MOD_RES
   <222> (1)..(1)
   <223> C6 acylation
<220>
   <221> MIST_FEATURE
   <222> (15)..(15)
   <223> X = Aib
<220>
   <221> MISC_FEATURE
   <222> (20)..(20)
   <223> X = Aib
<400> 242
   His Ser Asp Ala Val Phe Thr Asp Asn Tyr Thr Arg Leu Arg Xaa Gln
   1 5 10 15
   Val Ala Ala Xaa Lys Tyr Leu Gln Ser Ile Lys Gln Gly Gly Pro Ser
   20 25 30
   Ser Gly Ala Pro Pro Pro Ser
   35
<210> 243
   <211> 39
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (21)..(21)
   <223> X = Aib
<400> 243
   His Ser Asp Ala Val Phe Thr Asp Asn Tyr Thr Arg Leu Arg Xaa Gln
   1               5                   10                  15
   Val Ala Ala Lys Xaa Tyr Leu Gln Ser Ile Lys Gln Gly Gly Pro Ser
               20                  25                  30
   Ser Gly Ala Pro Pro Pro Ser
           35
<210> 244
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MOD_RES
   <222> (42)..(42)
   <223> AMIDATION
<400> 244
   His Ser Asp Ala Val Phe Thr Asp Asn Tyr Thr Arg Leu Arg Xaa Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Cys
           35                  40
<210> 245
   <211> 42
   <212> PRT
   <213> Artificial
<220>
   <223> synthetic construct
<220>
   <221> MOD_RES
   <222> (1)..(1)
   <223> c6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 245
   His Ser Asp Ala Val Phe Thr Asp Asn Tyr Thr Arg Leu Ala Xaa Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 246
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 246
   His Ser Asp Ala Val Phe Thr Asp Asn Tyr Thr Arg Leu Gln Xaa Gln
   1 5 10 15
   Val Ala Ala Xaa Lys Tyr Leu Gln Ser Ile Lys Asn Lys Arg Tyr Gly
   20 25 30
   Gly Pro Ser Ser Gly Ala Pro Pro Pro Ser
   35 40
<210> 247
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (14)..(14)
   <223> X = homoarginine
<400> 247
   His Ser Asp Ala Val Phe Thr Asp Asn Tyr Thr Arg Leu Xaa Lys Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 248
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Ornithine
<400> 248
   His Ser Asp Ala Val Phe Thr Asp Asn Tyr Thr Arg Leu Arg Xaa Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 249
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (14)..(14)
   <223> X = homoarginine
<220>
   <221> MISC_FEATURE
   <222> (15)..(15)
   <223> X = ornithine
<400> 249
   His Ser Asp Ala Val Phe Thr Asp Asn Tyr Thr Arg Leu Xaa Xaa Gln
   1 5 10 15
   Val Ala Ala Lys Lys Tyr Leu Gln Ser Ile Lys Asn Lys Arg Tyr Gly
   20 25 30
   Gly Pro Ser Ser Gly Ala Pro Pro Pro Ser
   35 40
<210> 250
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 250
   His Ser Glu Ala Val Phe Thr Glu Asn Tyr Thr Arg Leu Arg Xaa Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 251
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 251
   His Ser Asp Ala Val Phe Thr Asp Gln Tyr Thr Arg Leu Arg Xaa Gln
   1 5 10 15
   Val Ala Ala Xaa Lys Tyr Leu Gln Ser Ile Lys Gln Lys Arg Tyr Gly
   20 25 30
   Gly Pro Ser Ser Gly Ala Pro Pro Pro Ser
   35 40
<210> 252
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (14)..(14)
   <223> x = homoarginine
<220>
   <221> MISC_FEATURE
   <222> (15)..(15)
   <223> X = Aib
<220>
   <221> MISC_FEATURE
   <222> (20)..(20)
   <223> X = Aib
<400> 252
   His Ser Asp Ala Val Phe Thr Asp Asn Tyr Thr Arg Leu Xaa Xaa Gln
   1 5 10 15
   Val Ala Ala Xaa Lys Tyr Leu Gln Ser Ile Lys Asn Lys Arg Tyr Gly
   20 25 30
   Gly Pro Ser Ser Gly Ala Pro Pro Pro Ser
   35 40
<210> 253
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (15)..(15)
   <223> Xaa at position 15 is Aib
<220>
   <221> MISC_FEATURE
   <222> (20)..(20)
   <223> Xaa at position 20 is Aib
<400> 253
   His Ser Asp Ala Val Phe Thr Asp Asn Tyr Thr Arg Leu Leu Xaa Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 254
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 254
   His Ser Asp Ala Val Phe Thr Asp Asn Tyr Thr Arg Leu Lys Xaa Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 255
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (14)..(14)
   <223> X = Ornithine
<220>
   <221> MISC_FEATURE
   <222> (15)..(15)
   <223> X = Aib
<220>
   <221> MISC_FEATURE
   <222> (20)..(20)
   <223> X = Aib
<400> 255
   His Ser Asp Ala Val Phe Thr Asp Asn Tyr Thr Arg Leu Xaa Xaa Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 256
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (14)..(14)
   <223> X = Cit
<220>
   <221> MISC_FEATURE
   <222> (15)..(15)
   <223> X = Aib
<220>
   <221> MISC_FEATURE
   <222> (20)..(20)
   <223> X = Aib
<400> 256
   His Ser Asp Ala Val Phe Thr Asp Asn Tyr Thr Arg Leu Xaa Xaa Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 257
   <211> 42
   <212> PRT
   <213> Artificial
<220>
   <223> Synthetic construct
<220>
   <221> MOD_RES
   <222> (1)..(1)
   <223> c6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 257
   His Ser Asp Ala Val Phe Thr Asp Asn Tyr Thr Arg Leu Arg Xaa Lys
   1 5 10 15
   Val Ala Ala Xaa Lys Tyr Leu Gln Ser Ile Lys Asn Lys Arg Tyr Gly
   20 25 30
   Gly Pro Ser Ser Gly Ala Pro Pro Pro Ser
   35 40
<210> 258
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 258
   His Ser Asp Ala Val Phe Thr Asp Asn Tyr Thr Arg Leu Arg Xaa Gln
   1 5 10 15
   Ile Ala Ala Xaa Lys Tyr Leu Gln Ser Ile Lys Asn Lys Arg Tyr Gly
   20 25 30
   Gly Pro Ser Ser Gly Ala Pro Pro Pro Ser
   35 40
<210> 259
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 259
   His Ser Asp Ala Val Phe Thr Asp Asn Tyr Thr Arg Leu Arg Xaa Gln
   1               5                   10                  15
   Lys Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 260
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 260
   His Ser Asp Ala Val Phe Thr Asp Asn Tyr Thr Arg Leu Arg Xaa Gln
   1               5                   10                  15
   Ala Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 261
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (17)..(17)
   <223> X = Norleucine
<220>
   <221> MISC_FEATURE
   <222> (20)..(20)
   <223> X = Aib
<400> 261
   His Ser Asp Ala Val Phe Thr Asp Asn Tyr Thr Arg Leu Arg Xaa Gln
   1               5                   10                  15
   Xaa Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 262
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (12)..(12)
   <223> X = Ornithine
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(27)
   <223> X = ornithine
<220>
   <221> MISC_FEATURE
   <222> (29)..(29)
   <223> x = ornithine
<400> 262
   His Ser Asp Ala Val Phe Thr Asp Asn Tyr Thr Xaa Leu Arg Xaa Gln
   1               5                   10                  15
   Val Ala Ala Xaa Lys Tyr Leu Gln Ser Ile Xaa Asn Xaa Gly Gly Pro
               20                  25                  30
   Ser Ser Gly Ala Pro Pro Pro Ser
           35                  40
<210> 263
   <211> 40
   <212> PRT
   <213> Artificial
<220>
   <223> synthetic construct
<220>
   <221> MOD_RES
   <222> (1)..(1)
   <223> c6 acylation
<220>
   <221> MISC_FEATURE
   <222> (12)..(12)
   <223> X = ornithine
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(27)
   <223> X = ornithine
<220>
   <221> MISC_FEATURE
   <222> (29)..(29)
   <223> X = Ornithine
<220>
   <221> MOD_RES
   <222> (40)..(40)
   <223> AMIDATION
<400> 263
   His Ser Asp Ala Val Phe Thr Asp Asn Tyr Thr Xaa Leu Arg Xaa Gln
   1               5                   10                  15
   Val Ala Ala Xaa Lys Tyr Leu Gln Ser Ile Xaa Asn Xaa Gly Gly Pro
               20                  25                  30
   Ser Ser Gly Ala Pro Pro Pro Cys
           35                  40

<210> 264
   <211> 40
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (12)..(12)
   <223> x = homoarginine
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(27)
   <223> X = homoarginine
<220>
   <221> MISC_FEATURE
   <222> (29)..(29)
   <223> X = homoarginine
<400> 264
   His Ser Asp Ala Val Phe Thr Asp Asn Tyr Thr Xaa Leu Arg Xaa Gln
   1               5                   10                  15
   Val Ala Ala Xaa Lys Tyr Leu Gln Ser Ile Xaa Asn Xaa Gly Gly Pro
               20                  25                  30
   Ser Ser Gly Ala Pro Pro Pro Ser
           35                  40
<210> 265
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (12)..(12)
   <223> X = homoarginine
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(27)
   <223> X = homoarginine
<220>
   <221> MISC_FEATURE
   <222> (29)..(29)
   <223> X = homoarginine
<220>
   <221> MOD_RES
   <222> (40)..(40)
   <223> AMIDATION
<400> 265
   His Ser Asp Ala Val Phe Thr Asp Asn Tyr Thr Xaa Leu Arg Xaa Gln
   1               5                   10                  15
   Val Ala Ala Xaa Lys Tyr Leu Gln Ser Ile Xaa Asn Xaa Gly Gly Pro
               20                  25                  30
   Ser Ser Gly Ala Pro Pro Pro Cys
           35                  40
<210> 266
   <211> 39
   <212> PRT
   <213> Artificial
<220>
   <223> Synthetic construct
<220>
   <221> MISC_FEATURE
   <222> (12)..(12)
   <223> X = homoarginine
<220>
   <221> MISC_FEATURE
   <222> (15)..(15)
   <223> = Aib
<220>
   <221> MISC_FEATURE
   <222> (20)..(20)
   <223> X = Aib
<220>
   <221> MISC_FEATURE
   <222> (27)..(28)
   <223> X = homoarginine
<400> 266
   His Ser Asp Ala Val Phe Thr Asp Asn Tyr Thr Xaa Leu Arg Xaa Gln
   1 5 10 15
   Val Ala Ala Xaa Lys Tyr Leu Gln Ser Ile Xaa Xaa Gly Gly Pro Ser
   20 25 30
   Ser Gly Ala Pro Pro Pro Ser
   35
<210> 267
   <211> 39
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (12)..(12)
   <223> X = homoarginine
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(28)
   <223> X = homoarginine
<220>
   <221> MOD_RES
   <222> (39)..(39)
   <223> AMIDATION
<400> 267
   His Ser Asp Ala Val Phe Thr Asp Asn Tyr Thr Xaa Leu Arg Xaa Gln
   1               5                   10                  15
   Val Ala Ala Xaa Lys Tyr Leu Gln Ser Ile Xaa Xaa Gly Gly Pro Ser
               20                  25                  30
   Ser Gly Ala Pro Pro Pro Cys
           35
<210> 268
   <211> 39
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (12)..(12)
   <223> x = ornithine
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(28)
   <223> X = homoarginine
<400> 268
   His Ser Asp Ala Val Phe Thr Asp Asn Tyr Thr Xaa Leu Arg Xaa Gln
   1               5                   10                  15
   Val Ala Ala Xaa Lys Tyr Leu Gln Ser Ile Xaa Xaa Gly Gly Pro Ser
               20                  25                  30
   Ser Gly Ala Pro Pro Pro Ser
           35
<210> 269
   <211> 39
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (12)..(12)
   <223> X = Ornithine
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(28)
   <223> X = homoarginine
<220>
   <221> MOD_RES
   <222> (39)..(39)
   <223> AMIDATION
<400> 269
   His Ser Asp Ala Val Phe Thr Asp Asn Tyr Thr Xaa Leu Arg Xaa Gln
   1               5                   10                  15
   Val Ala Ala Xaa Lys Tyr Leu Gln Ser Ile Xaa Xaa Gly Gly Pro Ser
               20                  25                  30
   Ser Gly Ala Pro Pro Pro Cys
           35
<210> 270
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (12)..(12)
   <223> X = homoarginine
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(27)
   <223> X = homoarginine
<220>
   <221> MISC_FEATURE
   <222> (29)..(30)
   <223> X = homoarginine
<400> 270
   His Ser Asp Ala Val Phe Thr Asp Asn Tyr Thr Xaa Leu Arg Xaa Gln
   1               5                   10                  15
   Val Ala Ala Xaa Lys Tyr Leu Gln Ser Ile Xaa Asn Xaa Xaa Tyr Gly
               20                  25                  30
   Gly Pro Ser Ser Gly Ala Pro Pro Pro Ser
           35                  40
<210> 271
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (12)..(12)
   <223> X = homoarginine
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(27)
   <223> X = homoarginine
<220>
   <221> MISC_FEATURE
   <222> (29)..(30)
   <223> x = homoarginine
<220>
   <221> MOD_RES
   <222> (42)..(42)
   <223> AMIDATION
<400> 271
   His Ser Asp Ala Val Phe Thr Asp Asn Tyr Thr Xaa Leu Arg Xaa Gln
   1               5                   10                  15
   Val Ala Ala Xaa Lys Tyr Leu Gln Ser Ile Xaa Asn Xaa Xaa Tyr Gly
               20                  25                  30
   Gly Pro Ser Ser Gly Ala Pro Pro Pro Cys
           35                  40
<210> 272
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 272
   His Ser Asp Ala Val Phe Thr Asp Asn Tyr Thr Arg Leu Arg Xaa Gln
   1               5                   10                  15
   Val Ala Ala Xaa Ala Tyr Leu Gln Ser Ile Lys Asn Lys Arg Tyr Gly
               20                  25                  30
   Gly Pro Ser Ser Gly Ala Pro Pro Pro Ser
           35                  40
<210> 273
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (21)..(21)
   <223> X = Ornithine
<400> 273
   His Ser Asp Ala Val Phe Thr Asp Asn Tyr Thr Arg Leu Arg Xaa Gln
   1 5 10 15
   Val Ala Ala Xaa Xaa Tyr Leu Gln Ser Ile Lys Asn Lys Arg Tyr Gly
   20 25 30
   Gly Pro Ser Ser Gly Ala Pro Pro Pro Ser
   35 40
<210> 274
   <211> 42
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (12)..(12)
   <223> x = Ornithine
<220>
   <221> MISC_FEATURE
   <222> (15)..(15)
   <223> X = Aib
<220>
   <221> MISC_FEATURE
   <222> (20)..(20)
   <223> X = Aib
<400> 274
   His Ser Asp Ala Val Phe Thr Asp Asn Tyr Thr Xaa Leu Arg Xaa Gln
   1 5 10 15
   Val Ala Ala Xaa Lys Tyr Leu Gln Ser Ile Lys Asn Lys Arg Tyr Gly
   20 25 30
   Gly Pro Ser Ser Gly Ala Pro Pro Pro Ser
   35 40
<210> 275
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (12)..(12)
   <223> X = Aib
<220>
   <221> MISC_FEATURE
   <222> (15)..(15)
   <223> X = Aib
<220>
   <221> MISC_FEATURE
   <222> (20)..(20)
   <223> X = Aib
<400> 275
   His Ser Asp Ala Val Phe Thr Asp Asn Tyr Thr Xaa Leu Arg Xaa Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 276
   <211> 42
   <212> PRT
   <213> Artificial
<220>
   <223> synthetic construct
<220>
   <221> MOD_RES
   <222> (1)..(1)
   <223> C6 acylation
<220>
   <221> MISC_FEATURE
   <222> (13)..(13)
   <223> X = Aib
<220>
   <221> MISC_FEATURE
   <222> (15)..(15)
   <223> X = Aib
<220>
   <221> MISC_FEATURE
   <222> (20)..(20)
   <223> X = Aib
<400> 276
   His Ser Asp Ala Val Phe Thr Asp Asn Tyr Thr Arg Xaa Arg Xaa Gln
   1               5                   10                  15
   Val Ala Ala Xaa Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 277
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (28)..(28)
   <223> X = Aib
<400> 277
   His Ser Asp Ala Val Phe Thr Asp Asn Tyr Thr Arg Leu Arg Xaa Gln
   1               5                   10                  15
   Val Ala Ala Xaa Lys Tyr Leu Gln Ser Ile Lys Xaa Lys Gly Gly Pro
               20                  25                  30
   Ser Ser Gly Ala Pro Pro Pro Ser
           35                  40
<210> 278
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 278
   His Ser Asp Ala Val Phe Thr Asp Asn Tyr Thr Arg Leu Arg Xaa Gln
   1               5                   10                  15
   Val Ala Ala Xaa Lys Tyr Leu Gln Ser Ile Lys Pro Lys Gly Gly Pro
               20                  25                  30
   Ser Ser Gly Ala Pro Pro Pro Ser
           35                  40
<210> 279
   <211> 39
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<400> 279
   His Ser Asp Ala Val Phe Thr Asp Asn Tyr Thr Arg Leu Arg Xaa Gln
   1               5                   10                  15
   Val Ala Ala Xaa Lys Tyr Leu Gln Ser Ile Lys Lys Gly Gly Pro Ser
               20                  25                  30
   Ser Gly Ala Pro Pro Pro Ser
           35
<210> 280
   <211> 39
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(28)
   <223> X = ornithine
<400> 280
   His Ser Asp Ala Val Phe Thr Asp Asn Tyr Thr Arg Leu Arg Xaa Gln
   1               5                   10                  15
   Val Ala Ala Xaa Lys Tyr Leu Gln Ser Ile Xaa Xaa Gly Gly Pro Ser
               20                  25                  30
   Ser Gly Ala Pro Pro Pro Ser
           35
<210> 281
   <211> 39
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(28)
   <223> Lys is D-isomer
<400> 281
   His Ser Asp Ala Val Phe Thr Asp Asn Tyr Thr Arg Leu Arg Xaa Gln
   1               5                   10                  15
   Val Ala Ala Xaa Lys Tyr Leu Gln Ser Ile Lys Lys Gly Gly Pro Ser
               20                  25                  30
   Ser Gly Ala Pro Pro Pro Ser
           35
<210> 282
   <211> 39
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (28)..(28)
   <223> X = homoarginine
<400> 282
   His Ser Asp Ala Val Phe Thr Asp Asn Tyr Thr Arg Leu Arg Xaa Gln
   1               5                   10                  15
   Val Ala Ala Xaa Lys Tyr Leu Gln Ser Ile Lys Xaa Gly Gly Pro Ser
               20                  25                  30
   Ser Gly Ala Pro Pro Pro Ser
           35
<210> 283
   <211> 39
   <212> PRT
   <213> Artificial
<220>
   <223> synthetic construct
<220> RES
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (28)..(28)
   <223> X = Aib
<400> 283
   His Ser Asp Ala Val Phe Thr Asp Asn Tyr Thr Arg Leu Arg Xaa Gln
   1 5 10 15
   Val Ala Ala Xaa Lys Tyr Leu Gln Ser Ile Lys Xaa Gly Gly Pro Ser
   20 25 30
   Ser Gly Ala Pro Pro Pro Ser
   35
<210> 284
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(27)
   <223> X = Ornithine
<220>
   <221> MISC_FEATURE
   <222> (29)..(29)
   <223> x = Ornithine
<400> 284
   His Ser Asp Ala Val Phe Thr Asp Asn Tyr Thr Arg Leu Arg Xaa Gln
   1 5 10 15
   Val Ala Ala Xaa Lys Tyr Leu Gln Ser Ile Xaa Gln Xaa Gly Gly Pro
   20 25 30
   Ser Ser Gly Ala Pro Pro Pro Ser
   35 40
<210> 285
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (12)..(12)
   <223> X = ornithine
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(27)
   <223> X = Ornithine
<220>
   <221> MISC_FEATURE
   <222> (29)..(29)
   <223> X = ornithine
<400> 285
   His Ser Asp Ala Val Phe Thr Asp Asn Tyr Thr Xaa Leu Arg Xaa Gln
   1               5                   10                  15
   Leu Ala Ala Xaa Lys Tyr Leu Gln Ser Ile Xaa Asn Xaa Gly Gly Pro
                   20                  25                  30
   Ser Ser Gly Ala Pro Pro Pro Ser
           35                  40
<210> 286
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (12)..(12)
   <223> X = ornithine
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(27)
   <223> X = Ornithine
<220>
   <221> MISC_FEATURE
   <222> (29)..(29)
   <223> X = ornithine
<400> 286
   His Ser Asp Ala Val Phe Thr Asp Asn Tyr Thr Xaa Leu Arg Xaa Gln
   1               5                   10                  15
   Ile Ala Ala Xaa Lys Tyr Leu Gln Ser Ile Xaa Asn Xaa Gly Gly Pro
               20                  25                  30
   Ser Ser Gly Ala Pro Pro Pro Ser
           35                  40
<210> 287
   <211> 40
   <212> PRT
   <213> Artificial
<220>
   <223> Synthetic construct
<220>
   <221> MOD_RES
   <222> (1)..(1)
   <223> C6 acylation
<220>
   <221> MISC_FEATURE
   <222> (15)..(15)
   <223> X = Aib
<220>
   <221> MISC_FEATURE
   <222> (20)..(20)
   <223> X = Aib
<220>
   <221> MISC_FEATURE
   <222> (27)..(27)
   <223> X = Ornithine
<220>
   <221> MISC_FEATURE
   <222> (29)..(29)
   <223> X = ornithine
<400> 287
   His Ser Asp Ala Val Phe Thr Asp Asn Tyr Thr Ala Leu Arg Xaa Gln
   1               5                   10                  15
   Val Ala Ala Xaa Lys Tyr Leu Gln Ser Ile Xaa Asn Xaa Gly Gly Pro
               20                  25                  30
   Ser Ser Gly Ala Pro Pro Pro Ser
           35                  40
<210> 618
   <211> 9
   <212> PRT
   <213> Artificial
<220> .
   <223> Synthetic construct
<400> 618
   Ser Trp Cys Glu Pro Gly Trp Cys Arg
   1               5
<210> 619
   <211> 42
   <212> PRT
   <213> Artificial
<220>
   <223> Synthetic construct
<220>
   <221> MOD_RES
   <222> (1)..(1)
   <223> C6 acylation
<220>
   <221> MOD_RES
   <222> (10)..(10)
   <223> Tyr is methoxylated
<400> 619
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40
<210> 620
   <211> 42
   <212> PRT
   <213> Artificial
<220>
   <223> synthetic construct
<220>
   <221> MOD_RES
   <222> (1)..(1)
   <223> C6 acylation
<220>
   <221> MOD_RES
   <222> (22)..(22)
   <223> Tyr is methoxylated
<400> 620
   His Ser Asp Ala Val Phe Thr Asp Asn Tyr Thr Arg Leu Arg Lys Gln
   1               5                   10                  15
   Val Ala Ala Lys Lys Tyr Leu Gln Ser Ile Lys Asn Lys Arg Tyr Gly
               20                  25                  30
   Gly Pro Ser Ser Gly Ala Pro Pro Pro Ser
           35                  40